# EUROPEAN PATENT APPLICATION

(11) **EP 4 748 395 A1**
(43) Date of publication of application: **27.05.2026**
(21) Application number: 24842351.9
(22) Date of filing: 15.07.2024
(51) Int. Cl.: A61K 45/06, C12Q 1/6883, C12Q 1/68, C12N 15/11, C12N 15/12, A61K 48/00, A61P 25/00

(54) **THERAPY BASED ON INTRACEREBRAL PHOSPHATE HOMEOSTASIS PROMOTER**

(30) Priority: 19.07.2023 CN 202310890680; 20.10.2023 CN 202311370896
(71) Applicant: Shanghai Anyea Therapeutics Co., Ltd, Shanghai (CN)
(72) Inventor: CHENG, Xuewen, Shanghai 200031 (CN); XIONG, Zhi-Qi, Shanghai 200031 (CN); CHEN, Wan-Jin, Fuzhou, Fujian 350005 (CN); ZHAO, Miao, Fuzhou, Fujian 350005 (CN); CHEN, Lei, Shanghai 200031 (CN); ZENG, Yi-Heng, Fuzhou, Fujian 350005 (CN)
(74) Representative: Ipsilon
(86) International application number: PCT/CN2024/105509
(87) International publication number: WO 2025/016362

(57) **Abstract**

The present invention relates to the field of biomedicine, in particular, to the use of an intracerebral phosphate homeostasis promoter in cerebral calcification disorders. The intracerebral phosphate homeostasis promoter comprises an agent capable of regulating alternative splicing in an intron of the *SLC20A2* gene or an agent targeting astrocytes. In addition, further provided in the present invention are an agent capable of regulating alternative splicing in an intron of the *SLC20A2* gene, and the use thereof. Further provided in the present invention are cerebral calcification model based on alternative splicing in an intron of the human *SLC20A2* gene, and a preparation method thereof, and the use thereof.

## Description

### Cross-Reference to Related Applications

This application claims priority to Chinese Patent Application No. 202310890680.8 filed on July 19, 2023, and Chinese Patent Application No. 202311370896.8 filed on October 20, 2023, and the entire contents of which are incorporated herein by reference in their entirety.

### Technical Field

The present invention relates to the field of biomedicine, in particular, to the use of an intracerebral phosphate homeostasis promoter in cerebral calcification disorders. The intracerebral phosphate homeostasis promoter comprises an agent capable of regulating alternative splicing in an intron of the *SLC20A2* gene or an agent targeting astrocytes. In addition, further provided in the present invention are an agent capable of regulating alternative splicing in an intron of the *SLC20A2* gene, and the use thereof. Further provided in the present invention are a cerebral calcification model based on alternative splicing in an intron of the human *SLC20A2* gene, and a preparation method therefor and the use thereof.

### Background

Cerebral calcification is an age- and aging-related disease that is relatively common in clinical imaging. In cranial CT scans of the elderly individuals over 60 years old, over 20% show punctate calcification, and over 2-4% accompanied by larger patchy calcifications. In some neurodegenerative diseases, such as Alzheimer's disease (AD) and Parkinson's disease (PD), over 20% of patients with concurrent cerebral calcification, and the lesions tend to enlarge with age. Therefore, cerebral calcification is a clinically significant neurodegenerative disease with therapeutic needs.

Hereditary cerebral calcification is a neurodegenerative disease caused by genetic mutations, including various types, such as Primary Familial Brain calcification (PFBC) and Aicardi-Goutières Syndrome (AGS), etc. PFBC is a representative type among these, with an incidence of approximately 0.6%. The pathological features of PFBC are bilateral symmetrical calcification commonly found in areas such as the basal ganglia, thalamus, and cerebellar dentate nucleus, with calcification also occasionally found in the subcortical regions of some patients. As the disease progresses, calcification foci can coalesce into large patches. High-resolution brain tissue pathological imaging studies have found that calcium salt granules are primarily deposited in the perivascular spaces around terminal arterioles, venules, and capillaries. The clinical symptoms of hereditary cerebral calcification are diverse, mainly including movement disorders, psychiatric disorders, cognitive impairment, seizures, dizziness, headache, etc. In severe cases, personality and behavioral changes may occur, eventually leading to psychosis or dementia; some patients become disabled and unable to self-care, while others remain asymptomatic throughout their lives.

Hereditary cerebral calcification can occur sporadically (mostly due to de novo mutations) or be inherited within families, with high genetic heterogeneity. Over the past decade, human genetic studies have identified causative genes for PFBC including: *SLC20A2, XPR1, PDGFB, PDGFRB, MYORG,* and *JAM2.* These genes suggest two main possible pathogenic mechanisms for cerebral calcification: first, impaired integrity of the blood-brain barrier (BBB) structure or reduced pericyte coverage leading to increased BBB permeability. Patients related to this pathway may have mutations in genes such as *PDGFRB, PDGFB, JAM2,* etc., which are all expressed in cells constituting the neurovascular unit.

The second pathway involves impaired transport of inorganic phosphate (Pi) in the brain and the resulting imbalance in brain phosphate homeostasis (BPH), often showing significantly elevated cerebrospinal fluid (CSF) Pi levels. Patients belonging to this pathway may have mutations in the *SLC20A2, MYORG,* or *XPRI* genes. These genes encode the Na-Pi co-transporter PiT2 protein, the phosphate efflux transporter protein XPR1, and its regulator MYORG, respectively. Furthermore, statistical analysis of clinical calcification and symptoms indicates that compared to patients with gene mutations related to the BBB pathway, patients with mutations in Pi transport-related genes have larger calcifications and more severe clinical symptoms. Moreover, among all reported familial calcification patients, mutations in *SLC20A2* account for 61% of all genetic factors, *XPRI* for 6%, and MYORG mutations for 13%.

Currently, the main clinical strategy for PFBC is symptomatic treatment, with no effective drugs available to remove or reduce abnormal calcium deposits. In particular, targeting the mechanism of BPH imbalance may provide opportunities for developing corresponding clinical treatments.

Therefore, there is an urgent need in the field to develop a convenient and effective novel intervention strategy for cerebral calcification aimed at restoring phosphate homeostasis.

### Summary

In a first aspect, the present invention provides a use of an intracerebral phosphate homeostasis promoter in the manufacture of a medicament for preventing and/or treating a cerebral calcification disease.

In some embodiments, the intracerebral phosphate homeostasis promoter is an agent capable of regulating alternative splicing in an intron of the *SLC20A2* gene. In some embodiments, the alternative splicing comprises cryptic exon inclusion, and the agent regulates (e.g., inhibits or enhances) the splicing of the cryptic exon from pre-mRNA of the *SLC20A2* gene. In some embodiments, the agent thereby regulates (e.g., inhibits or enhances) the level of mature mRNA processed from the pre-mRNA, and/or regulates (e.g., inhibits or enhances) the expression of PiT2 protein.

In some embodiments, the alternative splicing comprises alternative splicing in intron 2. In some embodiments, the alternative splicing in intron 2 is a cryptic exon inclusion between exon 2 and exon 3. In some embodiments, the cryptic exon is located at c.289+901 to c.289+1035 of the *SLC20A2* gene. In some embodiments, the cryptic exon has the sequence as shown in SEQ ID NO: 2.

In some embodiments, the alternative splicing comprises alternative splicing in intron 10. In some embodiments, the alternative splicing in intron 10 is a cryptic exon inclusion between exon 10 and 11. In some embodiments, the cryptic exon is located at c.1795-1703 to c.1795-1627 of the *SLC20A2* gene. In some embodiments, the cryptic exon has the sequence as shown in SEQ ID NO: 3.

In some embodiments, the agent specifically binds to a target region in the *SLC20A2* gene or pre-mRNA thereof, wherein the target region is located within an intron region between two canonical exon regions, and wherein the intron region comprises the cryptic exon. In some embodiments, the target region is located within the region from up to about 100 nucleotides upstream of the cryptic exon to up to about 100 nucleotides downstream of the cryptic exon. In some embodiments, the target region is selected from a region spanning the cryptic exon splice site, a region within the cryptic exon, a region within 100 nucleotides upstream of the cryptic exon, or a region within 100 nucleotides downstream of the cryptic exon. In some embodiments, the target region is selected from a region upstream of the cryptic exon 5' splice acceptor site (SA), a region spanning the 5' splice acceptor site, a region within the cryptic exon, a region spanning the 3' splice donor site, or a region downstream of the 3' splice donor site (SD).

In some embodiments, the target region is located within the region c.289+801 to c.289+1135 of the *SLC20A2* gene or pre-mRNA thereof. In some embodiments, the target region is located within the sequence as shown in SEQ ID NO: 8.

In some embodiments, the target region is located within the region c.1795-1803 to c.1795-1527 of the *SLC20A2* gene or pre-mRNA thereof. In some embodiments, the target region is located within the sequence as shown in SEQ ID NO: 9.

In some embodiments, the agent is selected from an antisense oligonucleotide (ASO) or a gene editing tool (e.g., CRISPR/Cas, base editor, or non-Cas protein-dependent RNA editing tool). In some embodiments, the agent is antisense oligonucleotides (ASOs). In some embodiments, the agent is a DNA editor, such as CRISPR/Cas9. In some embodiments, the agent is an RNA editor, such as CRISPR/Cas13. In some embodiments, the agent is a base editor, such as Adenine/Cytosine base editor. In some embodiments, the agent is a non-Cas protein-dependent RNA editing tool.

In some embodiments, the agent is an antisense oligonucleotide (ASO) that specifically binds to the target region in *SLC20A2* pre-mRNA. In some embodiments, the ASO consists of 10~30 (e.g., 15~30, 15~25, 18~30, 18~25, 20~25) consecutive nucleotides; preferably consists of 18~25 consecutive nucleotides. In some embodiments, the ASO comprises a modification of backbone nucleotides and nucleotide linkage, a modification of the ribose, a modification of the nucleobase, or a combination thereof.

In some embodiments, the ASO specifically binds to a target region located within the region c.289+801 to c.289+1135 of *SLC20A2* pre-mRNA. In some embodiments, the ASO has at least 80% (e.g., at least 85%, at least 90%, at least 95%, at least 99%, or 100%) complementarity to a nucleotide sequence within SEQ ID NO: 8. In some embodiments, the ASO comprises a nucleotide sequence as shown in any one of SEQ ID NOs: 13-46, and 63-65, or a sequence having at least 80% (e.g., at least 85%, at least 90%, at least 95%, at least 99%, or 100%) identity thereto.

In some embodiments, the ASO specifically binds to a target region located within the region c.1795-1803 to c.1795-1527 of *SLC20A2* pre-mRNA. In some embodiments, the ASO has at least 80% (e.g., at least 85%, at least 90%, at least 95%, at least 99%, or 100%) complementarity to a nucleotide sequence within SEQ ID NO: 9. In some embodiments, the ASO comprises a nucleotide sequence as shown in any one of SEQ ID NOs: 47-61, and 66-68, or a sequence having at least 80% (e.g., at least 85%, at least 90%, at least 95%, at least 99%, or 100%) identity thereto.

In some embodiments, the agent is a gene editing tool, such as a DNA gene editing system or an RNA base editing tool. In some embodiments, the gene editing tool is a CRISPR/Cas system comprising a Cas protein and a guide RNA (gRNA), and the gRNA is capable of binding to the target region in the *SLC20A2* gene or pre-mRNA thereof. In some embodiments, the gRNA binds to the target region to recruit the binding of an editor complex and exert editing activity. In some embodiments, the target region is located within the region c.289+801 to c.289+1135 of *SLC20A2* pre-mRNA; or located within the region c.1795-1803 to c.1795-1527 of *SLC20A2* pre-mRNA. In some embodiments, the gene editing tool disrupts the function of the cryptic exon's splice acceptor (SA) or splice donor (SD) by mutating their sequences in the genome or pre-mRNA sequence, thereby inhibiting the integration of the cryptic exon into the mRNA. In some embodiments, the gene editing tool is a DNA base editor comprising (i) a fusion protein comprising a DNA base editing domain (e.g., a deaminase) and a DNA binding domain (e.g., a Cas protein, such as Cas9 or Cas12) and (ii) a guide RNA (gRNA), the gRNA capable of binding to the target region in the *SLC20A2* gene. In some embodiments, the gene editing tool is an RNA base editing tool. In some embodiments, the RNA base editing tool comprises: a CRISPR/Cas-based RNA base editor, or a non-CRISPR/Cas-dependent editor, such as an oligonucleotide-based base editor.

In some embodiments, the intracerebral phosphate homeostasis promoter is an astrocyte phosphate transport promoter.

In some embodiments, the intracerebral phosphate homeostasis promoter is selected from the group consisting of:
(Z1) a PiT2 protein, a nucleic acid molecule encoding PiT2 protein, an expression vector expressing PiT2 protein, a PiT2 promoter, or a combination thereof;
(Z2) a MYORG protein, a nucleic acid molecule encoding MYORG protein, an expression vector expressing MYORG protein, a MYORG promoter, or a combination thereof;
(Z3) a XPR1 protein, a nucleic acid molecule encoding XPR1 protein, an expression vector expressing XPR1 protein, an XPR1 promoter, or a combination thereof;
(Z4) any combination of Z1~Z3 above.

In some embodiments, the PiT2 protein comprises the amino acid sequence as shown in SEQ ID NO: 155. In some embodiments, the MYORG protein comprises the amino acid sequence as shown in SEQ ID NO: 154. In some embodiments, the XPR1 protein comprises the amino acid sequence as shown in SEQ ID NO: 153.

In some embodiments, the promoter described in Z1~Z3 comprises a small molecule compound, gene editing reagent, small nucleic acid drug (such as an antisense nucleotide drug), or a combination thereof.

In some embodiments, the astrocyte phosphate transport promoter is an expression vector comprising an exogenous gene and an astrocyte-selectively expressed enhancer element and/or an astrocyte-selectively expressed promoter operably linked thereto, and the exogenous gene is selected from a nucleic acid molecule encoding the PiT2 protein, MYORG protein, or XPR1 protein, or a combination thereof. In some embodiments, the exogenous gene is a nucleic acid molecule encoding the PiT2 protein. In some embodiments, the expression vector is a viral vector, such as AAV. In some embodiments, the astrocyte-selectively expressed promoter is *gfaABC1D.* In some embodiments, the *gfaABC1D* promoter comprises the sequence as shown in SEQ ID NO: 156.

In some embodiments, the astrocyte phosphate transport promoter is a small nucleic acid drug, and the small nucleic acid drug is conjugated to a targeting molecule which has targeting specificity to astrocyte. In some embodiments, the small nucleic acid drug is an ASO. In some embodiments, the small nucleic acid drug is fully or partially encapsulated within the targeting molecule, or conjugated to the targeting molecule. In some embodiments, the targeting molecule is selected from an antibody or antibody fragment thereof, a polysaccharide, an aptamer, or a nanoparticle.

In some embodiments, the cerebral calcification disease comprises Primary Familial Brain calcification (PFBC), Primary Basal Ganglia Calcification (PBGC), or other type of cerebral calcification related to intracerebral phosphate homeostasis deficiency (such as upregulation of cerebrospinal fluid inorganic phosphate). The other type of cerebral calcification disease may comprise other type of cerebral calcification caused by abnormal phosphate transport function in glial cell, such as some sporadic cerebral calcification cases, cerebral calcification caused by tumor or inflammation, and Aicardi-Goutières Syndrome (AGS).

In a second aspect, the present invention provides a method for preventing and/or treating a cerebral calcification disease, comprising administering an effective dose of an intracerebral phosphate homeostasis promoter to a subject in need thereof. In some embodiments, the intracerebral phosphate homeostasis promoter is as defined in the first aspect above. In some embodiments, the cerebral calcification disease comprises Primary Basal Ganglia Calcification (PBGC), Primary Familial Brain calcification (PFBC), or other type of cerebral calcification disease related to intracerebral phosphate homeostasis deficiency (such as upregulation of cerebrospinal fluid inorganic phosphate). The other type of cerebral calcification diseases may comprise other type of cerebral calcification caused by abnormal phosphate transport function in glial cell, such as some sporadic cerebral calcification cases, cerebral calcification caused by tumor or inflammation, and Aicardi-Goutières Syndrome (AGS).

In a third aspect, the present invention provides a medicine kit for treating a cerebral calcification disease, wherein the medicine kit comprises:
(a) a first container, and a first detection reagent for detecting a genetic mutation located in the first container, in which the first detection reagent is used for detecting the presence of the genetic mutation in a gene selected from the group consisting of: *PiT2, MYORG, XPR1,* or a combination thereof; and
(b) a second container, and a pharmaceutical composition located in the second container, in which the pharmaceutical composition comprises the astrocyte phosphate transport promoter as defined in the first aspect and a pharmaceutically acceptable carrier and/or excipient.

In some embodiments, optionally, the medicine kit may further comprise a third container, and a solution located in the third container for enhancing the dissolution or *in vivo* delivery of the drug in the second container.

In a fourth aspect, the present invention provides a method for screening a candidate drug for preventing and/or treating a cerebral calcification disease, wherein the method comprises the following steps:
(a) in a test group, adding a test drug to a culture system of astrocytes or cell line derived therefrom, and measuring the phosphate transport level (E1) and/or activity (A1) in the cells of the test group; in a control group, not adding the test drug to the culture system of the same cells, and measuring the phosphate transport level (E0) and/or activity (A0) in the cells of the control group;
wherein, if the phosphate transport level (E1) and/or activity (A1) in the cells of the test group is significantly higher than that of the control group, it indicates that the test drug promotes the expression and/or activity of phosphate transport level and is a candidate drug for preventing and/or treating the cerebral calcification disease.

In a fifth aspect, the present invention provides a use of an agent capable of regulating alternative splicing in an intron of the *SLC20A2* gene in the manufacture of a medicament for preventing and/or treating a disease related to PiT2 functional deficiency in a subject. Also provided is a method for preventing and/or treating a disease related to PiT2 functional deficiency in a subject, comprising administering an effective dose of an agent capable of regulating alternative splicing in an intron of the *SLC20A2* gene to a subject in need thereof.

In some embodiments, the agent capable of regulating alternative splicing in an intron of the *SLC20A2* gene is as defined in the first aspect.

In some embodiments, the PiT2 functional deficiency involves a mutation in the *SLC20A2* gene. In some embodiments, the mutation is selected from a non-synonymous mutation, nonsense mutation, frameshift mutation, splicing mutation, or structural variation. In some embodiments, the mutation is located in an exon region, and the subject is heterozygous for the mutation in the exon region. In some embodiments, the mutation is located in the target region within an intron, and the subject may be heterozygous, homozygous, or compound heterozygous for the intron mutation located within the target region. In some embodiments, the target region is located within the region c.289+801 to c.289+1135 of *SLC20A2* pre-mRNA, or within the region c.1795-1803 to c.1795-1527 of *SLC20A2* pre-mRNA. In some embodiments, the PiT2 functional deficiency is *SLC20A2* haploinsufficiency. In some embodiments, the disease related to PiT2 functional deficiency benefits from regulation of functional PiT2 protein or RNA expression.

In some embodiments, the disease is a cerebral calcification, such as Primary Familial Brain calcification (PFBC) or Primary Basal Ganglia Calcification (PBGC).

In some embodiments, the disease is a disease related to Pi balance abnormalities, such as a chronic kidney disease, cardiac valve calcification, arterial vascular calcification, and/or skeletal disease related to phosphate homeostasis imbalance.

In a sixth aspect, the present invention provides an agent capable of specifically binding to a target region in the *SLC20A2* gene or pre-mRNA thereof, and the target region is located within an intron region between two canonical exon regions, and wherein the intron region comprises a cryptic exon.

In some embodiments, the cryptic exon is located within intron 2; preferably, the cryptic exon is located at c.289+901 to c.289+1035 of the *SLC20A2* gene or pre-mRNA thereof; preferably, the target region is located within the region c.289+801 to c.289+1135 of the *SLC20A2* gene or pre-mRNA thereof.

In some embodiments, the cryptic exon is located within intron 10; preferably, the cryptic exon is located at c.1795-1703 to c.1795-1627 of the *SLC20A2* gene or pre-mRNA thereof; preferably, the target region is located within the region c.1795-1803 to c.1795-1527 of the *SLC20A2* gene or pre-mRNA thereof.

In some embodiments, the target region is as defined in the first aspect.

In some embodiments, the agent is the ASO as defined in the first aspect.

In a seventh aspect, the present invention provides a pharmaceutical composition comprising the agent capable of specifically binding to a target region in the *SLC20A2* gene or pre-mRNA thereof according to the sixth aspect or a combination thereof, and a pharmaceutically acceptable carrier and/or excipient.

In an eighth aspect, the present invention provides a use of the agent capable of specifically binding to a target region in the *SLC20A2* gene or pre-mRNA thereof according to the sixth aspect or the pharmaceutical composition according to the seventh aspect in the manufacture of a medicament for preventing and/or treating a disease related to PiT2 functional deficiency in a subject. Also provided is a method for preventing and/or treating a disease related to PiT2 functional deficiency in a subject, comprising administering the agent capable of specifically binding to a target region in the *SLC20A2* gene or pre-mRNA thereof according to the sixth aspect or the pharmaceutical composition according to the seventh aspect to a subject in need thereof.

In some embodiments, the disease related to PiT2 functional deficiency is a cerebral calcification, such as Primary Familial Brain calcification (PFBC) or Primary Basal Ganglia Calcification (PBGC).

In some embodiments, the disease related to PiT2 functional deficiency is a disease related to Pi balance abnormalities, such as chronic kidney disease, cardiac valve calcification, arterial vascular calcification, and/or skeletal diseases related to phosphate homeostasis imbalance.

In some embodiments, the medicament treats the disease by regulating the expression of PiT2 protein or functional RNA in the subject's cell.

In some embodiments, the subject comprises a mutation in the *SLC20A2* gene. In some embodiments, the mutation is selected from a non-synonymous mutation, nonsense mutation, frameshift mutation, splicing mutation, or structural variation. In some embodiments, the mutation is located in an exon region, and the subject is heterozygous for the mutation in the exon region. In some embodiments, the mutation is located in the target region within an intron, and the subject may be heterozygous, homozygous, or compound heterozygous for the intron mutation located within the target region. In some embodiments, the target region is located within the region c.289+801 to c.289+1135 of SLC20A2 pre-mRNA, or within the region c.1795-1803 to c.1795-1527 of *SLC20A2* pre-mRNA. In some embodiments, the PiT2 functional deficiency is *SLC20A2* haploinsufficiency.

In a ninth aspect, the present invention provides a method for regulating PiT2 protein expression in a cell, comprising contacting the cell *in vitro* with an agent capable of regulating alternative splicing in an intron of the *SLC20A2* gene. In some embodiments, the agent capable of regulating alternative splicing in an intron of the *SLC20A2* gene is as defined in the first aspect.

In a ninth aspect, the present invention provides a method for regulating PiT2 protein expression in a cell, comprising contacting the cell *in vitro* with the reagent according to the sixth aspect. In some embodiments, the cell comprises a mutation in the *SLC20A2* gene. In some embodiments, the mutation is selected from a non-synonymous mutation, nonsense mutation, frameshift mutation, splicing mutation, or structural variation; preferably, the mutation is located in an exon region, and the cell is heterozygous for the mutation in the exon region; preferably, the mutation is located in the target region within an intron, and the cell may be heterozygous, homozygous, or compound heterozygous for the intron mutation located within the target region.

In a tenth aspect, the present invention provides a method for identifying an agent capable of regulating PiT2 expression in a cell, the method comprising: i) identifying an agent targeting a target region in the PiT2 gene or pre-mRNA thereof, the target region being located within an intron region between two canonical exon regions, and wherein the intron region comprises a cryptic exon, and the target region is within the region from up to about 100 nucleotides upstream of the cryptic exon to up to about 100 nucleotides downstream of the cryptic exon; ii) delivering the agent identified in step i) to the cell; and iii) measuring the mRNA and protein expression levels of PiT2 in the cell of step ii).

In some embodiments, the method further comprises: iv) comparing the expression level measured in step iii) with the PiT2 expression level in a cell treated with a control method.

In some embodiments, the expression level is measured by RT-PCR, qPCR, or by Western blot.

In some embodiments, the target region is selected from a region upstream of the cryptic exon 5' splice acceptor site (SA), a region spanning the 5' splice acceptor site, a region within the cryptic exon, a region spanning the 3' splice donor site, or a region downstream of the 3' splice donor site (SD).

In some embodiments, the cryptic exon is located at c.289+901 to c.289+1035 of the *SLC20A2* gene; preferably, the target region is located within the region c.289+801 to c.289+1135 of the *SLC20A2* gene or pre-mRNA thereof; preferably, the target region is located within the sequence as shown in SEQ ID NO: 8.

In some embodiments, the cryptic exon is located at c.1795-1703 to c.1795-1627 of the *SLC20A2* gene; preferably, the target region is located within the region c.1795-1803 to c.1795-1527 of the *SLC20A2* gene or pre-mRNA thereof; preferably, the target region is located within the sequence as shown in SEQ ID NO: 9.

In an eleventh aspect, the present invention provides a method for preparing a cerebral calcification model, wherein the method comprises modifying a target genome such that the intron sequence between exon2 and exon3 in endogenous *SLC20A2* gene is wholly or partially replaced by the corresponding human *SLC20A2* gene intron genomic fragment, or such that the intron sequence between gene exon10 and exon11 in endogenous *SLC20A2* is wholly or partially replaced by the corresponding human *SLC20A2* gene intron genomic fragment, wherein the corresponding human *SLC20A2* gene intron genomic fragment comprises a mutation that alters *SLC20A2* gene splicing, thereby providing the cerebral calcification model.

In some embodiments, the corresponding human *SLC20A2* gene intron genomic fragment at least comprises a cryptic exon.

In some embodiments, the corresponding human *SLC20A2* gene intron genomic fragment at least comprises the region from about 100 nucleotides upstream of the cryptic exon to about 100 nucleotides downstream of the cryptic exon; for example, the replaced human *SLC20A2* gene intron genomic fragment between exon2 and exon3 comprises the sequence as shown in SEQ ID NO: 10; or the replaced human *SLC20A2* intron genomic fragment between exon 10 and exon11 comprises the sequence as shown in SEQ ID NO: 11.

In some embodiments, the mutation that alters *SLC20A2* gene splicing causes or enhances the alternative splicing in intron 2 or intron 10 as defined in the first aspect.

In some embodiments, the replacement method comprises: gene knockout, gene editing, RNA interference, epigenetic suppression, or any combination thereof.

In some embodiments, the model is an animal model, for example a mammalian model, such as a rodent model (e.g., mouse or rat model) or a non-human primate model.

In some embodiments, the model is a mammalian cell model, for example, human epidermal fibroblast or brain glial cell.

In a twelfth aspect, the present invention provides a genetically modified non-human animal, whose genome comprising: the intron sequence between endogenous *SLC20A2* gene exon2 and exon3 being replaced by the corresponding human *SLC20A2* gene intron genomic fragment, or the intron sequence between endogenous *SLC20A2* gene exon10 and exon11 being replaced by the corresponding human *SLC20A2* gene intron genomic fragment, wherein the corresponding human *SLC20A2* gene intron genomic fragment comprises a mutation that alters *SLC20A2* gene splicing.

In some embodiments, the mutation that alters *SLC20A2* gene splicing causes or enhances the alternative splicing in intron 2 or intron 10 as defined in the first aspect.

In some embodiments, the replaced human *SLC20A2* gene intron genomic fragment between exon2 and exon3 comprises the sequence as shown in SEQ ID NO: 10.

In some embodiments, the replaced human *SLC20A2* gene intron genomic fragment between exon10 and exon11 comprises the sequence as shown in SEQ ID NO: 11.

In some embodiments, the non-human animal is a rodent, such as a mouse or rat.

In some embodiments, the non-human animal is a non-human primate.

In some embodiments, the genetically modified non-human animal is an animal model of cerebral calcification.

In a thirteenth aspect, the present invention provides a genetically modified cell, whose genome comprising: the intron sequence between endogenous *SLC20A2* gene exon2 and exon3 being replaced by the corresponding human *SLC20A2* gene intron genomic fragment, or the intron sequence between endogenous *SLC20A2* gene exon10 and exon11 being replaced by the corresponding human *SLC20A2* gene intron genomic fragment, wherein the corresponding human *SLC20A2* gene intron genomic fragment comprises a mutation that alters *SLC20A2* gene splicing.

In some embodiments, the corresponding human *SLC20A2* gene intron genomic fragment at least comprises a cryptic exon.

In some embodiments, the corresponding human *SLC20A2* gene intron genomic fragment at least comprises the region from about 100 nucleotides upstream of the cryptic exon to about 100 nucleotides downstream of the cryptic exon; for example, the replaced human *SLC20A2* gene intron genomic fragment between exon2 and exon3 comprises the sequence as shown in SEQ ID NO: 10; or the replaced human *SLC20A2* gene intron genomic fragment between exon 10 and exon11 comprises the sequence as shown in SEQ ID NO: 11.

In some embodiments, the mutation that alters *SLC20A2* gene splicing causes or enhances the alternative splicing in intron 2 or intron 10 as defined in the first aspect.

In some embodiments, the corresponding human *SLC20A2* gene intron genomic fragment is introduced exogenously relative to the cell.

In some embodiments, the cell is a mammalian cell.

In some embodiments, the cell is a human epidermal fibroblast or brain glial cell.

In some embodiments, the genetically modified cell is a cell model of cerebral calcification.

In a fourteenth aspect, the present invention provides a use of a genetically modified non-human animal or cell, which comprises: (i) for indicating and analyzing the stage of cerebral calcification disease onset, or for use as a model for studying intracerebral phosphate homeostasis imbalance or cerebral calcification disease targets; (ii) for use as a model for screening a candidate drug or treatment regimen for treating a cerebral calcification disease or disease related to intracerebral phosphate homeostasis imbalance; and/or (iii) for use as a model for screening a candidate drug or treatment regimen for a disease related to phosphate homeostasis imbalance;
wherein, the genome of the genetically modified non-human animal or cell comprises: the intron sequence between endogenous *SLC20A2* gene exon2 and exon3 being replaced by the corresponding human *SLC20A2* gene intron genomic fragment, or the intron sequence between endogenous *SLC20A2* gene exon10 and exon11 being replaced by the corresponding human *SLC20A2* gene intron genomic fragment, wherein the corresponding human *SLC20A2* gene intron genomic fragment comprises a mutation that alters *SLC20A2* gene splicing.

In some embodiments, the corresponding human *SLC20A2* gene intron genomic fragment at least comprises a cryptic exon.

In some embodiments, the corresponding human *SLC20A2* gene intron genomic fragment at least comprises the region from about 100 nucleotides upstream of the cryptic exon to about 100 nucleotides downstream of the cryptic exon; for example, the replaced human *SLC20A2* gene intron genomic fragment between exon2 and exon3 comprises the sequence as shown in SEQ ID NO: 10; or the replaced human *SLC20A2* gene intron genomic fragment between exon 10 and exon11 comprises the sequence as shown in SEQ ID NO: 11.

In some embodiments, the mutation that alters *SLC20A2* gene splicing causes or enhances the alternative splicing in intron 2 or intron 10 as defined in the first aspect.

In a fifteenth aspect, the present invention provides a nucleic acid construct comprising a human *SLC20A2* gene minigene fragment sequence, wherein the minigene fragment sequence comprises exon2 and exon3 and the intron sequence between them, or comprising exon10 and exon11 and the intron sequence between them, wherein the intron sequence comprises a mutation that alters *SLC20A2* gene splicing.

In some embodiments, the nucleic acid construct comprises a promoter operably linked to the minigene fragment sequence.

In some embodiments, the mutation that alters *SLC20A2* gene splicing causes or enhances the alternative splicing in intron 2 or intron 10 as defined in the first aspect.

In some embodiments, the minigene fragment sequence comprises the sequence as shown in SEQ ID NO: 4 or 5.

In some embodiments, a polyA signal sequence is included downstream of the minigene fragment sequence.

In some embodiments, the nucleic acid construct further comprises a reporter gene, such as a fluorescent protein (e.g., GFP, mCherry) or luciferase.

In a sixteenth aspect, the present invention provides a method for selecting an agent capable of regulating *SLC20A2* mRNA splicing, comprising: providing (i) a cell comprising the nucleic acid construct according to the fifteenth aspect, (ii) the genetically modified non-human animal according to the twelfth aspect or the genetically modified cell according to the thirteenth aspect, or (iii) the cerebral calcification model obtained by the method according to the eleventh aspect; contacting a test reagent with any one of (i)-(iii); and detecting alternative splicing of *SLC20A2* mRNA.

In some embodiments, the cell is from, for example, a mammal, such as a rodent (e.g., mouse or rat), a non-human primate, or a human.

In some embodiments, a test reagent capable of inhibiting alternative splicing (e.g., reducing the amount of expression product with cryptic exon inclusion) is selected, thereby increasing the production of functional PiT2 protein.

In some embodiments, a test reagent capable of enhancing alternative splicing (e.g., increasing the amount of expression product with cryptic exon inclusion) is selected, thereby reducing the production of functional PiT2 protein.

In some embodiments, the detection comprises analyzing the length of the *SLC20A2* mRNA expression product.

In some embodiments, the alternative splicing is the alternative splicing in intron 2 as defined in the first aspect; preferably, the detection comprises PCR analysis using primers spanning exon2 and exon3; preferably, an amplicon comprising a cryptic exon inclusion of approximately 135 nt in length is produced.

In some embodiments, the alternative splicing is the alternative splicing in intron 10 as defined in the first aspect; preferably, the detection comprises PCR analysis using primers spanning exon10 and exon11; preferably, an amplicon containing a cryptic exon inclusion of approximately 76 nt in length is produced.

In some embodiments, the method comprises contacting a test reagent *in vitro* with a cell containing the nucleic acid construct and detecting alternative splicing of the nucleic acid construct; preferably, the detection comprises analyzing the length of the mRNA expression product of the nucleic acid construct or analyzing the expression of a reporter gene contained in the nucleic acid construct.

### Brief Description of the Drawings

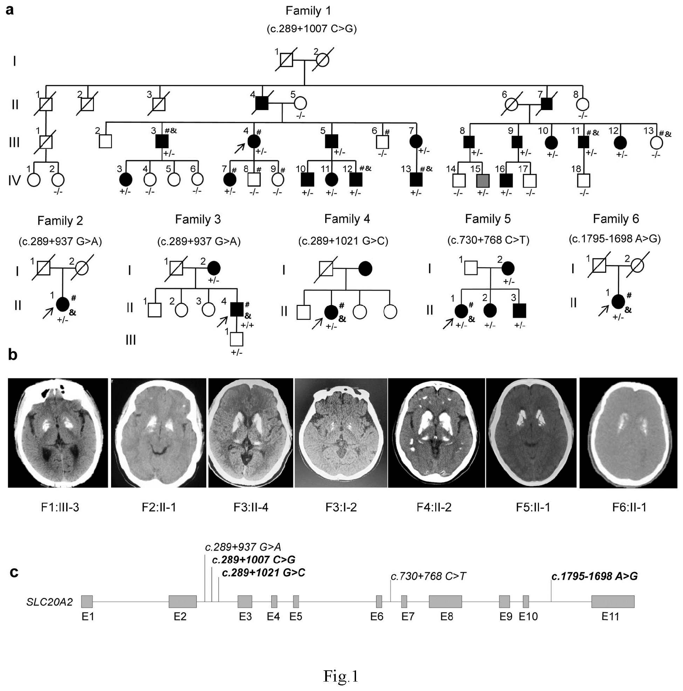

Figure 1: Detection of *SLC20A2* intron mutations in PFBC families by WGS.
   a, Pedigrees of six PFBC families with *SLC20A2* intron mutations. Solid symbols, open symbols, gray symbols, or slashed symbols represent individuals affected by cerebral calcification, individuals without cerebral calcification, individuals without symptoms, and deceased individuals, respectively. Symbols with a question mark (?) indicate individuals without brain CT imaging data. "#" and "&" indicate individuals selected for whole-exome sequencing (WES) or whole-genome sequencing (WGS), respectively. b, Representative brain CT images of patients from the six PFBC families. c, Distribution of five intron mutations in the *SLC20A2* locus. Light purple boxes indicate exons, with exon numbers marked below. Lines between exons indicate introns. In intron mutation labels, italicized and bolded text indicates mutations predicted not to produce new splicing acceptor or donor, suggesting they may function by affecting potential cryptic exons; while italicized but not bolded mutations may produce splicing acceptor or donor.

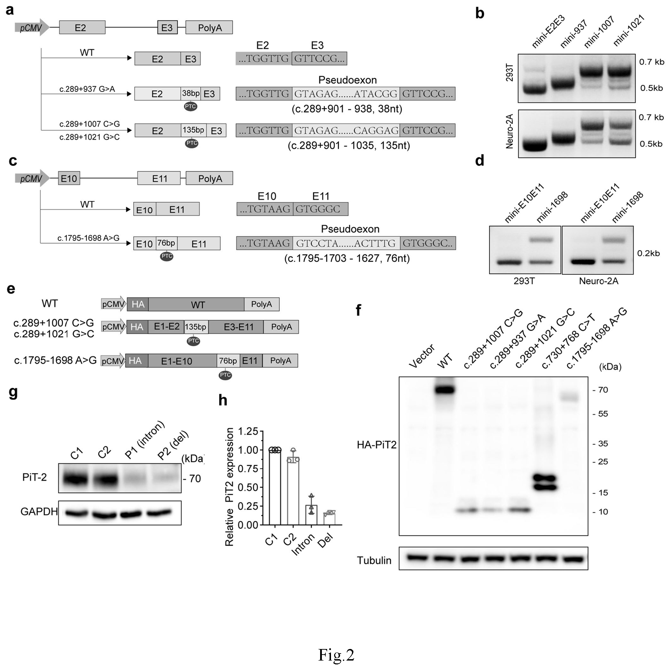

Figure 2: *SLC20A2* cryptic exons alter normal *SLC20A2* mRNA splicing and PiT2 protein expression due to intron mutations.
   a-b, Schematic diagram of minigene constructs and transcripts for *SLC20A2* intron mutations c.289+937 G>A and c.289+1007 C>G (a). (b) shows PCR detection of splicing product changes after intron mutations in the minigene. RT-PCR analysis of Neuro-2A cell samples transfected with *SLC20A2* minigenes carrying the mutations c.289+937 G>A, c.289+1007 C>G, and c.289+1021 G>C shows that the lower band represents correctly spliced exons, while the upper band represents PCR products of transcripts with a cryptic exon inserted between exon 2 and exon 3 of *SLC20A2.* c-d, Schematic diagram of *SLC20A2* minigene constructs containing human *SLC20A2* intron 10 with the c.1795-1698 A>G mutation (c) and PCR detection analysis of the transcripts (d). The lower band represents correctly spliced transcripts, while the upper band represents PCR products of transcripts with a cryptic exon inserted between exon 10 and exon 11 of *SLC20A2.* e, Schematic diagram of *SLC20A2* CDS constructs with abnormal splicing caused by two intron mutations (each with an N-terminal HA tag). f, After transfecting Neuro-2A cells with HA-labeled *SLC20A2* CDS constructs, Western blot (WB) analysis detects the impact of endogenous *SLC20A2* mutations on PiT2 protein products. g-h, Western blot results of PiT2 in fibroblast extracts from a PFBC patient carrying the c.289+1007 C>G mutation (P1), a PFBC patient with *SLC20A2* gene heterozygous deletion (P2), or healthy controls (C1 and C2) show that intron mutations lead to decreased PiT2 expression similar to exon mutations; (h) shows the statistical results.

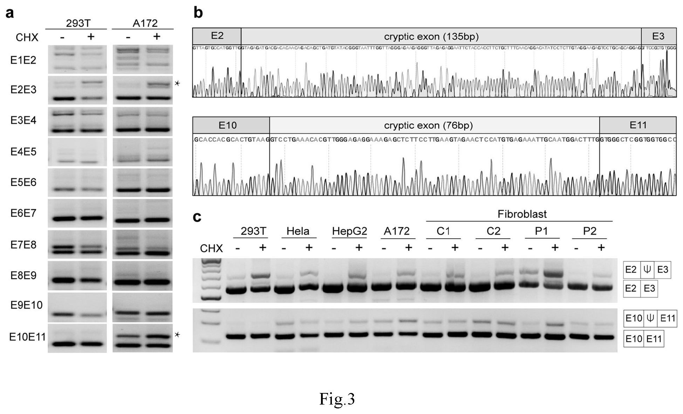

Figure 3: Presence of *SLC20A2* cryptic exons crpE2 and crpE10 in transcripts generated from human brain cells.
   a, RT-PCR analysis of whether cryptic exon inclusion events exist between adjacent exons of the *SLC20A2* gene mRNA in HEK293T and A172 cells. CHX represents cycloheximide (a widely used compound that can inhibit degradation of abnormal transcripts). Generally, the lower band represents normal transcripts; the upper band represents abnormal transcripts containing a certain cryptic exon (marked with an asterisk, named crpE2 and crpE10). b, Sanger sequencing of the upper bands for cryptic exons (crpE2 and crpE10) from HEK293T and A172 cells in Figure 3a reveals sequences identical to the cryptic exons found in the minigene experiments in Figure 2, showing these cryptic exons are endogenously present, but mutations significantly increase their probability of integration into mRNA. c, RT-PCR products for crpE2 and crpE10 from human cell lines comprising HEK293T, HeLa, HepG2, A172, as well as human fibroblasts from healthy controls (C1, C2); a PFBC patient with *SLC20A2* heterozygous c.289+1007 C>G mutation (P1); and a PFBC patient with *SLC20A2* heterozygous deletion (P2).

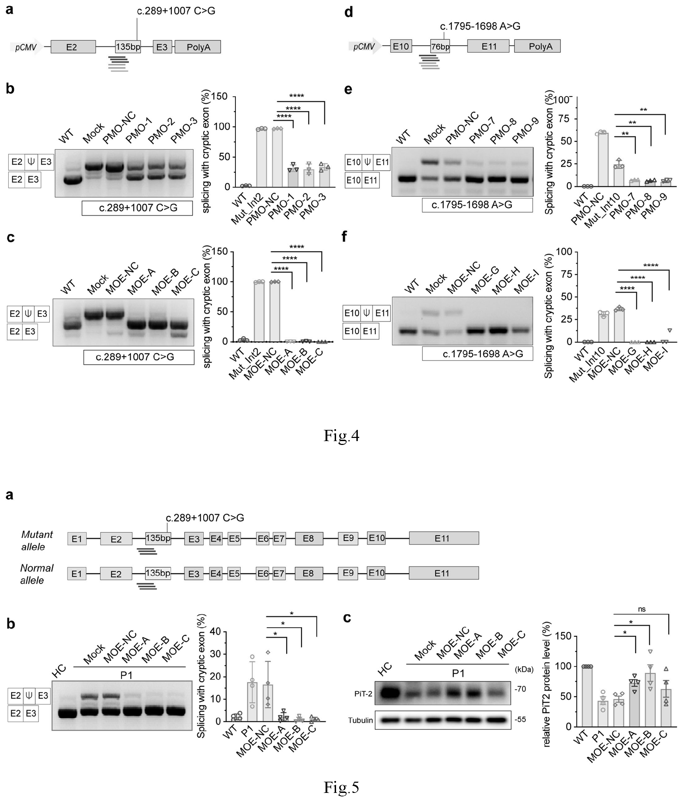

Figure 4: ASOs inhibit cryptic exon integration in *SLC20A2* minigene.
   a, Schematic diagram of PMO-modified ASOs (PMO-1, PMO-2, PMO-3) and MOE-modified ASOs (MOE-A, MOE-B, MOE-C) targeting the cryptic exon on the *SLC20A2* genomic sequence carrying the c.289+1007 C>G mutation in the minigene. b and c, compared to control ASOs (PMO-NC and MOE-NC), RT-PCR analysis and statistical results of crpE2 cryptic exon integration in Neuro-2A cells transfected with the *SLC20A2* c.289+1007 C>G minigene after intervention with PMO-modified ASOs (PMO-1, PMO-2, PMO-3) (b), or MOE-modified ASOs (MOE-A, MOE-B, MOE-C) (c). Cells were harvested for RNA extraction. The upper band represents unproductive transcripts with crpE2 (729 bp); the lower band represents normal transcripts (594 bp). d, Schematic of PMO-modified ASOs (PMO-7, PMO-8, PMO-9) and MOE-modified ASOs (MOE-G, MOE-H, MOE-I) targeting the cryptic exon on the *SLC20A2* genomic sequence carrying the c.1795-1698 mutation in the minigene. e and f, compared to control ASOs (PMO-NC and MOE-NC), RT-PCR analysis and statistical results of crpE10 cryptic exon integration in Neuro-2A cells transfected with the *SLC20A2* c.1795-1698 A>G mutation minigene after intervention with PMO-modified ASOs (PMO-7, PMO-8, PMO-9) (e), or MOE-modified ASOs (MOE-G, MOE-H, MOE-I) (f). Cells were harvested for RNA extraction. The upper band represents transcripts with crpE10 insertion (261 bp), the lower band represents normal transcripts (185 bp). Red lines represent MOE-modified ASOs; blue lines represent PMO-modified ASOs. The quantitation value (%) for splicing events with cryptic exon inclusion is calculated based on RT-PCR band intensity as follows: (Transcript with cryptic exon inclusion / (Transcript with cryptic exon inclusion + Normal transcript)) × 100. Relative PiT2 protein level is assessed using Tubulin as internal reference (i.e., PiT2/Tubulin). *P < 0.05, **P < 0.01, ***P < 0.001, ****P < 0.0001, Student's t-test.
Figure 5: ASOs inhibit cryptic exon integration in *SLC20A2* mRNA caused by intron mutations and upregulate PiT2 protein expression.
   a, Schematic showing that in cultured fibroblasts from a PFBC patient (P1) carrying the *SLC20A2* c.289+1007 C>G intron mutation, MOE-modified ASOs (MOE-A, MOE-B, MOE-C) can target the cryptic exon on the *SLC20A2* gene pre-mRNA transcribed from both the chromosome carrying the c.289+1007 C>G intron mutation and the normal chromosome without the mutation, thereby inhibiting abnormal *SLC20A2* splicing. b, RT-PCR detection and statistical analysis of crpE2 cryptic exon integration into *SLC20A2* mRNA levels in stably cultured fibroblasts derived from a PFBC patient (P1) carrying the *SLC20A2* c.289+1007 C>G intron mutation after treatment with MOE-modified ASOs (MOE-A, MOE-B, MOE-C, or control MOE-NC). Cells were harvested for RNA extraction. c, Western blot results and statistical analysis of PiT2 protein levels in P1-derived fibroblasts after intervention with MOE-modified ASOs (MOE-A, MOE-B, MOE-C) compared to MOE-NC. Red lines represent MOE-modified ASOs. The quantitation value (%) for splicing events with cryptic exon inclusion is calculated based on RT-PCR band intensity as follows: (Transcript with cryptic exon inclusion / (Transcript with cryptic exon inclusion + Normal transcript)) × 100. Relative PiT2 protein level is assessed using Tubulin as internal reference (i.e., PiT2/Tubulin). *P < 0.05, **P < 0.01, ***P < 0.001, ****P < 0.0001, Student's t-test.

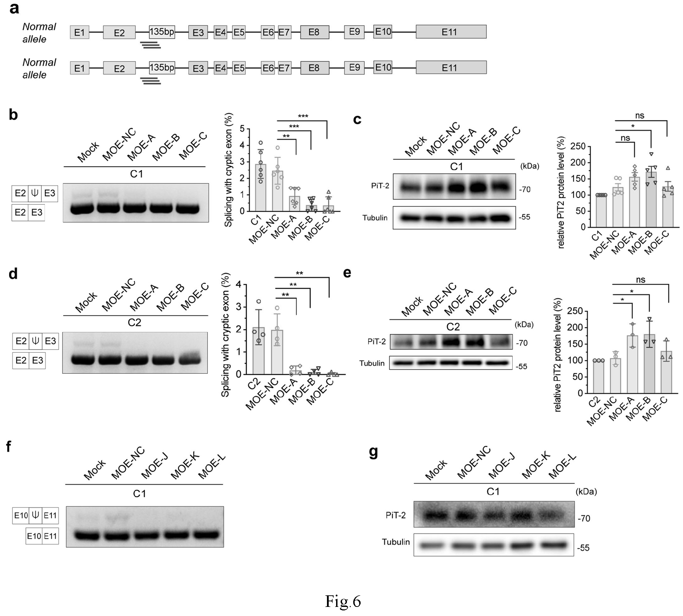

Figure 6: ASOs can also increase PiT2 expression by regulating cryptic exon-involved splicing events in *SLC20A2* in normal human fibroblasts.
   a, Schematic showing that in cultured human fibroblasts from healthy controls, on the *SLC20A2* gene pre-mRNA transcribed from normal chromosomes, MOE-modified ASOs (targeting crpE2: MOE-A, MOE-B, MOE-C; targeting crpE10: MOE-J, MOE-K, MOE-L) modulate splicing events of cryptic exons crpE2 or crpE10, thereby increasing *SLC20A2* mRNA and PiT2 protein expression. b, RT-PCR detection and statistical analysis of the expression level of SLC20A2 mRNA transcripts with crpE2 cryptic exon inclusion in human fibroblasts from a healthy control (C1) after treatment with MOE-modified ASOs (MOE-A, MOE-B, MOE-C, or control MOE-NC). The upper band is the transcript with crpE2 inclusion (699 bp), the lower band is the normal transcript (564 bp). c, Western blot results and statistical analysis of PiT2 protein levels in human fibroblasts from healthy control (C1) after intervention with MOE-modified ASOs (MOE-A, MOE-B, MOE-C) compared to MOE-NC. d, RT-PCR detection and statistical analysis of the expression level of SLC20A2 mRNA transcripts with crpE2 cryptic exon inclusion in human fibroblasts from a healthy control (C2) after treatment with MOE-modified ASOs (MOE-A, MOE-B, MOE-C, or control MOE-NC). The upper band is the transcript with crpE2 inclusion (699 bp), the lower band is the normal transcript (564 bp). e, Western blot results and statistical analysis of PiT2 protein levels in human fibroblasts from healthy control (C2) after intervention with MOE-modified ASOs (MOE-A, MOE-B, MOE-C) compared to MOE-NC. f, RT-PCR detection and statistical analysis of the expression level of SLC20A2 mRNA transcripts with crpE10 cryptic exon inclusion in human fibroblasts from a healthy control (C1) after treatment with MOE-modified ASOs (MOE-G, MOE-H, MOE-I) compared to MOE-NC. The upper band is the transcript with crpE10 inclusion (199 bp), the lower band is the normal transcript (123 bp). g, PiT2 protein levels in human fibroblasts from healthy control C1 after intervention with MOE-modified ASOs (MOE-G, MOE-H, MOE-I) compared to MOE-NC. The quantitation value (%) for splicing events with cryptic exon inclusion is calculated based on RT-PCR band intensity as follows: (Transcript with cryptic exon inclusion / (Transcript with cryptic exon inclusion + Normal transcript)) × 100. Relative PiT2 protein level is assessed using Tubulin as internal reference (i.e., PiT2/Tubulin). *P < 0.05, **P < 0.01, ***P < 0.001, ****P < 0.0001, Student's t-test.

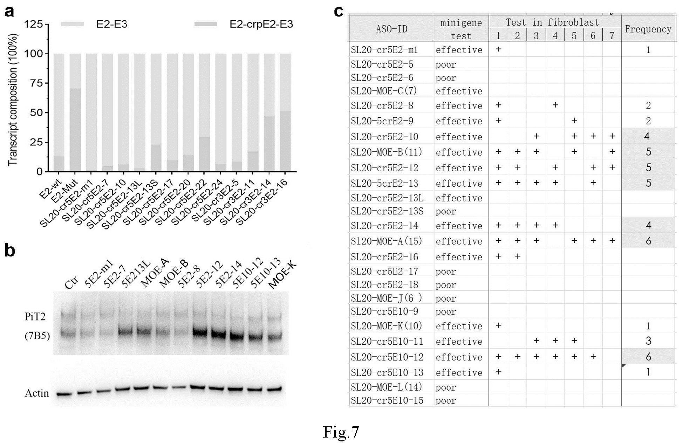

Figure 7: Expanded screening of ASO sequences regulating *SLC20A2* splicing.
   a, PCR analysis of splicing products expressed from N2A cells transfected with minigenes in response to ASOs, comprising new ASO sequences targeting crpE2 splicing modulation (cr5E2-m1, cr5E2-5, cr5E2-6, cr5E2-8, 5crE2-9, cr5E2-10, cr5E2-12, 5crE2-13, cr5E2-13L, cr5E2-13S, cr5E2-14, cr5E2-16, cr5E2-17, cr5E2-18, cr5E2-19, cr5E2-20, cr5E2-21, cr5E2-22, cr5E2-23, cr5E2-24, cr3E2-5, cr3E2-8, cr3E2-9, cr3E2-10, cr3E2-11, cr3E2-12, cr3E2-13, cr3E2-14, cr3E2-15, cr3E2-16), and new ASO sequences targeting crpE10 splicing modulation (cr5E10-9, cr5E10-11, cr5E10-12 ,cr5E10-13, cr5E10-15, cr5E10-16, cr5E10-19, cr3E10-9, cr3E10-14, cr3E10-15). Their sequences and relevant information are listed in the appendix table. b, Further screening of ASO sequences selected from minigene screening on healthy human fibroblasts. The effects of ASOs on regulating endogenous *SLC20A2* splicing were analyzed, assessed through repeated PCR and protein WB detection. Only partial sequence experimental results are shown above. c, Comprehensive analysis after two rounds of screening to obtain several ASO sequences with good efficacy.

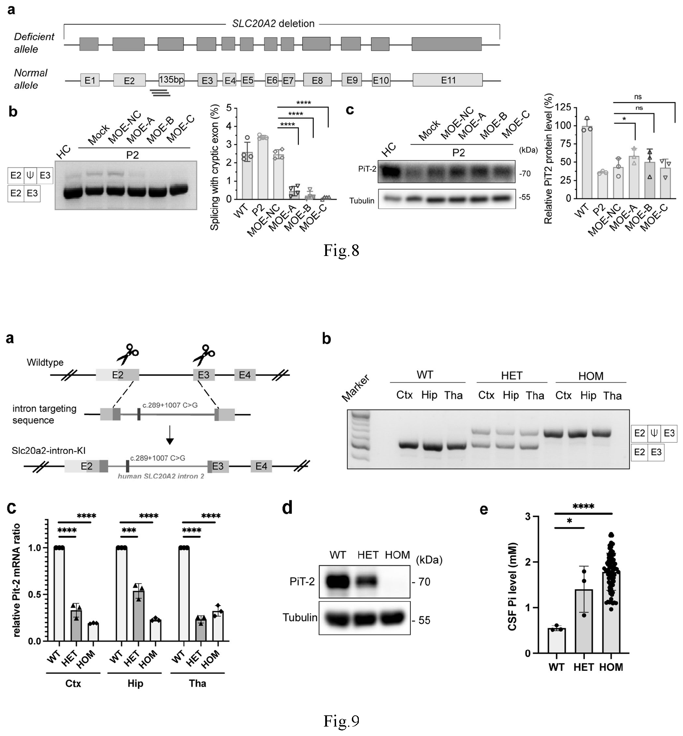

Figure 8: ASOs regulate *SLC20A2* mRNA splicing and upregulate PiT2 protein expression in a patient with *SLC20A2* heterozygous deletion.
   a. Schematic diagram shows that in cultured fibroblasts from a patient carrying a large genomic fragment heterozygous deletion of *SLC20A2* (P2), on the *SLC20A2* gene pre-mRNA transcribed from the other normal chromosome, MOE-modified ASOs (MOE-A, MOE-B, MOE-C) can be used to target splicing events of the cryptic exon crpE2 therein, thereby increasing *SLC20A2* mRNA and PiT2 protein expression. b, RT-PCR detection and statistical analysis of crpE2 cryptic exon integration into *SLC20A2* mRNA levels in stably cultured fibroblasts from a patient carrying a large genomic fragment heterozygous deletion of *SLC20A2* (P2) after treatment with MOE-modified ASOs (MOE-A, MOE-B, MOE-C) or control MOE-NC. Cells were harvested for RNA extraction. c, Western blot results and statistical analysis of PiT2 protein levels in P2 fibroblasts after intervention with MOE-modified ASOs (MOE-A, MOE-B, MOE-C) compared to MOE-NC. Red lines represent MOE-modified ASOs. The quantitation value (%) for splicing events with cryptic exon inclusion is calculated based on RT-PCR band intensity as follows: (Transcript with cryptic exon inclusion / (Transcript with cryptic exon inclusion + Normal transcript)) × 100. Relative PiT2 protein level is assessed using Tubulin as internal reference (i.e., PiT2/Tubulin). *P < 0.05, **P < 0.01, ***P < 0.001, ****P < 0.0001, Student's t-test.
Figure 9: Construction and *SLC20A2* expression analysis of humanized *SLC20A2* intron knock-in *(SLC20A2-KI)* model mice.
   a, Schematic diagram of the construction of *SLC20A2-KI* mouse model with the humanized SLC20A2 gene intron 2. CRISPR/Cas9 was used to create a nick at the genomic SLC20A2 intron 2 position, then homology-directed repair guided by the introduced targeting donor sequence replaced the mouse *SLC20A2* intron 2 and flanking bridging sequences to exon 2 and exon 3 with the human *SLC20A2* intron 2 sequence carrying the c.289+1007 C>G mutation. The final sequence produces transcripts that match the overall reading frame of the mouse Slc20a2. b, RT-PCR analysis of the expression level of SLC20A2 transcripts integrating the humanized crpE2 in brain tissues of heterozygous and homozygous *SLC20A2-KI* mice and wild-type mice. The upper band represents abnormal transcripts (crpE2) produced from the humanized intron 2 with the c.289+1007 C>G mutation; the lower band corresponds to normal *SLC20A2* transcripts. WT, wild-type; HET, heterozygous; HOM, homozygous; Ctx, cerebral cortex; Hip, hippocampus; Tha, thalamus. c and d, qPCR analysis of *SLC20A2* transcript levels (c) and Western blot results of PiT2 levels (d) in the brains of WT, HET, and HOM *SLC20A2-KI* mice both show that humanized mutation mice have Slc20a2 mRNA and PiT2 protein expression defects similar to Slc20a2 KO mice. e, Compared to wild-type mice, heterozygous and homozygous *SLC20A2-KI* mice show a graded increase in CSF Pi levels, similar to Slc20a2 KO mice.

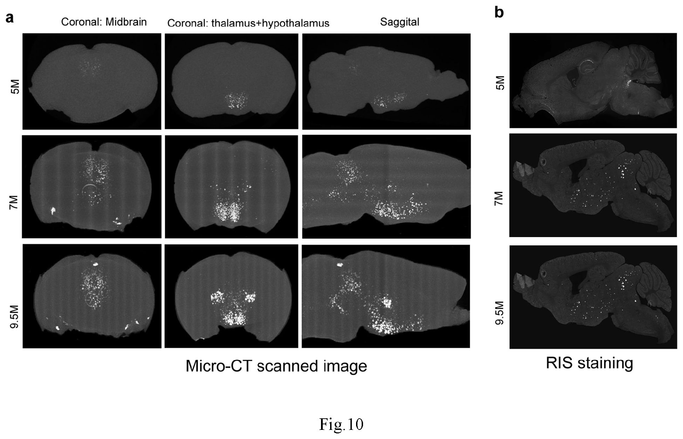

Figure 10: Micro-CT and RIS staining analysis of cerebral calcification in humanized *SLC20A2 (SLC20A2-KI)* model mice.
   a, Micro-CT scanning (voxel resolution: 9um x 9um x 9um) images of calcification effects in brains of homozygous *SLC20A2-KI* model mice at 5, 7, and 9.5 months of age, after PFA perfusion fixation. From left to right are coronal sections of the dorsal midbrain segment, coronal sections of the basal forebrain, and sagittal sections of one cerebral hemisphere. White dots represent calcified nodules. b, Cerebral calcification images acquired using VS120 microscope from brain sections of 5-, 7-, and 9.5-month-old homozygous *SLC20A2-KI* mice stained with the fluorescent dye RIS-AF647 (an imaging agent carrying an AlexaFluo-647nm fluorophore that binds to calcium bisphosphonate salts). Images are representative of analysis results from three mice per group. *P < 0.05, **P < 0.01, ***P < 0.001, ****P < 0.0001, Student's t-test. Scale bar, 1 mm.

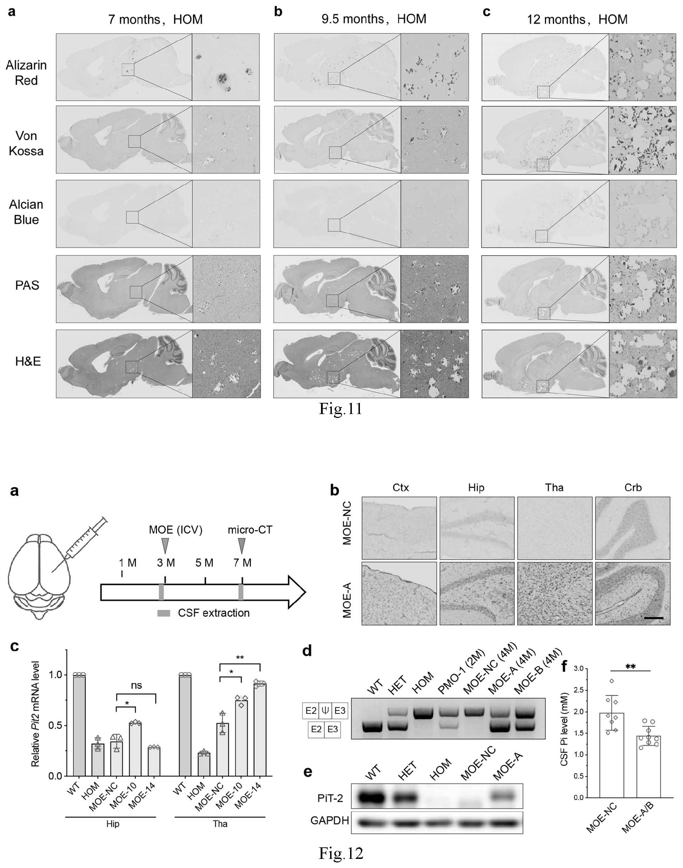

Figure 11: Histochemical staining analysis of cerebral calcification in humanized *SLC20A2 (SLC20A2-KI)* model mice.
   OCT frozen sections of fixed mouse brains from homozygous *SLC20A2-KI* mice at 7 months (a), 10 months (b), and 12 months (c) of age were subjected to five different histochemical stains, including alizarin red, Von Kossa, Alcian blue, H&E, and PAS. Among them, alizarin red and Von Kossa primarily show calcified deposits, while Alcian blue and PAS primarily show the attached mucopolysaccharide components in the calcification foci, which is a common feature of calcified deposits. Scale bar, 500 µm.
Figure 12: Intracerebroventricular intervention with ASO in *SLC20A2-KI* mice restores *SLC20A2* expression in the brain and reduces cerebrospinal fluid phosphate levels.
   a, Experimental design scheme for ASO intracerebroventricular (ICV) injection administration in homozygous *SLC20A2-KI* mice. Mice at 3 months of age were administered ICV injections of MOE-NC, MOE-A, or MOE-B, with treatment duration of 4 months. Doses for each MOE were 50µg, 100µg, 200µg, or 400µg. Brain tissues were collected from mice at 7 months of age to analyze cryptic exon inclusion in SLC20A2 transcripts, PiT2 protein levels, cerebrospinal fluid phosphate levels, and cerebral calcification. b, In situ hybridization analysis of the distribution of MOE-A throughout the mouse brain (cortex, hippocampus, thalamus, and cerebellum) 2 months after ICV injection in 5-month-old *SLC20A2-KI* mice. The RNA probe was designed to specifically bind to the sequence of MOE-A. MOE-A is stained red, DAPI is stained blue. c, Quantitative real-time PCR analysis of normal *SLC20A2* mRNA transcript expression in *SLC20A2-KI* mice 4 months after administration of MOE-NC, MOE-A, or MOE-B, compared to age-matched WT mice and untreated homozygous *SLC20A2-KI* mice. d, RT-PCR analysis of transcripts with cryptic exon crpE2 inclusion and normal *SLC20A2* transcripts 4 months after ICV injection of MOE-NC, MOE-A, and MOE-B. The topmost band represents mRNA transcripts with cryptic exon crpE2 inclusion (crpE2), while the bottom band represents normal *SLC20A2* transcripts. e, PiT2 protein levels in the thalamus of *SLC20A2-KI* mice 4 months after ICV injection of MOE-A, showing significant restoration of expression compared to control MOE and untreated mice, indicating the effectiveness of the ASO. f, Besides target gene expression, a biomarker directly related to cerebral calcification, i.e., cerebrospinal fluid inorganic phosphate levels, also showed a differential decrease, further indicating the effectiveness of the ASO.

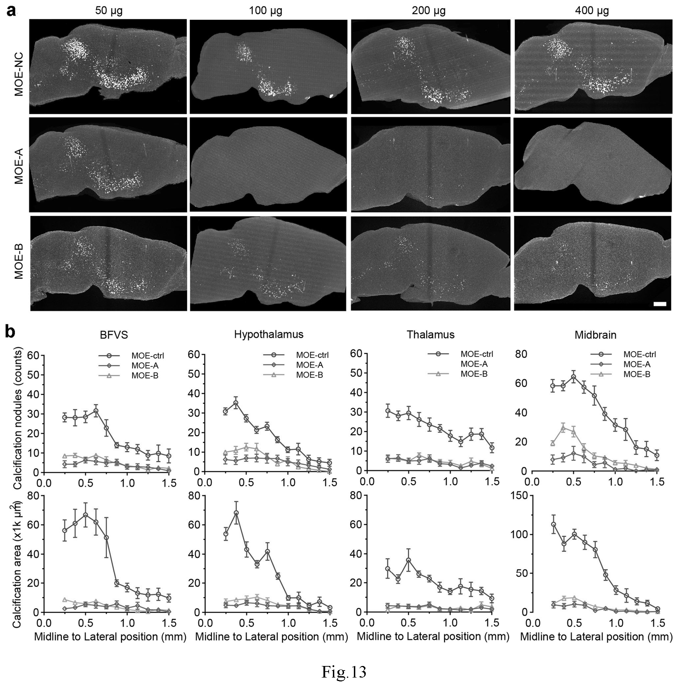

Figure 13: Intracerebroventricular intervention with ASO in *SLC20A2-KI* mice significantly inhibits the occurrence and progression of cerebral calcification.
   a, Micro-CT imaging of cerebral calcification with sagittal view in 3-month-old *SLC20A2-*KI mice 4 months after ICV injection of different doses (50, 100, 200, 400µg) of MOE-NC, MOE-A, and MOE-B; white bright spots are cerebral calcification nodules. b, Quantification of the number and area of calcification nodules distributed in the thalamus, hypothalamus, midbrain, and basal forebrain in 3-month-old *SLC20A2-*KI mice when they reached 7 months of age, 4 months after ICV injection of different doses (50, 100, 200, 400 µg) of MOE-NC, MOE-A, and MOE-B. Images are representative of analysis from two to three mice per group. *P < 0.05, **P < 0.01, ***P < 0.001, ****P < 0.0001, Student's t-test. Scale bar, 1mm.

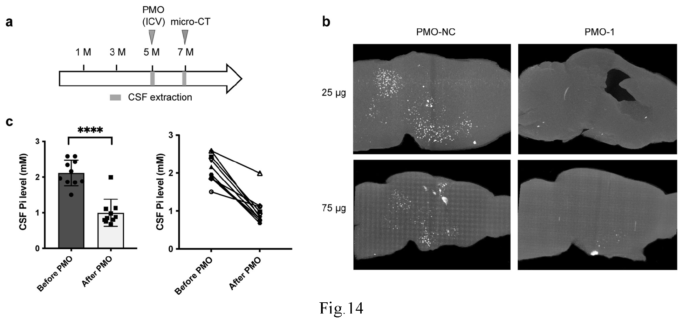

Figure 14: ICV injection of ASO drug can inhibit further progression of cerebral calcification in *SLC20A2-*KI mice that have already developed cerebral calcification.
   a, Experimental design for ICV injection of PMO-modified ASO in homozygous *SLC20A2-*KI mice. Mice at 5 months of age already had obvious calcification deposits in multiple brain regions. At this time point, ICV injections of PMO-NC or PMO-1 were administered, with an intervention period of 2 months. The dose for each PMO was 25µg or 75µg. Brain tissues were collected from mice at 7 months of age and analyzed for cryptic exon inclusion and cerebral calcification. CSF was extracted for analysis before PMO administration and at the end of administration (i.e., at 5 and 7 months). b, Changes in CSF Pi levels in homozygous *SLC20A2-*KI mice before and after administration of PMO-NC and PMO-1. c, Micro-CT imaging with sagittal view of cerebral calcification in 7-month-old homozygous *SLC20A2-*KI mice after intervention with control and therapeutic ASO at ICV injection doses of 25 µg and 75 µg. White bright spots are cerebral calcification nodules. Images are representative of analysis from two to three mice per group. ****P < 0.0001, Student's t-test. Scale bar, 1mm.

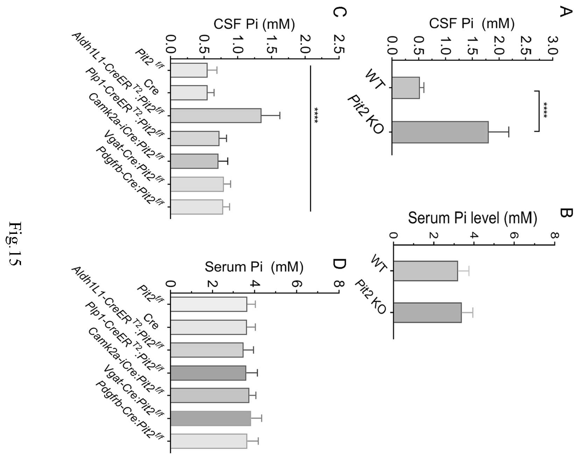

Figure 15: Impact of global or conditional knockout of Pit2 (Slc20a2) on brain phosphate homeostasis.
   Panel A shows that the average cerebrospinal fluid (CSF) inorganic phosphate (Pi) level in PiT2 global knockout *(Pit2* KO) mice is ~1.8 mM, showing a significant increase compared to the CSF Pi concentration in WT mouse brains (~0.5 mM). Panel B shows that serum inorganic phosphate (Pi) in PiT2 global knockout mice shows no significant change. Panel C shows changes in CSF Pi concentration in the brains of different cell-type-specific *Pit2* conditional knockout *(Pit2* cKO) mice. Among them, conditional knockout mice in astrocyte (obtained using *Aldh1L1-CreER*^{T2} induced by Tamoxifen) showed the most significant increase in CSF Pi (approximately 170% increase), with an average level of about 1.35 mM. Changes in CSF Pi in other conditional knockout mice, comprising in oligodendrocyte (*Plp1-CreER*^{T2}), excitatory neuron (*Camk2a-iCre*), inhibitory neuron *(Vgat-Cre),* and pericyte *(Pdgfrb-Cre),* were more moderate (increases ranging between 40-60%). This result indicates that astrocyte-mediated Pi transport is most important for maintaining brain phosphate homeostasis. Panel D shows no significant difference in serum Pi concentration among different conditional knockout mouse strains.

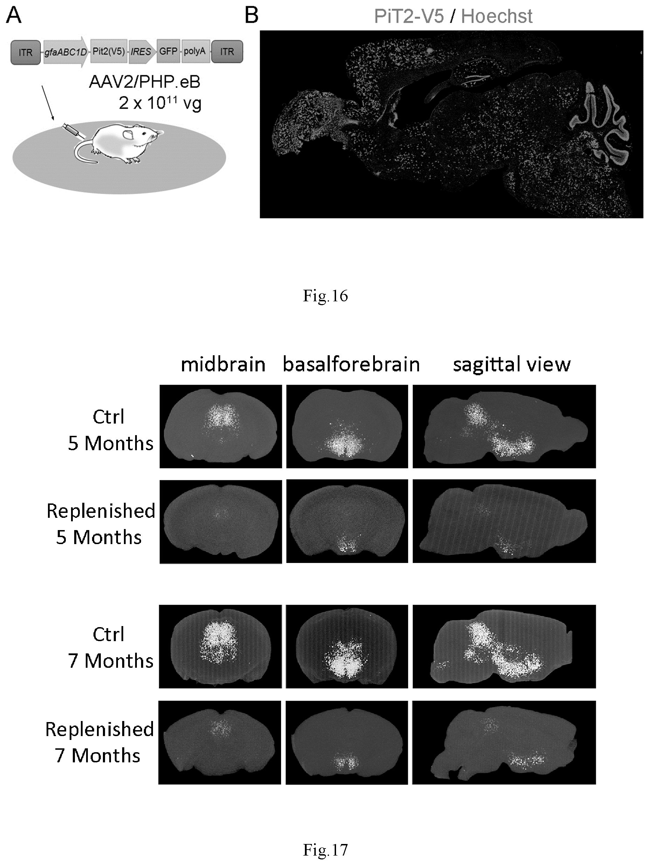

Figure 16:Selective PiT2 expression in brain astrocytes mediated by intravenous injection of AAV virus.
   Panel A shows the AAV serotype (PHP.eB) and expression vector structure diagram, as well as the mouse intravenous injection scheme. Panel B shows sagittal brain sections from mice one month after intravenous injection, prepared after PFA fixation. Immunofluorescence staining shows widespread expression of PiT2-V5 signal (red) in astrocytes throughout the mouse brain; blue Hoechst indicates nuclei.
Figure 17: Inhibitory effect on mouse cerebral calcification occurrence after AAV-mediated astrocyte-selective compensatory expression.
   Images obtained from microCT scanning of perfused mouse brains show that in the control virus group (expressing only GFP) of Pit2 KO mice, at 5 and 7 months of age, coronal and sagittal views of cerebral calcification reveal obvious distribution of calcification foci in multiple brain regions. Significant large-area calcification deposits are present in the dorsal midbrain, basal forebrain, thalamus, and hypothalamus; 7-month-old mice have larger calcification coverage area and larger calcification particles than 5-month-old mice. However, the brains of Pit2 KO mice in therapeutic AAV injection group (Replenished) at 5 and 7 months of age show significantly inhibited calcification.

### Detailed Description

### [I, Definitions]

In the present invention, unless otherwise stated, the scientific and technical terms used herein have the meanings commonly understood by those skilled in the art. Definitions and explanations of relevant terms are provided below for better understanding of the present invention.

As used herein, the term *"SLC20A2"* refers to the gene encoding solute carrier family 20 member 2 (see, e.g., human gene NCBI ID:6575), also known as *PiT2,* which encoding a protein product of a 652-amino-acid phosphate transporter called PiT2 protein, or PiT-2 protein (see, e.g., NP_006740). The mRNA sequence of *SLC20A2* is well-known to those skilled in the art, see, for example, NCBI: NM_006749, NM_001257181, or NM_001257180. As used herein, when describing positions on the mRNA sequence of the *SLC20A2* gene, for example, to describe the location of a cryptic exon, the sequence shown in NCBI: NM_006749 is used as the reference. An exemplary amino acid sequence of the PiT2 protein is as shown in SEQ ID NO: 155.

As used herein, the term *"XPRI"* refers to the gene encoding the phosphate efflux transporter protein XPR1. The sequences of the *XPR1* gene (e.g., gene ID: 9213) and the XPR1 protein (e.g., NP_004727.2) are known to those skilled in the art and can be found in public databases, such as HGNC:12827. An exemplary amino acid sequence of the XPR1 protein is as shown in SEQ ID NO: 153.

As used herein, the term *"MYORG"* refers to the gene encoding the regulator of the phosphate efflux transporter protein XPR1, MYORG. The sequences of the *MYORG* gene (e.g., gene ID: 57462) and the MYORG protein (e.g., NP_065753.2) are known to those skilled in the art and can be found in public databases, such as HGNC:19918. An exemplary amino acid sequence of the MYORG protein is as shown in SEQ ID NO: 154.

As used herein, the phrase "regulation of splicing" refers to a change affecting the level of any RNA or mRNA variant produced from the natural *SLC20A2* pre-mRNA. For example, regulation can mean, e.g., causing an increase or decrease in the level of abnormal *SLC20A2* mRNA containing cryptic exon inclusion, causing an increase or decrease in the level of normal full-length *SLC20A2* mRNA, and/or causing an increase or decrease in the level of abnormal *SLC20A2* RNA or mRNA containing a premature termination codon (nonsense codon). It is readily understood that any change in the ratio between *SLC20A2* splicing variants is also considered a result of splicing regulation. Each possibility represents a separate embodiment of the invention. In some embodiments, regulation means increasing the level of normal full-length *SLC20A2* mRNA and/or decreasing the level of abnormal (e.g., containing cryptic exon inclusion) *SLC20A2* mRNA.

As used herein, the terms "complementary" or "complementarity" refer to the ability of nucleic acids, such as oligonucleotides, polynucleotides, etc., to form base pairs with each other. Base pairing typically formed by hydrogen bonds between nucleotide units in antiparallel polynucleotide chains. Complementary polynucleotide chain can base pair in a Watson-Crick manner (e.g., A pairs with T, A pairs with U, C pairs with G) or in any other manner that allows duplex formation. As known to those skilled in the art, when RNA is used instead of DNA, the base considered complementary to adenosine is uracil rather than thymine. However, when U is indicated in the context of the present invention, unless otherwise stated, the ability to substitute T is implied. "At least partially complementary" means that two sequences may be fully complementary, or have no more than 6, 5, 4, 3, 2, or 1 mismatched base pairings overall, while retaining the ability to hybridize under relevant conditions. Those skilled in the art can determine the conditions most suitable for testing the complementarity of two sequences based on the ultimate application of the hybridized nucleotides. Such conditions may be, for example, stringent conditions, such as in an *in vitro* test system, 400 mM NaCl, 40 mM PIPES pH 6.4, 1 mM EDTA, at 50°C or 70°C for 12-16 hours, followed by washing. Other conditions, such as physiologically relevant conditions encountered *in vivo,* may also be applied.

As used herein, unless otherwise specified, the left end of a single-stranded nucleic acid (e.g., pre-mRNA transcript, oligonucleotide, ASO, etc.) sequence is the 5' end, and the leftward direction of a single- or double-stranded nucleic acid sequence is referred to as the 5' direction. Similarly, the right end or rightward direction of a nucleic acid sequence (single- or double-stranded) is the 3' end or 3' direction. Generally, a region or sequence in a nucleic acid that is 5' to a reference point is called "upstream", and a region or sequence in a nucleic acid that is 3' to a reference point is called "downstream". Generally, the 5' direction or end of an mRNA is the position of the initiation codon/start codon, while the 3' end or direction is the position of the termination codon. In some aspects, nucleotides upstream of a reference point in a nucleic acid may be represented by negative numbers, while nucleotides downstream of the reference point may be represented by positive numbers. For example, a reference point (e.g., an exon-exon junction in mRNA) may be denoted as the "zero" site, and the nucleotide directly adjacent to and upstream of the reference point is denoted as "minus one", e.g., "-1", while the nucleotide directly adjacent to and downstream of the reference point is denoted as "plus one", e.g., "+1".

As used herein, the term "pharmaceutically acceptable carrier and/or excipient" refers to carriers and/or excipients that are pharmacologically and/or physiologically compatible with the subject and the active ingredient, which are well-known in the art. "Pharmaceutically acceptable carriers and/or excipients" do not exert, or are not intended to exert, a therapeutic effect at the intended dosage. Such ingredients may serve functions such as: a) aiding in the processing of the drug delivery system during manufacturing, b) protecting, supporting, or enhancing the stability, bioavailability, or patient acceptability of the active ingredient, c) aiding in product identification, and/or d) enhancing any other attribute of the overall safety, effectiveness, delivery, etc. of the active ingredient during storage and use. For example, the siRNA of the present invention may be encapsulated by a delivery vector. "Pharmaceutically acceptable carriers and/or excipients" include, but are not limited to: pH adjusters, surfactants, adjuvants, ionic strength enhancers, diluents, osmotic pressure-maintaining agents, absorption-delaying agents, preservatives. For example, pH adjusters include but are not limited to phosphate buffers. Surfactants include but are not limited to cationic, anionic, or non-ionic surfactants, such as Tween-80. Ionic strength enhancers include but are not limited to sodium chloride. Preservatives include but are not limited to various antibacterial and antifungal agents, such as parabens, chlorobutanol, phenol, sorbic acid, etc. Osmotic pressure-maintaining agents include but are not limited to sugars, NaCl, and analogues thereof. Absorption-delaying agents include but are not limited to monostearates and gelatin. Diluents include but are not limited to water, aqueous buffers (e.g., buffered saline), alcohols, and polyols (e.g., glycerol), etc.

As used herein, the term "treatment" refers to methods implemented to achieve a beneficial or desired clinical outcome. For the purposes of the present invention, beneficial or desired clinical outcomes include, but are not limited to, alleviating symptoms, reducing the extent of disease, stabilizing (i.e., not worsening) the state of disease, delaying or slowing disease progression, improving or alleviating the state of disease, and relieving symptoms (whether partially or completely), whether detectable or undetectable. Furthermore, "treating" may also refer to prolonging survival compared to the expected survival period if not treated.

As used herein, the term "effective dose" refers to a dose sufficient to achieve or at least partially achieve the desired effect. For example, a therapeutically effective dose for a disease refers to a dose sufficient to cure or at least partially prevent the disease and its complications in a patient already suffering from the disease, for example, a dose necessary to improve at least one clinical parameter of the disease the patient suffers from or reduce the severity of at least one clinical parameter of the disease suffered. Determining such an effective dose is entirely within the capability of those skilled in the art. For instance, the dose effective for therapeutic use will depend on the severity of the disease to be treated, the overall state of the patient's own immune system, the general condition of the patient such as age, weight, and gender, the mode of drug administration, and other concomitant therapies, etc.

### II, Therapy Based on Regulating Alternative Splicing

In some aspects, the present invention relates to the regulation of alternative splicing and its applications in disease treatment, disease model preparation, etc.

Genetic studies have found that PFBC can be caused by autosomal heterozygous mutations in *SLC20A2, PDGFRB, PDGFB,* or *XPR1,* or by autosomal biallelic mutations in *MYORG, JAM2,* or *CMPK2*; in particular, dominant heterozygous mutations in the *SLC20A2* gene account for 61% of all reported PFBC cases, indicating that PFBC can be treated with gene therapy targeting the corresponding gene. However, insufficient understanding of PFBC pathogenesis and the lack of effective delivery tools have largely hindered the development of gene editing therapies for PFBC.

The inventors of the present application performed whole-genome sequencing (WGS) on 135 PFBC patients who were determined to be exon mutation or CNV negative by whole-exome sequencing (WES). They identified five *SLC20A2* gene intron mutations from six autosomal dominant PFBC families, and subsequently confirmed through minigene assays and patient-derived cell experiments that cryptic exon sequences exist within the intron sequences of *SLC20A2* pre-mRNA. The aforementioned intron mutations increase the probability of these cryptic exon sequences being integrated during mRNA processing; because these cryptic exon sequences contain premature termination codons, the abnormally spliced mRNA sequences cannot be translated into functional protein product PiT2. Thus, the inventors of the present application discovered a mechanism of premature termination of PiT2 translation due to previously unreported cryptic exon inclusion in abnormal *SLC20A2* mRNA splicing, providing a novel strategy for regulating *SLC20A2* gene mRNA splicing and expression.

Based on the above discoveries, the inventors of the present application demonstrated that ASO-mediated splicing modulation can enhance normal *SLC20A2* mRNA and PiT2 protein expression in cells containing *SLC20A2* intron mutations and in fibroblasts from patients with *SLC20A2* haploinsufficiency. Furthermore, in a humanized *SLC20A2* mouse model simulating SLC20A2-deficient PFBC patients, ASOs administered intracerebroventricularly (ICV) were able to reduce abnormal *SLC20A2* transcripts and inhibit the occurrence and progression of cerebral calcification.

Thus, the inventors of the present application provide a therapy for inhibiting cerebral calcification by regulating the *SLC20A2* alternative splicing process to reduce the inclusion of cryptic exons within introns, offering a promising treatment approach for diseases related to PiT2 functional deficiency (e.g., PFBC caused by *SLC20A2* haploinsufficiency).

Furthermore, based on the discovered alternative splicing in *SLC20A2* introns, the inventors of the present application further provide cellular and animal models of cerebral calcification and their preparation methods. These models can be used for indicating and analyzing the stage of cerebral calcification disease onset, serving as models for studying brain phosphate homeostasis imbalance or cerebral calcification disease targets, and for drug screening.

### II-A, Use of Regulating Alternative Splicing in Disease Treatment

In some aspects, the present invention relates to agents, methods, and use for regulating the splicing of *SLC20A2* gene pre-mRNA to restore or enhance the expression and function of the *SLC20A2* gene protein product PiT2.

One aspect of the present invention provides the use of an agent capable of regulating alternative splicing in introns of the *SLC20A2* gene for preventing and/or treating a disease related to PiT2 functional deficiency in a subject; or for use in the manufacture of a medicament for preventing and/or treating a disease related to PiT2 functional deficiency in a subject. The present invention also provides a method for preventing and/or treating a disease related to PiT2 functional deficiency in a subject, the method comprising administering an effective dose of an agent capable of regulating alternative splicing in introns of the *SLC20A2* gene to a subject in need thereof. The PiT2 functional deficiency comprises deficiency in expression level or activity.

In some embodiments, the alternative splicing comprises cryptic exon inclusion, and the agent regulates (e.g., inhibits or enhances) the splicing of the cryptic exon from the *SLC20A2* gene's pre-mRNA. In some embodiments, the agent thereby regulates (e.g., inhibits or enhances) the level of mature mRNA processed from the pre-mRNA, and/or regulates (e.g., inhibits or enhances) PiT2 protein expression.

In some embodiments, the agent inhibits cryptic exon inclusion in mature *SLC20A2* mRNA, increases the level of correctly spliced mature *SLC20A2* mRNA, decreases the level of abnormally spliced mature *SLC20A2* mRNA, and/or increases the production of functional PiT2 protein. Herein, the phrase "increasing the level of correctly spliced mature *SLC20A2* mRNA" refers to an increase in the level of correctly spliced mature *SLC20A2* mRNA after treatment with the agent provided by the present invention compared to the level before treatment or after control treatment.

In some embodiments, the agent promotes cryptic exon inclusion in mature *SLC20A2* mRNA, inhibits the level of correctly spliced mature *SLC20A2* mRNA, increases the level of abnormally spliced mature *SLC20A2* mRNA; wherein the cryptic exon sequence carries a premature termination codon (PTC), and the inclusion of this cryptic exon leads to a reduction or inhibition of functional PiT2 protein production. Herein, the phrase "decreasing the level of abnormally spliced mature *SLC20A2* mRNA" refers to a decrease in the level of abnormally spliced mature *SLC20A2* mRNA after treatment with the agent provided by the present invention compared to the level before treatment or after control treatment. The abnormally spliced mature *SLC20A2* mRNA comprises cryptic exon inclusion between exon 2 and exon 3 or between exon 10 and exon 11 as defined herein.

### Alternative Splicing in Intron 2

In some embodiments, the alternative splicing comprises alternative splicing in intron 2.

In some embodiments, the alternative splicing in intron 2 is the inclusion of a cryptic exon between exon 2 and exon 3 into the processed SLC20A2 mRNA sequence.

In some embodiments, the cryptic exon is located at c.289+901 to c.289+1035 of the *SLC20A2* gene.

In some embodiments, the c.289+1007 C>G mutation in the *SLC20A2* gene promotes the alternative splicing in intron 2.

In some embodiments, the cryptic exon has the sequence as shown in SEQ ID NO: 2.

In some embodiments, the agent inhibits alternative splicing in intron 2. As used herein, the phrase "inhibiting alternative splicing in intron 2" refers to reducing the incidence of including the 135nt length cryptic exon sequence (SEQ ID NO: 2) found in intron 2 of the *SLC20A2* gene into mature *SLC20A2* mRNA.

### Alternative Splicing in Intron 10

In some embodiments, the alternative splicing comprises alternative splicing in intron 10.

In some embodiments, the alternative splicing in intron 2 is the inclusion of a cryptic exon between exon 10 and exon 11 into the processed SLC20A2 mRNA sequence.

In some embodiments, the cryptic exon is located at c.1795-1703 to c.1795-1627 of the *SLC20A2* gene.

In some embodiments, the c.1795-1698 A>G mutation in the *SLC20A2* gene promotes the alternative splicing in intron 10.

In some embodiments, the cryptic exon has the sequence as shown in SEQ ID NO: 3.

In some embodiments, the agent inhibits alternative splicing in intron 10. As used herein, the phrase "inhibiting alternative splicing in intron 10" refers to reducing the incidence of including the 76nt length cryptic exon sequence (SEQ ID NO: 3) found in intron 10 of the *SLC20A2* gene into mature *SLC20A2* mRNA.

In some embodiments, the alternative splicing comprises the alternative splicing in intron 2 or alternative splicing in intron 10. In some embodiments, the agent is an agent that inhibits alternative splicing in intron 2, or an agent that inhibits alternative splicing in intron 10.

### Target Region

In some embodiments, the agent specifically binds to a target region in the *SLC20A2* gene or pre-mRNA thereof, and the target region is located within an intron region between two canonical exon regions, and wherein the intron region contains the cryptic exon.

In some embodiments, the target region is within the region from up to about 100 (e.g., up to about 90, up to about 80, up to about 70, up to about 60, up to about 50, up to about 40, up to about 30, up to about 20, up to about 10, up to about 9, up to about 8, up to about 7, up to about 6, up to about 5, up to about 4, up to about 3, up to about 2, up to about 1) nucleotides upstream of the cryptic exon to up to about 100 (e.g., up to about 90, up to about 80, up to about 70, up to about 60, up to about 50, up to about 40, up to about 30, up to about 20, up to about 10, up to about 9, up to about 8, up to about 7, up to about 6, up to about 5, up to about 4, up to about 3, up to about 2, up to about 1) nucleotides downstream of the cryptic exon.

In some embodiments, the target region is selected from a region spanning a cryptic exon splice site, a region within the cryptic exon, a region within 100 nucleotides upstream of the cryptic exon, or a region within 100 nucleotides downstream of the cryptic exon.

In some embodiments, the target region is selected from a region upstream of the cryptic exon 5' splice acceptor site (SA), a region spanning the 5' splice acceptor site, a region within the cryptic exon, a region spanning the 3' splice donor site, or a region downstream of the 3' splice donor site (SD).

In some embodiments, the target region is located within the region c.289+801 to c.289+1135 of the *SLC20A2* gene or its pre-mRNA.

In some embodiments, the target region is located within the sequence as shown in SEQ ID NO: 8.

In some embodiments, the target region is located within the region c.1795-1803 to c.1795-1527 of the *SLC20A2* gene or its pre-mRNA.

In some embodiments, the target region is located within the sequence as shown in SEQ ID NO: 9.

In some embodiments, the target region comprises a region spanning the cryptic exon's 5' splice acceptor site (SA). In some embodiments, the target region is within the region from up to about 25 (e.g., up to about 22, up to about 20, up to about 18, up to about 15, up to about 12, up to about 10, up to about 9, up to about 8, up to about 7, up to about 6, up to about 5, up to about 4, up to about 3, up to about 2, up to about 1) nucleotides upstream of the cryptic exon's 5' splice acceptor site to up to about 25 (e.g., up to about 22, up to about 20, up to about 18, up to about 15, up to about 12, up to about 10, up to about 9, up to about 8, up to about 7, up to about 6, up to about 5, up to about 4, up to about 3, up to about 2, up to about 1) nucleotides downstream of the cryptic exon's 5' splice acceptor site. In some embodiments, the target region is within the region from up to about 20 nucleotides upstream to up to about 20 nucleotides downstream of the cryptic exon's 5' splice acceptor site.

In some embodiments, the target region comprises a region spanning the cryptic exon's 3' splice donor site (SD). In some embodiments, the target region is within the region from up to about 25 (e.g., up to about 22, up to about 20, up to about 18, up to about 15, up to about 12, up to about 10, up to about 9, up to about 8, up to about 7, up to about 6, up to about 5, up to about 4, up to about 3, up to about 2, up to about 1) nucleotides upstream of the cryptic exon's 3' splice donor site to up to about 25 (e.g., up to about 22, up to about 20, up to about 18, up to about 15, up to about 12, up to about 10, up to about 9, up to about 8, up to about 7, up to about 6, up to about 5, up to about 4, up to about 3, up to about 2, up to about 1) nucleotides downstream of the cryptic exon's 3' splice donor site. In some embodiments, the target region is within the region from up to about 20 nucleotides upstream to up to about 20 nucleotides downstream of the cryptic exon's 3' splice donor site.

In some embodiments, the target region is located within the cryptic exon. In some embodiments, the target region or its vicinity contains a splicing regulatory element, such as a splicing enhancer element or a splicing silencer element.

In some embodiments, the target region is located within the intron region upstream or downstream of the cryptic exon. In some embodiments, the target region or its vicinity contains a splicing regulatory element, such as a branch site, a splicing enhancer element, or a splicing silencer element.

### Agents

In some embodiments, the agent is an antisense oligonucleotide (ASO). In some embodiments, the agent is a gene editing tool, such as a DNA or RNA editing system, e.g., CRISPR/Cas, base editors, or non-Cas protein-dependent RNA editing tool. In some embodiments, the agent is a DNA editor, such as CRISPR/Cas9. In some embodiments, the agent is an RNA editor, such as CRISPR/Cas13. In some embodiments, the agent is a base editor, such as an Adenine/Cytosine base editor. In some embodiments, the agent is a non-CRISPR/Cas-dependent RNA editing tool.

### ASO

In some embodiments, the agent is an antisense oligonucleotide (ASO) that specifically binds to a target region in the *SLC20A2* pre-mRNA. The ASO binds to the target region specifically via base complementarity, thereby exerting a function to modulate pre-mRNA splicing through a steric hindrance effect.

In some embodiments, the ASO consists of 10~30 (e.g., 15~30, 15~25, 18~30, 18~25, 20~25) consecutive nucleotides; preferably consists of 18~25 consecutive nucleotides.

Herein, the term ASO comprises any form of oligomeric molecule containing nucleic acid bases capable of hybridizing complementarily to nucleic acid bases on a target mRNA. ASOs may comprise naturally occurring nucleotides, nucleotide analogs, modified nucleotides, or any combination of the foregoing two or three. The term "naturally occurring nucleotide" comprises deoxyribonucleotides and ribonucleotides. The term "modified nucleotide" comprises nucleotides with modified or substituted sugar groups and/or with modified backbones. Chemical modifications of ASOs will be apparent to those skilled in the art.

In some embodiments, the ASO comprises modifications of backbone nucleotides and nucleotide linkages, modifications of the ribose, modifications of the nucleobase, or combinations thereof. In some embodiments, the backbone modification is selected from phosphorothioate linkages or phosphorodiamidate linkages. In some embodiments, the sugar moiety modifications include but are not limited to: 2'-O-methoxyethyl (2'-MOE), morpholino, 2'-O-methyl (2'-OMe), 2'-fluoro (2'-F), constrained ethyl (cEt), locked nucleic acid (LNA), peptide nucleic acid (PNA).

In some embodiments, each monomer of the ASO is modified in the same manner, for example, each linkage of the ASO's backbone contains a phosphorothioate linkage or each ribose moiety contains a 2'O-methyl modification. Such modifications present on each of the monomeric components of the ASO are referred to as "uniform modifications". In some embodiments, a combination of different modifications may be desired, for example, the ASO may comprise a combination of phosphorodiamidate linkages with sugar moieties containing a morpholine ring (morpholino). A combination of different modifications for an ASO is referred to as "mixed modifications" or "mixed chemistries".

In some embodiments, the ASO comprises 2'MOE modifications. In some embodiments, every nucleotide of the ASO has a 2'MOE modification. In some embodiments, the modifications of the ASO comprise a combination of 2'MOE and locked nucleic acid (LNA). In some embodiments, the ASO has a backbone with phosphorothioate linkages.

In some embodiments, the ASO comprises 2'-OMe modifications. In some embodiments, every nucleotide of the ASO has a 2'-OMe modification. In some embodiments, the modifications of the ASO comprise a combination of 2'-OMe and locked nucleic acid (LNA). In some embodiments, the ASO has a backbone with phosphorothioate linkages.

In some embodiments, the ASO comprises a combination of phosphorodiamidate linkages with sugar moieties containing a morpholine ring (morpholino), for example, it is a Phosphorodiamidate morpholino oligomer (PMO).

In some embodiments, the ASO specifically binds to a target region located within the region c.289+801 to c.289+1135 of *SLC20A2* pre-mRNA.

In some embodiments, the ASO has at least 80% complementarity to a nucleotide sequence within SEQ ID NO: 8. Herein, complementarity (the degree to which one polynucleotide is complementary to another) can be quantified by the proportion (e.g., percentage) of bases in the opposing strands that are expected to form hydrogen bonds with each other according to generally accepted base pairing rules. The sequence of the ASO need not be 100% complementary to the sequence of the target nucleic acid it hybridizes to. In some embodiments, an ASO may have at least 70%, at least 75%, at least 80%, at least 85%, at least 90%, at least 95%, at least 96%, at least 97%, at least 98%, or at least 99% sequence complementarity to the target region within the target nucleic acid sequence it targets. For example, an ASO where 18 of 20 nucleobases are complementary to the target region and will thus specifically hybridize would exhibit 90% complementarity.

In some embodiments, the ASO comprises a nucleotide sequence shown in any one of SEQ ID NOs: 13-46, 63-65, or a sequence having at least 80% (e.g., at least 85%, at least 90%, at least 95%, at least 99%, or 100%) identity thereto. In some embodiments, the ASO comprises a nucleotide sequence shown in any one of SEQ ID NOs: 19-22, 25, 26, or a sequence having at least 80% (e.g., at least 85%, at least 90%, at least 95%, at least 99%, or 100%) identity thereto.

In some embodiments, the ASO comprises a nucleotide sequence shown in any one of SEQ ID NOs: 13-46, or a sequence having at least 80% (e.g., at least 85%, at least 90%, at least 95%, at least 99%, or 100%) identity thereto, and comprises 2'MOE modifications and/or phosphorothioate linkages. In some embodiments, every nucleotide of the ASO has a 2'MOE modification, and the ASO has a backbone with phosphorothioate linkages.

In other embodiments, the ASO comprises a nucleotide sequence shown in any one of SEQ ID NOs: 63-65, or a sequence having at least 80% (e.g., at least 85%, at least 90%, at least 95%, at least 99%, or 100%) identity thereto, and the ASO is a PMO.

In some embodiments, the ASO specifically binds to a target region located within the region c.1795-1803 to c.1795-1527 of *SLC20A2* pre-mRNA.

In some embodiments, the ASO has at least 80% (e.g., at least 85%, at least 90%, at least 95%, at least 99%, or 100%) complementarity to a nucleotide sequence within SEQ ID NO: 9.

In some embodiments, the ASO comprises a nucleotide sequence shown in any one of SEQ ID NOs: 47-61, 66-68, or a sequence having at least 80% (e.g., at least 85%, at least 90%, at least 95%, at least 99%, or 100%) identity thereto. In some embodiments, the ASO comprises the nucleotide sequence as shown in SEQ ID NO: 50, or a sequence having at least 80% (e.g., at least 85%, at least 90%, at least 95%, at least 99%, or 100%) identity thereto.

In some embodiments, the ASO comprises a nucleotide sequence shown in any one of SEQ ID NOs: 47-61, or a sequence having at least 80% (e.g., at least 85%, at least 90%, at least 95%, at least 99%, or 100%) identity thereto, and comprises 2'MOE modifications and/or phosphorothioate linkages. In some embodiments, every nucleotide of the ASO has a 2'MOE modification, and the ASO has a backbone with phosphorothioate linkages.

In other embodiments, the ASO comprises a nucleotide sequence shown in any one of SEQ ID NOs: 66-68, or a sequence having at least 80% (e.g., at least 85%, at least 90%, at least 95%, at least 99%, or 100%) identity thereto, and the ASO is a PMO.

### Gene Editing Tools

In some embodiments, the agent is a gene editing tool, such as a DNA gene editing system or an RNA base editing tool, which typically comprises (i) a module with base editing activity and (ii) a module capable of binding to the target region in the *SLC20A2* gene. The gene editing tool inhibits the integration of the cryptic exon into mRNA by mutating the splice acceptor (SA) or splice donor (SD) sequences of the cryptic exon in the genomic or pre-mRNA sequence, thereby disrupting their SA or SD function.

Exemplary gene editing tools include, but are not limited to, CRISPR/Cas systems, DNA base editors, and RNA editing tools.

### CRISPR/Cas System

In some embodiments, the agent is a CRISPR/Cas system. The CRISPR/Cas system typically comprises a Cas protein and a guide RNA (gRNA), and the gRNA capable of binding to the target region in the *SLC20A2* gene or its pre-mRNA.

In some embodiments, the gRNA binds to the target region and thereby recruits the binding of the Cas enzyme to exert editing activity.

In some embodiments, the CRISPR system disrupts the function of the cryptic exon's splice acceptor (SA) or splice donor (SD) by mutating their sequences, thereby inhibiting the integration of the cryptic exon into the mRNA.

In some embodiments, the target region is located within the region c.289+801 to c.289+1135 of *SLC20A2* pre-mRNA.

In some embodiments, the target region is located within the region c.1795-1803 to c.1795-1527 of *SLC20A2* pre-mRNA.

### DNA Base Editors

In some embodiments, the agent is a DNA base editor. A DNA base editor typically comprises (i) a fusion protein comprising a DNA base editing domain (e.g., a deaminase) and a DNA-binding domain (e.g., a Cas protein, such as Cas9 or Cas12), and (ii) a guide RNA (gRNA) capable of binding to the target region in the *SLC20A2* gene.

In some embodiments, the gRNA binds to the target region and thereby recruits the binding of the fusion protein to exert base editing activity.

In some embodiments, the DNA base editor disrupts the function of the cryptic exon's splice acceptor (SA) or splice donor (SD) by causing base mutations in their sequences, thereby inhibiting the integration of the cryptic exon into the mRNA.

In some embodiments, the Cas protein is a catalytically inactive Cas protein or a Cas nickase protein.

In some embodiments, the base editor comprises: an ABE (Adenine Base Editor) or a CBE (Cytosine Base Editor), or novel editor obtained through molecular evolution/mutation screening.

### RNA Editing Tools

In some embodiments, the agent is an RNA editing tool. RNA editing tools include, for example: CRISPR/Cas-based RNA base editors, non-CRISPR/Cas-dependent editors, such as oligonucleotide-based base editors, etc.

In some embodiments, the CRISPR/Cas-based RNA base editor comprises (i) a fusion protein comprising an RNA base editing domain (e.g., an RNA deaminase) and an RNA-binding domain (e.g., Cas13), and (ii) a guide RNA (gRNA) capable of binding to the target region in the *SLC20A2* pre-mRNA. The gRNA binds to the target region and thereby recruits the binding of the fusion protein to exert base editing activity.

In some embodiments, the oligonucleotide-based base editor comprises (i) a sequence module capable of recruiting an endogenous RNA base editing protein (e.g., an RNA deaminase, such as ADAR) and (ii) a guide sequence module capable of binding to the target region in the *SLC20A2* pre-mRNA. The guide sequence module binds to the target region and thereby recruits the binding of the RNA base editing protein to exert base editing activity.

In some embodiments, the RNA editing tool disrupts the function of the cryptic exon's splice acceptor (SA) or splice donor (SD) by causing base mutations in their sequences in the pre-mRNA, thereby inhibiting the integration of the cryptic exon into the mRNA.

### Use

In some embodiments, the PiT2 functional deficiency involves a mutation in the *SLC20A2* gene. In some embodiments, the mutation is selected from a non-synonymous mutation, nonsense mutation, frameshift mutation, splicing mutation, or structural variation.

In some embodiments, the mutation is located in an exon region, and the subject is heterozygous for the mutation in the exon region.

In some embodiments, the mutation is an intron mutation, for example, a mutation within intron 2 and/or intron 10. In some embodiments, the mutation is located in the target region within an intron, and the subject may be heterozygous, homozygous, or compound heterozygous for the intron mutation located within the target region.

In some embodiments, the subject is a mammal. In some embodiments, the subject is a human.

In some embodiments, the PiT2 functional deficiency is *SLC20A2* haploinsufficiency.

In some embodiments, the medicament treats the disease by increasing the expression of PiT2 protein or functional RNA in the subject's cells. Therefore, in some embodiments, the diseases in the present invention can be any disease caused by or involving PiT2 functional deficiency, provided that the treatment of the disease would benefit from modulation (e.g., increase) of functional PiT2 protein or RNA expression.

In some embodiments, the disease related to PiT2 functional deficiency is cerebral calcification. In some embodiments, the cerebral calcification is Primary Familial Brain calcification (PFBC).

In some embodiments, the disease related to PiT2 functional deficiency is a disease related to Pi balance abnormalities. In some embodiments, the disease related to Pi balance abnormalities comprises chronic kidney disease, cardiac valve calcification, arterial vascular calcification, and/or skeletal diseases related to phosphate homeostasis imbalance.

In some embodiments, the agent described in any of the above embodiments can be formulated into dosage forms compatible with its intended route of administration, for example, via local administration (such as direct injection or implantation), systemic administration, or subcutaneous, intravenous, intraperitoneal, or parenteral routes, comprising intracranial (e.g., intracerebroventricular, meningeal, or intrathecal), intramuscular, transdermal, airway (aerosol), nasal, oral, rectal, or topical (comprising buccal and sublingual) administration.

In some embodiments, the agent is administered by inhalation, intranasal, intratracheal, or oropharyngeal inhalation. Formulations suitable for inhalational administration can be prepared by incorporating the required dose of active ingredient into an appropriate solvent, followed by sterile filtration. Typically, formulations for inhalation administration are sterile solutions at physiological pH and have low viscosity. Salts may be added to the formulation to balance tonicity. In some cases, surfactants or co-solvents may be added to increase the solubility of the active ingredient and improve aerosol properties. In some cases, excipients may be added to control viscosity to ensure the size and distribution of aerosolized droplets.

In some embodiments, the agent can be administered by injection, such as intravenous, intramuscular, subcutaneous, intradermal, intra-articular, intraocular, intraperitoneal, intracerebroventricular (e.g., administered via lumbar puncture), or topical administration. Pharmaceutical compositions suitable for injectable use include sterile aqueous solutions or dispersions and sterile powders for the extemporaneous preparation of sterile injectable solutions or dispersions. For intravenous administration, suitable carriers include physiological saline, bacteriostatic water, phosphate-buffered saline (PBS), etc. The pharmaceutical composition should be stable under the conditions of manufacture and storage and should be protected against the contaminating action of microorganisms such as bacteria and fungi. For example, a sterile injectable solution can be prepared by incorporating the required dose of the active ingredient in an appropriate solvent, and optionally, simultaneously incorporating other desired ingredients (comprising but not limited to, pH adjusters, surfactants, adjuvants, ionic strength enhancers, diluents, osmotic pressure-maintaining agents, absorption-delaying agents, preservatives, or any combination thereof), followed by sterile filtration. Furthermore, sterile injectable solutions can be prepared as sterile lyophilized powders (e.g., by vacuum drying or freeze-drying) for convenient storage and use.

In some embodiments, the agent is selected from ASOs, and the pharmaceutical composition is preferably configured as a solution, emulsion, microemulsion, foam, or a formulation containing liposomes (e.g., cationic liposomes or non-cationic liposomes). In some embodiments, the pharmaceutical composition is preferably configured for administration via intracerebroventricular injection (e.g., via lumbar puncture), intraperitoneal injection, intramuscular injection, intrathecal injection, subcutaneous injection, oral administration, intra-articular injection, intravitreal administration, subretinal injection, topical application, implantation, or intravenous injection.

In another aspect, the present invention provides a method for regulating PiT2 protein expression in a cell, comprising contacting the cell *in vitro* with an agent capable of regulating alternative splicing in introns of the *SLC20A2* gene as described above.

In some embodiments, the cell comprises a mutation in the *SLC20A2* gene. In some embodiments, the mutation is selected from a non-synonymous mutation, nonsense mutation, frameshift mutation, splicing mutation, or structural variation. In some embodiments, the cell is heterozygous for the mutation. In some embodiments, the cell is homozygous for the mutation, wherein the mutation is located within the target region.

### II-B, Agents Targeting Defined SLC20A2 Regions and Their Applications

In some aspects, the present invention relates to agents, methods, and applications for targeting defined regions in *SLC20A2* to restore or enhance the function of the *SLC20A2* gene product.

One aspect of the present invention provides an agent capable of specifically binding to a target region in the *SLC20A2* gene or its pre-mRNA, and the target region is located within an intron region between two canonical exon regions, and wherein the intron region contains a cryptic exon.

In some embodiments, the cryptic exon is located within intron 2.

In some embodiments, the cryptic exon is located at c.289+901 to c.289+1035 of the *SLC20A2* gene or its pre-mRNA.

In some embodiments, the c.289+1007 C>G mutation in the *SLC20A2* gene promotes cryptic exon inclusion in the intron 2.

In some embodiments, the cryptic exon has the sequence as shown in SEQ ID NO: 2.

In some embodiments, the target region is located within the region c.289+801 to c.289+1135 of the *SLC20A2* gene or its pre-mRNA.

In some embodiments, the target region is located within the sequence as shown in SEQ ID NO: 8.

In some embodiments, the cryptic exon is located within intron 10.

In some embodiments, the cryptic exon is located at c.1795-1703 to c.1795-1627 of the *SLC20A2* gene or its pre-mRNA.

In some embodiments, the c.1795-1698 A>G mutation in the *SLC20A2* gene promotes cryptic exon inclusion in the intron 10.

In some embodiments, the cryptic exon has the sequence as shown in SEQ ID NO: 3.

In some embodiments, the target region is located within the region c.1795-1803 to c.1795-1527 of the *SLC20A2* gene or its pre-mRNA.

In some embodiments, the target region is located within the sequence as shown in SEQ ID NO: 9.

In some embodiments, the target region is within the region from up to about 100 nucleotides upstream to up to about 100 nucleotides downstream of the cryptic exon.

In some embodiments, the target region is selected from a region spanning a cryptic exon splice site, a region within the cryptic exon, a region within 100 nucleotides upstream of the cryptic exon, or a region within 100 nucleotides downstream of the cryptic exon.

In some embodiments, the target region is selected from a region upstream of the cryptic exon 5' splice acceptor site (SA), a region spanning the 5' splice acceptor site, a region within the cryptic exon, a region spanning the 3' splice donor site, or a region downstream of the 3' splice donor site (SD).

In some embodiments, the target region comprises a region spanning the cryptic exon's 5' splice acceptor site (SA). In some embodiments, the target region is within the region from up to about 25 (e.g., up to about 22, up to about 20, up to about 18, up to about 15, up to about 12, up to about 10, up to about 9, up to about 8, up to about 7, up to about 6, up to about 5, up to about 4, up to about 3, up to about 2, up to about 1) nucleotides upstream of the cryptic exon's 5' splice acceptor site to up to about 25 (e.g., up to about 22, up to about 20, up to about 18, up to about 15, up to about 12, up to about 10, up to about 9, up to about 8, up to about 7, up to about 6, up to about 5, up to about 4, up to about 3, up to about 2, up to about 1) nucleotides downstream of the cryptic exon's 5' splice acceptor site. In some embodiments, the target region is within the region from up to about 20 nucleotides upstream to up to about 20 nucleotides downstream of the cryptic exon's 5' splice acceptor site.

In some embodiments, the target region comprises a region spanning the cryptic exon's 3' splice donor site (SD). Preferably, the target region is within the region from up to about 25 (e.g., up to about 22, up to about 20, up to about 18, up to about 15, up to about 12, up to about 10, up to about 9, up to about 8, up to about 7, up to about 6, up to about 5, up to about 4, up to about 3, up to about 2, up to about 1) nucleotides upstream of the cryptic exon's 3' splice donor site to up to about 25 (e.g., up to about 22, up to about 20, up to about 18, up to about 15, up to about 12, up to about 10, up to about 9, up to about 8, up to about 7, up to about 6, up to about 5, up to about 4, up to about 3, up to about 2, up to about 1) nucleotides downstream of the cryptic exon's 3' splice donor site. In some embodiments, the target region is within the region from up to about 20 nucleotides upstream to up to about 20 nucleotides downstream of the cryptic exon's 3' splice donor site.

In some embodiments, the target region is located within the cryptic exon. Preferably, the target region or its vicinity contains a splicing regulatory element, such as a splicing enhancer element or a splicing silencer element.

In some embodiments, the target region is located within the intron region upstream or downstream of the cryptic exon. Preferably, the target region or its vicinity contains a splicing regulatory element, such as a branch site, a splicing enhancer element, or a splicing silencer element.

In some embodiments, the target region is no longer than 100 nucleotides, for example, no longer than 90, 80, 70, 60, 50, 40, 30, or 25 nucleotides.

In some embodiments, the agent is selected from antisense oligonucleotides (ASOs) or gene editing tools (e.g., CRISPR/Cas, base editors, or non-Cas protein-dependent RNA editing tools).

In some embodiments, the agent is antisense oligonucleotides (ASOs). In some embodiments, the agent is a gene editing tool, such as a DNA or RNA editing system, e.g., CRISPR/Cas, base editors, or non-Cas protein-dependent RNA editing tools. In some embodiments, the agent is a DNA editor, such as CRISPR/Cas9. In some embodiments, the agent is an RNA editor, such as CRISPR/Cas13. In some embodiments, the agent is a base editor, such as Adenine/Cytosine base editor. In some embodiments, the agent is a non-Cas protein-dependent RNA editing tool.

### ASO

In some embodiments, the agent is an antisense oligonucleotide (ASO) that specifically binds to the target region in the *SLC20A2* pre-mRNA, thereby achieving splicing regulation and expression modulation of the SLC20A2 gene.

In some embodiments, the ASO consists of 10~30 (e.g., 15~30, 15~25, 18~30, 18~25, 20~25) consecutive nucleotides; preferably consists of 18~25 consecutive nucleotides.

In some embodiments, the ASO comprises modifications of backbone nucleotides and nucleotide linkages (e.g., phosphodiester bonds), modifications of the ribose, modifications of the nucleobase, or combinations thereof. In some embodiments, the backbone modification is selected from phosphorothioate linkages or phosphorodiamidate linkages. In some embodiments, the sugar moiety modifications include but are not limited to: 2'-O-methoxyethyl (2'-MOE), morpholino, 2'-O-methyl (2'-OMe), 2'-fluoro (2'-F), constrained ethyl (cEt), locked nucleic acid (LNA), peptide nucleic acid (PNA).

In some embodiments, each monomer of the ASO is modified in the same manner, for example, each linkage of the ASO's backbone contains a phosphorothioate linkage or each ribose moiety contains a 2'O-methyl modification. Such modifications present on each of the monomeric components of the ASO are referred to as "uniform modifications". In some embodiments, a combination of different modifications may be desired, for example, the ASO may comprise a combination of phosphorodiamidate linkages with sugar moieties containing a morpholine ring (morpholino). A combination of different modifications for an ASO is referred to as "mixed modifications" or "mixed chemistries".

In some embodiments, the ASO comprises 2'MOE modifications. In some embodiments, every nucleotide of the ASO has a 2'MOE modification. In some embodiments, the modifications of the ASO comprise a combination of 2'MOE and locked nucleic acid (LNA). In some embodiments, the ASO has a backbone with phosphorothioate linkages.

In some embodiments, the ASO comprises 2'-OMe modifications. In some embodiments, every nucleotide of the ASO has a 2'-OMe modification. In some embodiments, the modifications of the ASO comprise a combination of 2'-OMe and locked nucleic acid (LNA). In some embodiments, the ASO has a backbone with phosphorothioate linkages.

In some embodiments, the ASO comprises a combination of phosphorodiamidate linkages with sugar moieties containing a morpholine ring (morpholino), for example, it is a phosphorodiamidate morpholino oligomer (PMO).

In some embodiments, the ASO specifically binds to a target region located within the region c.289+801 to c.289+1135 of *SLC20A2* pre-mRNA.

In some embodiments, the ASO has at least 80%, at least 85%, at least 90%, at least 95%, at least 96%, at least 97%, at least 98%, or at least 99% sequence complementarity to a nucleotide sequence within SEQ ID NO: 8. In some embodiments, the ASO is 100% complementary to a nucleotide sequence within SEQ ID NO: 8.

In some embodiments, the ASO comprises a nucleotide sequence shown in any one of SEQ ID NOs: 13-46, 63-65, or a sequence having at least 80% (e.g., at least 85%, at least 90%, at least 95%, at least 99%, or 100%) identity thereto. In some embodiments, the ASO comprises a nucleotide sequence shown in any one of SEQ ID NOs: 19-22, 25, 26, or a sequence having at least 80% (e.g., at least 85%, at least 90%, at least 95%, at least 99%, or 100%) identity thereto.

In some embodiments, the ASO comprises a nucleotide sequence shown in any one of SEQ ID NOs: 13-46, or a sequence having at least 80% (e.g., at least 85%, at least 90%, at least 95%, at least 99%, or 100%) identity thereto, and comprises 2'MOE modifications and/or phosphorothioate linkages. In some embodiments, every nucleotide of the ASO has a 2'MOE modification, and the ASO has a backbone with phosphorothioate linkages.

In other embodiments, the ASO comprises a nucleotide sequence shown in any one of SEQ ID NOs: 63-65, or a sequence having at least 80% (e.g., at least 85%, at least 90%, at least 95%, at least 99%, or 100%) identity thereto, and the ASO is a PMO.

In some embodiments, the ASO specifically binds to a target region located within the region c.1795-1803 to c.1795-1527 of *SLC20A2* pre-mRNA.

In some embodiments, the ASO has at least 80%, at least 85%, at least 90%, at least 95%, at least 96%, at least 97%, at least 98%, or at least 99% sequence complementarity to a nucleotide sequence within SEQ ID NO: 9. In some embodiments, the ASO is 100% complementary to a nucleotide sequence within SEQ ID NO: 9.

In some embodiments, the ASO comprises a nucleotide sequence shown in any one of SEQ ID NOs: 47-61, 66-68, or a sequence having at least 80% (e.g., at least 85%, at least 90%, at least 95%, at least 99%, or 100%) identity thereto. In some embodiments, the ASO comprises the nucleotide sequence as shown in SEQ ID NO: 50, or a sequence having at least 80% (e.g., at least 85%, at least 90%, at least 95%, at least 99%, or 100%) identity thereto.

In some embodiments, the ASO comprises a nucleotide sequence shown in any one of SEQ ID NOs: 47-61, or a sequence having at least 80% (e.g., at least 85%, at least 90%, at least 95%, at least 99%, or 100%) identity thereto, and comprises 2'MOE modifications and/or phosphorothioate linkages. In some embodiments, every nucleotide of the ASO has a 2'MOE modification, and the ASO has a backbone with phosphorothioate linkages.

In other embodiments, the ASO comprises a nucleotide sequence shown in any one of SEQ ID NOs: 66-68, or a sequence having at least 80% (e.g., at least 85%, at least 90%, at least 95%, at least 99%, or 100%) identity thereto, and the ASO is a PMO.

### Gene Editing Tools

In some embodiments, the agent is a gene editing tool, such as a DNA gene editing system or an RNA base editing tool, which typically comprises (i) a module with base editing activity and (ii) a module capable of binding to the target region in the *SLC20A2* gene. The gene editing tool inhibits the integration of the cryptic exon into mRNA by mutating the splice acceptor (SA) or splice donor (SD) sequences of the cryptic exon in the genomic or pre-mRNA sequence, thereby disrupting their SA or SD function.

Exemplary gene editing tools include, but are not limited to, CRISPR/Cas systems, DNA base editors, and RNA editing tools.

### CRISPR/Cas System

In some embodiments, the agent is a CRISPR/Cas system. The CRISPR/Cas system typically comprises a Cas protein and a guide RNA (gRNA), the gRNA capable of binding to the target region in the *SLC20A2* gene or its pre-mRNA.

In some embodiments, the gRNA binds to the target region and thereby recruits the binding of the Cas enzyme to exert editing activity.

In some embodiments, the CRISPR system disrupts the function of the cryptic exon's splice acceptor (SA) or splice donor (SD) by mutating their sequences, thereby inhibiting the integration of the cryptic exon into the mRNA.

In some embodiments, the target region is located within the region c.289+801 to c.289+1135 of *SLC20A2* pre-mRNA.

In some embodiments, the target region is located within the region c.1795-1803 to c.1795-1527 of *SLC20A2* pre-mRNA.

### DNA Base Editors

In some embodiments, the agent is a DNA base editor. A DNA base editor typically comprises (i) a fusion protein comprising a DNA base editing domain (e.g., a deaminase) and a DNA-binding domain (e.g., a Cas protein, such as Cas9 or Cas12), and (ii) a guide RNA (gRNA) capable of binding to the target region in the *SLC20A2* gene.

In some embodiments, the gRNA binds to the target region and thereby recruits the binding of the fusion protein to exert base editing activity.

In some embodiments, the DNA base editor disrupts the function of the cryptic exon's splice acceptor (SA) or splice donor (SD) by causing base mutations in their sequences, thereby inhibiting the integration of the cryptic exon into the mRNA.

In some embodiments, the Cas protein is a catalytically inactive Cas protein or a Cas nickase protein.

In some embodiments, the base editor comprises: an ABE (Adenine Base Editor) or a CBE (Cytosine Base Editor), or novel editors obtained through molecular evolution/mutation screening.

### RNA Editing Tools

In some embodiments, the agent is an RNA editing tool. RNA editing tools include, for example: CRISPR/Cas-based RNA base editors, oligonucleotide-based base editors, etc.

In some embodiments, the CRISPR/Cas-based RNA base editor comprises (i) a fusion protein comprising an RNA base editing domain (e.g., an RNA deaminase) and an RNA-binding domain (e.g., Cas13), and (ii) a guide RNA (gRNA) capable of binding to the target region in the *SLC20A2* pre-mRNA. The gRNA binds to the target region and thereby recruits the binding of the fusion protein to exert base editing activity.

In some embodiments, the oligonucleotide-based base editor comprises (i) a sequence module capable of recruiting an endogenous RNA base editing protein (e.g., an RNA deaminase, such as ADAR) and (ii) a guide sequence module capable of binding to the target region in the *SLC20A2* pre-mRNA. The guide sequence module binds to the target region and thereby recruits the binding of the RNA base editing protein to exert base editing activity.

In some embodiments, the RNA editing tool disrupts the function of the cryptic exon's splice acceptor (SA) or splice donor (SD) by causing base mutations in their sequences, thereby inhibiting the integration of the cryptic exon into the mRNA.

In another aspect, the present invention provides a pharmaceutical composition comprising the agent as described above or a combination thereof, and a pharmaceutically acceptable carrier and/or excipient.

In some embodiments, the pharmaceutical composition is formulated into dosage forms compatible with its intended route of administration, for example, via local administration (such as direct injection or implantation), systemic administration, or subcutaneous, intravenous, intraperitoneal, or parenteral routes, comprising intracranial (e.g., intracerebroventricular, meningeal, or intrathecal), intramuscular, transdermal, airway (aerosol), nasal, oral, rectal, or topical (comprising buccal and sublingual) administration.

In some embodiments, the pharmaceutical composition is administered by inhalation, intranasal, intratracheal, or oropharyngeal inhalation. Formulations suitable for inhalational administration can be prepared by incorporating the required dose of active ingredient into an appropriate solvent, followed by sterile filtration. Typically, formulations for inhalation administration are sterile solutions at physiological pH and have low viscosity. Salts may be added to the formulation to balance tonicity. In some cases, surfactants or co-solvents may be added to increase the solubility of the active ingredient and improve aerosol properties. In some cases, excipients may be added to control viscosity to ensure the size and distribution of aerosolized droplets.

In some embodiments, the pharmaceutical composition can be administered by injection, such as intravenous, intramuscular, subcutaneous, intradermal, intra-articular, intraocular, intraperitoneal, intracerebroventricular (e.g., administered via lumbar puncture), or topical administration. Pharmaceutical compositions suitable for injectable use include sterile aqueous solutions or dispersions and sterile powders for the extemporaneous preparation of sterile injectable solutions or dispersions. For intravenous administration, suitable carriers include physiological saline, bacteriostatic water, phosphate-buffered saline (PBS), etc. The pharmaceutical composition should be stable under the conditions of manufacture and storage and should be protected against the contaminating action of microorganisms such as bacteria and fungi. For example, a sterile injectable solution can be prepared by incorporating the required dose of the active ingredient in an appropriate solvent, and optionally, simultaneously incorporating other desired ingredients (comprising but not limited to, pH adjusters, surfactants, adjuvants, ionic strength enhancers, diluents, osmotic pressure-maintaining agents, absorption-delaying agents, preservatives, or any combination thereof), followed by sterile filtration. Furthermore, sterile injectable solutions can be prepared as sterile lyophilized powders (e.g., by vacuum drying or freeze-drying) for convenient storage and use.

In some embodiments, the agent is selected from ASOs, and the pharmaceutical composition is preferably configured as a solution, emulsion, microemulsion, foam, or a formulation containing liposomes (e.g., cationic liposomes or non-cationic liposomes). In some embodiments, the pharmaceutical composition is preferably configured for administration via intracerebroventricular injection (e.g., via lumbar puncture), intraperitoneal injection, intramuscular injection, intrathecal injection, subcutaneous injection, oral administration, intra-articular injection, intravitreal administration, subretinal injection, topical application, implantation, or intravenous injection.

In another aspect, the present invention provides the use of the agent or pharmaceutical composition as described above for preventing and/or treating a disease related to PiT2 functional deficiency (e.g., deficiency in amount or activity) in a subject, or for use in the manufacture of a medicament for preventing and/or treating a disease related to PiT2 functional deficiency (e.g., deficiency in amount or activity) in a subject. The present invention also provides a method for preventing and/or treating a disease related to PiT2 functional deficiency in a subject, the method comprising administering an effective dose of the agent or pharmaceutical composition as described above to a subject in need thereof.

In some embodiments, the disease associated with PiT2 functional deficiency is cerebral calcification, such as Primary Familial Brain calcification (PFBC). In some embodiments, the disease associated with PiT2 functional deficiency is a disease related to Pi imbalance abnormality, such as chronic kidney disease, cardiac valve calcification, arterial vascular calcification, and/or bone diseases related to phosphate homeostasis imbalance. In some embodiments, the disease is treated by the drug through regulating (e.g., increasing) the expression of PiT2 protein or functional RNA in the subject's cells.

In some embodiments, the subject comprises a mutation in the *SLC20A2* gene. In some embodiments, the mutation is selected from a non-synonymous mutation, a nonsense mutation, a frameshift mutation, a splicing mutation, or a structural variation.

In some embodiments, the mutation is located in an exon region, and the subject is heterozygous for the mutation in the exon region.

In some embodiments, the mutation is an intron mutation, for example, a mutation within intron 2 and/or intron 10. In some embodiments, the mutation is located in the target region within an intron, and the subject can be heterozygous, homozygous, or compound heterozygous for the intron mutation located within the target region.

In some embodiments, the PiT2 functional deficiency is *SLC20A2* haploinsufficiency.

In some embodiments, the subject is a mammal. In some embodiments, the subject is a human.

In another aspect, the present invention also provides a method for regulating PiT2 protein expression in a cell, comprising contacting the cell with an agent or pharmaceutical composition as described above *in vitro.*

In some embodiments, the cell comprises a mutation in the *SLC20A2* gene. In some embodiments, the mutation is selected from a non-synonymous mutation, a nonsense mutation, a frameshift mutation, a splicing mutation, or a structural variation. In some embodiments, the mutation is located in an exon region, and the cell is heterozygous for the mutation in the exon region. In some embodiments, the mutation is an intron mutation, for example, a mutation within intron 2 and/or intron 10. In some embodiments, the mutation is located in the target region within an intron, and the cell can be heterozygous, homozygous, or compound heterozygous for the intron mutation located within the target region.

### II-C. Screening Methods

In some aspects, the present invention relates to screening methods for agents that regulate PiT2 expression.

One aspect of the present invention provides a method for identifying an agent capable of regulating PiT2 expression in a cell, the method comprising: i) identifying an agent targeting a target region in a PiT2 gene or its precursor mRNA, the target region located in an intron region between two canonical exon regions, and wherein the intron region contains a cryptic exon, the target region being within a region from up to about 100 nucleotides upstream of the cryptic exon to up to about 100 nucleotides downstream of the cryptic exon; ii) delivering the agent identified in step i) to the cell; and iii) measuring the PiT2 expression level in the cell of step ii).

In some embodiments, the method is performed *in vitro.*

In some embodiments, the method further comprises: iv) comparing the expression level measured in step iii) with the PiT2 expression level in a cell treated with a control method. An increase compared to the control group indicates that the agent is an activator. A decrease compared to the control group indicates that the agent is an inhibitor.

In some embodiments, the PiT2 expression level refers to the expression level of functional PiT2, for example, the mRNA level of correctly spliced products or the level of the protein encoded thereby.

In some embodiments, the expression level is measured by RT-PCR, qPCR, or by Western blot.

In some embodiments, the target region is selected from a region upstream of the 5' splice site (splicing acceptor site, SA) of the cryptic exon, a region spanning the 5' splice site, a region within the cryptic exon, a region spanning the 3' splice site, or a region downstream of the 3' splice site (splicing donor site, SD).

In some embodiments, the cryptic exon is located at c.289+901 to c.289+1035 of the *SLC20A2* gene. In some embodiments, the cryptic exon has a sequence as shown in SEQ ID NO: 2. In some embodiments, the target region is located within the region from c.289+801 to c.289+1135 of the *SLC20A2* gene or its pre-mRNA. In some embodiments, the target region is located within the sequence as shown in SEQ ID NO: 8.

In some embodiments, the cryptic exon is located at c.1795-1703 to c.1795-1627 of the *SLC20A2* gene. In some embodiments, the cryptic exon has a sequence as shown in SEQ ID NO: 3. In some embodiments, the target region is located within the region from c.1795-1803 to c.1795-1527 of the *SLC20A2* gene or its pre-mRNA. In some embodiments, the target region is located within the sequence as shown in SEQ ID NO: 9.

In some embodiments, the agent targets the target region in the *SLC20A2* precursor mRNA. In some embodiments, the agent is an antisense oligonucleotide (ASO). In some embodiments, the agent is an RNA editor, such as CRISPR/Cas13. In some embodiments, the agent is a non-Cas protein-dependent RNA editing tool.

In some embodiments, the agent targets the target region in the *SLC20A2* gene. In some embodiments, the agent is a DNA editor, such as CRISPR/Cas9. In some embodiments, the agent is a base editor, such as an Adenine/Cytosine base editor.

In some aspects, the present invention relates to screening methods for agents that modulate *SLC20A2* mRNA splicing.

One aspect of the present invention provides a nucleic acid construct comprising a minigene fragment sequence of the human *SLC20A2* gene, the minigene fragment sequence comprising exon 2 and exon 3 and the intron sequence between them, or comprising exon 10 and exon 11 and the intron sequence between them, wherein the intron sequence comprises a mutation that alters *SLC20A2* gene splicing.

In some embodiments, the nucleic acid construct comprises a promoter operably linked to the minigene fragment sequence.

In some embodiments, the mutation that alters *SLC20A2* gene splicing causes or enhances alternative splicing in intron 2. In some embodiments, the alternative splicing in intron 2 is the inclusion of a cryptic exon between exon 2 and 3. In some embodiments, the cryptic exon is located at c.289+901 to c.289+1035 of the *SLC20A2* gene.

In some embodiments, the cryptic exon has a sequence as shown in SEQ ID NO: 2.

In some embodiments, the mutation comprises the c.289+1007 C>G mutation of the *SLC20A2* gene.

In some embodiments, the minigene fragment sequence comprising exon 2 and exon 3 and the intron sequence between them comprises the sequence as shown in SEQ ID NO: 4.

In some embodiments, the alternative splicing comprises alternative splicing in intron 10.

In some embodiments, the alternative splicing in intron 10 is the inclusion of a cryptic exon between exon 10 and 11. In some embodiments, the cryptic exon is located at c.1795-1703 to c.1795-1627 of the *SLC20A2* gene.

In some embodiments, the cryptic exon has a sequence as shown in SEQ ID NO: 3.

In some embodiments, the mutation comprises the c.1795-1698 A>G mutation of the *SLC20A2* gene.

In some embodiments, the minigene fragment sequence comprising exon 10 and exon 11 and the intron sequence between them comprises the sequence as shown in SEQ ID NO: 5.

In some embodiments, the minigene fragment sequence comprises a polyA signal sequence downstream.

In some embodiments, the nucleic acid construct further comprises a reporter gene, such as a fluorescent protein (e.g., GFP, mCherry) or luciferase.

In another aspect, the present invention provides an expression vector comprising a nucleic acid construct as described above. Herein, the term "vector" refers to a nucleic acid vehicle into which a polynucleotide can be inserted. When a vector enables expression of a protein encoded by the inserted polynucleotide, the vector is called an expression vector. A vector can be introduced into a host cell via transformation, transduction, or transfection, causing the genetic material elements it carries to be expressed in the host cell. Vectors are well known to those skilled in the art and include, but are not limited to: plasmids; phagemids; cosmids; artificial chromosomes, such as Yeast Artificial Chromosomes (YAC), Bacterial Artificial Chromosomes (BAC), or P1-derived Artificial Chromosomes (PAC); bacteriophages such as λ phage or M13 phage; and animal viruses. Animal viruses that can be used as vectors include, but are not limited to, retroviruses (comprising lentiviruses), adenoviruses, adeno-associated viruses, herpesviruses (such as herpes simplex virus), poxviruses, baculoviruses, papillomaviruses, and papovaviruses (such as SV40). A vector can contain various elements controlling expression, comprising, but not limited to, promoter sequences, transcription initiation sequences, enhancer sequences, selection elements, and reporter genes. Additionally, a vector can also contain a replication origin.

In another aspect, the present invention provides a method for screening an agent capable of regulating *SLC20A2* mRNA splicing, comprising: providing (i) a cell comprising a nucleic acid construct as described above, (ii) a genetically modified non-human animal or cell of the present invention, or (iii) a cerebral calcification model obtained by the preparation method for a cerebral calcification model of the present invention; contacting a test agent with any one of (i)-(iii); and detecting alternative splicing of *SLC20A2* mRNA. In some embodiments, the method is for non-diagnostic therapeutic purposes. In some embodiments, the method is performed in vitro.

In some embodiments, the cell is derived from, for example, a mammal, such as a rodent (e.g., mouse or rat), a non-human primate, or a human.

In some embodiments, a test agent capable of inhibiting alternative splicing (e.g., reducing the amount of expression products containing cryptic exon inclusion) is selected, thereby increasing the production of functional PiT2 protein.

In some embodiments, a test agent capable of enhancing alternative splicing (e.g., increasing the amount of expression products containing cryptic exon inclusion) is selected, thereby reducing the production of functional PiT2 protein.

In some embodiments, the detection comprises analyzing the length of *SLC20A2* mRNA expression products.

In some embodiments, the alternative splicing is alternative splicing in intron 2. In some embodiments, the alternative splicing in intron 2 is the inclusion of a cryptic exon between exon 2 and 3. In some embodiments, the cryptic exon is located at c.289+901 to c.289+1035 of the *SLC20A2* gene. In some embodiments, the cryptic exon has a sequence as shown in SEQ ID NO: 2.

In some embodiments, the detection comprises performing PCR analysis using primers spanning exon 2 and exon 3. In some embodiments, an amplicon containing an inclusion of a cryptic exon with a length of about 135 nt is produced. In some embodiments, the primers comprise the forward primer as shown in SEQ ID NO: 123 and the reverse primer as shown in SEQ ID NO: 124.

In some embodiments, the alternative splicing comprises alternative splicing in intron 10. In some embodiments, the alternative splicing in intron 10 is the inclusion of a cryptic exon between exon 10 and 11. In some embodiments, the cryptic exon is located at c.1795-1703 to c.1795-1627 of the *SLC20A2* gene. In some embodiments, the cryptic exon has a sequence as shown in SEQ ID NO: 3.

In some embodiments, the detection comprises performing PCR analysis using primers spanning exon 10 and exon 11. In some embodiments, an amplicon containing an inclusion of a cryptic exon with a length of about 76 nt is produced. In some embodiments, the primers comprise the forward primer as shown in SEQ ID NO: 127 and the reverse primer as shown in SEQ ID NO: 128.

In some embodiments, the method comprises contacting a test agent in vitro with a cell comprising the nucleic acid construct and detecting alternative splicing of the nucleic acid construct. In some embodiments, the method comprises introducing (e.g., transfecting) the nucleic acid construct as described above or a vector comprising the nucleic acid construct into a cell to provide a cell comprising the nucleic acid construct. In some embodiments, the detection comprises analyzing the length of mRNA expression products of the nucleic acid construct as described above. In some embodiments, the detection comprises analyzing the expression of a reporter gene comprised in the nucleic acid construct. In some embodiments, expression of the reporter gene indicates alternative splicing. In some embodiments, expression of the reporter gene indicates correct splicing.

### II-D. Cerebral calcification Model and Its Preparation

In some aspects, the present invention relates to constructing a cerebral calcification model based on alternative splicing in an intron of the *SLC20A2* gene.

One aspect of the present invention provides a method for preparing a cerebral calcification model, wherein the method comprises modifying a target genome so that the intron sequence between endogenous *Slc20a2* gene exon 2 and exon 3 is replaced by the corresponding intron genomic fragment of the human *SLC20A2* gene, or the intron sequence between endogenous *Slc20a2* gene exon 10 and exon 11 is replaced by the corresponding intron genomic fragment of the human *SLC20A2* gene, wherein the corresponding intron genomic fragment of the human *SLC20A2* gene comprises a mutation that alters *SLC20A2* gene splicing, thereby providing a cerebral calcification model. In some embodiments, the target genome is homozygous or heterozygous for the replacement.

In some embodiments, the corresponding intron genomic fragment of the human *SLC20A2* gene comprises at least the cryptic exon. In some embodiments, the corresponding intron genomic fragment of the human *SLC20A2* gene comprises at least the region from about 100 nucleotides upstream to about 100 nucleotides downstream of the cryptic exon.

In some embodiments, the mutation that alters *SLC20A2* gene splicing causes or enhances alternative splicing in intron 2. In some embodiments, the alternative splicing in intron 2 is the inclusion of a cryptic exon between exon 2 and 3. In some embodiments, the cryptic exon is located at c.289+901 to c.289+1035 of the *SLC20A2* gene.

In some embodiments, the cryptic exon has a sequence as shown in SEQ ID NO: 2.

In some embodiments, the mutation comprises the c.289+1007 C>G mutation of the human *SLC20A2* gene.

In some embodiments, the replaced intron genomic fragment of the human *SLC20A2* gene between exon 2 and exon 3 comprises the sequence as shown in SEQ ID NO: 10.

In some embodiments, the alternative splicing comprises alternative splicing in intron 10.

In some embodiments, the alternative splicing in intron 10 is the inclusion of a cryptic exon between exon 10 and 11. In some embodiments, the cryptic exon is located at c.1795-1703 to c.1795-1627 of the human *SLC20A2* gene.

In some embodiments, the cryptic exon has a sequence as shown in SEQ ID NO: 3.

In some embodiments, the mutation comprises the c.1795-1698 A>G mutation of the human *SLC20A2* gene.

In some embodiments, the replaced intron genomic fragment of the human *SLC20A2* gene between exon 10 and exon 11 comprises the sequence as shown in SEQ ID NO: 11.

In some embodiments, the donor sequence for replacement has the following structure: Exon2(3'-partial)-intron-crpE2(or crpE10)-intron-exon3(5'-partial).

In some embodiments, the donor sequence for replacement has the following structure: Exon10(3'-partial)-intron-crpE2(or crpE10)-intron-exon11(5'-partial).

In some embodiments, the expression of the modified endogenous *Slc20a2* gene is under the control of regulatory elements at the endogenous *Slc20a2* gene locus in the target genome.

Methods for replacing the intron sequence of the endogenous *Slc20a2* gene with the corresponding intron sequence of the human *SLC20A2* gene can be performed using various suitable genome editing techniques known in the art. In some embodiments, the replacement method comprises: gene knockout, gene editing, RNA interference, epigenetic suppression, or any combination thereof.

In some embodiments, the gene knockout comprises: stem cell targeting technology based on homologous recombination, gene editing technology, chemical molecular-induced genomic mutagenesis, transposon site-specific inactivation, or any combination thereof.

In some embodiments, the gene editing comprises: CRISPR/Cas systems (e.g., Cas9, Cas12a (Cpf1)), TALEN, zinc finger nuclease, Base editing, or any combination thereof.

In some embodiments, the replacement method comprises introducing a targeting vector carrying an inclusion nucleic acid (e.g., the sequence of the corresponding intron genomic fragment of the human *SLC20A2* gene to be introduced) flanked by 5' and 3' homology arms into a cell. In some embodiments, the inclusion nucleic acid further contains a selectable marker gene (e.g., a self-deleting cassette containing a selectable marker gene), the gene can be flanked by or contain site-specific recombination sites (e.g., loxP, Frt, etc.). In some embodiments, the inclusion nucleic acid further contains a reporter gene.

In some embodiments, the model is a non-human animal model, and the target genome is the genome of a non-human animal.

In some embodiments, the model is a mammalian model. In some embodiments, the animal model is a rodent model, such as a mouse or rat model. In some embodiments, the animal model is a non-human primate model.

In some embodiments, the method comprises: modifying the genome of a non-human animal embryonic stem (ES) cell such that the modified genome contains the replacement in the endogenous *Slc20a2* gene; using the non-human animal ES cell containing the modified genome to produce a non-human animal, thereby obtaining a humanized cerebral calcification animal model. In some embodiments, the targeting vector can be introduced into the non-human animal ES cells by, for example, electroporation.

In some embodiments, the model is a cell model, and the target genome is the endogenous genome of a target cell. In some embodiments, the replaced corresponding intron genomic fragment of the human *SLC20A2* gene (with or without mutation) is exogenously introduced relative to the target cell.

In some embodiments, the cell is a mammalian cell. In some embodiments, the cell is a human dermal fibroblast or brain glial cell.

In some embodiments, the method comprises: modifying the genome of a target cell such that the modified genome contains the replacement in the endogenous *Slc20a2* gene; thereby obtaining a cell model comprising the modified genome.

In another aspect, the present invention provides a genetically modified non-human animal whose genome comprises: the intron sequence between endogenous *Slc20a2* gene exon 2 and exon 3 being replaced by the corresponding intron genomic fragment of the human *SLC20A2* gene, or the intron sequence between endogenous *Slc20a2* gene exon 10 and exon 11 being replaced by the corresponding intron genomic fragment of the human *SLC20A2* gene, wherein the corresponding intron genomic fragment of the human *SLC20A2* gene comprises a mutation that alters *SLC20A2* gene splicing. In some embodiments, the non-human animal is homozygous or heterozygous for the replacement.

In some embodiments, the corresponding intron genomic fragment of the human *SLC20A2* gene comprises at least the cryptic exon. In some embodiments, the corresponding intron genomic fragment of the human *SLC20A2* gene comprises at least the region from about 100 nucleotides upstream to about 100 nucleotides downstream of the cryptic exon.

In some embodiments, the mutation that alters *SLC20A2* gene splicing causes or enhances alternative splicing in intron 2. In some embodiments, the alternative splicing in intron 2 is the inclusion of a cryptic exon between exon 2 and 3. In some embodiments, the cryptic exon is located at c.289+901 to c.289+1035 of the human *SLC20A2* gene.

In some embodiments, the cryptic exon has a sequence as shown in SEQ ID NO: 2.

In some embodiments, the mutation comprises the c.289+1007 C>G mutation of the *SLC20A2* gene.

In some embodiments, the replaced intron genomic fragment of the human *SLC20A2* gene between exon 2 and exon 3 comprises the sequence as shown in SEQ ID NO: 10.

In some embodiments, the alternative splicing comprises alternative splicing in intron 10.

In some embodiments, the alternative splicing in intron 10 is the inclusion of a cryptic exon between exon 10 and 11. In some embodiments, the cryptic exon is located at c.1795-1703 to c.1795-1627 of the *SLC20A2* gene.

In some embodiments, the cryptic exon has a sequence as shown in SEQ ID NO: 3.

In some embodiments, the mutation comprises the c.1795-1698 A>G mutation of the *SLC20A2* gene.

In some embodiments, the replaced intron genomic fragment of the human *SLC20A2* gene between exon 10 and exon 11 comprises the sequence as shown in SEQ ID NO: 11.

In some embodiments, the non-human animal is a rodent, such as a mouse or rat.

In some embodiments, the non-human animal is a non-human primate.

In some embodiments, the genetically modified non-human animal is an animal model for cerebral calcification.

In another aspect, the present invention provides a genetically modified cell whose genome comprises: the intron sequence between endogenous *SLC20A2* gene exon 2 and exon 3 being replaced by the corresponding intron genomic fragment of the human *SLC20A2* gene, or the intron sequence between endogenous *SLC20A2* gene exon 10 and exon 11 being replaced by the corresponding intron genomic fragment of the human *SLC20A2* gene, wherein the corresponding intron genomic fragment of the human *SLC20A2* gene comprises a mutation that alters *SLC20A2* gene splicing. In some embodiments, the cell is homozygous or heterozygous for the replacement.

In some embodiments, the corresponding intron genomic fragment of the human *SLC20A2* gene comprises at least the cryptic exon. In some embodiments, the corresponding intron genomic fragment of the human *SLC20A2* gene comprises at least the region from about 100 nucleotides upstream to about 100 nucleotides downstream of the cryptic exon.

In some embodiments, the corresponding intron genomic fragment of the human *SLC20A2* gene is exogenously introduced relative to the cell. "Exogenously introduced" refers to nucleic acids originating from outside the target cell. Exogenous nucleic acids can be artificially or recombinantly produced, or isolated from heterologous cells and introduced into the target cell.

In some embodiments, the mutation that alters *SLC20A2* gene splicing causes or enhances alternative splicing in intron 2. In some embodiments, the alternative splicing in intron 2 is the inclusion of a cryptic exon between exon 2 and 3. In some embodiments, the cryptic exon is located at c.289+901 to c.289+1035 of the *SLC20A2* gene.

In some embodiments, the cryptic exon has a sequence as shown in SEQ ID NO: 2.

In some embodiments, the mutation comprises the c.289+1007 C>G mutation of the *SLC20A2* gene.

In some embodiments, the replaced intron genomic fragment of the human *SLC20A2* gene between exon 2 and exon 3 comprises the sequence as shown in SEQ ID NO: 10.

In some embodiments, the alternative splicing comprises alternative splicing in intron 10.

In some embodiments, the alternative splicing in intron 10 is the inclusion of a cryptic exon between exon 10 and 11. In some embodiments, the cryptic exon is located at c.1795-1703 to c.1795-1627 of the *SLC20A2* gene.

In some embodiments, the cryptic exon has a sequence as shown in SEQ ID NO: 3.

In some embodiments, the mutation comprises the c.1795-1698 A>G mutation of the *SLC20A2* gene.

In some embodiments, the replaced intron genomic fragment of the human *SLC20A2* gene between exon 10 and exon 11 comprises the sequence as shown in SEQ ID NO: 11.

In some embodiments, the cell is a mammalian cell.

In some embodiments, the cell is a human dermal fibroblast or brain glial cell.

In some embodiments, the genetically modified cell is a cell model for cerebral calcification.

### II-E. Use of Cerebral calcification Model

In some aspects, the present invention also relates to use of the above-mentioned cerebral calcification model in disease research, drug screening, etc.

One aspect of the present invention provides the use of the cerebral calcification model of the present invention, or the genetically modified non-human animal or the genetically modified cell, for indicating and analyzing the onset stage of cerebral calcification disease, or for use as a model for studying brain phosphate homeostasis imbalance or cerebral calcification disease targets.

In another aspect, the present invention provides the use of the cerebral calcification model of the present invention, or the genetically modified non-human animal or the genetically modified cell, for use as a model for screening candidate drugs or treatment regimens for treating cerebral calcification disease or diseases related to brain phosphate homeostasis imbalance.

In some embodiments, the disease is a cerebral calcification disease.

In some embodiments, the drug is a drug for preventing or treating cerebral calcification.

In some embodiments, the drug is a drug for restoring brain phosphate homeostasis.

In another aspect, the present invention provides the use of the cerebral calcification model of the present invention, or the genetically modified non-human animal or the genetically modified cell, for use as a model for screening candidate drugs or treatment regimens for diseases related to phosphate homeostasis imbalance.

In some embodiments, the phosphate homeostasis imbalance-related diseases include, but are not limited to, chronic kidney disease, cardiac valve calcification, arterial vascular calcification, and/or bone diseases related to phosphate homeostasis imbalance.

In some embodiments, the drug is a drug that regulates *SLC20A2* mRNA splicing thereby regulating the expression level of its encoded product PiT2.

In some embodiments, the drug is a drug that regulates *SLC20A2* mRNA splicing thereby regulating the function of its encoded product PiT2.

In some embodiments, the drug inhibits *SLC20A2* mRNA alternative splicing thereby increasing the production of functional PiT2 protein.

In some embodiments, the drug promotes *SLC20A2* mRNA alternative splicing thereby inhibiting the production of functional PiT2 protein.

In another aspect, the present invention provides a method for screening candidate drugs or treatment regimens for treating cerebral calcification disease or diseases related to brain phosphate homeostasis imbalance, the method comprising: administering the candidate drug or treatment regimen to the cerebral calcification model of the present invention, or the genetically modified non-human animal or the genetically modified cell; evaluating whether the candidate drug or treatment regimen is capable of (i) increasing the expression level of *SLC20A2* full-length mRNA transcript and/or its encoded protein product PiT2, (ii) increasing the phosphate transport capacity of target cells (e.g., brain glial cells, such as astrocytes), and/or (iii) reducing the level of inorganic phosphate in cerebrospinal fluid.

In some embodiments, the disease is a cerebral calcification disease.

In some embodiments, the drug is a drug for preventing or treating cerebral calcification.

In some embodiments, the drug is a drug for restoring brain phosphate homeostasis.

In some embodiments, the candidate drug or treatment regimen is capable of increasing the expression level of *SLC20A2* full-length mRNA transcript and/or its encoded protein product PiT2, for example, upregulating by at least 30%, at least 50%, at least 100%, at least 150%, at least 200%, etc. In some embodiments, the candidate drug or treatment regimen is capable of increasing the expression level of correctly spliced *SLC20A2* mRNA transcript and/or its encoded functional PiT2 protein product, for example, upregulating by at least 30%, at least 50%, at least 100%, at least 150%, at least 200%, etc.

In some embodiments, the candidate drug or treatment regimen is capable of increasing the phosphate transport capacity of target cells (e.g., brain glial cells, such as astrocytes), for example, increasing by at least 30%, at least 50%, at least 100%, at least 150%, at least 200%, etc.

In some embodiments, the candidate drug or treatment regimen is capable of reducing the level of inorganic phosphate in cerebrospinal fluid, for example, downregulating abnormally elevated inorganic phosphate levels in cerebrospinal fluid by at least 75%, at least 60%, at least 50%, at least 40%, at least 30%, at least 25%, for example, down to levels close to normal reference values (e.g., healthy controls).

In another aspect, the present invention provides a method for screening candidate drugs or treatment regimens for treating diseases related to phosphate homeostasis imbalance, the method comprising: administering the candidate drug or treatment regimen to the cerebral calcification model of the present invention, or the genetically modified non-human animal or the genetically modified cell; evaluating whether the candidate drug or treatment regimen is capable of (i) increasing the expression level of *SLC20A2* full-length mRNA transcript and/or its encoded protein product PiT2, (ii) increasing the phosphate transport capacity of target cells (e.g., brain glial cells, such as astrocytes), and/or (iii) reducing the level of inorganic phosphate in cerebrospinal fluid.

In some embodiments, the phosphate homeostasis imbalance-related diseases include, but are not limited to, chronic kidney disease, cardiac valve calcification, arterial vascular calcification, and/or bone diseases related to phosphate homeostasis imbalance.

In some embodiments, the candidate drug or treatment regimen is capable of increasing the expression level of *SLC20A2* full-length mRNA transcript and/or its encoded protein product PiT2, for example, upregulating by at least 30%, at least 50%, at least 100%, at least 150%, at least 200%, etc. In some embodiments, the candidate drug or treatment regimen is capable of increasing the expression level of correctly spliced *SLC20A2* mRNA transcript and/or its encoded functional PiT2 protein product, for example, upregulating by at least 30%, at least 50%, at least 100%, at least 150%, at least 200%, etc.

In some embodiments, the candidate drug or treatment regimen is capable of increasing the phosphate transport capacity of target cells (e.g., brain glial cells, such as astrocytes), for example, increasing by at least 30%, at least 50%, at least 100%, at least 150%, at least 200%, etc.

In some embodiments, the candidate drug or treatment regimen is capable of reducing the level of inorganic phosphate in cerebrospinal fluid, for example, downregulating abnormally elevated inorganic phosphate levels in cerebrospinal fluid by at least 75%, at least 60%, at least 50%, at least 40%, at least 30%, at least 25%, for example, down to levels close to normal reference values (e.g., healthy controls).

In some embodiments, the drug is a drug that regulates *SLC20A2* mRNA splicing thereby regulating the expression level of its encoded product PiT2.

In some embodiments, the drug is a drug that regulates *SLC20A2* mRNA splicing thereby regulating the function of its encoded product PiT2.

In some embodiments, the drug inhibits *SLC20A2* mRNA alternative splicing thereby increasing the production of functional PiT2 protein.

In some embodiments, the drug promotes *SLC20A2* mRNA alternative splicing thereby inhibiting the production of functional PiT2 protein.

In some embodiments, the disease associated with PiT2 functional deficiency is cerebral calcification, such as Primary Familial Brain calcification (PFBC). In some embodiments, the disease associated with PiT2 functional deficiency is a disease related to Pi imbalance abnormality, such as chronic kidney disease, cardiac valve calcification, arterial vascular calcification, and/or bone diseases related to phosphate homeostasis imbalance. In some embodiments, the disease is treated by the drug through regulating (e.g., increasing) the expression of PiT2 protein or functional RNA in the subject's cells.

In some embodiments, the subject comprises a mutation in the *SLC20A2* gene. In some embodiments, the mutation is selected from a non-synonymous mutation, a nonsense mutation, a frameshift mutation, a splicing mutation, or a structural variation.

In some embodiments, the mutation is located in an exon region, and the subject is heterozygous for the mutation in the exon region.

In some embodiments, the mutation is an intron mutation, for example, a mutation within intron 2 and/or intron 10. In some embodiments, the mutation is located in the target region within an intron, and the subject can be heterozygous, homozygous, or compound heterozygous for the intron mutation located within the target region.

In some embodiments, the PiT2 functional deficiency is *SLC20A2* haploinsufficiency.

In some embodiments, the subject is a mammal. In some embodiments, the subject is a human.

In another aspect, the present invention also provides a method for regulating PiT2 protein expression in a cell, comprising contacting the cell with an agent or pharmaceutical composition as described above in vitro.

In some embodiments, the cell comprises a mutation in the *SLC20A2* gene. In some embodiments, the mutation is selected from a non-synonymous mutation, a nonsense mutation, a frameshift mutation, a splicing mutation, or a structural variation. In some embodiments, the mutation is located in an exon region, and the cell is heterozygous for the mutation in the exon region. In some embodiments, the mutation is an intron mutation, for example, a mutation within intron 2 and/or intron 10. In some embodiments, the mutation is located in the target region within an intron, and the cell can be heterozygous, homozygous, or compound heterozygous for the intron mutation located within the target region.

### II-F. Beneficial Effects and Summary

The present invention firstly discovered and identified previously unreported cryptic exon inclusions in *SLC20A2* mRNA abnormal splicing, and thereby invented agents and methods for regulating this abnormal splicing. In cerebral calcification patient cells and the *SLC20A2-KI* mouse model, regulating this abnormal splicing can upregulate the expression of the SLC20A2 gene product PiT2 protein, reverse the increase in CSF Pi levels, thereby inhibiting the progression of cerebral calcification.

In the examples of the present invention, the method for regulating *SLC20A2* abnormal splicing to upregulate the expression of the *SLC20A2* gene product PiT2 protein can be mainly achieved by antisense oligonucleotides (ASOs). However, based on the methodological logic and implementation pathways described in the present invention, those skilled in the art will understand that the present invention can also be achieved by DNA base editing to permanently alter the *SLC20A2* mRNA splicing pattern at the genomic level, or by RNA base editing to alter the *SLC20A2* mRNA splicing pattern at the RNA level.

Furthermore, because PiT2 is highly distributed in the brain, kidneys, cardiovascular system, and bones, and participates in Pi homeostasis, regulating PiT2 expression through splicing regulation can also be used to restore other syndromes related to Pi imbalance abnormalities, comprising chronic kidney disease, cardiovascular arteriosclerosis, and bone diseases.

Particularly, the method of the present invention for regulating *SLC20A2* mRNA abnormal splicing can still increase PiT2 protein expression levels in healthy cells, indicating that this method can rescue *SLC20A2* haploinsufficiency not limited to specific mutation forms, thereby rescuing PiT2 expression in patients with non-intron mutations causing *SLC20A2* loss-of-function mutations, who constitute a larger population proportion.

Based on this, the present invention provides a brand-new strategy based on regulating *SLC20A2* gene mRNA splicing to treat *SLC20A2* haploinsufficiency, which holds important clinical value for the treatment of cerebral calcification disease or diseases related to phosphate homeostasis imbalance.

### III. Astrocyte-Targeted Therapy

In some aspects, the present invention relates to astrocyte-targeted gene replacement therapy and use for rebuilding brain phosphate homeostasis, comprising inhibiting the occurrence of cerebral calcification in individuals with PiT2 mutations, and delaying or halting the progression of cerebral calcification in PBC (PFBC) or phosphate homeostasis imbalance-related cerebral calcification patients.

Through extensive and in-depth research and extensive screening, the inventors have for the first time developed an astrocyte-targeted gene replacement therapy that can effectively intervene in the progression of cerebral calcification. Through thorough experiments, the inventors have for the first time elucidated the pathogenic mechanism by which mutations and/or abnormalities in genes and/or molecules such as PiT2, MYORG, XPR1 lead to cerebral calcification, which comprises dysfunction of astrocyte phosphate transport caused by these mutations and/or abnormalities, leading to brain phosphate homeostasis imbalance, which in turn leads to cerebral calcification. At the methodological level, the present invention provides a strategy to restore brain phosphate homeostasis and inhibit calcification occurrence or block its progression by increasing the expression levels of PiT2, MYORG, or XPR1 genes within brain astrocytes.

In the examples provided by the present invention, a recombinant AAV virus capable of selectively expressing PiT2 protein in astrocytes was constructed. Experimental results showed that after intravenous injection of a recombinant AAV virus capable of crossing the blood-brain barrier, and the expression module it carries for expressing the PiT2 protein can selectively express the PiT2 protein, the product of the replacement gene, in astrocytes within the mouse brain. Consequently, brain astrocytes in *Pit2*-KO mice receiving this expression compensation achieved reconstruction of brain phosphate homeostasis, thereby effectively inhibiting the occurrence and progression of cerebral calcification from its root cause. The strategy revealed by the present invention also supports, as alternative implementation forms, that delivering gene expression promoters to astrocytes, such as antisense oligonucleotide (ASO) drugs, or RNA splicing modification based on gene/base editing, to modulate the expression levels of *PiT2, MYORG,* or *XPR1* in astrocytes, can similarly achieve the goal of repairing brain phosphate homeostasis and intervening in cerebral calcification. These data indicate that the gene replacement therapy of the present invention can be used in humans to repair brain phosphate homeostasis, inhibit cerebral calcification occurrence or halt further progression of cerebral calcification, and improve related neurological symptoms, possessing outstanding clinical translational value.

### III-(A). Astrocyte Phosphate Transport Promoters

One aspect of the present invention provides astrocyte phosphate transport promoters. Herein, the astrocyte phosphate transport promoter refers to an agent capable of promoting phosphate transport in astrocytes. The agent can, for example, achieve astrocyte-specific expression, such as using astrocyte-specific expression elements (e.g., promoters). Alternatively, it can specifically target astrocytes, such as specifically binding to specific receptors on astrocyte membranes.

In some embodiments, the brain phosphate homeostasis promoter is selected from the group consisting of:
(Z1) PiT2 protein, a nucleic acid molecule encoding the PiT2 protein, an expression vector expressing the PiT2 protein, a PiT2 promoter, or a combination thereof;
(Z2) MYORG protein, a nucleic acid molecule encoding the MYORG protein, an expression vector expressing the MYORG protein, a MYORG promoter, or a combination thereof;
(Z3) XRP1 protein, a nucleic acid molecule encoding the XRP1 protein, an expression vector expressing the XRP1 protein, an XRP1 promoter, or a combination thereof;
(Z4) any combination of Z1 to Z3 above.

In some embodiments, the promoter in any one of Z1 to Z3 is capable of activating or upregulating the expression of the gene, and/or activating or enhancing the activity of the protein product of the gene.

In some embodiments, the promoter can exert its activating effect through any mechanism, for example, by activating gene expression at the RNA or protein level (e.g., enhancing gene transcription, and/or enhancing translation of the gene's mRNA product).

In some embodiments, the promoter enhances its biological function by activating the expression level of the gene. In such embodiments, expression level measurement can be performed at the nucleic acid level or the protein level. Methods for measuring expression at the nucleic acid level include but are not limited to Northern blot, PCR, RT-PCR, or real-time RT-PCR. Methods for measuring expression at the protein level include but are not limited to Western blot or polyacrylamide gel electrophoresis combined with protein staining techniques such as Coomassie brilliant blue or silver staining, mass spectrometry, ELISA, etc.

In some embodiments, the promoter in any one of Z1 to Z3 comprises small molecule compounds, gene editing reagents (e.g., base editing reagents), small nucleic acid drugs (such as antisense oligonucleotide drugs), or a combination thereof. In some embodiments, the promoter is a small molecule compound. In some embodiments, the promoter is a gene editing reagent (e.g., a base editing reagent). In some embodiments, the promoter is a small nucleic acid drug (such as an antisense oligonucleotide drug).

In some embodiments, the astrocyte phosphate transport promoter is selected from promoters that regulate the expression levels of *PiT2, MYORG,* or *XPR1* in astrocytes.

In some embodiments, the astrocyte phosphate transport promoter according to any of the preceding embodiments can achieve astrocyte-specific expression, such as enabled by astrocyte-specific expression elements (e.g., promoters). In such embodiments, the astrocyte phosphate transport promoter can be an expression vector comprising a exogenous gene and an astrocyte-selective enhancer element and/or an astrocyte-selective promoter operably linked thereto, the exogenous gene selected from nucleic acid molecules encoding PiT2 protein, MYORG protein, or XRP1 protein, or a combination thereof.

In some embodiments, the exogenous gene is a nucleic acid molecule encoding the PiT2 protein.

In some embodiments, the astrocyte-selective promoter is *gfaABC1D.* In some embodiments, the astrocyte-selective promoter is a promoter derived from genes such as *ALDH1L1, AQP4, S100B.*

In some embodiments, the astrocyte phosphate transport promoter is an expression vector (e.g., a viral vector, such as AAV) comprising a nucleic acid molecule encoding the PiT2 protein and an astrocyte-selective promoter (e.g., *gfaABC1D*) operably linked thereto.

In some embodiments, the expression vector is a viral vector, for example, a lentiviral vector, adenoviral vector, adeno-associated viral vector, poxviral vector (e.g., vaccinia virus vector), herpes simplex viral vector.

In some embodiments, the expression vector is AAV. Adeno-associated virus (AAV), also known as adeno-associated virus, belongs to the *Parvoviridae* family, genus *Dependoparvovirus,* and is currently one of the simplest-structured single-stranded DNA defective viruses discovered. The wild-type AAV genome consists of linear single-stranded DNA (ssDNA) of approximately 4.7 kb, flanked by inverted terminal repeats (ITRs), while the sequence between the two ITR elements comprises the coding sequences for Rep and Cap proteins. Regulated by multiple alternative promoters and splice variants, the Rep gene can express four different proteins involved in viral genome replication, transcription, and integration. With the help of an important assembly-activating protein AAP expressed in open reading frame 2 (ORF2) of the Cap gene, the three structural capsid proteins VP1/VP2/VP3 expressed by the Cap gene assemble into an icosahedral capsid of 60 subunits at a ratio of 1:1:10. Because adeno-associated virus (AAV) is smaller than other viral vectors, has no or only weak immunogenicity, extremely low genome integration probability, and can transfect both dividing and non-dividing cells, it is considered an ideal delivery vector for gene therapy strategies.

The viral ITR elements contain cis-acting sequences required for genome replication and packaging. Therefore, AAV can be engineered into a viral delivery vector by removing all viral coding gene sequences between the ITR elements and replacing them with foreign DNA sequences encoding genes of interest. These foreign DNA sequences can encode conventional expression cassettes to express selected target genes in target cells. These engineered recombinant adeno-associated viral vectors (rAAVs), due to their good safety profile, broad host cell range (dividing and non-dividing cells), low immunogenicity, and long-term expression of exogenous genes in vivo, are regarded as one of the most promising gene transfer vectors and are widely used in gene therapy and vaccine research worldwide. After more than a decade of research, the biological characteristics of recombinant AAV have been well understood, especially in terms of their application effects in various cells, tissues, and *in vivo* experiments. In medical research, rAAV is used for gene therapy research of various diseases (comprising *in vivo* and *in vitro* experiments).

AAV vectors can be prepared using standard methods in the art. The production, purification, and use of multiple serotypes of adeno-associated viruses have undergone numerous tests in laboratories and animal and human experiments. Methods for purifying vectors can be found in various technical books and biotechnology method articles, and also, for example, in U.S. Patent Nos. 6,566,118, 6,989,264, and 6,995,006, and the disclosures of which are incorporated herein by reference in their entirety. Preparation of recombinant AAV vectors is described, for example, in PCT Application No. PCT/US2005/027091, the disclosure of which is incorporated herein by reference in its entirety. Embodiments using recombinant AAV vectors for in vitro and in vivo delivery of target genes have been described in literature and patents (see, e.g., International Patent Application Publications Nos. WO91/18088 and WO93/09239; U.S. Patent Nos. 4,797,368, 6,596,535, and 5,139,941; and European Patent No. 0488528, all of which are incorporated herein by reference in their entirety). These patents describe engineered AAV plasmids of various sources (strains, serotypes, mutant optimized strains, etc.) with the original rep and/or cap genes removed from the AAV genome, used to load foreign target genes, for producing recombinant AAV viruses and for use in delivering target genes *in vitro* (into cultured cells) or *in vivo* (directly into organisms). For safety considerations, AAV viruses for therapeutic purposes must be replication-defective. The production of these recombinant AAVs is generally prepared by co-transfecting two plasmids into a stable cell line infected with a human helper virus (e.g., adenovirus): Plasmid A is a plasmid with inverted terminal repeat (ITR) elements at both ends, loaded with a specific promoter and the coding sequence of the target expression gene; Plasmid B is a plasmid carrying the AAV genome replication gene (rep) and capsid protein coding gene (cap). In some production systems, for higher safety requirements, the replication gene (rep) and capsid protein coding gene (cap) can also be constructed on separate plasmids.

In some embodiments, to obtain better delivery and expression effects targeting specific tissues and cell types, recombinant AAV virus expression vectors can be combined with viral capsid protein coding plasmids of different serotypes. These serotypes or capsid protein coding genes are derived from different wild-type serotypes (including but not limited to AAV1, AAV2, AAV3, AAV4, AAV5, AAV6, AAV7, AAV8, AAV9, AAV10, AAV11, AAV12, AAV13, AAV14, AAV15, and AAV16 AAV capsid proteins), or can be derived coding sequences of these capsid proteins that have undergone mutation screening or molecular biology-directed evolution modification. Therefore, the present disclosure may use recombinant AAV viruses comprising any of the serotypes described above, or AAV serotypes developed in the future with better effects. Basic methods for developing, screening, and producing such viral capsid protein serotypes are known in the art and described in published academic literature and U.S. Patent No. 6,596,535.

In some embodiments, the AAV (particularly the capsid protein) is selected from blood-brain barrier-penetrating serotype AAVs, such as PHP.eB or PHP.AX.

In some embodiments, the astrocyte phosphate transport promoter is an AAV (such as PHP.eB or PHP.AX) comprising a nucleic acid molecule encoding the PiT2 protein and an astrocyte-selective promoter (e.g., *gfaABC1D*) operably linked thereto.

In other embodiments, the astrocyte phosphate transport promoter according to any of the preceding embodiments can specifically target astrocytes, such as specifically binding to specific receptors on astrocyte membranes. In such embodiments, the astrocyte phosphate transport promoter is linked to a targeting molecule possessing targeting specificity to astrocytes. In some embodiments, the astrocyte phosphate transport promoter is completely or partially encapsulated within the targeting molecule, or conjugated to the targeting molecule. In some embodiments, the targeting molecule is selected from antibodies or antibody fragments thereof, polysaccharides, aptamers, or nanoparticles.

In some embodiments, the astrocyte phosphate transport promoter is a small nucleic acid drug, the small nucleic acid drug being linked to a targeting molecule possessing targeting specificity to astrocytes.

In some embodiments, the small nucleic acid drug is an ASO. In some embodiments, the ASO upregulates the expression levels of *PiT2, MYORG,* or *XPR1.*

In some embodiments, the astrocyte phosphate transport promoter can be a gene editing or base editing formulation capable of performing target gene mRNA splicing regulation, linked to a targeting molecule possessing targeting specificity to astrocytes as described above, thereby regulating the expression levels of *PiT2, MYORG,* or *XPR1* in astrocytes. In some embodiments, the gene editing or base editing formulation typically comprises (i) a module with base editing activity and (ii) a module capable of binding to the target gene. Exemplary gene editing tools include but are not limited to CRISPR/Cas systems, DNA base editors, RNA editing tools.

### III-(B). Expression Vectors and Host Cells

One aspect of the present invention provides a plasmid construct and its design rationale for expressing a phosphate transport promoter (such as PiT2 protein) in astrocytes, which comprises a promoter for selectively driving target gene transcription in astrocytes and an open reading frame sequence encoding the phosphate transport promoter (such as PiT2 protein). Using the provided sequence information, a skilled person can construct nucleic acid sequences or expression plasmids suitable for transduction into target cells using published molecular cloning techniques.

In some embodiments, the vector contains a nucleic acid sequence encoding the PiT2 protein and is constructed in the form of an expression cassette familiar to those skilled in the art, which can be used to introduce into and express the target gene in astrocytes of human or primate brains, and thus serve as a therapeutic vector. Vectors can be viral or non-viral (e.g., plasmids, or LNP-packaged mRNA sequences). Viral vectors include vectors derived from the following types of viruses: adenovirus, adeno-associated virus (AAV), retrovirus, lentivirus, herpesvirus, vaccinia virus, MMLV, GaLV, simian immunodeficiency virus (SIV), HIV, poxvirus, and SV40. Preferably, the viral vector is replication defective, although it is contemplated that it can be replication deficient, replication competent, or conditionally replication competent. Viral vectors typically can remain in an episomal state without integrating into the host cell genome.

A preferred viral vector for delivering a nucleic acid sequence encoding the PiT2 protein to astrocytes is an AAV vector, such as a serotype AAV that can penetrate the blood-brain barrier via intravenous injection, such as PHP.eB or PHP.AX proven effective in mice. Intravenous injection to penetrate the blood-brain barrier can achieve efficient viral delivery throughout the brain; other methods, such as injection via the lymphatic system, or ventricular/spinal lumbar puncture, are still under development. Some can achieve whole-brain delivery in mice and may potentially achieve human brain delivery in the future. Using specific AAV serotypes (AAV2, AAV5, AAV9, etc.), or artificially modified versions of any of these serotypes (comprising AAV PHP.eB, AAV.CPP.16, and AAV.CPP.21 vectors), combined with cell type-specific promoters (such as *gfaABC1D*), can achieve selective expression in target cell types. Of course, with further development of viral technology, or through modification of serotypes alone to achieve astrocyte-specific infection, dependence on selective promoters could be reduced.

Viral vectors have the ability to enter cells, while non-viral vectors such as plasmids can be complexed with other adjuvants to assemble virus-like or lipid carriers, which can also be taken up by target cells. Such technologies have been proven effective in some human tissues; in the near future, such technologies may be prepared for selective delivery to astrocytes throughout the brain. Such adjuvants include polycationic liposomes, lipid nanoparticles (LNP), etc. Vectors for use in the present invention are preferably suitable for in vivo use, and more preferably suitable for use in humans.

The vector will preferably contain one or more regulatory sequences to direct the expression of the nucleic acid sequence in astrocytes. The vector may be operably linked to regulatory sequences in a manner that comprises one or more sequence modules such as promoters, introns, enhancers, translation regulatory elements, RNA stability regulatory sequences, ligand-inducible expression regulatory sequences, transcription termination signals, polyadenylation sequences, origins of replication, nucleic acid restriction sites, and homologous recombination sites. The vector may also include selective screening markers, for example, to support efficient viral assembly of the vector in a virus production system (e.g., 293 cells), or elements for inducible expression in astrocytes, or elements for negative regulation of target gene expression in non-astrocytes.

"Operably linked" means that the nucleic acid sequence is linked to other sequence modules in a certain order or structure based on their functional roles, so that they regulate the expression or function of the target gene in a temporally and spatially ordered manner as designed. For example, a nucleic acid sequence operably linked to a promoter will have transcriptional regulatory functions influenced by or influencing the promoter activity.

A promoter mediates the transcriptional expression of the downstream coding open reading frame nucleic acid sequence of the target gene linked thereto. The promoter can be constitutive or inducible. The promoter can direct selective expression in astrocytes, or also include oligodendrocytes and their precursor cells. In the latter case, promoter design screening needs to pass expression validation in both astrocytes and oligodendrocytes. Suitable promoters are key to achieving astrocyte-specific expression in the present invention. For example, in the mouse model described in the present invention, astrocyte-selective expression relies on *gfaABC1D* to drive transcription of the *PIT2* coding gene; some studies have verified that the selectivity of the *gfaABC1D* promoter is conserved and usable in human neural cells. Considering that single-cell studies are promoting the screening of more optional promoters, achieving astrocyte-selective expression may employ more promoters in the future, such as promoters derived from genes like *ALDH1L1, AQP4, S100B* and subjected to screening and modification.

From a technical feasibility perspective, various expression vectors can be applied to achieve PiT2 protein expression in astrocytes (preferably primate, more preferably human astrocytes). The present invention preferentially chooses adeno-associated virus as the expression vector. The present invention provides a method for constructing a recombinant adeno-associated virus expression vector loaded with the PiT2 coding sequence. This method can quickly and stably construct AAV expression vectors carrying the PiT2 coding sequence and package them to obtain therapeutic AAV virus preparations.

The present invention also provides a host cell comprising the vector as described above.

The present invention also provides an AAV vector packaging system, comprising:
an exogenous gene expression plasmid: comprising a nucleic acid sequence encoding a phosphate transport promoter (such as PiT2 protein) and an astrocyte-specific promoter (such as *gfaABC1D*) operably linked thereto; preferably, also comprising inverted terminal repeat sequences (ITRs) located at both ends; and
a packaging plasmid: comprising Rep and Cap genes.

In some embodiments, the exogenous gene expression plasmid is AAV2. In some embodiments, the packaging plasmid expresses PHP.eB serotype capsid protein.

In some embodiments, the AAV vector packaging system further comprises a helper plasmid comprising genes assisting Rep and Cap expression.

The present invention also provides a host cell comprising the AAV vector packaging system as described above. In some embodiments, the host cell is a genetically engineered cell used for producing AAV virus particles. Preferably, the host cell is a mammalian cell (preferably human, more preferably human astrocyte) for increasing the expression level of PiT2 protein.

### III-(C). Gene Therapy Vectors

One aspect of the present invention provides gene therapy vectors, particularly gene therapy vectors for preventing and/or treating cerebral calcification disease. The gene therapy vector is selected from the expression vectors as described above that achieve selective expression of a phosphate transport promoter (such as PiT2 protein) in astrocytes.

In some embodiments, the gene therapy vector is a viral expression vector. In some embodiments, the viral expression vector is an adeno-associated virus (AAV) vector. Techniques for serotype and promoter combinations achieving whole-brain astrocyte-specific expression have been realized in mice, such as using the combination of PHP.eB or PHP.AX with promoter gfaABC1D; however, the effectiveness of this combination in primates or humans has not been effectively proven and still requires continued engineering screening. However, it can be predicted that it may be modified from the following listed serotypes AAV1, 2, 3, 4, 5, 6, 7, 8, 9, and 10, or AAV vectors composed of chimeric AAVs derived therefrom such as AAV2-AAV3, AAVhu.14, AAV3a/3b, AAVHSC15, AAV-HSC17, AAVhu.37, CHt-P6, AAV2.5, AAV6.2, AAV2i8, AAV-HSC15/17, AAVM41, AAV9.45, AAV6(Y445F/Y731F), AAV2.5T, AAV-HAE1/2, AAV clone 32/83, AAVShH10, AAV2(Y->F), AAV8(Y733F), AAV2.15, AAV2.4, AAVM41, AAVr3.45, as well as some serotypes derived from non-human primates, such as AAVrh.10, AAVrh32.33, AAVrh.8. Screening for more effective delivery serotypes relies on directed evolution and screening of capsid protein coding sequences, especially in situ screening and validation performed in tissues and cell types of interest.

Currently, gene therapy vectors are mainly administered via intravenous injection to achieve whole-brain astrocyte delivery; with technological advancements, it is not excluded that other injection routes may also achieve efficient infection and expression in whole-brain astrocytes, comprising via head and neck lymphatic systems, nasal sinuses, ventricular injection, or lumbar puncture injection.

### III-(D). Formulations and Compositions

One aspect of the present invention provides a formulation or composition, the formulation or composition comprising (a) an effective dose of a brain phosphate homeostasis promoter as an active ingredient, and (b) a pharmaceutically acceptable carrier or excipient. In this text, the brain phosphate homeostasis promoter comprises astrocyte phosphate transport promoters.

In some embodiments, the formulation or composition is a pharmaceutical composition.

In some embodiments, the formulation or composition can promote and/or repair astrocyte phosphate transport function, thereby maintaining and/or restoring brain phosphate homeostasis, preventing and/or treating cerebral calcification caused by brain phosphate homeostasis defects.

In some embodiments, the formulation or composition is used for treating genetic cerebral calcification. The genetic cerebral calcification is Primary Familial Brain calcification (PFBC). The formulations of the present invention may be applicable to the treatment of other types of cerebral calcification triggered by glial cell phosphate transport dysfunction, such as some sporadic cerebral calcification cases, calcification caused by tumors or inflammation, and Aicardi-Goutières syndrome (AGS).

For convenient clinical application, the formulation or composition can be contained in an injection administration container or a disposable injection device (such as an injection needle). The injection administration device may contain a single administration dose of the formulation or composition. The injection administration device can be included in a kit for convenient storage and use. During transportation, the small container containing the drug suspension should be placed under refrigeration conditions (such as dry ice or a refrigerated shipping box). For long-term storage, it should be kept in a -80°C freezer; for short-term storage, it can be kept in a -20°C or 4°C refrigerator.

In some embodiments, the "active ingredient" in the formulation or composition refers to the gene therapy vector as described above, for example, a viral vector (comprising adeno-associated viral vectors). The "active ingredient", formulation, and/or composition can be used for treating cerebral calcification. "Effective dose" refers to a dose of the active ingredient sufficient to significantly improve major clinical observation indicators (such as cerebrospinal fluid phosphate levels, other biomarkers of calcification), the progression of cerebral calcification, or the severity of main symptoms, preferably without causing serious side effects.

In some embodiments, the formulation or composition can be liquid or solid, for example, powder, gel, or paste. Preferably, the composition is a liquid, preferably an injectable liquid. Suitable excipients will be known to those skilled in the art, or have been proven reliable and useful through clinical trials.

In some embodiments, the formulation or composition can be administered via intravenous injection, or other validated effective injection methods, to achieve whole-brain astrocyte delivery and expression. In any administration mode, preferably, the formulation or composition is provided as an injectable liquid. Preferably, the injection liquid can be provided as a concentrate for dilution before injection, or as a ready-to-use injection solution loaded in a syringe.

In some embodiments, the formulation or composition may comprise physiologically acceptable sterile aqueous or nonaqueous solutions, dispersions, suspensions, or emulsions, and sterile powders for reconstitution into sterile injectable solutions or dispersions. Suitable aqueous and nonaqueous carriers, diluents, solvents, or excipients include water, ethanol, polyols, and suitable mixtures thereof.

### III-(E). Therapeutic Applications

The present invention provides methods of using astrocyte phosphate transport promoters to inhibit the occurrence of cerebral calcification in advance, or to treat cerebral calcification, particularly genetic cerebral calcification. From an applicability perspective, this method is applicable to all populations with brain phosphate homeostasis imbalance caused by glial cell phosphate transport defects. This population comprises patients with Primary Familial Brain calcification (PFBC) related to mutations in the *PiT2, MYORG,* or *XPR1* genes, and may also include non-PFBC type cerebral calcification driven by brain phosphate homeostasis imbalance. The method comprises introducing the astrocyte phosphate transport promoter of the present invention, for example, a vector containing optimized sequences encoding *PiT2, MYORG,* or *XPR1*, into astrocytes for expression. The method may comprise administering a nucleic acid expression vector, or a promoter that regulates the expression levels of *PiT2, MYORG,* or *XPR1* in astrocytes, such as an antisense oligonucleotide (ASO) drug, or a gene editing or base editing formulation capable of performing target gene mRNA splicing regulation, to brain astrocytes via intravenous injection (or other technical means).

The astrocyte phosphate transport promoter of the present invention can be administered alone or in combination with other therapeutic drugs (e.g., formulated in the same pharmaceutical composition or separate pharmaceutical compositions).

In some embodiments, using the astrocyte phosphate transport promoter can improve the pathological process of cerebral calcification, or alleviate one or more symptoms of the disease, comprising rebuilding astrocyte phosphate transport capacity, repairing brain phosphate homeostasis imbalance, and reducing the occurrence of cerebral calcification.

In some embodiments, the method comprises introducing a nucleic acid sequence encoding PiT2, *MYORG,* or *XPR1* protein into brain astrocytes. Preferably, the method comprises contacting cells with a vector (preferably a virus, more preferably an adeno-associated virus) containing a nucleic acid sequence encoding the PiT2 protein. Preferably, the cells are astrocytes.

In some embodiments, in the method, an optimized nucleic acid sequence encoding PiT2, *MYORG,* or *XPR1,* or a promoter that can regulate the expression of PiT2, *MYORG,* or *XPR1* genes, is delivered to astrocytes and selectively expressed in astrocytes to rebuild astrocyte phosphate transport capacity, repair brain phosphate homeostasis imbalance, and ultimately inhibit the further progression of cerebral calcification in the brain, alleviating related clinical symptoms.

In some aspects, the present invention provides methods for preventing and/or treating cerebral calcification disease, comprising administering an effective dose of a brain phosphate homeostasis promoter to a subject in need thereof. Also provided is the use of a brain phosphate homeostasis promoter for preparing a drug for preventing and/or treating cerebral calcification disease.

In some embodiments, the brain phosphate homeostasis promoter is the astrocyte phosphate transport promoter as described above.

In some embodiments, the prevention comprises inhibiting the calcification in genetically mutation-carrying individuals before calcification occurs. In some embodiments, the mutation comprises mutations in *PiT2, MYORG, XRP1,* or a combination thereof. In some embodiments, the calcification comprises cerebral calcification.

In some embodiments, the treatment comprises inhibiting and/or delaying further progression in individuals who have developed calcification.

In some embodiments, the treatment comprises reversing and/or repairing brain phosphate homeostasis imbalance and/or cerebral calcification.

In some embodiments, the astrocyte phosphate transport promoter is an expression vector expressing a exogenous gene, the exogenous gene selected from the group consisting of: *PiT2, MYORG, XRP1,* or a combination thereof. In some embodiments, the expression vector comprises a viral vector, such as AAV. In some embodiments, the exogenous gene is operably linked to an astrocyte-selective enhancer element and/or an astrocyte-selective promoter. In some embodiments, the promoter is *gfaABC1D.* In some embodiments, the promoter is a promoter derived from genes such as *ALDH1L1, AQP4, S100B* and subjected to screening and modification.

In some embodiments, the astrocyte phosphate transport promoter comprises: small molecule compounds, gene editing reagents, small nucleic acid drugs, or a combination thereof. In some embodiments, the promoter is linked to a targeting molecule possessing targeting specificity to astrocytes. In some embodiments, the promoter is completely or partially encapsulated within the targeting molecule, or conjugated to the targeting molecule. In some embodiments, the targeting molecule is selected from antibodies or antibody fragments thereof, polysaccharides, aptamers, or nanoparticles.

In some embodiments, the astrocyte phosphate transport promoter is a small nucleic acid drug. In some embodiments, the small nucleic acid drug comprises antisense oligonucleotides (ASOs).

In some embodiments, the astrocyte phosphate transport promoter is a gene editing reagent. In some embodiments, the gene editing reagent comprises a base editing reagent.

In some embodiments, the prevention or treatment comprises: introducing the astrocyte phosphate transport promoter described herein into astrocytes of the subject.

In some embodiments, the cerebral calcification disease comprises cerebral calcification caused by abnormal brain phosphate homeostasis. As used herein, "phosphate homeostasis abnormality", "phosphate homeostasis imbalance", "phosphate homeostasis dysregulation", and "phosphate homeostasis defect" are used interchangeably. As used herein, "phosphate transport dysregulation" and "phosphate transport defect" are used interchangeably.

In some embodiments, the cerebral calcification disease comprises Primary familial brain calcification (PFBC) or Primary Basal Ganglia Calcification (PBGC).

In some embodiments, the drug is administered to a subject selected from the group consisting of: PFBC patients carrying mutations in *PIT2, MYORG, XPR1* genes, or a combination thereof; or patients with other types of cerebral calcification disease accompanied by brain phosphate homeostasis defects (such as upregulated cerebrospinal fluid inorganic phosphate). The other types of cerebral calcification disease may include other types of cerebral calcification triggered by glial cell phosphate transport dysfunction, such as some sporadic cerebral calcification cases, calcification caused by tumors or inflammation, and Aicardi-Goutières syndrome (AGS).

In some embodiments, the method further comprises: detecting gene mutations in the subject's body in *PiT2, MYORG, XRP1,* other pathogenic genes and/or molecules regulating *PiT2* and/or *XPR1* phosphate transporter expression and function, or a combination thereof; if the detection result indicates the presence of the mutations in the subject's body, then implementing the above therapy on the subject.

In some embodiments, the method further comprises: collecting cells from the subject, differentiating the cells into astrocytes, detecting the phosphate transport function of the astrocytes; if the detection result indicates that the astrocyte phosphate transport capacity C1 from the subject is significantly lower than the astrocyte phosphate transport capacity C0 of normal individuals, then implementing the above therapy on the subject. In some embodiments, "significantly lower" means that the ratio (C1/C0) of the astrocyte phosphate transport capacity C1 derived from the subject to the astrocyte phosphate transport capacity C0 of normal individuals is ≤2/3, preferably ≤1/2; more preferably ≤1/4.

In some embodiments, the brain phosphate homeostasis defect comprises astrocyte phosphate transport defect in the brain.

In some embodiments, the cerebral calcification disease comprises cerebral calcification disease caused by brain phosphate homeostasis imbalance.

In some embodiments, for subjects (or patients) with cerebral calcification disease due to brain phosphate homeostasis imbalance, their astrocyte phosphate transport capacity C1 is significantly lower than the astrocyte phosphate transport capacity C0 of normal individuals. In some embodiments, "significantly lower" means that the ratio of astrocyte phosphate transport capacity C1 to the astrocyte phosphate transport capacity C0 of normal individuals (C1/C0) is ≤2/3, preferably ≤1/2; more preferably ≤1/4.

In some embodiments, the subject has a genetic mutation in genes selected from the group consisting of: *PiT2, MYORG, XRP1,* other pathogenic genes and/or molecules regulating *PiT2* and/or *XPR1* phosphate transporter expression and function, or a combination thereof. In some embodiments, the genetic mutation comprises exonic mutations, intronic mutations, 5'-UTR mutations, 3'-UTR mutations, or a combination thereof.

In some embodiments, the subject comprises animal models. In some embodiments, the subject comprises mammals. In some embodiments, the subject comprises humans. In some embodiments, the subject comprises non-human mammals. In some embodiments, the subject is a non-human mammal. In some embodiments, the subject comprises primates. In some embodiments, the subject comprises rodents. In some embodiments, the subject comprises humans, mice, rats, monkeys, or a combination thereof.

In some embodiments, the administration to the subject comprises: intravenous injection, subcutaneous injection, head and neck lymphatic injection, nasal sinus administration, cerebral ventricular injection, brain parenchyma injection, spinal lumbar puncture injection, or a combination thereof.

In some aspects, the present invention also provides a kit for preventing and/or treating cerebral calcification disease. The kit comprises:
(a) a first container, and a first detection reagent for detecting gene mutations located in the first container, the first detection reagent used for detecting whether genetic mutations exist in genes selected from the group consisting of: *PiT2, MYORG, XRP1*, or a combination thereof; and
(b) a second container, and a pharmaceutical composition located in the second container, the pharmaceutical composition comprising a drug component that can modulate and repair brain phosphate homeostasis imbalance and a pharmaceutically acceptable carrier or excipient.

In some embodiments, the drug component comprises a brain phosphate homeostasis promoter.

In some embodiments, the drug component comprises the astrocyte phosphate transport promoter described herein.

In some embodiments, the kit further comprises: (c) a third container, and a solution located in the third container for assisting in dissolving or *in vivo* delivery of the pharmaceutical composition in the second container.

In some embodiments, the kit optionally further comprises: (d) a fourth container, and a fourth detection reagent located in the fourth container for detecting astrocyte phosphate transport function.

In some embodiments, the pharmaceutical composition in the second container is administered to a subject selected from the group consisting of: PFBC patients carrying mutations in *PIT2, MYORG, XPR1* genes, or a combination thereof; or patients with other types of cerebral calcification disease accompanied by brain phosphate homeostasis defects (such as upregulated cerebrospinal fluid inorganic phosphate). In some embodiments, the brain phosphate homeostasis defect comprises astrocyte phosphate transport defect in the brain. In some embodiments, the cerebral calcification disease comprises cerebral calcification disease caused by brain phosphate homeostasis imbalance. In some embodiments, for subjects (or patients) with cerebral calcification disease due to brain phosphate homeostasis imbalance, their astrocyte phosphate transport capacity C1 is significantly lower than the astrocyte phosphate transport capacity C0 of normal individuals. In some embodiments, "significantly lower" means that the ratio of astrocyte phosphate transport capacity C1 to the astrocyte phosphate transport capacity C0 of normal individuals (C1/C0) is ≤2/3, preferably ≤1/2; more preferably ≤1/4. In some embodiments, the subject has a genetic mutation in genes selected from the group consisting of: *PiT2, MYORG, XRP1,* other pathogenic genes and/or molecules regulating *PiT2* and/or *XPR1* phosphate transporter expression and function, or a combination thereof. In some embodiments, the genetic mutation comprises exonic mutations, intron mutations, 5'-UTR mutations, 3'-UTR mutations, copy number variations (CNVs), or a combination thereof.

In some embodiments, using the first detection reagent to detect whether gene mutations exist in the body of the required subject in *PiT2, MYORG, XRP1,* other pathogenic genes and/or molecules regulating *PiT2* and/or *XPR1* phosphate transporter expression and function, or a combination thereof; if the detection result indicates the presence of the mutations in the subject's body, then the subject is considered suitable for treatment with the pharmaceutical composition in the kit.

In some embodiments, collecting cells from the required subject, differentiating the cells into astrocytes, using the fourth detection reagent to detect the phosphate transport function of the astrocytes; if the detection result indicates that the astrocyte phosphate transport capacity C1 of the subject is significantly lower than the astrocyte phosphate transport capacity C0 of normal individuals, then the subject is considered suitable for treatment with the pharmaceutical composition in the kit. In some embodiments, "significantly lower" means that the ratio (C1/C0) of the astrocyte phosphate transport capacity C1 derived from the subject to the astrocyte phosphate transport capacity C0 of normal individuals is ≤2/3, preferably ≤1/2; more preferably ≤1/4. In some embodiments, the cells include primary cells and passaged cells. In some embodiments, the cells include somatic cells, germ cells, gametes, stem cells. In some embodiments, the stem cells include: totipotent stem cells, pluripotent stem cells, and unipotent stem cells. In some embodiments, the stem cells are induced pluripotent stem cells (iPSCs). In some embodiments, the cells include: embryonic stem cells, adipose stem cells, neural stem cells, mesenchymal stem cells, oligodendrocyte progenitor cells, hematopoietic stem cells, immune cells (such as T cells, B cells, NK cells). In some embodiments, the cells include: healthy normal human cells, cancerous human cells. In some embodiments, the cells include: suspension cells, adherent cells. In some embodiments, the cells do not include embryonic stem cells. In some embodiments, the cells do not include human embryonic stem cells.

In some embodiments, the subject comprises animal models. In some embodiments, the subject comprises mammals. In some embodiments, the subject comprises humans. In some embodiments, the subject comprises non-human mammals. In some embodiments, the subject is a non-human mammal. In some embodiments, the subject comprises primates. In some embodiments, the subject comprises rodents. In some embodiments, the subject comprises humans, mice, rats, monkeys, or a combination thereof.

In some embodiments, the first container and second container, third container, fourth container are the same or different containers.

In some embodiments, the pharmaceutical composition in the second container comprises a single and/or compound formulation containing a guide RNA or its expression vector, and/or a gene editing protein or its expression vector. In some embodiments, the gene editing protein comprises a base editing protein. In some embodiments, the single and/or compound formulation in the second container may be packaged in different drug containers due to drug transportation and storage needs.

In some embodiments, the pharmaceutical composition in the second container is a single formulation containing other drugs helpful for treating cerebral calcification disease.

In some embodiments, the dosage form of the pharmaceutical composition in the second container is selected from the group consisting of: lyophilized formulations, liquid formulations, or a combination thereof.

In some embodiments, the dosage form of the pharmaceutical composition in the second container comprises: injectable dosage forms, transdermal absorption dosage forms, intranasal inhalation dosage forms, or a combination thereof.

In some embodiments, the kit further contains instructions.

### III-(F). Drug Screening

The present invention provides a method for screening candidate drugs for preventing and/or treating cerebral calcification disease, the method comprising the steps:
(a) in a test group, adding a test drug to a culture system of astrocytes, and observing the phosphate transport level (E1) and/or activity (A1) in the cells of the test group; in a control group, not adding the test compound to a culture system of the same cells, and observing the phosphate transport level (E0) and/or activity (A0) in the cells of the control group;
wherein, if the phosphate transport level (E1) and/or activity (A1) of the cells in the test group is significantly higher than that of the control group, it indicates that the test drug is a candidate drug for preventing and/or treating cerebral calcification disease that promotes the expression and/or activity of phosphate transport.

In some embodiments, the test drug is a compound.

In some embodiments, the "significantly higher" means E1/E0 ≥ 1.5, preferably ≥ 2.

In some embodiments, the "significantly higher" means A1/A0 ≥ 1.5, preferably ≥ 2.

In some embodiments, the cells include astrocytes.

In some embodiments, the cells are cells cultured in vitro.

In some embodiments, the method comprises step (b): administering the candidate drug determined in step (a) to a mammalian model to determine its effects on the mammal. In some embodiments, the mammal is a mammal suffering from cerebral calcification disease.

In some embodiments, the method is non-diagnostic and non-therapeutic.

### III-(G). Exemplary Schemes

The present invention relates to the following exemplary embodiments:
Embodiment 1. Use of a brain phosphate homeostasis promoter, characterized in that it is used for preparing a drug for preventing and/or treating cerebral calcification disease.
Embodiment 2. The use according to Embodiment 1, characterized in that the brain phosphate homeostasis promoter is selected from the group consisting of:
   (Z1) a PiT2 protein, a nucleic acid molecule encoding the PiT2 protein, an expression vector expressing the PiT2 protein, a PiT2 promoter, or a combination thereof;
   (Z2) a MYORG protein, a nucleic acid molecule encoding the MYORG protein, an expression vector expressing the MYORG protein, a MYORG promoter, or a combination thereof;
   (Z3) a XRP1 protein, a nucleic acid molecule encoding the XRP1 protein, an expression vector expressing the XRP1 protein, an XRP1 promoter, or a combination thereof;
   (Z4) any combination of Z1 to Z3 above.
Embodiment 3. The use according to Embodiment 1, characterized in that the promoter comprises small nucleic acid drugs.
Embodiment 4. The use according to Embodiment 1, characterized in that the cerebral calcification disease comprises Primary familial brain calcification (PFBC).
Embodiment 5. A formulation and composition, characterized in that the formulation and composition contain a brain phosphate homeostasis promoter.
Embodiment 6. The formulation and composition according to Embodiment 5, characterized in that the brain phosphate homeostasis promoter is selected from the group consisting of:
   (Z1) a PiT2 protein, a nucleic acid molecule encoding the PiT2 protein, an expression vector expressing the PiT2 protein, a PiT2 promoter, or a combination thereof;
   (Z2) a MYORG protein, a nucleic acid molecule encoding the MYORG protein, an expression vector expressing the MYORG protein, a MYORG promoter, or a combination thereof;
   (Z3) a XRP1 protein, a nucleic acid molecule encoding the XRP1 protein, an expression vector expressing the XRP1 protein, an XRP1 promoter, or a combination thereof;
   (Z4) any combination of Z1 to Z3 above.
Embodiment 7. A kit for treating cerebral calcification disease, characterized in that the kit comprises:
   (a) a first container, and a first detection reagent located in the first container for detecting gene mutations, the first detection reagent used for detecting whether genetic mutations exist in genes selected from the group consisting of: *PiT2, MYORG, XRP1,* or a combination thereof; and
   (b) a second container, and a pharmaceutical composition located in the second container, the pharmaceutical composition comprising a drug component that can modulate and repair brain phosphate homeostasis imbalance and a pharmaceutically acceptable carrier.
Embodiment 8. The kit according to Embodiment 7, characterized in that the kit further comprises: (c)a third container, and a solution located in the third container for assisting in dissolving or *in vivo* delivery of the drug in the second container.
Embodiment 9. A method for screening candidate compounds for preventing and/or treating cerebral calcification disease, characterized in that the method comprises the steps:
   (a) in a test group, adding a test compound to a culture system of astrocytes, and observing the phosphate transport level (E1) and/or activity (A1) in the cells of the test group; in a control group, not adding the test compound to a culture system of the same cells, and observing the phosphate transport level (E0) and/or activity (A0) in the cells of the control group;
   wherein, if the phosphate transport level (E1) and/or activity (A1) of the cells in the test group is significantly higher than that of the control group, it indicates that the test compound is a candidate compound for preventing and/or treating cerebral calcification disease that promotes the expression and/or activity of phosphate transport.
Embodiment 10. Method according to Embodiment 9, characterized in that the method comprises step (b): administering the candidate compound determined in step (a) to a mammalian model to determine its effects on the mammal.

### III-(H). Beneficial Effects and Summary

The present invention for the first time discovered that the astrocyte-mediated phosphate transport process, comprising the uptake of inorganic phosphate (Pi) from the extracellular interstitial fluid by the PiT2 transporter expressed on the glial cell leaflet membranes, and the excretion of Pi taken up by glial cells to the outside of the brain through the XPR1 transporter on the end-foot membrane structure at the blood-brain barrier exit, is an important link in brain phosphate homeostasis regulation. Conditional knockout experiments in mouse animal models indicate that when astrocyte-mediated phosphate transport capacity is blocked compared to other cell types, the phosphate level in cerebrospinal fluid shows the most significant increase, i.e., homeostasis imbalance. Meanwhile, expression studies based on in situ hybridization show that astrocytes highly express the phosphate uptake transporter PiT2 and the excretion transporter XPR1; whereas neurons highly express another uptake transporter, PiT1.

Building on the discovery in item (1), by delivering viruses to astrocytes in Pit2-KO mice to specifically express the PiT2 protein within astrocytes, the brain phosphate transport level in Pit2-KO mice can be significantly reduced, and the occurrence and progression of cerebral calcification therein can be inhibited.

Methodologically, based on the core cellular pathological mechanism that cerebral calcification occurs due to phosphate homeostasis imbalance, the present invention, by selectively expressing PiT2 on astrocytes, rebuilds astrocyte phosphate transport capacity, repairs the imbalanced brain phosphate homeostasis, and fundamentally eliminates the underlying driving factors for calcification.

It should be noted that this method is not merely treating the progression of cerebral calcification but repairing brain phosphate homeostasis. In a patient's brain, initially, due to genetic mutations or acquired factors affecting, brain phosphate homeostasis becomes imbalanced, and then this imbalance induces the occurrence of cerebral calcification. Therefore, the most fundamental treatment of this method is directed towards repairing the imbalanced brain phosphate homeostasis. In the future, when clinical experts recognize phosphate homeostasis imbalance as a disease, just like elevated blood sugar is diabetes, or elevated blood lipids is a pathology requiring immediate treatment, therapeutic strategy of this method may benefit a broader audience.

Furthermore, the core methodological strategy elucidated by this method is targeting astrocytes to repair brain phosphate homeostasis imbalance. Those skilled in the art will understand that this method can be based on different delivery strategies and can be further optimized in the future with technological advancements.

### Examples

The present invention is now described with reference to the following examples intended to illustrate (but not limit) the invention.

Those skilled in the art know that the examples describe the invention by way of illustration and are not intended to limit the scope claimed in this application. Those skilled in the art will understand that various modifications and changes can be made to the details according to all published teachings, and such changes fall within the scope of protection of the invention. The full scope of the invention is given by the appended claims and any equivalents thereof. Unless otherwise specified, experimental methods in the examples are conventional methods. For those not specifying specific conditions, they are performed according to conventional conditions or conditions recommended by the manufacturer. Reagents or instruments not indicating the manufacturer are generally conventional products available through commercial purchase.

### Materials and Methods

### 1. Subject Recruitment, Whole Genome Sequencing, and Genetic Analysis

778 patients with suspected PFBC were recruited from the Department of Neurology, The First Affiliated Hospital of Fujian Medical University between 2012-2023. The PFBC diagnostic criteria were as follows: a) calcifications detected by CT scan are bilateral and symmetrical in the basal ganglia and/or dentate nuclei; b) the total calcification score (TCS) exceeds previously reported age-specific thresholds; c) no biochemical abnormalities, with normal serum concentrations of calcium, phosphate, and parathyroid hormone (PTH); d) no infectious, toxic, or traumatic causes. Neurological examination and clinical assessment were performed by at least two senior neurologists. For all suspected patients, whole-exome sequencing (WES) was first used to exclude cases with mutations in the entire coding regions and exon segments of known PFBC-associated genes such as *SLC20A2, PDGFRB, PDGFB, XPR1, MYORG, JAM2,* and *CMPK2,* and then identified PFBC-associated intron mutation cases and pedigrees from the remaining sample set. This study was approved by the Institutional Review Board of The First Affiliated Hospital of Fujian Medical University, and all participants provided written informed consent before enrollment.

For whole-genome sequencing, after obtaining blood genomic DNA from cerebral calcification patients, paired-end DNA libraries (Illumina Truseq library construction) were prepared from human DNA samples according to the instructions of the relevant sequencing library kit manufacturer. The constructed DNA libraries were subjected to next-generation sequencing with 150bp paired-end reads using the Illumina NovaSeq 6000. The average sequencing depth ranged from 31.35 to 57.77, with 90.1% to 99.2% of the whole genome covered at least 20 times. Then, the sequencing results (without barcodes) were aligned to the human reference genome hg19 using SpeedSeq. Subsequently, calling analysis of single nucleotide variants and insertions/deletions (Indels) was performed using the Genome Analysis Toolkit v4.1. Potential genomic structural variants and copy number variants in the patient genome were extracted via SpeedSeq; or ANNOVAR was used for annotating single nucleotide mutations, indels, structural variants, and copy number variants.

For parametric linkage analysis, DNA samples were isolated from whole blood of 45 family members from Family 1 and subjected to whole-genome linkage analysis using the HumanOmniZhongHua-8 BeadChip (Illumina). Genotype calling and quality control were processed with the GenomeStudio genotyping module (Illumina). SNPs with Mendelian errors or calling rates below 100% were removed. Parametric linkage analysis was performed using Merlin (v1.1.2), specifying a rare dominant model with a rare disease allele frequency of 0.0001.

### 2. Construction of Minigene Plasmids

To establish a rapid analysis of splicing events involving potential cryptic exon via RT-PCR, DNA fragments of human *SLC20A2* gene genomic sequences containing wild-type or mutant exon 2 - intron 2 - exon 3, exon 6 - intron 6 - exon 7, and exon 10 - intron 10 - exon 11 were cloned into a minigene backbone plasmid driven by the CMV promoter. To test potential changes in the *SLC20A2* mRNA translation product due to cryptic exon inclusion associated with mutations, the corresponding *SLC20A2* cDNA containing cryptic exon inclusion was cloned into the pCMV6-Entry plasmid (Origene) using the ClonExpress MultiS One Step Cloning Kit (Vazyme). Then, mutations in patients were generated via overlap PCR or the Mut Express II fast mutagenesis kit (Vazyme). Primers for amplifying these fragments are listed in Table 3.

### 3. Antisense Oligonucleotides (ASOs)

Antisense oligonucleotide technology is a drug development strategy based on chemically modified short oligonucleotides (12-24 nt) that bind to their RNA targets via Watson-Crick base pairing. ASO sequences were designed according to ASO design guidelines. ASOs with MOE and PS modifications (2'-O-methoxyethyl sugar with a phosphorothioate backbone) were synthesized and HPLC purified by Integrated DNA Technologies. ASOs with PMO modifications (phosphorodiamidate morpholino backbone) were synthesized by Gen-Tools, LLC. All ASOs were dissolved in nuclease-free water for *in vitro* cell culture experiments and in artificial cerebrospinal fluid (aCSF) for *in vivo* mouse experiments. Detailed sequences are provided in Table 1.

### 4. Construction of the SLC20A2-KI Mouse Model

To construct a humanized *SLC20A2* mouse model, CRISPR/Cas9 technology was used to replace the genomic fragment from exon 2 to exon 3 of the mouse *SLC20A2* gene with the genomic sequence of frame-matched human *SLC20A2* gene carrying the c.289+1007 C>G mutation. Briefly, a targeting sequence with bilateral 1.4 kb homology arms was co-microinjected with sgRNAs and Cas9 RNA into the uterus of C57BL/6J mice. To screen for founder (F0) mice with the correct knock-in sequence, genomic DNA extracted from mouse tails was used for genotyping. Germline transmission of F0 KI mice was verified by crossing with C57BL/6J wild-type mice to produce F1 KI mice. To further verify targeted mice with correct homologous recombination, long-range sequencing and southern-blot analysis were also performed on F1 mice. Heterozygous and homozygous *SLC20A2-KI* mice were selected for biochemical and phenotypic analysis. sgRNA sequences and genotyping primers are listed in Table 3.

### 5. Cell Culture and Transfection

HEK293T, Neuro-2A, A172, HepG2, HeLa cells (from the Cell Bank of the Chinese Academy of Sciences) and primary human fibroblasts were cultured in DMEM supplemented with 10% (vol/vol) fetal bovine serum (FBS) in 6-well or 12-well plates (Corning). Human fibroblasts were derived from PFBC patients with heterozygous *SLC20A2* c.289+1007 C>G mutation and heterozygous *SLC20A2* deletion, as well as from healthy volunteer donors as control.

For plasmid transfection, approximately 3 µg of minigene or cDNA plasmids were transfected into HEK293T and Neuro-2A cells using Lipofectamine 3000 (Thermo Fisher). For Western blot, cells were harvested 24 hours post-transfection. For splicing analysis using RT-PCR, cells were transfected with MOE- or PMO-modified ASOs after 24 hours of culture.

For MOE-modified ASOs, cells were transfected using Lipofectamine 3000 (Thermo Fisher) at a final concentration of 100 nM for 48 hours. For PMO-modified ASOs, cells were transfected with 4 µM PMO using 6 µL/mL Endo-Porter (Gene-Tools, LLC) for 48 hours. To inhibit NMD-mediated RNA degradation, cultured human fibroblasts was incubated with cycloheximide (CHX, 50 µg/mL) for 6 hours before harvest.

### 6. RNA Extraction and Splicing Analysis

Total RNA was extracted from HEK293T, Neuro-2A, HepG2, HeLa, A172 cells, or human fibroblasts using TRIzol (Invitrogen) according to the manufacturer's instructions, then 1 µg RNA was reverse transcribed into cDNA using the iScript cDNA Synthesis Kit (Bio-Rad). For splicing analysis, transcribed cDNA was used for PCR with primer pairs spanning corresponding exons of the *SLC20A2* gene; primer sequences are listed in Table 3.

### 7. Protein Extraction and Western-blot Analysis

Cells or mouse brains were collected in RIPA lysis buffer containing protease inhibitors, and lysates were centrifuged at 14,000g for 15 minutes. To detect PiT2, protein extracts were incubated at 37°C for 10 minutes, then separated using 9% SDS-PAGE gels and blotted onto PVDF membranes. Membranes were blocked with 5% skim milk, then detected with PiT2-7B5 (1:1000, monoclonal antibody obtained by immunizing mice with the PiT2 protein loop region), HA-tag (1:5000, #3724, Cell Signaling), GAPDH (1:3000, KC-5G5, KangChen), α-Tubulin (1:5000, 66031-1-Ig, Proteintech), and corresponding HRP-labeled secondary antibodies (1:2000, Cell Signaling). Bands were visualized using ECL (TIANGEN) and measured with Image J software.

### 8. Intracerebroventricular (ICV) Injection of ASO in Mice

All animal surgeries were performed under standard aseptic conditions. Mice were anesthetized with isoflurane (5% for induction, 1.5% for maintenance), placed in a small animal stereotaxic frame (Model 68528, RWD life science), and body temperature was maintained with a heating pad. At the incision site of *SLC20A2-KI* mice, 2% lidocaine was injected, and hair was removed using depilatory cream. After disinfecting the scalp at the surgical site three times with betadine and 75% alcohol, a midline incision was made, the scalp was removed to expose the skull, and the periosteum and other soft tissues attached to the skull were cleaned with cotton swabs and a scalpel. After the skull dried, tissue adhesive (3M Vetbond Tissue Adhesive) was used to glue the skin around the skull to the exposed skull edge. The skull plane was adjusted to the stereotaxic horizontal level, and the bregma was identified. A layer of blue light-curable self-etching adhesive (3M ESPE Single Bond Universal, 3M Deutschland Gmbh) was applied on and around the frontal bone to aid fixation. Then, a custom head plate was fixed flat onto the frontal bone using dental cement. While keeping the dura intact, a small circular craniotomy was carefully drilled (AP +0.25mm; ML -0.75mm). The dura was protected and covered with antibiotic ointment and fast-curing silicone elastomer. Mice were moved to a specially designed fixation plate under 1.5% isoflurane, and cerebrospinal fluid was collected from the tympanic cavity. Afterwards, mice were allowed to recover for at least one week before ASO injection.

For ICV injection, mice were anesthetized with isoflurane (5% for induction, 1% for maintenance), and the mouse head was fixed in a stereotaxic instrument with a heating pad to maintain body temperature. ASO powder was dissolved in filtered custom-modified artificial cerebrospinal fluid (125 mM NaCl, 20 mM NaHCO₃, 3 mM KCl, 1.2 mM MgSO₄, 1 mM NaH₂PO₄.H₂O, 1.5 mM CaCl₂) to a working concentration of 25 µg/µL. A total dose of 50 µg, 100 µg, 200 µg, or 400 µg of *SLC20A2*-ASO or control-ASO was injected into the right lateral ventricle (central coordinates: AP +0.25 mm; ML -0.75 mm; DV -1.85 mm) using a Nano-injector (Nanoject III, Drummond Scientific Company) at a rate of 1 nL/s. Glass pipettes were withdrawn at least 10 minutes after injection. The craniotomy was sealed with blue light-curable flowable resin to avoid infection. Mice were allowed to recover from anesthesia on a heating pad before being returned to their cages.

### 9. CSF Collection and Pi Measurement

According to established laboratory protocols, mice were fixed using a stereotaxic frame (Model 68528, RWD life science). Another ultra-micro-scale three-way adjustable stand was used to hold the collection needle, which was loaded with a drawn glass needle, to puncture into the ventricular cistern from the medulla oblongata behind the brain of anesthetized adult mice. Then, slight negative pressure was applied to guide CSF into the glass needle. Approximately 5-10 µL of CSF was collected, then centrifuged for 5 minutes (2000 g, 4°C) to remove cellular debris. The concentration of inorganic phosphate in CSF was detected using the PiColorLock gold phosphate detection kit (303-0030, Innova Biosciences), then read at 635 nm using a SpectraMax (Molecular Devices).

### 10. In Situ Hybridization (ISH) to Detect ASO Distribution in Mouse Brain

All reagents used in ISH analysis were prepared with diethyl pyrocarbonate (DEPC). Seven-month-old *SLC20A2-KI* mice treated with MOE-A and MOE-NC were anesthetized with chloral hydrate, perfused with 4% PFA, and sequentially dehydrated with 15% and 30% sucrose. Brain sections of 18-µm thickness were prepared for ISH using the miRNAscope^{™} Assay Red (Advanced Cell Diagnostics, Cat.No.324500) and a custom-designed probe for MOE-A (Advanced Cell Diagnostics, Cat.No.1123181-S1) according to the manufacturer's instructions. Whole-brain expression of MOE-A was imaged using a VS120 microscope (Olympus, Japan) with a 40× objective.

### 11. micro-CT and Histochemical Staining Analysis of Cerebral calcification

To assess cerebral calcification, *SLC20A2*-KI and WT mice were anesthetized with chloral hydrate and perfused with 4% PFA in PBS. For micro-CT scanning, three-dimensional images of mouse brains were obtained using an X-ray micro-CT scanner (Bruker, Skyscan 1172); paraffin-embedded mouse brains were evaluated in high-resolution mode with an X-ray tube voltage of 90 kV, current of 88 µA, and voxel size of 72 µm.

For histochemical staining of cerebral calcification, after dehydration through a graded series of ethanol, mouse brains were paraffin-embedded, and 4-micron-thick sections were prepared. Evaluation of cerebral calcification was performed according to widely recognized conventional methods in the field: these staining methods included alizarin red, Von Kossa, Alcian blue, periodic acid-Schiff (PAS), and H&E. Each analysis was performed in triplicate.

### 13. Statistical Analysis

Data are presented as mean ± standard error of the mean (SEM). Statistical significance was assessed by Student's t-test. Differences were considered statistically significant at <0.05, denoted as follows: *p < 0.05, **p < 0.01, ***p < 0.001, and ****p < 0.0001.

### Example 1: Identification of SLC20A2 Intron Mutations in PFBC Patients and Analysis of Causation of SLC20A2 Splicing and Translation Abnormalities thereof

After years of diligent work, the inventors accumulated hundreds of cerebral calcification cases and constructed pedigree maps. Through genetic analysis using exome sequencing, causative mutations in exons of known PFBC genes (*SLC20A2, PDGFB, PDGFRB, XPR1, MYORG, and JAM2*) were found in 33 out of 53 (62.2%) PFBC families. Additionally, exon segment deletions flanking the *SLC20A2* gene were identified in another 3 families via CNV analysis. Among the remaining 17 families, linkage analysis was performed on a large family (Family 1) (Fig. 1a) where no exon mutations were detected. Association mapping analysis using the Affymetrix 250K Nsp SNP chip on genomic DNA samples from 45 members of this family revealed a 32 Mb segment on chromosome 8 (hg19 chr8: 29042453-61341711) linked to the calcification phenotype in the family, with an LOD score of 4.5. Further analysis via WGS revealed a deep-intron variant, c.289+1007 C>G, in *SLC20A2,* and Sanger sequencing verified the co-segregation of this variant with cerebral calcification in the family. *In silico* evaluation of this variant using SpliceAI showed an acceptor gain score of 0.11 and a donor gain score of 0.11, indicating possible introduction of an unreported cryptic exon from splicing within intron 2 of *SLC20A2* (Table 2).

Using similar WGS analysis and Sanger sequencing methods to screen other PFBC family patients lacking clear genetic diagnoses, three additional genetic variants in deep intron regions of the *SLC20A2* gene were found in four families (Fig. 1a): c.289+937 G>A in intron 2 (Family 2 and Family 3), c.730+768 C>T in intron 6 (Family 4), and c.1795-1698 A>G in intron 10 (Family 5). These four intron mutations (Fig. 1c) were not registered in major human population genetics databases and were all predicted to affect splicing by SpliceAI analysis (Table 2). Also found was that in these five families, these mutations co-segregated with cerebral calcification (Fig. 1b). The age of the subjects ranged from 30 to 60 years. Four of them (Family 1, 3, 4, 5) reported headaches, and one (Family 2) had dizziness. CT imaging showed moderate to severe cerebral calcification (TCS, 16-39) in the basal ganglia and dentate nuclei (Family 1-5), thalamus (Family 1-3), and subcortical white matter (Family 2) (Fig. 1b). All patients had normal serum calcium, phosphate, and PTH levels (Table 2). These results support that *SLC20A2* intron variants may be genetic causes of PFBC.

In mammals, it is now well established that the 5' splice donor site and 3' splice acceptor site of introns are conserved. Intron mutations affect mRNA processing by disrupting splice sites and/or splicing regulatory elements, or by altering the secondary structure of the mRNA molecule. Further sequence prediction suggested that the c.289+937 G>A mutation in intron 2 and the c.730+768 C>T mutation in intron 6 may directly create new splice acceptors or donors, thereby altering the original splicing mechanism; however, the c.289+1007 C>G mutation in intron 2 and the c.1795-1698 A>G mutation in intron 10 did not create characteristic splice acceptors or donors, suggesting they might act through other mechanisms, such as regulating the inclusion probability of cryptic exons.

To validate genetic predictions, Minigene analysis was firstly performed to explore how intron mutations found in 2 families alter the splicing of SLC20A2 cryptic introns. Specifically, *SLC20A2* genomic sequences (comprising the linker sequence of exon 2-3 upstream and downstream of intron 2, and the linker sequence of exon 10-11 upstream and downstream of intron 10) were cloned into a backbone plasmid driven by the CMV promoter to construct wild-type minigene plasmid, then site-directed mutagenesis was used to generate point mutations corresponding to the c.289+937 G>A and c.1795-1698 A>G intron mutations from the 2 PFBC families (Fig. 2a, c).

Minigene assays confirmed that for Family 1 and Family 5, although the c.289+1007 C>G and c.1795-1698 A>G mutations did not lead to de novo splice sites, RT-PCR and Sanger sequencing showed increased splicing inclusion of 135-nt and 76-nt cryptic exons within intron 2 and intron 10, respectively (Fig. 2b, d). In contrast, cells expressing wild-type *SLC20A2* minigenes without these mutations did not show detectable levels of abnormal transcripts in the above mutant minigene assays, implying these mutations likely alter the cryptic exon inclusion probability by affecting adjacent splicing regulatory elements.

Furthermore, by inserting the above 135-nt and 76-nt cryptic exons into the sequence positions corresponding to the N-terminal HA-tagged *SLC20A2* CDS, their impact on the protein product was further analyzed. According to sequence prediction, the inclusion of these two cryptic exons introduced premature termination codons (PTCs) in the new reading frame (Fig. 2g), thereby preventing the translation of functional PiT2 protein. In cultured Neuro-2A cells, transfection of HA-tagged *SLC20A2 minigene* constructs containing these two cryptic exons, or constructs containing neoteric exons generated by the other two de novo splice acceptors/donors, showed truncated PiT2 protein products by Western blot consistent with predictions (Fig. 2h). Empirically, due to the PTC mutations in these abnormal transcripts *in vivo,* triggering the NMD degradation mechanism for abnormal transcripts might not ultimately produce significantly expressed protein products. To this end, comparing with fibroblasts from healthy controls, a significant decrease in full-length PiT2 levels showed in fibroblasts derived from patients carrying the c.289+1007 C>G mutation (Fig. 2i). These findings collectively support a novel and important molecular genetic pathological mechanism: intron mutations in the SLC20A2 gene lead to increased inclusion of endogenous cryptic exons during splicing, causing *SLC20A2* haploinsufficiency and triggering cerebral calcification in mutation carriers from PFBC families.

### Example 2: Endogenous Cryptic Exons Exist in the Human Brain and Participate in Forming SLC20A2 Alternative Splicing Isoforms

*In vitro* evidence from the above Example suggests that the c.289+1007 C>G and c.1795-1698 A>G mutations have the ability to introduce cryptic exons during mRNA splicing in intron 2 and intron 10, and *in silico* analysis proved they were not predicted to create splice sites. An important question is whether these cryptic exons inherently exist in human cells, but under normal splicing conditions, these abnormal splicing isoforms produced at a lower proportion were degraded.

HEK293T cells (a kidney-derived cell line) and A172 cells (a brain-derived cell line) were used to explore whether potential splicing-regulated cryptic exons exist in each intron of *SLC20A2.* RT-PCR analysis of RNA transcripts in cells treated with CHX to inhibit degradation of abnormally spliced transcripts revealed cryptic exon inclusion events between exon 2 and exon 3 (c.289+901~c.289+1035, named "crpE2") in both HEK293T and A172 cells; and prominent cryptic exon inclusion events between exon 10 and exon 11 (c.1795-1703~c.1795-1628, named "crpE10") were observed in A172 cells (Fig. 3a, b). It is noted that the expression levels of these abnormal splicing transcripts containing cryptic exons crpE2 or crpE10 were sensitive to cycloheximide (CHX), a small molecule inhibitor used to assess potential involvement of the NMD pathway, suggesting that transcripts incorporating crpE2 or crpE10 are likely degraded via the NMD pathway in the brain under normal conditions. Sanger sequencing of crpE2 and crpE10 confirmed they were the 135-nt and 76-nt splicing products from minigenes carrying the c.289+1007 C>G and c.1795-1698 A>G mutations, respectively (Fig. 3b). These results indicate that the inclusion of these two cryptic exons indeed occurs naturally in human cells (and is markedly increased when the intron mutations c.289+1007 C>G and c.1795-1698 A>G are present).

RT-PCR analysis on a broader range of human cells (HeLa cells, HepG2 cells, HEK293T cells, A172 cells, and human dermal fibroblasts) further verified that splicing inclusion of crpE2 and crpE10 in SLC20A2 mRNA transcripts can occur in different cell types from various tissue origins (Fig. 3c), suggesting this splicing mechanism can be used in scenarios aimed at intervening in diseases by regulating PiT2 expression.

### Example 3: ASOs Can Correct crpE2- or crpE10-Involved Abnormal Splicing and Increase Production Efficiency of Functional PiT2 Protein Translation

To evaluate whether ASOs can inhibit cryptic exon-dependent alternative splicing, *SLC20A2* minigene constructs were transfected into Neuro-2a cells and co-transfected ASO formulations targeting the splice sites of crpE2 and/or crpE10 to suppress the production level of abnormally spliced transcripts. Among various ASO modifications, 2'-O-methoxyethyl (MOE) and phosphorodiamidate morpholino oligomers (PMO) were the most common modification used in approved neurological ASO drugs, and ASOs with these two types of modifications were used for this analysis. For minigenes containing the c.289+1007 C>G mutation, 4 µM PMO-ASOs (PMO-1, PMO-2, PMO-3) (PMO-1: 65.38 ± 3.081%; PMO-2: 67.17 ± 5.045%; PMO-3: 63.27 ± 3.078%) and 100 nM MOE-ASOs (MOE-A, MOE-B, MOE-C) (MOE-A: 99.64 ± 0.2714%; MOE-B: 99.34 ± 0.4810%; MOE-C: 99.76 ± 0.2441%) significantly reduced the degree of crpE2 splicing inclusion (Fig. 4a-c). For minigenes with the c.1795-1698 A>G mutation, crpE10 splicing inclusion was also greatly reduced by 4 µM PMO-ASOs (PMO-7, PMO-8, PMO-9) (PMO-7: 17.51 ± 2.599%; PMO-8: 18.58 ± 2.594%; PMO-9: 19.40 ± 2.619%) or 100 nM MOE-ASOs (MOE-G, MOE-H, MOE-I) (MOE-G: 36.76 ± 1.073%; MOE-H: 36.76 ± 1.073%; MOE-I: 36.76 ± 1.073%) (Fig. 4d-f). These results support the potential application value of ASO intervention to regulate PiT2 expression for treating cerebral calcification diseases.

To further test the effect of ASOs on correcting endogenous abnormal splicing, MOE-ASOs were transfected into human fibroblasts derived from PFBC patients carrying intron mutations. Dermal fibroblasts from a patient (P1) carrying a heterozygous *SLC20A2* c.289+1007 C>G mutation were firstly cultured. Compared to the vehicle group or control MOE-ASO (MOE-NC), MOE-A and MOE-B significantly removed crpE2 inclusion in *SLC20A2* mRNA (MOE-A: 14.35 ± 5.2645%; MOE-B: 15.62 ± 5.206%; MOE-C: 15.48 ± 5.200%) (Fig. 5a,b) and led to increased PiT2 protein levels (MOE-A: 21.24 ± 7.787%; MOE-B: 29.53 ± 10.26%; MOE-C: 6.632 ± 12.11%) (Fig. 5c). These findings support ASO-mediated inhibition of abnormal splicing as a feasible approach to rescue *SLC20A2* expression insufficiency in PFBC patients with intron mutations.

Considering that ASOs can modulate nonproductive alternative splicing of the wild-type allele and increase the level of normal SLC20A2 transcripts, to verify this issue from another perspective, fibroblasts from two healthy volunteer donors (C1, C2) were cultured. ASO cell intervention experiments showed (Fig. 6a-e) that MOE-A and MOE-B also exhibited splicing regulatory effects on *SLC20A2* mRNA and could upregulate PiT2 protein expression levels in healthy control fibroblasts (C1, C2). MOEs targeting crpE10 (MOE-J, MOE-K, MOE-L) also reduced crpE10 inclusion and increased PiT2 protein expression in wild-type fibroblasts (Fig. 6f-g). This result indicates that for autosomal dominant haploinsufficiency disease target genes, the normal gene on the other chromosome without pathogenic mutations can be regulated by ASO intervention to modulate its expression and achieve the goal of altering disease progression. Given the expanded practical application value, the screening of ASO sequences was expanded on fibroblasts from healthy controls to include new ASO sequences targeting crpE2 splicing regulation (cr5E2-m1, cr5E2-5, cr5E2-6, cr5E2-8, 5crE2-9, cr5E2-10, cr5E2-12, 5crE2-13, cr5E2-13L, cr5E2-13S, cr5E2-14, cr5E2-16, cr5E2-17, cr5E2-18, cr5E2-19, cr5E2-20, cr5E2-21, cr5E2-22, cr5E2-23, cr5E2-24, cr3E2-5, cr3E2-8, cr3E2-9, cr3E2-10, cr3E2-11, cr3E2-12, cr3E2-13, cr3E2-14, cr3E2-15, cr3E2-16), and new ASO sequences targeting crpE10 splicing regulation (cr5E10-9, cr5E10-11, cr5E10-12 ,cr5E10-13, cr5E10-15, cr5E10-16, cr5E10-19, cr3E10-9, cr3E10-14, cr3E10-15). Their sequences and related information are listed in Table 1. First, minigenes were transfected into N2A cells, and the response of minigene-expressed splicing products to ASOs was analyzed by PCR to filter out most sequences with poor effects (Fig. 7a, c). Next, to evaluate the effect of the remaining ASO sequences on regulating endogenous SLC20A2 splicing, ASOs were transfected into healthy human fibroblasts. After multiple rounds of PCR and protein WB detection (Fig. 7b,c), 7 ASO sequences were finally identified and showed relatively good effects in promoting SLC20A2 or PiT2 expression. They included ASO sequences targeting crpE2 splicing regulation: cr5E2-10, MOE-B (cr5E2-11), cr5E2-12, cr5E2-13, cr5E2-14, MOE-A (cr5E2-15), and the ASO sequence targeting crpE10 splicing regulation: cr5E10-12. Limited by detection conditions, this result cannot exclude that ASO sequences targeting other regions described in the patent may also have good effects; here only demonstrated the analytical process of the invention.

In PFBC disease, heterozygous dominant mutations in the SLC20A2 gene account for 60%, highlighting that ASO intervention can be used to rescue *SLC20A2* haploinsufficiency not limited to specific mutation forms (comprising intron mutations, exonic mutations, transcriptional regulatory region mutations, etc.). To test whether MOE-A and MOE-B can also rescue PiT2 expression in patients with non-intron mutations causing *SLC20A2* loss-of-function mutations, which constitute a larger population proportion, dermal fibroblasts from a patient (P2) were cultured with heterozygous genomic deletion covering a segment of *SLC20A2.* Compared to solvent or control ASO, MOE-A and MOE-B could largely reduce the production of endogenous crpE2-inserted *SLC20A2* mRNA (MOE-A: 2.024 ± 0.1775%; MOE-B: 2.267 ± 0.16915%; MOE-C: 2.386 ± 0.1278%) (Fig. 8a, b) and promote a significant increase in PiT2 protein (MOE-A: 15.99 ± 4.225%; MOE-B: 13.53 ± 4.951%; MOE-C: 11.25 ± 4.507%) (Fig. 8c). In summary, these results indicate that ASO-mediated correction of cryptic exon-dependent alternative splicing can rescue *SLC20A2* haploinsufficiency caused by intron mutations, and even other non-intron mutations.

### Example 4: Construction of Humanized SLC20A2 Intron Mouse Model (SLC20A2-KI) and Progressive Cerebral calcification Analysis

To evaluate whether ASO therapy can effectively intervene in the occurrence and progression of cerebral calcification at the animal level, a humanized mouse model was developed. Under design condition of maintaining reading frame-matching, the entire human intron 2 and flanking junction sequences carrying the *SLC20A2* c.289+1007 C>G mutation were knocked in (Fig. 9a), causing the transcripts of the chimeric SLC20A2 gene in the mouse model to contain the crpE2 cryptic exon, which can be bound and intervened by by ASO preparations designed for humans. Specifically, using CRISPR/Cas9 technology combined with simultaneously introduced targeting DNA fragment, the genomic fragment from exon 2 to exon 3 of the mouse *SLC20A2* gene was replaced with the a frame-matched human *SLC20A2* gene sequence carrying the c.289+1007 C>G mutation. Briefly, a targeting sequence with bilateral 1.4 kb homology arms was co-microinjected with sgRNAs and Cas9 RNA into the uterus of C57BL/6J mice. To screen for founder (F0) mice with the correct knock-in sequence, genomic DNA extracted from mouse tails was used for genotyping. Germline transmission of F0 KI mice was verified by crossing with C57BL/6J wild-type mice to produce F1 KI mice. To further verify targeted mice with correct homologous recombination, long-range sequencing and southern-blot analysis were performed on F1 mice. Heterozygous and homozygous *SLC20A2-KI* mice were selected for biochemical and phenotypic analysis.

In heterozygous and homozygous KI mice, RT-PCR and sequencing analysis observed significantly increased crpE2 inclusion in *SLC20A2* mRNA (Fig. 9b), accompanied by significantly decreased *SLC20A2* mRNA and PiT2 protein levels in these animals (Fig. 9c, d). Analysis of inorganic phosphate in cerebrospinal fluid showed that both heterozygous and homozygous *SLC20A2-KI* mice had significantly increased levels of inorganic phosphate (Pi) in the cerebrospinal fluid (CSF) (Fig. 9e), thereby metabolically recapitulating the brain Pi balance disturbance in *SLC20A2* mutated PFBC patients. This indicates successful application of the *SLC20A2* c.289+1007 C>G mutation in the construction of *SLC20A2* haploinsufficiency model.

Subsequently, micro-CT, RIS-AF647 fluorescent staining that recognizes calcification deposits (Fig. 10a, b), and histological staining (including alizarin red, Von Kossa, Alcian blue, periodic acid-Schiff (PAS), and H&E) were used to examine cerebral calcification in *SLC20A2-KI* mice. MicroCT scans showed (Fig. 10a) that approximately 71% of *SLC20A2-KI* mice began to develop distinct symmetrical cerebral calcifications in bilateral basal forebrain, hypothalamic, and dorsal midbrain regions at 5 months of age, which was 3 months later than that in *SLC20A2*-KO mice. At 7 months of age, calcification deposits in homozygous *SLC20A2-KI* mice were widely distributed in many brain regions, such as the basal forebrain, thalamus, hypothalamus, midbrain, and cerebellar dentate nucleus regions (Fig. 10a). Moreover, at 9.5 months of age, both the number and volume of calcified nodules continued to increase, with significant larger calcified deposits appearing in the thalamus, highly similar to PFBC patients. Using RIS-AF647 fluorescent staining that recognizes calcification deposits, calcification deposits in brain regions similar to those in microCT could also be seen (Fig. 10b), but fluorescently displayed calcification foci were clearer and suitable for quantitative statistics. Furthermore, histological staining analyses, including alizarin red, Von Kossa, Alcian blue, periodic acid-Schiff (PAS), and H&E, further showed that the calcification foci in the mouse model were highly consistent in morphology with human cerebral calcification deposits (Fig. 11), indicating they are driven by the same pathological mechanism. These results indicate that humanized *SLC20A2-KI* mouse model can recapitulate *SLC20A2* haploinsufficiency, brain Pi disturbance, and the cerebral calcification distribution pattern of mutated PFBC patients with *SLC20A2* mutations.

### Example 5: ASO Intervention Effectively Inhibits Cerebral calcification in SLC20A2-KI Mice

Given that mutations in the *SLC20A2* gene are the most common cause of PFBC, humanized *SLC20A2-*KI mouse model thus represents a valuable model for testing novel ASO-based therapies to treat cerebral calcification. We termed this Calcification Repression by ASO Corrected Expression (CRACE) therapy. Four doses of MOE-ASOs (50, 100, 200, 400 µg) were designed to assess whether single-dose ASO intracerebroventricular injection-mediated splicing correction could inhibit cerebral calcification in *SLC20A2-*KI mice before 3 months of calcification (Fig. 12a). Due to their significant efficacy in rescuing PiT2 expression in cell experiments, MOE-A and MOE-B (collectively referred to as therapeutic MOEs) were selected for *in vivo* administration.

Four months after a single 200 µg ICV injection of MOE-A, in situ hybridization using a probe specifically recognizing MOE-A showed a whole-brain distribution of ASO signals (Fig. 12b), indicating that MOEs can effectively diffuse and exert long-term effects in the brain. Regarding target gene expression, compared to *SLC20A2-*KI mice injected with control MOE, homozygous *SLC20A2-*KI mice injected with therapeutic MOEs showed a significant decrease in abnormal *SLC20A2* transcripts containing crpE2 inclusion and a clear increase in normal *SLC20A2* transcripts in the thalamus, and consequently, the level of the translation product PiT2 protein was also significantly increased (Fig. 12c, d, e). Simultaneously, after 4 months of intervention with 100 µg and 200 µg therapeutic MOE-ASOs, CSF Pi levels in *SLC20A2-*KI mice also significantly decreased (Fig. 12f), suggesting that abnormal brain phosphate homeostasis was restored.

Regarding cerebral calcification, four months after ICV administration of 100-400 µg therapeutic MOE-ASOs at 3 months of age, micro-CT scans of 7-month-old mouse brains showed significant reductions in both volume and number of cerebral calcification foci in the thalamus, hypothalamus, midbrain, and dorsal forebrain (Fig. 13a, b). Therefore, MOE-modified ASO-mediated splicing correction can prevent the occurrence of cerebral calcification in humanized *SLC20A2-*KI mice modeling PFBC.

To further evaluate the intervention effect of ASO ICV injection on mice that have already developed calcification, ASO ICV injection in 5-month-old homozygous *SLC20A2-*KI mice were performed. According to the results in Fig. 9, calcification had already appeared in the mouse brain at this time. Previous cell experiments showed that both MOE- and PMO-modified ASOs could restore SLC20A2 expression. In this animal experiment, 25 µg and 75 µg PMO-ASOs were used for 5-month-old homozygous *SLC20A2-*KI mice (Fig. 14a). At the end of PMO-1 administration, a significant increase in the expression level of normal *SLC20A2* transcripts in the thalamus of *SLC20A2-*KI mice was detected (Fig. 14b), and CSF Pi concentration also significantly decreased (Fig. 14c). Similarly, micro-CT showed that compared to PMO-NC, single intervention with 25 µg or 75 µg PMO-1 significantly reduced the distribution of cerebral calcification in 7-month-old *SLC20A2-*KI mice (Fig. 15d). Overall, these findings indicate that CRACE therapy can effectively increase PiT2 expression, restore brain Pi balance, and inhibit or reduce cerebral calcification in *SLC20A2-*KI mice. Therefore, these data and the invention support that ASO-mediated CRACE therapy can be used for cerebral calcification treatment in PFBC patients.

### Example 6: Expression Characteristics of Hereditary Cerebral Calcification-Related Genes in Mouse Brain

Given the abnormal brain Pi homeostasis found in PFBC patients with mutations in the Pi transporter genes *PIT2* or *XPR1,* the expression patterns of the *SLC20* sodium-phosphate co-transporter genes *Pit1* (*Slc20a1*) and *Pit2* (*Slc20a2*) and the Pi efflux protein gene *Xpr1* in the mouse brain were analyzed. Using publicly available mouse brain single-cell RNA-seq datasets (Source a: https://betsholtzlab.org/Publications/databases.html; Source b: http://celltypes.org/brain/), cell-type expression heatmaps for *Pit1, Pit2, Xpr1,* and the PFBC-related glycosidase *Myorg* were generated. The single-cell expression heatmaps suggested that both astrocytes and neurons show enriched expression of *Pit1, Pit2,* and *Xpr1,* implying active phosphate transport in both these cell types.

To validate the expression of Pi transporters in astrocytes and neurons, the RNAscope in situ hybridization (ISH) method was used to study the expression of *Pit1* and *Pit2* in adult mouse brains, focusing on regions known for calcification deposits in PFBC patients, such as the thalamus, cerebellum, striatum, and hypothalamus. Astrocytes were labeled by immunostaining of the marker protein S100b. In the cerebellum, Bergmann cells (a specific subtype of astrocytes) did not show *Pit1* ISH signal but displayed strong *Pit2* signal. In the thalamus, *Pit2* ISH signal was also highly expressed in all astrocytes, while only a few (<20%) astrocytes expressed lower-intensity *Pit1* signal. This preferential expression of *Pit2* over *Pit1* in astrocytes was consistent across all four brain regions and was confirmed by dual in situ hybridization with another astrocyte marker gene, *Aldh1l1.* Additionally, *Olig1*-positive oligodendrocytes and oligodendrocyte precursor cells (OPCs) also expressed *Pit2* but not *Pit1.*

In contrast, glutamatergic (*Vglut2+*) and GABAergic (*Gad1+*) neurons expressed strong *Pit1* and relatively weak *Pit2.* Considering that oligodendrocytes expressed mild levels of *Xpr1,* these results indicate a binary expression pattern: high expression of *Pit1* and *Xpr1* in neurons, and high expression of *Pit2* and *Xpr1* in astrocytes. Combined with the fact that 61% of reported PFBC patients have *PIT2* but no *PIT1* mutations, the discovery of preferential *Pit2* expression in astrocytes suggests the importance of astrocyte-mediated brain Pi homeostasis imbalance in the pathogenesis of cerebral calcification.

### Example 7: Preparation of Mouse Animal Models

*Slc20a2^{tm1aWtsi}* mice (MGI:4431725) were crossed with *Actin-Flp* mice (RRID: IMSR_JAX:005703) to remove the *En2a-LacZ-PGK-Neo* cassette, generating *Pit2* flox mice, or with *Ella-Cre* mice (RRID: IMSR_JAX:003724) to remove the *PGK-Neo-flox-Exon3* cassette, generating *Pit2* KO mice. *Pit1* (*Slc20a1*) flox mice were purchased from the KOAP engineered mouse collection (T013241, GemPharmatech, China). Subsequently, they were bred with wild-type C57BL/6 mice (SLAC Animals, China) for at least three generations. Conditional Cre mouse lines were obtained from: *Aldh1l1-CreER^{T2}* prepared by Baljit S. Khakh's lab (PMID: 27939582, RRID:IMSR_JAX:029655); *Plp1-CreER^{T2}* provided by Jia-Wei Zhou's lab (RRID:IMSR_JAX:005975); *Pdgfrb-Cre* prepared by Volkhard Lindner's lab (PMID: 21557454); *Camk2a-iCre* prepared by G. Schutz's lab (PMID: 11668676); and *Vgat-Ires-Cre* generated by Jax Mice Laboratory (IMSR_JAX:016962). Mouse genotyping was performed using Taq polymerase (Vazyme, China). Tamoxifen (20mg/ml in corn oil) was administered intraperitoneally at a dose of 1 mg/10g body weight, once daily for 5 consecutive days.

Animal care and experimental procedures were approved by the Animal Care and Use Committee of the Center for Excellence in Brain Science and Intelligence Technology, Chinese Academy of Sciences.

### Example 8: Impact of PiT2 Knockout in Astrocytes on Brain Phosphate Homeostasis

### 8.1 Serum, Cerebrospinal Fluid Preparation, and Pi Concentration Measurement

Blood was collected from the orbital sinus of adult mice and allowed to stand at room temperature for at least 1 hour, then centrifuged (2000xg, 15 minutes) to prepare serum. Cerebrospinal fluid (CSF) was collected from the cisterna magna of anesthetized adult mice and centrifuged for 5 minutes (2000xg, 4°C) to remove cellular debris. The concentrations of inorganic phosphate in serum and CSF were detected using the PiColorLock Free Inorganic Phosphate (Pi) Assay Kit (303-0030, Innova Biosciences). Absorbance was then read at 635 nm using a SpectraMax spectrophotometer (Molecular Devices), and Pi concentration was calculated based on a standard curve.

### 8.2 Plasmid Construction, Virus, and Delivery

The open reading frames (ORFs) of human *MYORG, PIT2,* or *XPR1* expression plasmids were derived from expression plasmids from Origene Company. For *in vivo* expression, PiT2 and XPR1 expression cassettes were inserted downstream of the *gfaABC1D* promoter in an AAV2 expression backbone. This expression plasmid was combined with a plasmid for the PHP.eB serotype capsid protein (Taitools, Inc, Shanghai) for AAV viruses packaging. For intraparenchymal injection, diluted AAV2/5 virus with an initial titer of 1x10¹³ was injected into the thalamic region of adult mice at a rate of 60 nL/min using a Nano-injector (Nanoject III, Drummond Scientific Company). For whole-brain re-expression in astrocytes, 100 µL of virus solution containing 2x10¹¹ of AAV5.PH.eB capsids was injected via the tail vein or retro-orbital sinus into each 8-week-old (50-56 days) mouse.

### 8.3 Immunostaining, Antibodies, and Microscopy Imaging

Mouse brain sections (20/25 µm) fixed with 4% PFA were first permeabilized with 1X PBS containing 0.5% Triton X-100, then blocked with PBS containing 0.1% Triton X-100 and 5% BSA, followed by incubation with corresponding primary antibodies (4°C) overnight. The next day, the brain sections were washed three times with PBS for 10 min each, incubated with secondary antibodies at room temperature for 2h, stained with Hoechst, washed 3×10 min in PBS, and finally fixed onto slides. Antibodies used included: mouse-anti-V5 (R96025, Invitrogen), Rat-anti-HA (11867423001, Roche), Mouse-anti-Calnexin (ab10286, Abcam), Chicken-anti-GFP (ab13970, Abcam), Rabbit-anti-AQP4 (AB3594, Millipore), Rabbit-anti-Col4 (ab6586, Abcam), Rabbit-anti-S100β (ab41548, ab52642, Abcam). Fluorescence images were acquired using an FV3000 confocal laser scanning microscope (Olympus). Parameters: 488/568/647 nm fluorescence channels, 40× or 60× objective lens, zoom factor 3-5, z-step 0.4 µm.

### 8.4 Micro-CT Imaging

Mouse brain samples fixed with 4% PFA were scanned using a high-resolution micro-CT scanner (Skyscan 1172, Bruker). Scanning parameters were as follows: source voltage 40 kV, current 250 µA, exposure time 210 ms; rotation step 0.6°; camera pixel size 9×9×9 µm. Images were reconstructed using the NRecon program supported by the GPUReconServer engine, outputting BMP format images (pixel size 5 µm). Reconstruction parameters: beam hardening correction 33%, ring artifact reduction 8, Gaussian smoothing kernel 2. The output BMP image series was then imported into Imaris software and converted to TIF series (pixel size 20 µm). Subsequently, the TIF image stacks were imported into ImageJ for positional alignment to output coronal and sagittal sections of mouse Micro-CT images. Finally, based on standard atlas coordinates, sagittal sections within 0.1-1.6 mm lateral to the midline (75 slices, 1.5 mm thickness) were superimposed to project a sagittal image of cerebral calcifications in that region. Additionally, coronal images of cerebral calcifications were projected from sections covering the hindbrain (75 slices, 1.5 mm thickness) or basal forebrain and hypothalamus (150 slices, 3 mm thickness).

### 8.5 Fluorescence Imaging of Calcification Deposits

Mouse brains after micro-CT scanning were frozen in OCT reagents and sectioned into 25 µm thick slices using a cryostat microtome. Brain slices selected from specific locations were incubated with 0.2 µM RIS-AF647 dye (BV500101, Biovinc, USA) for 5 h, then stained with Hoechst and fixed on glass slides. Calcification images were acquired using a VS120 slide scanner system (Olympus) in fluorescence mode with a 20× objective lens. For quantitative analysis of cerebral calcification, RIS-stained channels of VS120 images were batch-converted into single-channel grayscale TIF images. Within four defined regions of interest (ROI), the number and area of calcified nodules were analyzed using the "analyze particles" function in ImageJ software. Each slice was registered to corresponding lateral coordinates based on the sagittal brain region topology from the adult mouse brain atlas. Statistical analysis of the number and area of calcified nodules between groups was performed using GraphPad Prism.

### 8.6 Data Statistics

All data are presented as mean ± standard deviation; data presented as mean ± s.e.m. are noted in figure legends. Two-tailed t-test was used for statistical analysis between two groups; One-way ANOVA was used for multiple groups. * indicates p < 0.01, ** indicates p < 0.05, *** indicates p < 0.001, **** indicates p < 0.0001. For all tests, p < 0.05 (**) was considered statistically significant.

### 8.7 Results

The impact of PiT2 knockout in astrocytes on brain phosphate homeostasis is shown in Fig.15. Fig. 15A shows that CSF inorganic phosphate (Pi) levels were significantly elevated in PiT2 global knockout mice (about 1.8 mM) compared to WT mice (about 0.5 mM). Fig.15B shows no significant change in serum Pi levels in PiT2 global knockout mice. Fig.15C shows CSF Pi concentrations in different cell type-specific PiT2 conditional knockout mice. Astrocyte-specific conditional knockout (*Aldh1L1-CreER^{T2}*) mice showed the most pronounced increase (170%) in CSF Pi, reaching 1.35 mM. Other conditional knockout mice, comprising oligodendrocyte (*Plp1-CreER^{T2}*), excitatory neuron (*Camk2a-iCre*), inhibitory neuron (*Vgat-Cre*), and pericyte (*Pdgfrb-Cre*) mice, showed only moderate increases (40-60%) in CSF Pi. Fig.15D shows no significant differences in serum Pi concentrations among different conditional knockout mouse strains.

The results of - selective PiT2 expression in the brain astrocytes mediated by intravenous injection of AAV are shown in Figure 16. Immunofluorescence shows widespread expression of PiT2-V5 signal (red) in astrocytes throughout the mouse brain, with blue Hoechst staining the nuclei.

The inhibitory effect of AAV-mediated selective astrocyte compensatory expression on cerebral calcification in mice is shown in Figure 17. MicroCT images (coronal and sagittal views) of *Pit2* KO mice in the control group (Ctrl) at 5 and 7 months of age showed significant large area of calcification deposits in the dorsal midbrain, basal forebrain, thalamus, and hypothalamus. Calcification coverage area and particle size were greater in 7-month-old mice compared to 5-month-old mice. However, calcification was significantly inhibited in *Pit2* KO mice in therapeutic AAV injection group (Replenished) at both 5 and 7 months of age.

### 8.8 Discussion

Examples 5-8 analyzed the expression characteristics of homologous genes of specific hereditary cerebral calcification-related genes, particularly those involved in phosphate transport and metabolism, such as *PIT1, PIT2, XPR1,* and *MYORG,* in mouse brains using in situ hybridization. It was discovered that astrocytes exhibit selective high expression of specific gene combinations. Specifically, astrocytes highly express the Na-Pi co-transporter *Pit2* (*Slc20a2*) and *Xpr1,* but *Pit1* (*Slc20a1*) is not expressed or is expressed in very few astrocytes. Furthermore, significant elevation of CSF inorganic phosphate (Pi) levels was observed in mice with conditional knockout of the *Pit2* gene in astrocytes. Therefore, glial cells, particularly astrocytes, are confirmed as the cell type most closely associated with phosphate transport metabolism and the regulation of brain phosphate homeostasis in cerebral calcification disorders.

Based on the above cellular mechanism discovery, a method currently achievable for widespread infection of specific cell types throughout the brain-intravenous injection of AAV viruses-was employed. After penetrating the blood-brain barrier, these viruses can selectively express the compensatory human *PIT2* protein product in astrocytes within the mouse brain. It was further discovered that successful compensatory expression of *PIT2* in brain astrocytes significantly inhibited cerebral calcification in mice, indicating that restoring brain phosphate homeostasis is an effective strategy for inhibiting cerebral calcification, possessing outstanding clinical translational value.

Based on the world's first discovery of the important physiological mechanism of astrocyte-mediated regulation of brain phosphate homeostasis, and the finding in mouse models that expression compensation in astrocytes can effectively restore phosphate homeostasis in *Pit2* KO mouse brains and effectively inhibit the occurrence and progression of cerebral calcification, this approach can serve as an innovative therapy for cerebral calcification intervention, bringing hope to a broad population of PFBC patients.

### Appendix

**Table 1: ASO Sequences**

| ASO-ID | Sequence (5'-3') | Modification | Target |
|---|---|---|---|
| MOE-NC | GTCATCTCTACCTAGAGGCA (SEQ ID NO: 12) | MOE+PS | Negative control |
| SL20-cr5E2-m1 | TAGAGGCAAGCAGAAAAAGA (SEQ ID NO: 13) | MOE+PS | 5'-SA of crpE2 |
| SL20-cr5E2-5 | TCTACCTAGAGGCAAGCAGA (SEQ ID NO: 14) | MOE+PS | 5'-SA of crpE2 |
| SL20-cr5E2-6 | CTCTACCTAGAGGCAAGCAG (SEQ ID NO: 15) | MOE+PS | 5'-SA of crpE2 |
| SL20-MOE-C(7) | TCTCTACCTAGAGGCAAGCA (SEQ ID NO: 16) | MOE+PS | 5'-SA of crpE2 |
| SL20-cr5E2-8 | ATCTCTACCTAGAGGCAAGC (SEQ ID NO: 17) | MOE+PS | 5'-SA of crpE2 |
| SL20-cr5E2-9 | CATCTCTACCTAGAGGCAAG (SEQ ID NO: 18) | MOE+PS | 5'-SA of crpE2 |
| SL20-cr5E2-10 | TCATCTCTACCTAGAGGCAA (SEQ ID NO: 19) | MOE+PS | 5'-SA of crpE2 |
| SL20-MOE-B(11) | GTCATCTCTACCTAGAGGCA (SEQ ID NO: 20) | MOE+PS | 5'-SA of crpE2 |
| SL20-cr5E2-12 | CGTCATCTCTACCTAGAGGC (SEQ ID NO: 21) | MOE+PS | 5'-SA of crpE2 |
| SL20-cr5E2-13 | TCGTCATCTCTACCTAGAGG (SEQ ID NO: 22) | MOE+PS | 5'-SA of crpE2 |
| SL20-cr5E2-13L | TCGTCATCTCTACCTAGAGGCA (SEQ ID NO: 23) | MOE+PS | 5'-SA of crpE2 |
| SL20-cr5E2-13 S | TCGTCATCTCTACCTAGA (SEQ ID NO: 24) | MOE+PS | 5'-SA of crpE2 |
| SL20-cr5E2-14 | GTCGTCATCTCTACCTAGAG (SEQ ID NO: 25) | MOE+PS | 5'-SA of crpE2 |
| S120-MOE-A(15) | TGTCGTCATCTCTACCTAGA (SEQ ID NO: 26) | MOE+PS | 5'-SA of crpE2 |
| SL20-cr5E2-16 | GTGTCGTCATCTCTACCTAG (SEQ ID NO: 27) | MOE+PS | 5'-SA of crpE2 |
| SL20-cr5E2-17 | TGTGTCGTCATCTCTACCTA (SEQ ID NO: 28) | MOE+PS | 5'-SA of crpE2 |
| SL20-cr5E2-18 | TTGTGTCGTCATCTCTACCT (SEQ ID NO: 29) | MOE+PS | 5'-SA of crpE2 |
| SL20-cr5E2-19 | GTTGTGTCGTCATCTCTACC (SEQ ID NO: 30) | MOE+PS | 5'-SA of crpE2 |
| SL20-cr5E2-20 | TGTTGTGTCGTCATCTCTAC (SEQ ID NO: 31) | MOE+PS | 5'-SA of crpE2 |
| SL20-cr5E2-21 | CTGTTGTGTCGTCATCTCTA (SEQ ID NO: 32) | MOE+PS | 5'-SA of crpE2 |
| SL20-cr5E2-22 | TCTGTTGTGTCGTCATCTCTA (SEQ ID NO: 33) | MOE+PS | 5'-SA of crpE2 |
| SL20-cr5E2-23 | GTCTGTTGTGTCGTCATCTC (SEQ ID NO: 34) | MOE+PS | 5'-SA of crpE2 |
| SL20-cr5E2-24 | TGTCTGTTGTGTCGTCATCT (SEQ ID NO: 35) | MOE+PS | 5'-SA of crpE2 |
| SL20-cr3E2-5 | TACACCAGCATCTTACCTCCT (SEQ ID NO: 36) | MOE+PS | 3'-SD of crpE2 |
| SL20-cr3E2-8 | CCAGCATCTTACCTCCTGCT (SEQ ID NO: 37) | MOE+PS | 3'-SD of crpE2 |
| SL20-cr3E2-9 | CAGCATCTTACCTCCTGCTG (SEQ ID NO: 38) | MOE+PS | 3'-SD of crpE2 |
| SL20-cr3E2-10 | AGCATCTTACCTCCTGCTGC (SEQ ID NO: 39) | MOE+PS | 3'-SD of crpE2 |
| SL20-cr3E2-11 | GCATCTTACCTCCTGCTGCA (SEQ ID NO: 40) | MOE+PS | 3'-SD of crpE2 |
| SL20-cr3E2-12 | CATCTTACCTCCTGCTGCAG (SEQ ID NO: 41) | MOE+PS | 3'-SD of crpE2 |
| SL20-cr3E2-13 | ATCTTACCTCCTGCTGCAGG (SEQ ID NO: 42) | MOE+PS | 3'-SD of crpE2 |
| SL20-cr3E2-14 | TCTTACCTCCTGCTGCAGGA (SEQ ID NO: 43) | MOE+PS | 3'-SD of crpE2 |
| SL20-cr3E2-15 | CTTACCTCCTGCTGCAGGAC (SEQ ID NO: 44) | MOE+PS | 3'-SD of crpE2 |
| SL20-cr3E2-16 | TTACCTCCTGCTGCAGGACT (SEQ ID NO: 45) | MOE+PS | 3'-SD of crpE2 |
| SL20-MOE-J(6) | TAGGACCTTAATACATAATGA (SEQ ID NO: 46) | MOE+PS | 3'-SD of crpE2 |
| SL20-cr5E10-9 | TTTTAGGACCTTAATACATA (SEQ ID NO: 47) | MOE+PS | 5'-SA of crpe10 |
| SL20-MOE-K(10) | GTTTTAGGACCTTAATACAT (SEQ ID NO: 48) | MOE+PS | 5'-SA of crpE10 |
| SL20-cr5E10-11 | TGTTTTAGGACCTTAATACA (SEQ ID NO: 49) | MOE+PS | 5'-SA of crpE10 |
| SL20-cr5E10-12 | GTGTTTTAGGACCTTAATAC (SEQ ID NO: 50) | MOE+PS | 5'-SA of crpE10 |
| SL20-cr5E10-13 | CGTGTTTTAGGACCTTAATA (SEQ ID NO: 51) | MOE+PS | 5'-SA of crpE10 |
| SL20-MOE-L(14) | ACGTGTTTTAGGACCTTAAT (SEQ ID NO: 52) | MOE+PS | 5'-SA of crpE10 |
| SL20-cr5E10-15 | AACGTGTTTTAGGACCTTAA (SEQ ID NO: 53) | MOE+PS | 5'-SA of crpE10 |
| SL20-cr5E10-16 | CAACGTGTTTTAGGACCTTA (SEQ ID NO: 54) | MOE+PS | 5'-SA of crpE10 |
| SL20-cr5E10-19 | TCCCAACGTGTTTTAGGACCT (SEQ ID NO: 55) | MOE+PS | 5'-SA of crpE10 |
| SL20-cr3E10-9 | TGGTGATTTACCAAAGTCCA (SEQ ID NO: 56) | MOE+PS | 3'-SD of crpE10 |
| SL20-cr3E10-14 | TGATTTACCAAAGTCCATTGCA (SEQ ID NO: 57) | MOE+PS | 3'-SD of crpE10 |
| SL20-cr3E10-15 | TTTACCAAAGTCCATTGCAA (SEQ ID NO: 58) | MOE+PS | 3'-SD of crpE10 |
| MOE-G | ACGTGTTTCAGGACCTTAAT (SEQ ID NO: 59) | MOE+PS | 5'-SA of crpE10 |
| MOE-H | GTGTTTCAGGACCTTAAT (SEQ ID NO: 60) | MOE+PS | 5'-SA of crpE10 |
| MOE-I | GTTTCAGGACCTTAATACAT (SEQ ID NO: 61) | MOE+PS | 5'-SA of crpE10 |
| PMO-NC | CCTCTTACCTCAGTTACAATTTATA (SEQ ID NO: 62) | PMO | Negative control |
| PMO-1 | TCATCTCTACCTAGAGGCAAGCAGA (SEQ ID NO: 63) | PMO | 5'-SA of crpE2 |
| PMO-2 | CATCTCTACCTAGAGGCAAGCAGAA (SEQ ID NO: 64) | PMO | 5'-SA of crpE2 |
| PMO-3 | ATCTCTACCTAGAGGCAAGCAGAAA (SEQ ID NO: 65) | PMO | 5'-SA of crpE2 |
| PMO-7 | TTCAGGACCTTAATACATAATGACA (SEQ ID NO: 66) | PMO | 5'-SA of crpE10 |
| PMO-8 | GTTTCAGGACCTTAATACATAATGA (SEQ ID NO: 67) | PMO | 5'-SA of crpE10 |
| PMO-9 | TTTCAGGACCTTAATACATAATGAC (SEQ ID NO: 68) | PMO | 5'-SA of crpE10 |

**Table 2: PFBC Family Information**

| | Family 1 | Family 2 | Family 3 | Family 4 | Family 5 |
|---|---|---|---|---|---|
| gene | SLC20A2 | | | | |
| variation (gDNA) | chr8:42328613 G>C | chr8:42328683 C>T | chr8:42328683 C>T | chr8:42301396 G>A | chr8:42277183 T>C |
| variation (cDNA, NM 006749.4) | c.289+1007 C>G | c.289+937 G>A | c.289+937 G>A | c.730+768 C>T | c.1795-1698 A>G |
| genotype | Heterozygous | Heterozygous | Homozygous | Heterozygous | Heterozygous |

| location | Intron 2 | Intron 2 | Intron 2 | Intron 6 | Intron 10 |
|---|---|---|---|---|---|
| SpliceAI | 0.11 | 0.5 | 0.5 | 0.72 | 0.33 |
| gnomAD genomes (all) | Not present | Not present | Not present | Not present | Not present |
| gnomAD exomes (all) | Not present | 8.10045e-06 (1/123450) | 8.10045e-06 (1/123450) | Not present | Not present |
| ExAC | Not present | Not present | Not present | Not present | Not present |
| 1000 Genomes | Not present | Not present | Not present | Not present | Not present |
| Symptom | Headache | Dizziness | Headache | Headache | Headache |
| Serum calcium(mmol/L) | NA | 2.25 | NA | 2.51 | 2.35 |
| Serum phosphate(mmol/L) | NA | 1.00 | NA | 1.13 | 1.29 |
| PTH (pmol/L) | NA | 6.69 | NA | 3.19 | 3.47 |
| TCS | 16 | 39 | 34 | 22 | 16 |

**Table 3: Primer Sequences and sgRNA Sequences**

| **Name** | **Sequence (5'-3')** | **Application** |
|---|---|---|
| **E2E3-mini-fragment-1** | F: TAGGGCGGCCGGGAATTGGATCACAGGAGGAGACATCACAG (SEQ ID NO: 69) | Minigene construction E2-I2-E3 |
| | R: GATATGTCCTGTTGAAAGCAG (SEQ ID NO: 70) | |
| **E2E3-mini-fragment-2** | F: GCTTTCAACAGGACATATCCTCTTG (SEQ ID NO: 71) | Minigene construction E2-I2-E3 |
| | R: CGACCTAGGGAAAGTGGGGA (SEQ ID NO: 72) | |
| **E2E3-mini-fragment-3** | F: CCCCACTTTCCCTAGGTCGT (SEQ ID NO: 73) | Minigene construction E2-I2-E3 |
| | R: GCGATCGCAGATCCTTGCGGCCTTACCAATCTTGACAAGCTCCAT (SEQ ID NO: 74) | |
| **E2E3-mini-mut1** | F: CTTTCAACAGGACATATCGTCTTGTAGGAAGAGTCCTGC (SEQ ID NO: 75) | Introduce mutationE2-I2-E3 (c.289+1007 C>G) |
| | R: CAAGACGATATGTCCTGTTGAAAGCAGAAGGTGGTAGAAT (SEQ ID NO: 76) | |
| **E2E3-mini-mut2** | F: GTATACAGGTAATTTGGTTAGGGAGAAGAGGGTTAGAG (SEQ ID NO: 77) | Introduce mutationE2-I2-E3 (c.289+937 G>A) |
| | R: CTAACCAAATTACCTGTATACATCAGCTGTCTGTTGTGTCGTC (SEQ ID NO: 78) | |
| **E2E3-mini-mut3** | F: CTTGTAGGAAGACTCCTGCAGCAGGAGGTAAGATGCTGGT (SEQ ID NO: 79) | Introduce mutationE2-I2-E3 (c.289+1021 G>C) |
| | R: TGCAGGAGTCTTCCTACAAGAGGATATGTCCTGTTGAAAGC (SEQ ID NO: 80) | |
| **E6E7-mini-fragment-1** | F: TGCTCGGCCTTGTTCTCCC (SEQ ID NO: 81) | Minigene construction E6-I6-E7 |
| | R: AAATGCCAGCATGCAGAATAGG (SEQ ID NO: 82) | |
| **E6E7-mut-mini-fragment-1** | F: TGCTCGGCCTTGTTCTCCC (SEQ ID NO: 83) | Minigene construction E6-I6-E7 (c.730+768 C>T) |
| | R: AAAAATACCAGCATGCAGAATAGGTGC (SEQ ID NO: 84) | |
| **E6E7-mini-fragment-2** | F: CCTATTCTGCATGCTGGCATTT (SEQ ID NO: 85) | Minigene construction E6-I6-E7 |
| | R: GCATGTGGCTTCAAGGCTTG (SEQ ID NO: 86) | |
| **E6E7-mut-mini-fragment-2** | F: CCTATTCTGCATGCTGGTATTT (SEQ ID NO: 87) | Minigene construction E6-I6-E7 (c.730+768 C>T) |
| | R: GCATGTGGCTTCAAGGCTTG (SEQ ID NO: 88) | |
| **E6E7-mini-fragment-3** | F: CAAGCCTTGAAGCCACATGC (SEQ ID NO: 89) | Minigene construction E6-I6-E7 |
| | R: TCTTGTTGCCCAAGGGGAGTG (SEQ ID NO: 90) | |
| **E6E7-mini-fragment-4** | F: ACTCCCCTTGGGCAACAAGA (SEQ ID NO: 91) | Minigene construction E6-I6-E7 |
| | R: TCTTGACCTCGTGAACTGCTCG (SEQ ID NO: 92) | |
| **E6E7-mini-fragment-5** | F: AGCAGTTCACGAGGTCAAGAA (SEQ ID NO: 93) | Minigene construction E6-I6-E7 |
| | R: GGGTCAGATCCGAATACCACAG (SEQ ID NO: 94) | |
| **E6E7-mini-fragment-6** | F: GAACTGTGGTATTCGGATCTGACCC (SEQ ID NO: 95) | Minigene construction E6-I6-E7 |
| | R: CGTATGCAGCCCGAGGGTG (SEQ ID NO: 96) | |
| **E2E3-135bp-fragment-1** | F: ATGTACCCATACGATGTTCCAGATTACGCTGCCATGGATGAGTAT (SEQ ID NO: 97) | cDNA plasmid construction E2E3 +135bp |
| | R: GTCTGTTGTGTCGTCATCTCTACCAACCATGGCACTAACT (SEQ ID NO: 98) | |
| **E2E3-135bp-fragment-2** | F: AGTTAGTGCCATGGTTGGTAGAGATGACGACACAACAGAC (SEQ ID NO: 99) | cDNA plasmid construction E2E3 +135bp |
| | R: CAGCTGCCACACAGCGGAACCTCCTGCTGCAGGACTCTTC (SEQ ID NO: 100) | |
| **E2E3-135bp-fragment-3** | F: GAAGAGTCCTGCAGCAGGAGGTTCCGCTGTGTGGCAGCTG (SEQ ID NO: 101) | cDNA plasmid construction E2E3 +135bp |
| | R: GAGCGGCCGCGTACGCGTCACATATGGAAGGATCC (SEQ ID NO: 102) | |
| **E2E3-38bp-fragment-1** | F: ATGTACCCATACGATGTTCCAGATTACGCTGCCATGGATGAGTAT (SEQ ID NO: 103) | cDNA plasmid construction E2E3 +38bp |
| | R: AACCTGTATACATCAGCTGTCTGTTGTGTCGTCATCTCTACCAACCATGGCACTAACTT (SEQ ID NO: 104) | |
| **E2E3-38bp-fragment-2** | F: GGTAGAGATGACGACACAACAGACAGCTGATGTATACAGGTTCCGCTGTGTGGCAGCT (SEQ ID NO: 105) | cDNA plasmid construction E2E3 +38bp |
| | R: GAGCGGCCGCGTACGCGTCACATATGGAAGGATCC (SEQ ID NO: 106) | |
| **E6E7-190bp-fragment-1** | F: ATGTACCCATACGATGTTCCAGATTACGCTGCCATGGATGAGTAT (SEQ ID NO: 107) | cDNA plasmid construction E6E7 +190bp |
| | R: TGGGTTGTTTCCACTTTGGGCTGTTATTTTCCTCCGCATC (SEQ ID NO: 108) | |
| **E6E7-190bp-fragment-2** | F: GATGCGGAGGAAAATAACAGCCCAAAGTGGAAACAACCCA (SEQ ID NO: 109) | cDNA plasmid construction E6E7 +190bp |
| | R: ACCTTCTTTTTGTAATTTGCCAGCATGCAGAATAGGTGCT (SEQ ID NO: 110) | |
| **E6E7-190bp-fragment-3** | F: AGCACCTATTCTGCATGCTGGCAAATTACAAAAAGAAGGT (SEQ ID NO: 111) | cDNA plasmid construction E6E7 +190bp |
| | R: GAGCGGCCGCGTACGCGTCACATATGGAAGGATCC (SEQ ID NO: 112) | |
| **5'-sgRNA-7** | CACCGCGGTTCCGAAGGAAT (SEQ ID NO: 113) | Slc20a2-KI mouse construction |
| **3'-sgRNA-10** | TCGGGGACTCACTGCATCGT (SEQ ID NO: 114) | Slc20a2-KI mouse construction |
| **SLC20A2-KI-genotype-F0** | F: CAGGTCCTGCCTCCTCTTGTGAATG (SEQ ID NO: 115) | Genotyping of mutant Slc20a2-KI (F0) mice |
| | R: GGGTTTCGAGGAGCTGGCTCGTTT (SEQ ID NO: 116) | |
| **5'-probe** | ACCGTAACCAAACGAACTGTACCGACAGTAGTTTGAAAGCCAGCAGTTAGCAGTTTC TTCAGACTTCAACACTGTCCAGCGCTTTCCAGAGCAGACTCACTGTTTACAAGAACT CTTGGCCTCCCGAAGTTAGCAACCTCAGATTTTGTTTATTTAAAGAATTTCTGTGAAA GAAAAGGACCCCAGAGCCCATTTTCCAAAATGGCCATGGACGGGTATCTGTGGATGG TCATTTTGGGTTTCATTATTGCTTTCATCCTGGCGTTTTCAGTCGGTGCCAACGATGTT GCCAAT (SEQ ID NO: 117) | Southern-blot analysis of Mutant Slc20a2-KI (F1) mice |
| **3'-probe** | CTTCACTGAGTATTCCTTTCATTCCTTCAGTCGCCTCCTGGTTTATATCGCCACTTCTTT CTGGCTTCATGTCCGGCGTGCTGTTCATACTAATCAGAATGTTCATCCTTACTAAGGTG AGTGTGGCGGCTTCTCTGGTTTCTGGGTTGTACTAAGTTTTAGAACTGATTCTGGTTCT ACTCAGTAGAAGCTAGTACCAGTACCAGTGGCTCGGCAGGAGACCTGTACTTTCATTG CCAGGACTCACCTGGAGTTTGAGTCACGGGTGTTCAGGAAGGGTCAGAAGCCTCAGG GAACGGCCAAGACTCTCCCGGGCCTTGCACACAAGGGCGCTTCCAGCCAGGCTCTATA ATTTACTTCGTTAGCCCAAGCTCCCAGAAATTGGGCTTAGACCTCAAGAATGAGGTGAT TATTTCCTGGTTGTCAA (SEQ ID NO: 118) | Southern-blot analysis of Mutant Slc20a2-KI (F1) mice |
| **SLC20A2-KI-genotype-WT** | F: CCCGAAGTTAGCAACCTCAGATTT (SEQ ID NO: 119) | Genotyping of wild-type alleles in Slc20a2-KI (F1) mice |
| | R: TCCCACTGGTGCCAATGGAGG (SEQ ID NO: 120) | |
| **SLC20A2-KI-genotype-Mut** | F: CCCGAAGTTAGCAACCTCAGATTT (SEQ ID NO: 121) | Genotyping of the mutant allele in Slc20a2-KI (F1) mice |
| | R: AAATGTGAAACATCCAACCAG (SEQ ID NO: 122) | |
| **E2E3-minigene-RT** | F: GCGGCCGGGAATTGGATC (SEQ ID NO: 123) | Minigene splice analysis |
| | R: TCAGCTGCCACACAGCGGAA (SEQ ID NO: 124) | |
| **E6E7-minigene-RT** | F: CGGGAATTGGATCCTGCTC (SEQ ID NO: 125) | Minigene splice analysis |
| | R: ACCTTACTGAGGCTTTCGTC (SEQ ID NO: 126) | |
| **E10E11-minigene-RT** | F: CGGCCGGGAATTGGATCC (SEQ ID NO: 127) | Minigene splice analysis |
| | R: AGGCCACGAAGATGTTCCGA (SEQ ID NO: 128) | |
| **E2E3-mouse-RT** | F: TGGCCATGGACGGGTATCT (SEQ ID NO: 129) | Slc20a2-KI mouse splice analysis |
| | R: GTATGAACAGCACGCCGGA (SEQ ID NO: 130) | |
| **Ms-Pit2-qPCR** | F: CCTTACTAAGGAGGACCCTG (SEQ ID NO: 131) | qPCR analysis of Ms Pit2 |
| | R: TGGACACACGAATAGCCACA (SEQ ID NO: 132) | |
| **E1E2-endogeneous-RT** | F: CTCGGCCTAATGTGGTAGGAT (SEQ ID NO: 133) | Endogenous splice analysis |
| | R: GGGAAAGCTGTGATGTCTCCTC (SEQ ID NO: 134) | |
| **E2E3-endogeneous-RT** | F: CACAGCTTTCCCAGATCGGG (SEQ ID NO: 135) | Endogenous splice analysis |
| | R: TCAGCTGCCACACAGCGGAA (SEQ ID NO: 136) | |
| **E3E4-endogeneous-RT** | F: AGGATTCTCACTGGTCGCAATC (SEQ ID NO: 137) | Endogenous splice analysis |
| | R: GCAGGCCAGACATGAAACCA (SEQ ID NO: 138) | |
| **E4E5-endogeneous-RT** | F: TTTCATGTCTGGCCTGCTG (SEQ ID NO: 139) | Endogenous splice analysis |
| | R: GGTAGCAGCATAGAATACTGGG (SEQ ID NO: 140) | |
| **ESE6-endogeneous-RT** | F: TCCGGGCACTCCCAGTATTC (SEQ ID NO: 141) | Endogenous splice analysis |
| | R: TGAGGGCTATGGCCCACAT (SEQ ID NO: 142) | |
| **E6E7-endogeneous-RT** | F: GCCATAGCCCTCATTTCCTT (SEQ ID NO: 143) | Endogenous splice analysis |
| | R: ACTCGTGATAAAGCACCTTCT (SEQ ID NO: 144) | |
| **E7E8-endogeneous-RT** | F: GGTGCCAAGGCTAATGATGAC (SEQ ID NO: 145) | Endogenous splice analysis |
| | R: TGGGTCATGGACAGTGCTCT (SEQ ID NO: 146) | |
| **E8E9-endogeneous-RT** | F: CTGTTTCGGGTCCTTTGCTCA (SEQ ID NO: 147) | Endogenous splice analysis |
| | R: ATAAAACAGCAGCCAGACGG (SEQ ID NO: 148) | |
| **E9E10-endogeneous-RT** | F: TGATCCAGACCATGGGGAA (SEQ ID NO: 149) | Endogenous splice analysis |
| | R: TGACTGGAAGCCCGATGTT (SEQ ID NO: 150) | |
| **E10E11-endogeneous-RT** | F: TCAGCACCACGCACTGTAAG (SEQ ID NO: 151) | Endogenous splice analysis |
| | R: CCACAGGGACGGTCACGAA (SEQ ID NO: 152) | |

**Table 4: Other Sequences**

| SEQ ID NO: | Description | Sequence (5'-3') |
|---|---|---|
| 1 | Mature *SLC20A2* mRNA with normal splicing | AGCGTGTTCCTGCCTGCTGCTTGGCTGCAGTTAATTTTAGCCGGGGTTAGTTCTATGAGTTGTAAAAGTAGAAGTTAGCAA GCTGAAATGTGACTTCTCGGCCTAATGTGGTAGGATATCTATTTTAGACTTCAGATTGTGTTTGTGATCAGGAGAGCAGTCT TCTGAAGAATTGTGGCTCAGTTTTCTTGGAAACTACTTGAGGAGACATCACAGCTTTCCCAGATCGGGAGGAAAAATATGG AATGTGTTTTACCGCTGACTGAACACAACCAAATGAACTGTCCTGACAGTAGTTTGCAAACCAGCAGCTAGCAGTTTGTCC AGCCTCTAACATTGTCCAGCACTTTCCAGAGCAAACTCACTGTTTACAAGAACTCTTGGCCTTACGAAGTTTATAACCTCA AGCTTTGTTTATTTAAAATATTCCTGCAAAAGAAAAGTACCCGGCACCCACTTTCCAAAATGGCCATGGATGAGTATTTGTG GATGGTCATTTTGGGTTTCATCATAGCTTTCATCTTGGCCTTTTCTGTTGGTGCAAACGATGTTGCCAACTCCTTTGGTACAG CCGTGGGCTCTGGTGTGGTGACCTTGAGGCAGGCATGCATTTTAGCTTCAATATTTGAAACCACCGGCTCCGTGTTACTAG GCGCCAAAGTAGGAGAAACCATTCGCAAAGGTATCATTGACGTGAACCTGTACAACGAGACGGTGGAGACTCTCATGGCT GGGGAAGTTAGTGCCATGGTTGGTTCCGCTGTGTGGCAGCTGATTGCTTCCTTCCTGAGGCTTCCAATCTCAGGAACGCAC TGCATTGTGGGTTCTACTATAGGATTCTCACTGGTCGCAATCGGTACCAAAGGTGTGCAGTGGATGGAGCTTGTCAAGATT GTTGCTTCTTGGTTTATATCTCCACTGTTGTCTGGTTTCATGTCTGGCCTGCTGTTTGTACTCATCAGAATTTTCATCTTAAAA AAGGAAGACCCTGTTCCCAATGGCCTCCGGGCACTCCCAGTATTCTATGCTGCTACCATAGCAATCAATGTCTTTTCCATCA TGTACACAGGAGCACCAGTGCTCGGCCTTGTTCTCCCCATGTGGGCCATAGCCCTCATTTCCTTTGGTGTCGCCCTCCTGTT CGCTTTTTTTGTGTGGCTCTTCGTGTGTCCGTGGATGCGGAGGAAAATAACAGGCAAATTACAAAAAGAAGGTGCTTTATC ACGAGTATCTGACGAAAGCCTCAGTAAGGTTCAGGAAGCAGAGTCCCCAGTATTTAAAGAGCTACCAGGTGCCAAGGCTA ATGATGACAGCACCATCCCGCTCACGGGAGCAGCAGGGGAGACACTGGGGACCTCGGAAGGCACTTCTGCGGGCAGCCA CCCTCGGGCTGCATACGGAAGAGCACTGTCCATGACCCATGGCTCTGTGAAATCGCCCATCTCCAACGGCACCTTCGGCTT CGACGGCCACACCAGGAGCGACGGTCATGTGTACCACACCGTGCACAAAGACTCGGGGCTCTACAAAGATCTGCTGCAC AAAATCCACATCGACAGGGGCCCCGAGGAGAAGCCAGCCCAGGAAAGCAACTACCGGCTGCTGCGCCGAAACAACAGTT ACACCTGCTACACCGCAGCCATTTGTGGGCTGCCAGTGCACGCCACCTTTCGAGCTGCGGACTCATCGGCCCCAGAGGAC AGTGAGAAGCTGGTGGGCGACACCGTGTCCTACTCCAAGAAGAGGCTGCGCTACGACAGCTACTCGAGCTACTGTAACGC GGTGGCAGAGGCGGAGATCGAGGCGGAGGAGGGCGGCGTGGAGATGAAGCTGGCGTCGGAGCTGGCCGACCCTGACCA GCCGCGAGAGGACCCTGCAGAGGAGGAGAAGGAGGAGAAGGACGCACCCGAGGTTCACCTCCTGTTCCATTTCCTGCAG GTCCTCACCGCCTGTTTCGGGTCCTTTGCTCACGGCGGCAATGACGTGAGTAATGCCATCGGTCCCCTGGTAGCCTTGTGG CTGATTTACAAACAAGGCGGGGTAACGCAAGAAGCAGCTACACCCGTCTGGCTGCTGTTTTATGGAGGAGTTGGAATCTG CACAGGCCTCTGGGTCTGGGGGAGAAGAGTGATCCAGACCATGGGGAAGGACCTCACTCCCATCACGCCGTCCAGCGGC TTCACGATCGAGCTGGCCTCAGCCTTCACAGTGGTGATCGCCTCCAACATCGGGCTTCCAGTCAGCACCACGCACTGTAA GGTGGGCTCGGTGGTGGCCGTGGGCTGGATCCGCTCCCGCAAGGCTGTGGACTGGCGCCTCTTTCGGAACATCTTCGTGG CCTGGTTCGTGACCGTCCCTGTGGCTGGGCTGTTCAGCGCTGCTGTCATGGCTCTTCTCATGTATGGGATCCTTCCATATGT GTGATTTGTCTTCTTCCAGCTGCAAACAGCTAAAGGGATGGTCTGGTGTTGGCGTGTGGGAGACATGTGTGCTCGTGCCGC ACATACACATCCTGGCCGTGCACGGCTCTCTCATGACCAGCTCTCTGCCTCCCTTCCAGGAGGCTCCATCCCACACTGTTC ACCCAGGCTGCGGAGACTCACCTTCCCGAGCTAACTTAACTACTGTACATAATAATATGTATTAAACTGGTATCGTGGTGATA TAATGTGGTGCAGTTACTTATATATTAAATATCTATTGTATCCATAGAATAGGCAGCATTATTTCAAACATATTCAAGTTGGGA GTGGAGATCATTGCCTAGAAGTCAATATTCAATAAATCTTGTACATAACTATTTCGATGGCAAATGTTAAGCCTTCTAAAAGG AAAGTGTAGATTGGAAAATGATTTTTTTTCCAAATGATGTTTTTGCCTTCTAATATACTGTAAGGTAATGAGCTTCAGAACA GGCAACCTGACCCTGCAGAGGTCGCGTGCTGTGGGATGACAGCGGGACGGGAGCTCACAAGTGCTTTCACTGAAGATTT GTTCATATACTGTGTATTGATTGTTGTGTAATATATCATCATTGCTTTTGTAAATACGTAAAACTGTAATTTTTTAATGGTGTGC TTCCCTTATACTTTTTTGATCAGAGAATTTTGGAAAGTACCAAAGAAGCAGGGGAATCATTGGCCAGTGTTACGTTTTCACA TTGTCTGTCTCCCACCCTCACTGATCACGCCTGCCCCAGAGCAGTGTGTGGCGGTGACACCGTCACCCAGCATGCGCCAC GCCGTGGCTCCCACCAGCAGTGCCACCGCCACCACACCCCAGATCCCACCCACCTTGCAGTGGCCTTTCCTTGTCATCAG AGTAGAGAATGCACAGGTGTTGGTGAGGGCGTGTGGCTGAGCACTACATGTCAAGTCCAGAGTCAGTTTCTATCCCAATTC TCCCTGCAGCCTGAAGAACGGATCCTTGTCTCCAATGTCAGCACAAAGGAGGCTTTTTCTGTGCTTTGACATTCTAGCACT TCAGGGATGAGAGGGAGGGAGAATCCTGGATGCTGGATGGAGTATTTCTCTGAGGCCCACACAAAGCTGGACACCCCCAG GCTCTACTCCATCCCATTGGAGTCTCTTCTTTTTTTGATAGCGGGAGGGAGGAAGTACGACTAATGTTGGAGCCTGAAACTA TGGAAATGCTGCTAAAATTTTTATATTGACAAACATTTTCTTGGTACTTCATTGTCATTTTTCATTAATCAACCATATTAAATTT ATAATAAAAAATGCCCCTCAGAA |
| 2 | Cryptic exon of event crpE2 (135-nt) | GTAGAGATGACGACACAACAGACAGCTGATGTATACGGGTAATTTGGTTAGGGAGAAGAGGGTTAGAGAGGAATTCT ACCACCTTCTGCTTTCAACAGGACATATCCTCTTGTAGGAAGAGTCCTGCAGCAGGAG |
| 3 | Cryptic exon of event crpE10 (76-nt) | GTCCTAAAACACGTTGGGAGAGGAAAGAGCTCTTCCTTGAAGTAGAACTCCATGTGAGAAATTGCAATGGACTTTG |
| 4 | Minigenes construct for event crpE2 | GAGGAGACATCACAGCTTTCCCAGATCGGGAGGAAAAATATGGAATGTGTTTTACCGCTGACTGAACACAACCAAAT GAACTGTCCTGACAGTAGTTTGCAAACCAGCAGCTAGCAGTTTGTCCAGCCTCTAACATTGTCCAGCACTTTCCAGA GCAAACTCACTGTTTACAAGAACTCTTGGCCTTACGAAGTTTATAACCTCAAGCTTTGTTTATTTAAAATATTCCTGCA AAAGAAAAGTACCCGGCACCCACTTTCCAAAATGGCCATGGATGAGTATTTGTGGATGGTCATTTTGGGTTTCATCAT AGCTTTCATCTTGGCCTTTTCTGTTGGTGCAAACGATGTTGCCAACTCCTTTGGTACAGCCGTGGGCTCTGGTGTGGT GACCTTGAGGCAGGCATGCATTTTAGCTTCAATATTTGAAACCACCGGCTCCGTGTTACTAGGCGCCAAAGTAGGAG AAACCATTCGCAAAGGTATCATTGACGTGAACCTGTACAACGAGACGGTGGAGACTCTCATGGCTGGGGAAGTTAGT GCCATGGTTGGTGAGTAGCCTCTTCTCTATACCGATGGGACATCCGCCCACTGACCCGCTTCCCTGCAGTACTTTGCC CATATCCATAAATGTGATTTTTGCTTTGGCTTTTCAAAAACTGGTTGGATGTTTCACATTTACTCTCTTATTTTTGCTTTC TGCATATTAATTTATGATTTTTAAAGTGTTTAAATATCAGGCAACCATTTATTTTCTGAACTTTTGAGCAAACTTGGTTG CTAAAATAGATTTCAGCTAAGTGAATACTTTTCAGAAAGTACAGAAAAAGACCTTTGTAGAACAGCGCTTTATTATTT TAGAGTTGTTTGAAGGTTGTCTAAGTGAATGTGTTTTCAAGGTTGATGTATGAAATTTCCAGGAAGGTTGATTTAGAT TTTTTTCTTAAGGTTTTTCATCAATTAGGAGGCAATGTTTTGAAAACTTGGATGTCAAAAATAATATTTATTAGTCTAAC TTTTATATTCACAGTTTAAGTTCTAAAGAAGTTACCAAATAAATCTTAAGTGCGTGTGTTTCAAGAAATTCTGGTCTCT TACATTATTTGTCATCCTTTTGTTGGCTTTTGTTTTGATTTTCTGATTCTACCTCTGGGAGCTCCATTTGGTATGTTTGTT TTGTTTTCCGGCCAAAAAATAATGTGCGTGTTGCCTGGTGATAGCATGACCTTTTATTGCAGTTTCAAGTTGTCTACAT GAAGTGTGGAGGTGTGTGAACTGTGTGAATAAGCAGAGTGGCTGGTTGGACAGAGTGATGTCCCCATGGCCTAAGC GGACACAGATCTCGGCCCCGTGGGTCTTCCCATCACTGCCCTCGCTACGACAGAAAAGAACCTTCCCGGCAGGCGT GTGTTAAGTGCAGCTGACAGGATTCTTTTTCTGCTTGCCTCTAGGTAGAGATGACGACACAACAGACAGCTGATGTAT ACGGGTAATTTGGTTAGGGAGAAGAGGGTTAGAGAGGAATTCTACCACCTTCTGCTTTCAACAGGACATATCCTCTTG TAGGAAGAGTCCTGCAGCAGGAGGTAAGATGCTGGTGTACTAAGAATACAGCCACATTCCCCCGAGTTCTTTATTTTG TCACTTACATTTTTTCTTTTCTTTTTTTTTTTTTTTTTGAGACAGAGTCTTGCTCTGTGGCCCAGGCTGGAGTGCAATA GTGTGATCTCCGCTCACTGCCACCTCCACCTCCTGGGTTCAAGCTATTCTCTCACCTCACCCTCCGGAGTAGCTGAGA CTAAAGGCGTGTGCCACCGCACCTGGCTAATTTTTGTATTTTTAGTAGAGACAGGGTTTCACCATGTTGGCCAGGCTA |
| | | GTCTTGAACTCCTGACCTCAAGCAATCCACCCACCATCACCTCTCAAAGTGCTGGGATTACAGGCGTGAGCCACCAC ACCTGGCCTGTCACATTTCTTAAACACCTGGAATTTAGAGATTATTGACCAAGTTCACAATGGCCCAATCTCATGTTGT TCGAGGCGTCTGATAAGAAGCAGCCTGTTGTACTAAGGAATTGCATATTCACCAAGCACTGTAGGCCAGTAACACAC ACCAGGGAGCTCCTACTTCTCAGCTTGACGCTTTGTGTTGTAAAGATGTCAGGTTCAACTTTGGGAGGCCCCCGAGA TGGGCAGACCACTTGATCCCAGGAGTTTGAGACCAGTCTGGGCAACATAGGGAGACCCTGTCTGTACAAAAAAACT AAAAAATTAGCTGGGCATGGTGGCACATGCCTGTAGTCCTAGCTGCTTGGGAGGCTGAAGTGGGAGGATTGCTTGAG CCCAGGAGGTTGAGGCTGCAGTGAGTTGTGATTGTGCCACTGCACTCCAGCATGGGCGACATAGCCAGACCCTGTCT CAAAAAAAAAAAAAAAAAAATCAGGTTCAAGATCACTTTCATTCCAACAACTATAGTCATTATTGAATTTTACTTACC TTCATGGAACATATGTATATCTCAGCATAGTCAGATATAATGGAAATTTCTCTTAGAAAAATAACCGTTCTTTGTAAAAT TCACCTTTTTAGTTGTGATAGATATCTAAGATTTAATTCCATCAAGTGGAAATTAAGATTTTTCTAGTCAGGAAAATCT GACTAGATGTGTTGAAAGTATGGGACAAATCAAGAAAAACACTGACAAAATTAAATCTTTTTTTTTTTTTTTGAGACG GAATCTCGCTTTGTTGCCCAGGCTGGAGTGCAGTGGTGTGATCTCGGCTCACTGCAACCTCTGCCTCCTGGGTTCAA GCGATTCTCCTGCCTCAGCCTCCTAAGTAGCTGGGATTACAGGCACCCGACACCACACCCAGCTAATTTTTGTATTTT TGGTAGAGATGGGGTTTCACCATGTTAGTCAGGCTGGTCTCAAACTCCTGACCTCGTGATCCTCCCACCTTGGCCTCC CAAAGTGCTGGGATTACAGGCGTGAGCCACCACACCCGGCCACAAAGTTAAGTCTTAACTGAAGTTTTTCCTTTTGA TGGATAAACTTTAGAGGCATGGTTCTGCTTATTAAAGAAATTTGTTAGGGAAGCCACGCTGAAGACCTCCGTGTGAC AGAAGGGTTCATGAACAAGGCTTCATCTGCTCACTCTTTCTGCAGACGGGATCATTTCTCCGTTCCCCACCCCTGGCG TTTGGCTCTTTACTGAAAGAATGAAGGATACTTACCTCAACTAGAATTTGGGTTTACCAAAGCAATTTGGGGTTTTGC TTGAATAACTTGATTTTGTTTGCTTTGGTTTTTAAACTTTTTTTTTTTTGGTATGTGGTTAAATGAATCTCAAAGCCTAT TTTGTTGGGAATTTGTATGAGTTACCTAGATTCATAAAATCGGGTTCTTTGCCCTGGGAGCTATAAATTTAAGATGGTG GTGTAATTACCATTGGCCAAAACCACAGCTGATTCTGTCCAGATGCCTGAATTCTCCAGTCTTCCTCTTCTAAATCCAA CAGTCCTTTTTTCTTCTCCCTCTTTCCCTCTGTCCTTAATTCTATTCTGTTTGCTTCTTTTGTTTTCTCTGTTAGTCACTT CTCCCCAAGCCCCTGGGCGTCTGTGCTTCCTGACAGAGTTGTGTTAGAGTTAAGGTGATTGGGTAGAAATTATTGCTG CCAGTGAAACGTCCTGAACAAGTCATCTTGTGGGACTAGCTCAGCCCTAAAGCAGTAGTCTGTCCATTTTGTAGGAA ACCACCAATGGTTCCAAAAATCTGAGAATTCAAAACTCTGCATCTTTTTTTGTGGAAAGACCCACACTAGCACAAAC GAGCCAGCTCCTCGAAACCCTTAAGAAGCACACAGCCCCCTTCCAGCTCCCTCTGCCCAGCCACACTCTGCCACCCC ACCCCCGCCCGGCAGCCTCCCTGGAGCCTGAGCCTTTGCTCTGCCGTTTCTTTCATCTGCACGGGAATGGCGGCCTC TGAGATGCCCATCTCCCCACTTTCCCTAGGTCGTGGCTTGCACTCTTATCCCATGCTTCATCTCTTTCCTCTGTCAGAT GCCATTATCTTACATTAAAGTATGTCTTTTTCCCTCTGTTCTTCCTGACACTAACATGATCCCATTTGTCTTCAGACACC CCTGAGGCTAAGGTGCTATCACCTACTCCCAGGCCCTCTTGCAGGTATTTCCTGGCCCCACCAGGCCCTCCCTCAGCT GTCCCCTGTCTCCTCCCCTCCCTGCTATCCTCTCTCCTCCTTCTTCCCTCTCTCAGGTGGTCCCATTACTCCCTACCTTT CTTTACAAACTCTCCTCCTCCAGGATGTATTTTGTAAAGTCAGCCACAGAAAATATAGCAGCTTTCAGGAAGGACAAA TCTTATGGTCTGAATTCAATTGGGTGGGTTGATATCACTGTCTCACATCATGAGATTATTTTAATTGTAAGAATATAGGC TGGTAGCGGTGGCTCACACCTGTAATCCGAGCACTTTGGGAGGCCGAGGCAGGCGGATCATGAGGTCAGGAGATTG AGACCATCTTGGCTGACGCGGTGAAACCCCGTCTCTACTAAAAATACAAAAAATTAGCCGGGCGTGGTAGCGGGCAC CTGTAGTCCCAGCTACTCGGGAGGCTGAGGCAGGAGAATGGCGTGAACCCAGGAGGCAGAGCTTGCAGTGAGCCG AGATTGCGCCTGGGCAACAGAGCAAGACCCCGTCTAAAAAAAAAAAAAAGAATATAAACCATATCATGTATTTTCTA AGCTATGAATCCGTGCTATGAAACGTGCTCCAAACCCTGGCTTTCTTTTTAAAACCACTAGTCCCCGAGGAGCCCAG GGCTCAGATGTGGAGGCTTAAGGAGGTGTCCGCGGGTAGGCCTGGCGCTGCCATGGAGCCTGATATGGGTTTGGCAG CACAAGGCTGGCCCCTTCTCCTCCCAACGCACGCCTTAAGGATTGACACAGGGTCACAGCCAGCCTCAGCTCAAAG |
| | | GTCAGCACGCCTAGCACTCATGGCAGCAACTGTGTGTGTTGCTTCATGGACTGGGTCCTGCACAGCCATAAATGATC CTCCTGCAACTCAGATGCCTTTGAAGGAAATGCCAGGGGTCCCCTGAAAAGTGCTGCTGCTGCCAGGCAACCAGGT TTCCAGAATTAACGTTTGGGATTAATAACGTGCCCAGCTTATCAGCCAGGCCCTTTGTTCCCCTACAGTTCCATGGGA GCTTTTCAACAACTGCTTCTGAAAGTGATCTCTCCCGGTTGATGCTTTTAATACTCACTACAAAATCTGATCTGCTTAT GAAAATATTTGAAGTGAACTGTAGCCTAGAAAAACATGAAACCTAGAAAAGAGTTGGTGAAATTGAGGTAAAAGGA CACAAAGCCAACACTAATGAGAACCTCTTAACAACAAGGAATCAGACACACAAATAAAAAGTCCAACATTCTGGGT AGGATCTGAGAGCAGATGCCCAAATTTGGGTTAAATATCATAGTTATTTCTCATAGGAGAGGCACCATCAACAAGCAG TATGGGCTGGGTGCAGTGGCTCACTGCATCCAGTGAGCCTGTCATCCCAGCACTTTGGGAGGCCATGGTGGGAAAAT GGCTTGAGCCCAGGAGTTCAAGACCAGCCAGGGCAACATAGCAAGACCCCCGTCTCTGCAAAAAAATTTTAATAATT AGCTGAGTGTGGCAGCTCATGCCTGTAATCCCAGGGCTTTGGGAGGCTGAAGCAGGAGGATTGCTTAAGGCCAGGA GTTCCAAACCAGCCTGGGTAACATAGCAAAATCCTGTCTCTGCAAAAAAATTTAAAAATTAACCAGGTGTGGTGGTG CACACTTGTAATCTCAGCTGCTTGGGAGGCTGAGACAGGAGGATCATTTGAGTCCAGGAGTTTAAGGCTGCAGTGAG CCATGATTTTGCAGCTGTACTCCAGCCTGGGCAAGAGTGAGACGCTGTCTCTACAAAAGAAAAAAAGAAAAGAAAA GAAAAAAGCAACATGGTATTATACTCTCTTATATAGATAATATGACCTAGCCAATATCTGGTCCTTTTCATTATGTGACT GTAAGGCAGAACTACAACAAAATCTTTTCCCAATATATTGCCTTTCTAGTCTTTCTGTGTTCATTCGAGACTTTAATCT CTTGCTTGGTTTTGGCCAAAATTCACCCCAGAACAAGTTCATACACAGCACCCTCACTGAAAACTGACAAGGGCAGT CAGTGTGTAGCACCAGGAGACTTGCCTTTAAATGCATAATCTGGCCGGACACAATGCCTCACGCATGTAATCCCAGCA CTTTGGGAGGCCAAGGCGGGTAGATCACTTGAGGTTAGGGGTTCGAGACCAGCCTGGCTAACATAGTGAGACCCCAT CTTTACTAAAAATACAAAAATTAGCCAGAGGGCTTGCTTGAACCCAGGAGGCAGAGGTTGCAGTGAGCCAAGATCA GGCCACTGCACTCCAGCCTGGGCAACAGAGCAGGACTAGCTTCAAAAAATAAATAAATAACCATCATCCCTGTCCCC TCTGTGTTGAGGAAGGCAGAAGCTTTCCTTTAACTGAAGTTAATTGAGTGATTTTTGTCCAGGCTGTAGCGCTTTGTA AAGAAACAATTCACAGAAAATTAAACTCTGGGAGATGTTGTTATGGAAAACATTTGGATTTCTTCCCTTGGTGTCTGC ACCTCTGTACTCTGTATCTGATGGTTTTTTGTCCTTCAATCTGTAGGTTCCGCTGTGTGGCAGCTGATTGCTTCCTTCCT GAGGCTTCCAATCTCAGGAACGCACTGCATTGTGGGTTCTACTATAGGATTCTCACTGGTCGCAATCGGTACCAAAGG TGTGCAGTGGATGGAGCTTGTCAAGATTG |
| 5 | Minigenes construct for event crpE10 | CGGCTTCACGATCGAGCTGGCCTCAGCCTTCACAGTGGTGATCGCCTCCAACATCGGGCTTCCAGTCAGCACCACGC ACTGTAAGGTAGGCCCCTGCTAGCCGGGAGCCCTTCCCCAGGCCGCTGGGACAGGTCCAGGGCCACCGGAGCGCAT GCAAGGGAAGCTGTAGGGCTCTGTAGATGGTTGTGCAAAATGCAGCTCCAGGTTCTCCAGGTCTCCGGGACCTTGTA TTCCTCCATCTGTTCCCTCCACCGTCCTCCTCTCGGCAGGACGACACTATCTCTCGTTCAGACCTGGCTTTTCCAAGG CAGAATGCCAGGGCTGGCCTGCCTGGGACTGGCCTGCTATCCTCCCACTGTTTCCACAGTGGAGTTTGATTGGCATCC TGTGTGCGCTGGGCATACCTGTGGGCTCACTTGGGAGCCTGCCACTTGGTCCAGCCAATGAGGGGACTGGGGGTTG ACAGTGTTAGAAATTAACACAAATCATCTCTGGAATTGTAGGACACAGTTGTTTCTGGTTTTGTTCCTTATATGATGTT CAAAGTGACTATATTGCCATAAATTCCCCACGAGGGGTAATTGCTGATACTGACCACTGCGGAGTGCTCTGAAACCTG CAGCTGGACAAGAGAGGGCTCCTGGTCATGGTGTGGAAAGTTGGAGCTGCAGGGAGGAGCCCTGAAGACCAGTGA AGAGGAAATGGGTTCCGGGAGTTTCAGGGAGTGATCCACAGGTGGCCAGGGTGGGGAGGACCAGAACCCAGGTGG GGAGGACCAGAGCCCCGGTCTCATCACCCAACTCTGCCCAGTATCTGCAGATACTTCTAAAATAGCTCAGGTGAGCC TCTGTATACAGACAAACTCACCAGTCTCAACAAAGCGAAAGTCACTTTGAGATTTTTCCTAGGTTCATTCATTTGTATT ATTCAAGAAAAACTAATTGGTGTTTGCTGCTTGGGAGCAGAGCTCTCAGCACCAGGAGGAATATATGAGCCCTCAAA ACAGACACAGCCACCCACGTTTCTATTTACTCACAATTTTAAGATTCCAGCCAGTATGTTTAAAAGACAACGCCAAGC AAAGGCAAGTGGCTTCATGCTTCAAACACCCAGGTCAGGTGGCCCTCACAAGAGTATCTGGAGTGGCCCAGCAGCG |
| | | TGTTTCAAAGCTGGGGCTCCACGCATGAAGAATCAGCCTCAGTGGAAAGAGTTTGTTTTTTCCTTTTTTGCTACCATA TACAGAAATACTTCAATTTCCACAAAGTTTCTGTATACAAATCCCCCCACTTACTGATCATCAGGAGAGTGCTGGGTA CCGTGCTCAACACCACACGCATGGCACACCGTTCCCTCTCCACAGCAAGCCTGTGGGGGCGGAACTGTCACTCCCA GTCCACAGGTGAGGAGACTGGGTTCCCTCAGAGTTGAGCCGCTTCCCACATGCACACAGCCACATGTGATAGATCCA GGATTCAGATCTTCACCTCAGGCCACACACCTTCTCGTTCAGGTCCCCATCAGCACCATTGTGGTGCCCGACCATGGC CAAGGTGCCTGGGGCAAGCACTCCACTGGTGGTCTCCCCAAGAAGCTTCCCCAGCTTCATAGATGAGGAAACTGAG GCAGAGGAAAGTTAAGTAACGTGCCCGAGATTACACAGCCAGGGAGCAGCCCAGCTGAGGCTCCCACATGGACGGC ATGGCCAGCGTCCTCCTCCCCGGCTCCGCCTGGCAAGCATGGGGAAGGAAGGCAGGAAGAGGTGCAGGTGTGCCCG CCACCCATTCATTGTGAGTTTCCTAATGTGGGTTATTTAAAACTTACCAGAGAAGCACATATTCACTGAAATATTTCAA ACAATACAAGACTATAAGGAATGAAAAGTGACATCTTCTCTGGACTCCACATCACTTCCATTCACTGTGGTGTGTGGA GCGTGGAGGAGACGCGTGTCACTAAATACATTCTTTTCCAAGGACACGGGCTTATGACTTTTCTATAGGCATCAGTTG TCTTTTGGAATGCTTTTAAACAGATGTAATGCCATCTTTTTAATGATTAACCTACACTAAAGCAAGACTGCTGAAGGCA GACTCTTCTACACCAGTTGAAATAGCTTTTTTTTGTTTTGTTTTGTTTTGTTTTGTTTTGTTTTTGAGATGGAGTCTCAC TCTCATCCAGGCTGGAGTGCGGTGGCATGGTCCCAGCTCACCGCAGCCTCTGCCTCCTGAGTTCAAGCAGTTCTCCT GCCTCAGCCTCCGGGTAGCTGAGATTACAGGCATGCACCACCACACCCAGCTAATTTTTGTATTTTTAGTAGAGACAT GGCTTCACCATGTTGGCAAGGCTGGTCTCGAACTCCTGACCTCAGGTGATCTGCCTGCCTCAGCCTCCCAAAGTGCT GGGATTACAGGCGTGAGCCACCGTGCCTGGCCTGAAAGAGCTTTTGAAAAAACAGCACAGGTTTGGTTCTTCCAGG TTGGTGCTACTTAGCTAACTGTATGAGCAGTCTAATCCGTTTGTATGTATACTGTATACAGTGTTGGAATGGACAGTAC TGATTTTGCTAATAACACTAAACTCTAGAATACAGTTTATAGGAATAGATGAAAAGTATAAAATGCTTATTTAAAAAGC CAAACTCTTGAATTATCTATATGCATGATTTCCCCAATACTGTATTGATATATATACTATAATGATGTGTGACTAAGAGAT TTACATCCAGTTTGTACAGGACCTTAGTGAATAGATGTATATGTTATCATTATCCTAAAATACCTGTACAAAGTACATGA TAAGGTTTTTTTTTTGAGATGGAGTCTCGCTCTGTCACCCAGGCTGGAGTGCAGTGGCGCCATCTCCACTCACTGCAA GCTCCGCCTCCTGGGTTCATGCCATTCTCCTGCCTCAGCTTCCTGAGTAGCTGGGACTACAGGTGCCCACCACCAGCT ACTTTTTTATATTTTTAGTAGAGACGGGGTTTCACCGTGTTAGCCAGGATGGTCTCGATCTCCTGACCTCGTGATCCGC CCGCCTCAGCCTCCCAAAGTGCTGGGATTACAGACATGAGCCACCGTGCCCAGCCAGTTTTGTTTCCTTTGAGCTGG TGATGAGTAGTATTAGGATAGCTTCTTGGCACTTAAAGCAGGTCTCCCTGAATGGGACTGCAGCAGGGTGCCATCTAT AAAATTGACAAGACAGGTCACAACTTGGATTTGAGTAAGTTTGTCCGATATCATTGATCGTGGACTTCTGGACAAAG CCTAGAGCTATTCCCTGTGAGCACTAAGGACAGAGTCTCAGCTCTCAGGAACTTGCCGTGTAGTTGGAGAGCAGATG GCTCTCATCAGAGTCCAAATAATAGAAGTGCTGAGAAGAGCTCACAGTTGTCATTACCCCATAACACTTATTCTCTCA TATCACATATTTCCCCTGCACACAACAGTCCCCAAATGGCAAACGTGGGATGAACTATGAGAGCTGTGGGATGGCGT GAAGAATGGGGCCCTCTGTAAGTGAGACTGTGAATCTGGAAACATTGAAAGGGTATTGATGTCCACATAGAGTCATA GTTGAATTGCATCAGTTTTCAGGATAATTATCCTGTGTTTGATTTTTAAGTTTCATGCTTAGAGCCATCTAATAGAAAGT AGGGAGAAGTTGGGGACTTGGCAGGAAATAAGATGGAAACTATTGTTTAAAATGAGGTTTTTAATGTATCTAGGTAGA TGTTATTGATTTATGCTTCCTTCTAAGTGTACATGGCTAAGTGCATAGATTTGTTTTGTTTTGTTTTGTTTGAGACAGAG TCTTGCTTTGTGGCCCAGGCTGGAGTGCAGTGGCTTGATCTTGGCTCACTGCGACCTCCGCCTCCTGGGTTCAAGCA GTTTTCCTGCCTCAGCCTCCTGAGTAGCTGAGATTACAGGCATGCACCACCATGGTTGGCTAATTTTTGTATTTTTAGT AGAAATGGGGTTTCTCCATGTTAGCCAGGCTGGTCTTGAACTCCTGACCTCAAGTGGTCTGCCTGCCTCGGCCTCCC AAAGTGCTGGAATTACAGGCATGAGCCAGTGCGCCCGGCCTAAGTGCATAGTTTCAATTTTGTCGTAGCCTATTTCAA AACATTTATGAAACCATACTATATTATGTGATATATGAACTGAAGGTATAACTTGGGAAAAAACAATTAGGAAAGTCCT GTTATCACATTTTATTCCAGAAGGCATATATAAGGCAGTCTGTTTGATGAGTTACACCCTCTGTATGTATTCTGGTTTTA |
| | | TATATTTTGTATTTCATATCAGAGGACTCAGTTTATCAGATATTTTCCTGGAATAATACCTTTGAGTAGGGTCAAAAGCC TTAATTGGCCAGGTACGGTGGCTCATGCCTATAATCCTAGCTACTCGGGAGGCTGAGGCCGGAGAATCGCTTGAACCC GGGAGGCGGAGGTTGTGGTGAGTGGAGATTGCACCATTGCACTTCAGCCTGGGCAACAAGAGCAAAACTATCCCCC CCCGCACCCCCCAAAAAAAGCCTTAATTGAAGACACAGAAACCGGTTTTGAAAATAAAAGAATCCTCAGATGTTATG TTTCAGCAATTCGTTCATCATTCTTCCCTTCTGTGACCCTTAATCACGTTTGGTCTTTAGCCCTAGCAGATAACACACA GTGCCACTACTGAGCACAGTTGCTCATGTGTGTGCTCTTTAATAGGAATTTGGAAGTCATATTCTGCAGAATGTTTTAA TTCCATACTGGGTGGATTGAGATTATTCCTGTTTGTGGATCACCCACATTGTCAGTCCTCACTGATGACATAGATGATG ATTCAACCACCATTTCAATTTGGAACAGCATACAGGGAACACTACTGATTTCAAATGTAGGATTATAATTTCTAATTTA ATTTCTAATTCTTGTTCTCTGGTCCCCCTTCTTCTCAACAATTTTTTGCTATAAGTACCTGATGATGGCCTTAAACTGGA AACACACTCGATTTATTAAAAGGGAAGTTAGAACACAGCAAAAAAATAACATTTTACATTCACGGAAGACATGATCA GTAGCAAAGACATAGTACTCTGGATCTTTTATGAATCAAATAACATGTAACTAAATACATAAAATTGTTAAGAACTCAG AAATTACTGTCTTAGCGTATGATAAATGAAGTATAGAAAAAGTATGTGGGAATATGAAGAATTTGAACGTTTATTTAGA ACAGCATAATTAATAAATATGTACAAGTTTTTACGCTACTGATCATATGCCTTCCTGTCAAGCATCGTGAAACATTTATA AAAATCCTAAAATCAGAAGACCATGATAAAAATCTCTATAAAATTTTAAAAACAGACCTGACATGGTGGCTCACACCT GTAATCCCAGCACTTTGGGAGGCAGAGGTAGGAGGATCGCTTGAGCCCAGAAGTTTGAAACCAGCCTGGGGCAACA TAGTGAGACCCTGTCTCTATTTTATAAAAGTTAAAATTTAAATTAAAAAAAAAAAAAGTTTAAAGATAAGTTGTGCTA ACCATGATCTCTAATTACAATAAAATTAAACTAGAAATTAATTATATATTGAAATAAGAATGTATCCCAATGGAGACTTT TTAATGCTTTTTTAAGGTAACAATTGAATTAAGGAGTTTAGAAAAATACAAGGATAGCTTTCTTGGAAAATAATAAAA ACCAAATGTGTGTGTCTGTGGGTGGATACAAACAGCAAAACCTGTGGCAACCAAGTTATGCTAAGATGAAAATTCAT ATCTTTAAATGGTTTAATTGATCAAAGGGAAAATAAAATAACCAAGTATTAAAATTAGGATTTTGTAAAAAGTCCAGG ACAGTAAGAATTGAGGAAACAGGTTGTAAGAAATATAGAAGCAAAATTAATTTAAAACAAAATAAGAAGGGGAGAG GAAAGTGGCTACTAACAAAATGTCAGATCACATCAAGGAAAAGAGGGAGGGGAAGGAGGAAGACATTGTAACAAC AGAGAAGAGAACTGTTTCCATTATTAAATAAGCCACACAGCTATATGCTAAGAATTTGAAAACCCATGTGAATTATTTT CTGCAACAATATACGTGACAAAGAAATATTGGCCTAAGAAGTAGAAAAAAGTGGAAAATCTGACCCAGAACAATAG GATGAAATTAAGGGAGTTCTCCAAAAATAATTGTTCCCACCTAAAAAAGCAAAAAGGGGCTAGGGGTGGGGCGGGT GAGGATACTGCTGACTAGAGGAATTCATTAGAATCTTTTCCATTTAAACAAAAAAAAACCCAGCTCTCACACGATCTC AACTTTCCCACAATGTAGGAAGAAAAACAAAACTAAAAAACCTCTCCCATTTGTGCTATGGCGTACACTGAATAACC CTAATACCAAACCTAATTTAAAATATCACAAACAATGAAATCTGTTTACCGATGAATATTGATTGTAAAAACTTTAAGC AAGATGCTGAAAAATGACATCAACAATATTTGGCTGGGCATGGTGGCTCGTGCCTGTAATCCCAGCACTTTGGGAGG CTGAGGTGGGCAGATCATCTGAGGTCAGGAGTTCGAGACCAGCCTGGCCAACATGGCAAAACCCCGTCTCTACTAA AAATATAAAAATTAGGCAAGTGTGGTGGTGGGTGCCTGTAATCCCAGCTACTAGGGAGGCTGAGGCAGGAGAATCGC TTGAACCCAGGAGGTGGAGGTTGCAGTGAGCTGAGATTGTGCCACTGCACTCCAGCCTGGGCGACAGAGCAAGACT CTATCTCAAAACAAAACAATACTATACAAGAACCTTTCAGCAGGACCAAATTGATCAAGATTACCAGAAAGTGGGCT CCCTTCGTGACAGGGCAGGACTGCATCACCCACCACCAGCAGGCTGTGCAGGGCAAGGTCCTCGCAGGGGCTCAAG TTGGGTATATAGGCAGCTTCAGTATAATATGCCTTAGCAATCCACTTAAAAAAAAAAAGATCTCTGTTGAGATATAAGC CAAAAAGACATTTGATGATATTCAGTATTTCTTTTTTTTTGAGACGGAGTTTCACTCGTTACCCAGGCTGGAGTGCCAT AATGGCATGATCTCAGCTCACCACAACCTCCACCTCCCGGGTTCAAGCAATTCTCCTGCCTCAGCCTCCCGAGTAGCT GGGATTACAGGTGTGCAGCACCACACCCAGCTAATTTTGTATTTTTAGTAGAGACGGGGTTTCTCCATGTTGGTCAGG CTGGTCTCGAACTCCTGACCTCAGGTGATCTGCCCGCCTCGGCCTCCCAAAGTGCTGGGATTACAGGCATGAGCCAC CGCACCCGGCCGATATTCAGTATTTCTAAAACACTACTAGAGTTCGTAAGAGTGACCAAGTCCAAGGTAAAAATAGA |
| | | AAAATCCAGCACTTGTTCATCAGCAATAGCCAATTAGAAAATGTAACGGGAGGGAAAAAAACTGCCACCATAATTGC AGCAAAAATAAAAGTTATCTCAGAATAATCCTAATAAGCACTGTGAGTGTCATCAGGCACATGGTGTGTTCTTATAGT CCCAGCTACTCAGGAGGCTGAAGGTACGAGGACAGCCTGAACCCAGGAGTTCAAGACCAGTCTAGGCAACATAGTA AGACCCCATCTCTAAACAACTAAATAAATAATTTTTTAAAAAAGAAATGTGAGTGATATATATGGAAGAGGCACAAAA CTGCTCAGAGGTATAAAAAGATAATCTGAATATGAGACGTAATGTGCTATTGGATGGGAAAACCAGATGTTGTAATGA AGTCACTTCTCCCTAAATACAACCTGAACAGGATTTTACTTGGAATTTTACAAAACGAGACTAAAGAATACATTTTGG TGAAATAAATTATCAGCAATATAAAATGATCTAACAAGAAAACTGAAAGGAGGAACTAGCCTTACCTGACGTATAATG TCATATAAGGAAACAAAAGTCAAGAGTAGGTCCTGGGAGGAGGGGCTAGAACTGGGTGTTAGTGTTTTATGGGGCA GTTTCCCCGTGGGATGATGAAAAAGTTCTGGAGACAGATTGTGGTGATGGTTTGCAAAACAATGTGATTGTACTTAAT GCCTCTAAATGGTATGCTGAAAAATTACTAAAACTGTAAGTTTTATGTTATGTATATCTTACCACAATAATTTTTAAAAG TAGATACCTGGATAAGAACAGAGTAGATAGCAATGGATGGAACCTGTTCTATATGAGCATGTATTTTGAAAAAGGGAA AATTTAACCAGTAAGTGTTTGAAAGGTTGAATAGCTGACTGTCAGTCTAGAAAAACAAAATTAGACCACTCTCAAAA TACATGAACATAAATCTGATGGCTTAAATAATATTTTAAAAATATTCTTAAATTTAGAGTAAAATGGAATTTTATTTTAG AAAAATAAAATGGAATTAAATATTTATCAAATATTCAAGCCAAGGATAAATCACAGAAGAAAATTATAGCATATTTTAT TGCATGAAGTTAGAAACTTGAAAGTGAAACAAAGCAAAATACTACCTTAAGAAAATGTGTATTCGATATCACAGAGT TAATATTATTCAGTGAAGACTACACATAAATAAATAAGGAACACATTAGATTTTAGTATGTAAATGGACAAAATACATG AAGAGACAATTTTTTTTTTTTTTTGAGACGGAGTTTTACTCTGTCACCCAGGCTGGAGTGCAGTGGTGCCATCTCGGC TCACTGCAACCTCCACCTCCCCAGTTCAAGCGATTCTCCTGCCTCAGCCACCCAAGTAGCTGGGACTACAGGTGTGC ACCACCACGCCTAGCTAATTTTTTGTATTTTTAGTAGAGATGGGGTTTCACCCTGTTAGCCAGCATGGTCTTGATCTCC TGACCTTGTATCCGCCCGCCTTGGCCTCCCAAAGTGCTGGGATTACAAGCATGAGCCACTGCACCTGGCCGACAATT TGTATATGTGAAAGCTAGAGAGAGTGAAATTCAGTAAATATATTTTTTAAAGTTCAGCCTTAATTGTAATTAAAGATAC GCAAATTAAAACAGCAACAGTGCATCTTTTTGTCGGTTAATTAGCCAAAAAAAGAGCAACTTACATGATATCATGTGC TGATATTCACAGATTATGGATGACACTACATTAACAGCTATTTATAAAACTATTTGTCATTATGTATTAAGGTCCTAAAA CACGTTGGGAGAGGAAAGAGCTCTTCCTTGAAGTAGAACTCCATGTGAGAAATTGCAATGGACTTTGGTAAATCACC ATTTTATTTTATTTATTTTTTATTTTATTTTTTTTTGAGACTGAGTCTCATTCTGTTGTCCAGGCTGGAGTGCAGTATCGC GATCTTGGCTCACTGCAACCTCCACCTCCCAGGTTCAAGTGAGTAGCTGGGATTCCAGGTATGCACCACCACACCTG GCTAATTTTTTTTATTTTTAGTAGAGACAGGGTTTCACCATGTTGGTCAGGCTGGTCTCGAACTCCTGACCTCAGGTGA TCCACCCGCCTCGGCCTCCTGAAGTGCTGAGATTACAGGCGTGAGCCACCATGCCCGGCCTGGTAAATCACCATTTTA TAGCCATCGTCATAAACATTGGATTGTGAAATCTTTAGCAGAAGGGGAAAGTGAGGTTTGATAAGATGCAGGATATTT TAATCATATTAAAAAGTCTCTTCACAGATTGTGGCAAATGTCAAAAAATAGTGAATTGGGTAAAGAGAAAACAGGAG TTCTTGCAGCTTTTCTCTAAGTTTTGAATTATTTCAAGATACATTTTAAATGTTCCCTGTTAACTACAGTCGATTTCAGG GAATTTTTTTTTTTTTTTTTTTTTTTGAGACAGAGTCTCTCTCTGTCGCCCAGGTTGGAGTGCAGTGGCATGATCTCCG CTCACTGCAAGCTCAATGCAAGCTCCGCCTCCCAGGTTCACGCCATTCTCCCGCCTCAGCCTCCCAAGTAGCTGGGA CTACAGGCGCCTGCCACCACGCCTGGCTAATTTTTGGTATTCTTAGTAGAGACGGGGTTTCACCGTGTTAGCCAGGAT GGTCTCCATCTCCTGACCTCGTGATCCGCCCACCTCAGCCTCCCAAAGTGCTGGGATTACAGGCGGAAGCCACCGCG CACGGCCGGGAATATTATTTTTAAAGCCAAAATCCAACCTGGGCACAGTGGCTCACGCCTGTAATCCCAGCACTTTGG GAGGCTGAGGCGGGCCGATCACTTGAGGCCAGGAGTTTGAGACCAGCCTGACCAACATGGCGAAACCCCATCTCTA CTAAAAAATATGAAAATTAGCTGGATGTGGTGGTGCTTGCCTGTAATCCCACCTACTCAGGAGGCTGAGACAGGAGA ATCGCTTGAACCTGGGAGACATAGCTTGCAGTGAGCTGAGATTGCACCACTGTACTCCAGCACTTTGGGAGGCCAAG GAGGGAGGGTCACTTGAGGTGAGGAGTTCCACATCAGCGTGGCCAACATGGTGAAACCCCGTCTCTACTAAAAATAT |
| | | GAAAATTAGCCAAGGGTGATGGCATGCGCCTGTAATCTCAGCTGCTCAGGAGGCTGAGGCAGGAGAATTGCTTGAAC CTGGGAGATGGAGGTTGCAGTAAGCTGAGATCACACCACTGCACTCCAGCCTGGGTGAAGAGTGAGACTGTCTCAA AACAACTATATAAAACAACTATATAAAGAGCTATATGCTGAAGAGAAGAATCCCCAAACAATTCTACCTAAAATCTAA GCTATTTTAAAAGCTTTAACAAATGTAGGGGACTGGGTAATGTTGTACTAGAGCTTGTTTCTGAGATTAGCCCATGTAC ATTGGTAGAGTGTAGCCTTTGTGGCTCACCTGTTTTTACTTTGCAATGTCTGCTCCCACAGGTGGGCTCGGTGGTGGC CGTGGGCTGGATCCGCTCCCGCAAGGCTGTGGACTGGCGCCTCTTTCGGAACATCTTCGTGGCCTGGTTCGTGACCG TCCCTGTGGCTGGGCTGTTCAGCGCTGCTGTCATGGCTCTTCTCATGTATGGGATCCTTCCATATGTGTGA |
| 6 | Abnormal mRNA containing cryptic exon in event crpE2 | AGCGTGTTCCTGCCTGCTGCTTGGCTGCAGTTAATTTTAGCCGGGGTTAGTTCTATGAGTTGTAAAAGTAGAAGTTAGCAA GCTGAAATGTGACTTCTCGGCCTAATGTGGTAGGATATCTATTTTAGACTTCAGATTGTGTTTGTGATCAGGAGAGCAGTCT TCTGAAGAATTGTGGCTCAGTTTTCTTGGAAACTACTTGAGGAGACATCACAGCTTTCCCAGATCGGGAGGAAAAATATGG AATGTGTTTTACCGCTGACTGAACACAACCAAATGAACTGTCCTGACAGTAGTTTGCAAACCAGCAGCTAGCAGTTTGTCC AGCCTCTAACATTGTCCAGCACTTTCCAGAGCAAACTCACTGTTTACAAGAACTCTTGGCCTTACGAAGTTTATAACCTCA AGCTTTGTTTATTTAAAATATTCCTGCAAAAGAAAAGTACCCGGCACCCACTTTCCAAAATGGCCATGGATGAGTATTTGTG GATGGTCATTTTGGGTTTCATCATAGCTTTCATCTTGGCCTTTTCTGTTGGTGCAAACGATGTTGCCAACTCCTTTGGTACAG CCGTGGGCTCTGGTGTGGTGACCTTGAGGCAGGCATGCATTTTAGCTTCAATATTTGAAACCACCGGCTCCGTGTTACTAG GCGCCAAAGTAGGAGAAACCATTCGCAAAGGTATCATTGACGTGAACCTGTACAACGAGACGGTGGAGACTCTCATGGCT GGGGAAGTTAGTGCCATGGTTGGTAGAGATGACGACACAACAGACAGCTGATGTATACGGGTAATTTGGTTAGGGAGAAG AGGGTTAGAGAGGAATTCTACCACCTTCTGCTTTCAACAGGACATATCCTCTTGTAGGAAGAGTCCTGCAGCAGGAGGTTC CGCTGTGTGGCAGCTGATTGCTTCCTTCCTGAGGCTTCCAATCTCAGGAACGCACTGCATTGTGGGTTCTACTATAGGATTC TCACTGGTCGCAATCGGTACCAAAGGTGTGCAGTGGATGGAGCTTGTCAAGATTGTTGCTTCTTGGTTTATATCTCCACTGT TGTCTGGTTTCATGTCTGGCCTGCTGTTTGTACTCATCAGAATTTTCATCTTAAAAAAGGAAGACCCTGTTCCCAATGGCCT CCGGGCACTCCCAGTATTCTATGCTGCTACCATAGCAATCAATGTCTTTTCCATCATGTACACAGGAGCACCAGTGCTCGGC CTTGTTCTCCCCATGTGGGCCATAGCCCTCATTTCCTTTGGTGTCGCCCTCCTGTTCGCTTTTTTTGTGTGGCTCTTCGTGTG TCCGTGGATGCGGAGGAAAATAACAGGCAAATTACAAAAAGAAGGTGCTTTATCACGAGTATCTGACGAAAGCCTCAGTA AGGTTCAGGAAGCAGAGTCCCCAGTATTTAAAGAGCTACCAGGTGCCAAGGCTAATGATGACAGCACCATCCCGCTCACG GGAGCAGCAGGGGAGACACTGGGGACCTCGGAAGGCACTTCTGCGGGCAGCCACCCTCGGGCTGCATACGGAAGAGCA CTGTCCATGACCCATGGCTCTGTGAAATCGCCCATCTCCAACGGCACCTTCGGCTTCGACGGCCACACCAGGAGCGACGG TCATGTGTACCACACCGTGCACAAAGACTCGGGGCTCTACAAAGATCTGCTGCACAAAATCCACATCGACAGGGGCCCCG AGGAGAAGCCAGCCCAGGAAAGCAACTACCGGCTGCTGCGCCGAAACAACAGTTACACCTGCTACACCGCAGCCATTTG TGGGCTGCCAGTGCACGCCACCTTTCGAGCTGCGGACTCATCGGCCCCAGAGGACAGTGAGAAGCTGGTGGGCGACACC GTGTCCTACTCCAAGAAGAGGCTGCGCTACGACAGCTACTCGAGCTACTGTAACGCGGTGGCAGAGGCGGAGATCGAGG CGGAGGAGGGCGGCGTGGAGATGAAGCTGGCGTCGGAGCTGGCCGACCCTGACCAGCCGCGAGAGGACCCTGCAGAGG AGGAGAAGGAGGAGAAGGACGCACCCGAGGTTCACCTCCTGTTCCATTTCCTGCAGGTCCTCACCGCCTGTTTCGGGTCC TTTGCTCACGGCGGCAATGACGTGAGTAATGCCATCGGTCCCCTGGTAGCCTTGTGGCTGATTTACAAACAAGGCGGGGTA ACGCAAGAAGCAGCTACACCCGTCTGGCTGCTGTTTTATGGAGGAGTTGGAATCTGCACAGGCCTCTGGGTCTGGGGGAG AAGAGTGATCCAGACCATGGGGAAGGACCTCACTCCCATCACGCCGTCCAGCGGCTTCACGATCGAGCTGGCCTCAGCCT TCACAGTGGTGATCGCCTCCAACATCGGGCTTCCAGTCAGCACCACGCACTGTAAGGTGGGCTCGGTGGTGGCCGTGGGC TGGATCCGCTCCCGCAAGGCTGTGGACTGGCGCCTCTTTCGGAACATCTTCGTGGCCTGGTTCGTGACCGTCCCTGTGGCT GGGCTGTTCAGCGCTGCTGTCATGGCTCTTCTCATGTATGGGATCCTTCCATATGTGTGATTTGTCTTCTTCCAGCTGCAAAC AGCTAAAGGGATGGTCTGGTGTTGGCGTGTGGGAGACATGTGTGCTCGTGCCGCACATACACATCCTGGCCGTGCACGGC |
| | | TCTCTCATGACCAGCTCTCTGCCTCCCTTCCAGGAGGCTCCATCCCACACTGTTCACCCAGGCTGCGGAGACTCACCTTCC CGAGCTAACTTAACTACTGTACATAATAATATGTATTAAACTGGTATCGTGGTGATATAATGTGGTGCAGTTACTTATATATTA AATATCTATTGTATCCATAGAATAGGCAGCATTATTTCAAACATATTCAAGTTGGGAGTGGAGATCATTGCCTAGAAGTCAAT ATTCAATAAATCTTGTACATAACTATTTCGATGGCAAATGTTAAGCCTTCTAAAAGGAAAGTGTAGATTGGAAAATGATTTTT TTTCCAAATGATGTTTTTGCCTTCTAATATACTGTAAGGTAATGAGCTTCAGAACAGGCAACCTGACCCTGCAGAGGTCGCG TGCTGTGGGATGACAGCGGGACGGGAGCTCACAAGTGCTTTCACTGAAGATTTGTTCATATACTGTGTATTGATTGTTGTGT AATATATCATCATTGCTTTTGTAAATACGTAAAACTGTAATTTTTTAATGGTGTGCTTCCCTTATACTTTTTTGATCAGAGAATT TTGGAAAGTACCAAAGAAGCAGGGGAATCATTGGCCAGTGTTACGTTTTCACATTGTCTGTCTCCCACCCTCACTGATCAC GCCTGCCCCAGAGCAGTGTGTGGCGGTGACACCGTCACCCAGCATGCGCCACGCCGTGGCTCCCACCAGCAGTGCCACC GCCACCACACCCCAGATCCCACCCACCTTGCAGTGGCCTTTCCTTGTCATCAGAGTAGAGAATGCACAGGTGTTGGTGAG GGCGTGTGGCTGAGCACTACATGTCAAGTCCAGAGTCAGTTTCTATCCCAATTCTCCCTGCAGCCTGAAGAACGGATCCTT GTCTCCAATGTCAGCACAAAGGAGGCTTTTTCTGTGCTTTGACATTCTAGCACTTCAGGGATGAGAGGGAGGGAGAATCC TGGATGCTGGATGGAGTATTTCTCTGAGGCCCACACAAAGCTGGACACCCCCAGGCTCTACTCCATCCCATTGGAGTCTCT TCTTTTTTTGATAGCGGGAGGGAGGAAGTACGACTAATGTTGGAGCCTGAAACTATGGAAATGCTGCTAAAATTTTTATATT GACAAACATTTTCTTGGTACTTCATTGTCATTTTTCATTAATCAACCATATTAAATTTATAATAAAAAATGCCCCTCAGAA |
| 7 | Abnormal mRNA containing cryptic exon in event crpE10 | AGCGTGTTCCTGCCTGCTGCTTGGCTGCAGTTAATTTTAGCCGGGGTTAGTTCTATGAGTTGTAAAAGTAGAAGTTAGCAA GCTGAAATGTGACTTCTCGGCCTAATGTGGTAGGATATCTATTTTAGACTTCAGATTGTGTTTGTGATCAGGAGAGCAGTCT TCTGAAGAATTGTGGCTCAGTTTTCTTGGAAACTACTTGAGGAGACATCACAGCTTTCCCAGATCGGGAGGAAAAATATGG AATGTGTTTTACCGCTGACTGAACACAACCAAATGAACTGTCCTGACAGTAGTTTGCAAACCAGCAGCTAGCAGTTTGTCC AGCCTCTAACATTGTCCAGCACTTTCCAGAGCAAACTCACTGTTTACAAGAACTCTTGGCCTTACGAAGTTTATAACCTCA AGCTTTGTTTATTTAAAATATTCCTGCAAAAGAAAAGTACCCGGCACCCACTTTCCAAAATGGCCATGGATGAGTATTTGTG GATGGTCATTTTGGGTTTCATCATAGCTTTCATCTTGGCCTTTTCTGTTGGTGCAAACGATGTTGCCAACTCCTTTGGTACAG CCGTGGGCTCTGGTGTGGTGACCTTGAGGCAGGCATGCATTTTAGCTTCAATATTTGAAACCACCGGCTCCGTGTTACTAG GCGCCAAAGTAGGAGAAACCATTCGCAAAGGTATCATTGACGTGAACCTGTACAACGAGACGGTGGAGACTCTCATGGCT GGGGAAGTTAGTGCCATGGTTGGTTCCGCTGTGTGGCAGCTGATTGCTTCCTTCCTGAGGCTTCCAATCTCAGGAACGCAC TGCATTGTGGGTTCTACTATAGGATTCTCACTGGTCGCAATCGGTACCAAAGGTGTGCAGTGGATGGAGCTTGTCAAGATT GTTGCTTCTTGGTTTATATCTCCACTGTTGTCTGGTTTCATGTCTGGCCTGCTGTTTGTACTCATCAGAATTTTCATCTTAAAA AAGGAAGACCCTGTTCCCAATGGCCTCCGGGCACTCCCAGTATTCTATGCTGCTACCATAGCAATCAATGTCTTTTCCATCA TGTACACAGGAGCACCAGTGCTCGGCCTTGTTCTCCCCATGTGGGCCATAGCCCTCATTTCCTTTGGTGTCGCCCTCCTGTT CGCTTTTTTTGTGTGGCTCTTCGTGTGTCCGTGGATGCGGAGGAAAATAACAGGCAAATTACAAAAAGAAGGTGCTTTATC ACGAGTATCTGACGAAAGCCTCAGTAAGGTTCAGGAAGCAGAGTCCCCAGTATTTAAAGAGCTACCAGGTGCCAAGGCTA ATGATGACAGCACCATCCCGCTCACGGGAGCAGCAGGGGAGACACTGGGGACCTCGGAAGGCACTTCTGCGGGCAGCCA CCCTCGGGCTGCATACGGAAGAGCACTGTCCATGACCCATGGCTCTGTGAAATCGCCCATCTCCAACGGCACCTTCGGCTT CGACGGCCACACCAGGAGCGACGGTCATGTGTACCACACCGTGCACAAAGACTCGGGGCTCTACAAAGATCTGCTGCAC AAAATCCACATCGACAGGGGCCCCGAGGAGAAGCCAGCCCAGGAAAGCAACTACCGGCTGCTGCGCCGAAACAACAGTT ACACCTGCTACACCGCAGCCATTTGTGGGCTGCCAGTGCACGCCACCTTTCGAGCTGCGGACTCATCGGCCCCAGAGGAC AGTGAGAAGCTGGTGGGCGACACCGTGTCCTACTCCAAGAAGAGGCTGCGCTACGACAGCTACTCGAGCTACTGTAACGC GGTGGCAGAGGCGGAGATCGAGGCGGAGGAGGGCGGCGTGGAGATGAAGCTGGCGTCGGAGCTGGCCGACCCTGACCA GCCGCGAGAGGACCCTGCAGAGGAGGAGAAGGAGGAGAAGGACGCACCCGAGGTTCACCTCCTGTTCCATTTCCTGCAG |
| | | GTCCTCACCGCCTGTTTCGGGTCCTTTGCTCACGGCGGCAATGACGTGAGTAATGCCATCGGTCCCCTGGTAGCCTTGTGG CTGATTTACAAACAAGGCGGGGTAACGCAAGAAGCAGCTACACCCGTCTGGCTGCTGTTTTATGGAGGAGTTGGAATCTG CACAGGCCTCTGGGTCTGGGGGAGAAGAGTGATCCAGACCATGGGGAAGGACCTCACTCCCATCACGCCGTCCAGCGGC TTCACGATCGAGCTGGCCTCAGCCTTCACAGTGGTGATCGCCTCCAACATCGGGCTTCCAGTCAGCACCACGCACTGTAA GGTCCTAAAACACGTTGGGAGAGGAAAGAGCTCTTCCTTGAAGTAGAACTCCATGTGAGAAATTGCAATGGACTTTGGTG GGCTCGGTGGTGGCCGTGGGCTGGATCCGCTCCCGCAAGGCTGTGGACTGGCGCCTCTTTCGGAACATCTTCGTGGCCTG GTTCGTGACCGTCCCTGTGGCTGGGCTGTTCAGCGCTGCTGTCATGGCTCTTCTCATGTATGGGATCCTTCCATATGTGTGA TTTGTCTTCTTCCAGCTGCAAACAGCTAAAGGGATGGTCTGGTGTTGGCGTGTGGGAGACATGTGTGCTCGTGCCGCACAT ACACATCCTGGCCGTGCACGGCTCTCTCATGACCAGCTCTCTGCCTCCCTTCCAGGAGGCTCCATCCCACACTGTTCACCC AGGCTGCGGAGACTCACCTTCCCGAGCTAACTTAACTACTGTACATAATAATATGTATTAAACTGGTATCGTGGTGATATAAT GTGGTGCAGTTACTTATATATTAAATATCTATTGTATCCATAGAATAGGCAGCATTATTTCAAACATATTCAAGTTGGGAGTGG AGATCATTGCCTAGAAGTCAATATTCAATAAATCTTGTACATAACTATTTCGATGGCAAATGTTAAGCCTTCTAAAAGGAAA GTGTAGATTGGAAAATGATTTTTTTTCCAAATGATGTTTTTGCCTTCTAATATACTGTAAGGTAATGAGCTTCAGAACAGGCA ACCTGACCCTGCAGAGGTCGCGTGCTGTGGGATGACAGCGGGACGGGAGCTCACAAGTGCTTTCACTGAAGATTTGTTCA TATACTGTGTATTGATTGTTGTGTAATATATCATCATTGCTTTTGTAAATACGTAAAACTGTAATTTTTTAATGGTGTGCTTCCC TTATACTTTTTTGATCAGAGAATTTTGGAAAGTACCAAAGAAGCAGGGGAATCATTGGCCAGTGTTACGTTTTCACATTGTC TGTCTCCCACCCTCACTGATCACGCCTGCCCCAGAGCAGTGTGTGGCGGTGACACCGTCACCCAGCATGCGCCACGCCGT GGCTCCCACCAGCAGTGCCACCGCCACCACACCCCAGATCCCACCCACCTTGCAGTGGCCTTTCCTTGTCATCAGAGTAG AGAATGCACAGGTGTTGGTGAGGGCGTGTGGCTGAGCACTACATGTCAAGTCCAGAGTCAGTTTCTATCCCAATTCTCCCT GCAGCCTGAAGAACGGATCCTTGTCTCCAATGTCAGCACAAAGGAGGCTTTTTCTGTGCTTTGACATTCTAGCACTTCAGG GATGAGAGGGAGGGAGAATCCTGGATGCTGGATGGAGTATTTCTCTGAGGCCCACACAAAGCTGGACACCCCCAGGCTCT ACTCCATCCCATTGGAGTCTCTTCTTTTTTTGATAGCGGGAGGGAGGAAGTACGACTAATGTTGGAGCCTGAAACTATGGA AATGCTGCTAAAATTTTTATATTGACAAACATTTTCTTGGTACTTCATTGTCATTTTTCATTAATCAACCATATTAAATTTATAA TAAAAAATGCCCCTCAGAA |
| 8 | ASO target sequence of event crpE2 (c.289+801 to c.289+1135) | TGGGTCTTCCCATCACTGCCCTCGCTACGACAGAAAAGAACCTTCCCGGCAGGCGTGTGTTAAGTGCAGCTGACAGG ATTCTTTTTCTGCTTGCCTCTAGGTAGAGATGACGACACAACAGACAGCTGATGTATACGGGTAATTTGGTTAGGGAG AAGAGGGTTAGAGAGGAATTCTACCACCTTCTGCTTTCAACAGGACATATCCTCTTGTAGGAAGAGTCCTGCAGCAG GAGGTAAGATGCTGGTGTACTAAGAATACAGCCACATTCCCCCGAGTTCTTTATTTTGTCACTTACATTTTTTCTTTTC TTTTTTTTTTTTTTTTTGAGACAG |
| 9 | ASO target sequence of event crpE10 (c.1795-1803 to c.1795-1527) | AAAAGAGCAACTTACATGATATCATGTGCTGATATTCACAGATTATGGATGACACTACATTAACAGCTATTTATAAAAC TATTTGTCATTATGTATTAAGGTCCTAAAACACGTTGGGAGAGGAAAGAGCTCTTCCTTGAAGTAGAACTCCATGTGA GAAATTGCAATGGACTTTGGTAAATCACCATTTTATTTTATTTATTTTTTATTTTATTTTTTTTTGAGACTGAGTCTCATT CTGTTGTCCAGGCTGGAGTGCAGTATCGCGATCTTGGC |
| 10 | Humanized mouse model donor sequence of crpE2 | TCCTTTGGTACAGCCGTGGGCTCTGGTGTGGTGACCTTGAGGCAGGCATGCATTTTAGCTTCAATATTTGAAACCACC GGCTCCGTGTTACTAGGCGCCAAAGTAGGAGAAACCATTCGCAAAGGTATCATTGACGTGAACCTGTACAACGAGAC GGTGGAGACTCTCATGGCTGGGGAAGTTAGTGCCATGGTTGGTGAGTAGCCTCTTCTCTATACCGATGGGACATCCGC CCACTGACCCGCTTCCCTGCAGTACTTTGCCCATATCCATAAATGTGATTTTTGCTTTGGCTTTTCAAAAACTGGTTGG ATGTTTCACATTTACTCTCTTATTTTTGCTTTCTGCATATTAATTTATGATTTTTAAAGTGTTTAAATATCAGGCAACCAT TTATTTTCTGAACTTTTGAGCAAACTTGGTTGCTAAAATAGATTTCAGCTAAGTGAATACTTTTCAGAAAGTACAGAA |
| | | AAAGACCTTTGTAGAACAGCGCTTTATTATTTTAGAGTTGTTTGAAGGTTGTCTAAGTGAATGTGTTTTCAAGGTTGA TGTATGAAATTTCCAGGAAGGTTGATTTAGATTTTTTTCTTAAGGTTTTTCATCAATTAGGAGGCAATGTTTTGAAAAC TTGGATGTCAAAAATAATATTTATTAGTCTAACTTTTATATTCACAGTTTAAGTTCTAAAGAAGTTACCAAATAAATCTT AAGTGCGTGTGTTTCAAGAAATTCTGGTCTCTTACATTATTTGTCATCCTTTTGTTGGCTTTTGTTTTGATTTTCTGATT CTACCTCTGGGAGCTCCATTTGGTATGTTTGTTTTGTTTTCCGGCCAAAAAATAATGTGCGTGTTGCCTGGTGATAGCA TGACCTTTTATTGCAGTTTCAAGTTGTCTACATGAAGTGTGGAGGTGTGTGAACTGTGTGAATAAGCAGAGTGGCTG GTTGGACAGAGTGATGTCCCCATGGCCTAAGCGGACACAGATCTCGGCCCCGTGGGTCTTCCCATCACTGCCCTCGC TACGACAGAAAAGAACCTTCCCGGCAGGCGTGTGTTAAGTGCAGCTGACAGGATTCTTTTTCTGCTTGCCTCTAGGT AGAGATGACGACACAACAGACAGCTGATGTATACGGGTAATTTGGTTAGGGAGAAGAGGGTTAGAGAGGAATTCTAC CACCTTCTGCTTTCAACAGGACATATCGTCTTGTAGGAAGAGTCCTGCAGCAGGAGGTAAGATGCTGGTGTACTAAG AATACAGCCACATTCCCCCGAGTTCTTTATTTTGTCACTTACATTTTTTCTTTTCTTTTTTTTTTTTTTTTTGAGACAGA GTCTTGCTCTGTGGCCCAGGCTGGAGTGCAATAGTGTGATCTCCGCTCACTGCCACCTCCACCTCCTGGGTTCAAGCT ATTCTCTCACCTCACCCTCCGGAGTAGCTGAGACTAAAGGCGTGTGCCACCGCACCTGGCTAATTTTTGTATTTTTAG TAGAGACAGGGTTTCACCATGTTGGCCAGGCTAGTCTTGAACTCCTGACCTCAAGCAATCCACCCACCATCACCTCT CAAAGTGCTGGGATTACAGGCGTGAGCCACCACACCTGGCCTGTCACATTTCTTAAACACCTGGAATTTAGAGATTAT TGACCAAGTTCACAATGGCCCAATCTCATGTTGTTCGAGGCGTCTGATAAGAAGCAGCCTGTTGTACTAAGGAATTGC ATATTCACCAAGCACTGTAGGCCAGTAACACACACCAGGGAGCTCCTACTTCTCAGCTTGACGCTTTGTGTTGTAAA GATGTCAGGTTCAACTTTGGGAGGCCCCCGAGATGGGCAGACCACTTGATCCCAGGAGTTTGAGACCAGTCTGGGC AACATAGGGAGACCCTGTCTGTACAAAAAAACTAAAAAATTAGCTGGGCATGGTGGCACATGCCTGTAGTCCTAGCT GCTTGGGAGGCTGAAGTGGGAGGATTGCTTGAGCCCAGGAGGTTGAGGCTGCAGTGAGTTGTGATTGTGCCACTGC ACTCCAGCATGGGCGACATAGCCAGACCCTGTCTCAAAAAAAAAAAAAAAAAAATCAGGTTCAAGATCACTTTCAT TCCAACAACTATAGTCATTATTGAATTTTACTTACCTTCATGGAACATATGTATATCTCAGCATAGTCAGATATAATGGA AATTTCTCTTAGAAAAATAACCGTTCTTTGTAAAATTCACCTTTTTAGTTGTGATAGATATCTAAGATTTAATTCCATCA AGTGGAAATTAAGATTTTTCTAGTCAGGAAAATCTGACTAGATGTGTTGAAAGTATGGGACAAATCAAGAAAAACAC TGACAAAATTAAATCTTTTTTTTTTTTTTTGAGACGGAATCTCGCTTTGTTGCCCAGGCTGGAGTGCAGTGGTGTGAT CTCGGCTCACTGCAACCTCTGCCTCCTGGGTTCAAGCGATTCTCCTGCCTCAGCCTCCTAAGTAGCTGGGATTACAGG CACCCGACACCACACCCAGCTAATTTTTGTATTTTTGGTAGAGATGGGGTTTCACCATGTTAGTCAGGCTGGTCTCAA ACTCCTGACCTCGTGATCCTCCCACCTTGGCCTCCCAAAGTGCTGGGATTACAGGCGTGAGCCACCACACCCGGCCA CAAAGTTAAGTCTTAACTGAAGTTTTTCCTTTTGATGGATAAACTTTAGAGGCATGGTTCTGCTTATTAAAGAAATTTG TTAGGGAAGCCACGCTGAAGACCTCCGTGTGACAGAAGGGTTCATGAACAAGGCTTCATCTGCTCACTCTTTCTGCA GACGGGATCATTTCTCCGTTCCCCACCCCTGGCGTTTGGCTCTTTACTGAAAGAATGAAGGATACTTACCTCAACTAG AATTTGGGTTTACCAAAGCAATTTGGGGTTTTGCTTGAATAACTTGATTTTGTTTGCTTTGGTTTTTAAACTTTTTTTTT TTTGGTATGTGGTTAAATGAATCTCAAAGCCTATTTTGTTGGGAATTTGTATGAGTTACCTAGATTCATAAAATCGGGT TCTTTGCCCTGGGAGCTATAAATTTAAGATGGTGGTGTAATTACCATTGGCCAAAACCACAGCTGATTCTGTCCAGAT GCCTGAATTCTCCAGTCTTCCTCTTCTAAATCCAACAGTCCTTTTTTCTTCTCCCTCTTTCCCTCTGTCCTTAATTCTAT TCTGTTTGCTTCTTTTGTTTTCTCTGTTAGTCACTTCTCCCCAAGCCCCTGGGCGTCTGTGCTTCCTGACAGAGTTGTG TTAGAGTTAAGGTGATTGGGTAGAAATTATTGCTGCCAGTGAAACGTCCTGAACAAGTCATCTTGTGGGACTAGCTCA GCCCTAAAGCAGTAGTCTGTCCATTTTGTAGGAAACCACCAATGGTTCCAAAAATCTGAGAATTCAAAACTCTGCATC TTTTTTTGTGGAAAGACCCACACTAGCACAAACGAGCCAGCTCCTCGAAACCCTTAAGAAGCACACAGCCCCCTTCC AGCTCCCTCTGCCCAGCCACACTCTGCCACCCCACCCCCGCCCGGCAGCCTCCCTGGAGCCTGAGCCTTTGCTCTGC |
| | | CGTTTCTTTCATCTGCACGGGAATGGCGGCCTCTGAGATGCCCATCTCCCCACTTTCCCTAGGTCGTGGCTTGCACTC TTATCCCATGCTTCATCTCTTTCCTCTGTCAGATGCCATTATCTTACATTAAAGTATGTCTTTTTCCCTCTGTTCTTCCTG ACACTAACATGATCCCATTTGTCTTCAGACACCCCTGAGGCTAAGGTGCTATCACCTACTCCCAGGCCCTCTTGCAGG TATTTCCTGGCCCCACCAGGCCCTCCCTCAGCTGTCCCCTGTCTCCTCCCCTCCCTGCTATCCTCTCTCCTCCTTCTTC CCTCTCTCAGGTGGTCCCATTACTCCCTACCTTTCTTTACAAACTCTCCTCCTCCAGGATGTATTTTGTAAAGTCAGCC ACAGAAAATATAGCAGCTTTCAGGAAGGACAAATCTTATGGTCTGAATTCAATTGGGTGGGTTGATATCACTGTCTCA CATCATGAGATTATTTTAATTGTAAGAATATAGGCTGGTAGCGGTGGCTCACACCTGTAATCCGAGCACTTTGGGAGG CCGAGGCAGGCGGATCATGAGGTCAGGAGATTGAGACCATCTTGGCTGACGCGGTGAAACCCCGTCTCTACTAAAAA TACAAAAAATTAGCCGGGCGTGGTAGCGGGCACCTGTAGTCCCAGCTACTCGGGAGGCTGAGGCAGGAGAATGGCG TGAACCCAGGAGGCAGAGCTTGCAGTGAGCCGAGATTGCGCCTGGGCAACAGAGCAAGACCCCGTCTAAAAAAAA AAAAAAGAATATAAACCATATCATGTATTTTCTAAGCTATGAATCCGTGCTATGAAACGTGCTCCAAACCCTGGCTTTC TTTTTAAAACCACTAGTCCCCGAGGAGCCCAGGGCTCAGATGTGGAGGCTTAAGGAGGTGTCCGCGGGTAGGCCTG GCGCTGCCATGGAGCCTGATATGGGTTTGGCAGCACAAGGCTGGCCCCTTCTCCTCCCAACGCACGCCTTAAGGATT GACACAGGGTCACAGCCAGCCTCAGCTCAAAGGTCAGCACGCCTAGCACTCATGGCAGCAACTGTGTGTGTTGCTT CATGGACTGGGTCCTGCACAGCCATAAATGATCCTCCTGCAACTCAGATGCCTTTGAAGGAAATGCCAGGGGTCCCC TGAAAAGTGCTGCTGCTGCCAGGCAACCAGGTTTCCAGAATTAACGTTTGGGATTAATAACGTGCCCAGCTTATCAG CCAGGCCCTTTGTTCCCCTACAGTTCCATGGGAGCTTTTCAACAACTGCTTCTGAAAGTGATCTCTCCCGGTTGATGC TTTTAATACTCACTACAAAATCTGATCTGCTTATGAAAATATTTGAAGTGAACTGTAGCCTAGAAAAACATGAAACCT AGAAAAGAGTTGGTGAAATTGAGGTAAAAGGACACAAAGCCAACACTAATGAGAACCTCTTAACAACAAGGAATCA GACACACAAATAAAAAGTCCAACATTCTGGGTAGGATCTGAGAGCAGATGCCCAAATTTGGGTTAAATATCATAGTTA TTTCTCATAGGAGAGGCACCATCAACAAGCAGTATGGGCTGGGTGCAGTGGCTCACTGCATCCAGTGAGCCTGTCAT CCCAGCACTTTGGGAGGCCATGGTGGGAAAATGGCTTGAGCCCAGGAGTTCAAGACCAGCCAGGGCAACATAGCAA GACCCCCGTCTCTGCAAAAAAATTTTAATAATTAGCTGAGTGTGGCAGCTCATGCCTGTAATCCCAGGGCTTTGGGAG GCTGAAGCAGGAGGATTGCTTAAGGCCAGGAGTTCCAAACCAGCCTGGGTAACATAGCAAAATCCTGTCTCTGCAA AAAAATTTAAAAATTAACCAGGTGTGGTGGTGCACACTTGTAATCTCAGCTGCTTGGGAGGCTGAGACAGGAGGATC ATTTGAGTCCAGGAGTTTAAGGCTGCAGTGAGCCATGATTTTGCAGCTGTACTCCAGCCTGGGCAAGAGTGAGACGC TGTCTCTACAAAAGAAAAAAAGAAAAGAAAAGAAAAAAGCAACATGGTATTATACTCTCTTATATAGATAATATGACC TAGCCAATATCTGGTCCTTTTCATTATGTGACTGTAAGGCAGAACTACAACAAAATCTTTTCCCAATATATTGCCTTTCT AGTCTTTCTGTGTTCATTCGAGACTTTAATCTCTTGCTTGGTTTTGGCCAAAATTCACCCCAGAACAAGTTCATACAC AGCACCCTCACTGAAAACTGACAAGGGCAGTCAGTGTGTAGCACCAGGAGACTTGCCTTTAAATGCATAATCTGGCC GGACACAATGCCTCACGCATGTAATCCCAGCACTTTGGGAGGCCAAGGCGGGTAGATCACTTGAGGTTAGGGGTTCG AGACCAGCCTGGCTAACATAGTGAGACCCCATCTTTACTAAAAATACAAAAATTAGCCAGAGGGCTTGCTTGAACCC AGGAGGCAGAGGTTGCAGTGAGCCAAGATCAGGCCACTGCACTCCAGCCTGGGCAACAGAGCAGGACTAGCTTCA AAAAATAAATAAATAACCATCATCCCTGTCCCCTCTGTGTTGAGGAAGGCAGAAGCTTTCCTTTAACTGAAGTTAATT GAGTGATTTTTGTCCAGGCTGTAGCGCTTTGTAAAGAAACAATTCACAGAAAATTAAACTCTGGGAGATGTTGTTATG GAAAACATTTGGATTTCTTCCCTTGGTGTCTGCACCTCTGTACTCTGTATCTGATGGTTTTTTGTCCTTCAATCTGTAG GTTCCGCTGTGTGGCAGCTGATTGCTTCCTTCCTGAGGCTTCCAATCTCAGGAACGCACTGCATTGTGGGTTCTACTA TAGGATTCTCACTGGTCGCAATCGGT |
| 11 | Humanized mouse model donor sequence of crpE10 | GCCTCCAACATCGGGCTTCCAGTCAGCACCACGCACTGTAAGGTAGGCCCCTGCTAGCCGGGAGCCCTTCCCCAGGC CGCTGGGACAGGTCCAGGGCCACCGGAGCGCATGCAAGGGAAGCTGTAGGGCTCTGTAGATGGTTGTGCAAAATGC |
| | | AGCTCCAGGTTCTCCAGGTCTCCGGGACCTTGTATTCCTCCATCTGTTCCCTCCACCGTCCTCCTCTCGGCAGGACGA CACTATCTCTCGTTCAGACCTGGCTTTTCCAAGGCAGAATGCCAGGGCTGGCCTGCCTGGGACTGGCCTGCTATCCTC CCACTGTTTCCACAGTGGAGTTTGATTGGCATCCTGTGTGCGCTGGGCATACCTGTGGGCTCACTTGGGAGCCTGCC ACTTGGTCCAGCCAATGAGGGGACTGGGGGTTGACAGTGTTAGAAATTAACACAAATCATCTCTGGAATTGTAGGAC ACAGTTGTTTCTGGTTTTGTTCCTTATATGATGTTCAAAGTGACTATATTGCCATAAATTCCCCACGAGGGGTAATTGCT GATACTGACCACTGCGGAGTGCTCTGAAACCTGCAGCTGGACAAGAGAGGGCTCCTGGTCATGGTGTGGAAAGTTG GAGCTGCAGGGAGGAGCCCTGAAGACCAGTGAAGAGGAAATGGGTTCCGGGAGTTTCAGGGAGTGATCCACAGGT GGCCAGGGTGGGGAGGACCAGAACCCAGGTGGGGAGGACCAGAGCCCCGGTCTCATCACCCAACTCTGCCCAGTAT CTGCAGATACTTCTAAAATAGCTCAGGTGAGCCTCTGTATACAGACAAACTCACCAGTCTCAACAAAGCGAAAGTCA CTTTGAGATTTTTCCTAGGTTCATTCATTTGTATTATTCAAGAAAAACTAATTGGTGTTTGCTGCTTGGGAGCAGAGCT CTCAGCACCAGGAGGAATATATGAGCCCTCAAAACAGACACAGCCACCCACGTTTCTATTTACTCACAATTTTAAGAT TCCAGCCAGTATGTTTAAAAGACAACGCCAAGCAAAGGCAAGTGGCTTCATGCTTCAAACACCCAGGTCAGGTGGC CCTCACAAGAGTATCTGGAGTGGCCCAGCAGCGTGTTTCAAAGCTGGGGCTCCACGCATGAAGAATCAGCCTCAGT GGAAAGAGTTTGTTTTTTCCTTTTTTGCTACCATATACAGAAATACTTCAATTTCCACAAAGTTTCTGTATACAAATCC CCCCACTTACTGATCATCAGGAGAGTGCTGGGTACCGTGCTCAACACCACACGCATGGCACACCGTTCCCTCTCCAC AGCAAGCCTGTGGGGGCGGAACTGTCACTCCCAGTCCACAGGTGAGGAGACTGGGTTCCCTCAGAGTTGAGCCGCT TCCCACATGCACACAGCCACATGTGATAGATCCAGGATTCAGATCTTCACCTCAGGCCACACACCTTCTCGTTCAGGT CCCCATCAGCACCATTGTGGTGCCCGACCATGGCCAAGGTGCCTGGGGCAAGCACTCCACTGGTGGTCTCCCCAAGA AGCTTCCCCAGCTTCATAGATGAGGAAACTGAGGCAGAGGAAAGTTAAGTAACGTGCCCGAGATTACACAGCCAGG GAGCAGCCCAGCTGAGGCTCCCACATGGACGGCATGGCCAGCGTCCTCCTCCCCGGCTCCGCCTGGCAAGCATGGG GAAGGAAGGCAGGAAGAGGTGCAGGTGTGCCCGCCACCCATTCATTGTGAGTTTCCTAATGTGGGTTATTTAAAACT TACCAGAGAAGCACATATTCACTGAAATATTTCAAACAATACAAGACTATAAGGAATGAAAAGTGACATCTTCTCTGG ACTCCACATCACTTCCATTCACTGTGGTGTGTGGAGCGTGGAGGAGACGCGTGTCACTAAATACATTCTTTTCCAAGG ACACGGGCTTATGACTTTTCTATAGGCATCAGTTGTCTTTTGGAATGCTTTTAAACAGATGTAATGCCATCTTTTTAATG ATTAACCTACACTAAAGCAAGACTGCTGAAGGCAGACTCTTCTACACCAGTTGAAATAGCTTTTTTTTGTTTTGTTTT GTTTTGTTTTGTTTTGTTTTTGAGATGGAGTCTCACTCTCATCCAGGCTGGAGTGCGGTGGCATGGTCCCAGCTCACC GCAGCCTCTGCCTCCTGAGTTCAAGCAGTTCTCCTGCCTCAGCCTCCGGGTAGCTGAGATTACAGGCATGCACCACC ACACCCAGCTAATTTTTGTATTTTTAGTAGAGACATGGCTTCACCATGTTGGCAAGGCTGGTCTCGAACTCCTGACCT CAGGTGATCTGCCTGCCTCAGCCTCCCAAAGTGCTGGGATTACAGGCGTGAGCCACCGTGCCTGGCCTGAAAGAGC TTTTGAAAAAACAGCACAGGTTTGGTTCTTCCAGGTTGGTGCTACTTAGCTAACTGTATGAGCAGTCTAATCCGTTTG TATGTATACTGTATACAGTGTTGGAATGGACAGTACTGATTTTGCTAATAACACTAAACTCTAGAATACAGTTTATAGG AATAGATGAAAAGTATAAAATGCTTATTTAAAAAGCCAAACTCTTGAATTATCTATATGCATGATTTCCCCAATACTGTA TTGATATATATACTATAATGATGTGTGACTAAGAGATTTACATCCAGTTTGTACAGGACCTTAGTGAATAGATGTATATG TTATCATTATCCTAAAATACCTGTACAAAGTACATGATAAGGTTTTTTTTTTGAGATGGAGTCTCGCTCTGTCACCCAG GCTGGAGTGCAGTGGCGCCATCTCCACTCACTGCAAGCTCCGCCTCCTGGGTTCATGCCATTCTCCTGCCTCAGCTTC CTGAGTAGCTGGGACTACAGGTGCCCACCACCAGCTACTTTTTTATATTTTTAGTAGAGACGGGGTTTCACCGTGTTA GCCAGGATGGTCTCGATCTCCTGACCTCGTGATCCGCCCGCCTCAGCCTCCCAAAGTGCTGGGATTACAGACATGAG CCACCGTGCCCAGCCAGTTTTGTTTCCTTTGAGCTGGTGATGAGTAGTATTAGGATAGCTTCTTGGCACTTAAAGCAG GTCTCCCTGAATGGGACTGCAGCAGGGTGCCATCTATAAAATTGACAAGACAGGTCACAACTTGGATTTGAGTAAGT TTGTCCGATATCATTGATCGTGGACTTCTGGACAAAGCCTAGAGCTATTCCCTGTGAGCACTAAGGACAGAGTCTCAG |
| | | CTCTCAGGAACTTGCCGTGTAGTTGGAGAGCAGATGGCTCTCATCAGAGTCCAAATAATAGAAGTGCTGAGAAGAGC TCACAGTTGTCATTACCCCATAACACTTATTCTCTCATATCACATATTTCCCCTGCACACAACAGTCCCCAAATGGCAA ACGTGGGATGAACTATGAGAGCTGTGGGATGGCGTGAAGAATGGGGCCCTCTGTAAGTGAGACTGTGAATCTGGAA ACATTGAAAGGGTATTGATGTCCACATAGAGTCATAGTTGAATTGCATCAGTTTTCAGGATAATTATCCTGTGTTTGAT TTTTAAGTTTCATGCTTAGAGCCATCTAATAGAAAGTAGGGAGAAGTTGGGGACTTGGCAGGAAATAAGATGGAAAC TATTGTTTAAAATGAGGTTTTTAATGTATCTAGGTAGATGTTATTGATTTATGCTTCCTTCTAAGTGTACATGGCTAAGT GCATAGATTTGTTTTGTTTTGTTTTGTTTGAGACAGAGTCTTGCTTTGTGGCCCAGGCTGGAGTGCAGTGGCTTGATC TTGGCTCACTGCGACCTCCGCCTCCTGGGTTCAAGCAGTTTTCCTGCCTCAGCCTCCTGAGTAGCTGAGATTACAGG CATGCACCACCATGGTTGGCTAATTTTTGTATTTTTAGTAGAAATGGGGTTTCTCCATGTTAGCCAGGCTGGTCTTGAA CTCCTGACCTCAAGTGGTCTGCCTGCCTCGGCCTCCCAAAGTGCTGGAATTACAGGCATGAGCCAGTGCGCCCGGCC TAAGTGCATAGTTTCAATTTTGTCGTAGCCTATTTCAAAACATTTATGAAACCATACTATATTATGTGATATATGAACTG AAGGTATAACTTGGGAAAAAACAATTAGGAAAGTCCTGTTATCACATTTTATTCCAGAAGGCATATATAAGGCAGTCT GTTTGATGAGTTACACCCTCTGTATGTATTCTGGTTTTATATATTTTGTATTTCATATCAGAGGACTCAGTTTATCAGATA TTTTCCTGGAATAATACCTTTGAGTAGGGTCAAAAGCCTTAATTGGCCAGGTACGGTGGCTCATGCCTATAATCCTAGC TACTCGGGAGGCTGAGGCCGGAGAATCGCTTGAACCCGGGAGGCGGAGGTTGTGGTGAGTGGAGATTGCACCATTG CACTTCAGCCTGGGCAACAAGAGCAAAACTATCCCCCCCCGCACCCCCCAAAAAAAGCCTTAATTGAAGACACAGA AACCGGTTTTGAAAATAAAAGAATCCTCAGATGTTATGTTTCAGCAATTCGTTCATCATTCTTCCCTTCTGTGACCCTT AATCACGTTTGGTCTTTAGCCCTAGCAGATAACACACAGTGCCACTACTGAGCACAGTTGCTCATGTGTGTGCTCTTT AATAGGAATTTGGAAGTCATATTCTGCAGAATGTTTTAATTCCATACTGGGTGGATTGAGATTATTCCTGTTTGTGGATC ACCCACATTGTCAGTCCTCACTGATGACATAGATGATGATTCAACCACCATTTCAATTTGGAACAGCATACAGGGAAC ACTACTGATTTCAAATGTAGGATTATAATTTCTAATTTAATTTCTAATTCTTGTTCTCTGGTCCCCCTTCTTCTCAACAAT TTTTTGCTATAAGTACCTGATGATGGCCTTAAACTGGAAACACACTCGATTTATTAAAAGGGAAGTTAGAACACAGCA AAAAAATAACATTTTACATTCACGGAAGACATGATCAGTAGCAAAGACATAGTACTCTGGATCTTTTATGAATCAAAT AACATGTAACTAAATACATAAAATTGTTAAGAACTCAGAAATTACTGTCTTAGCGTATGATAAATGAAGTATAGAAAA AGTATGTGGGAATATGAAGAATTTGAACGTTTATTTAGAACAGCATAATTAATAAATATGTACAAGTTTTTACGCTACT GATCATATGCCTTCCTGTCAAGCATCGTGAAACATTTATAAAAATCCTAAAATCAGAAGACCATGATAAAAATCTCTAT AAAATTTTAAAAACAGACCTGACATGGTGGCTCACACCTGTAATCCCAGCACTTTGGGAGGCAGAGGTAGGAGGATC GCTTGAGCCCAGAAGTTTGAAACCAGCCTGGGGCAACATAGTGAGACCCTGTCTCTATTTTATAAAAGTTAAAATTTA AATTAAAAAAAAAAAAAGTTTAAAGATAAGTTGTGCTAACCATGATCTCTAATTACAATAAAATTAAACTAGAAATTA ATTATATATTGAAATAAGAATGTATCCCAATGGAGACTTTTTAATGCTTTTTTAAGGTAACAATTGAATTAAGGAGTTTA GAAAAATACAAGGATAGCTTTCTTGGAAAATAATAAAAACCAAATGTGTGTGTCTGTGGGTGGATACAAACAGCAAA ACCTGTGGCAACCAAGTTATGCTAAGATGAAAATTCATATCTTTAAATGGTTTAATTGATCAAAGGGAAAATAAAATA ACCAAGTATTAAAATTAGGATTTTGTAAAAAGTCCAGGACAGTAAGAATTGAGGAAACAGGTTGTAAGAAATATAGA AGCAAAATTAATTTAAAACAAAATAAGAAGGGGAGAGGAAAGTGGCTACTAACAAAATGTCAGATCACATCAAGGA AAAGAGGGAGGGGAAGGAGGAAGACATTGTAACAACAGAGAAGAGAACTGTTTCCATTATTAAATAAGCCACACAG CTATATGCTAAGAATTTGAAAACCCATGTGAATTATTTTCTGCAACAATATACGTGACAAAGAAATATTGGCCTAAGAA GTAGAAAAAAGTGGAAAATCTGACCCAGAACAATAGGATGAAATTAAGGGAGTTCTCCAAAAATAATTGTTCCCACC TAAAAAAGCAAAAAGGGGCTAGGGGTGGGGCGGGTGAGGATACTGCTGACTAGAGGAATTCATTAGAATCTTTTCCA TTTAAACAAAAAAAAACCCAGCTCTCACACGATCTCAACTTTCCCACAATGTAGGAAGAAAAACAAAACTAAAAAA CCTCTCCCATTTGTGCTATGGCGTACACTGAATAACCCTAATACCAAACCTAATTTAAAATATCACAAACAATGAAATC |
| | | TGTTTACCGATGAATATTGATTGTAAAAACTTTAAGCAAGATGCTGAAAAATGACATCAACAATATTTGGCTGGGCAT GGTGGCTCGTGCCTGTAATCCCAGCACTTTGGGAGGCTGAGGTGGGCAGATCATCTGAGGTCAGGAGTTCGAGACC AGCCTGGCCAACATGGCAAAACCCCGTCTCTACTAAAAATATAAAAATTAGGCAAGTGTGGTGGTGGGTGCCTGTAA TCCCAGCTACTAGGGAGGCTGAGGCAGGAGAATCGCTTGAACCCAGGAGGTGGAGGTTGCAGTGAGCTGAGATTGT GCCACTGCACTCCAGCCTGGGCGACAGAGCAAGACTCTATCTCAAAACAAAACAATACTATACAAGAACCTTTCAGC AGGACCAAATTGATCAAGATTACCAGAAAGTGGGCTCCCTTCGTGACAGGGCAGGACTGCATCACCCACCACCAGC AGGCTGTGCAGGGCAAGGTCCTCGCAGGGGCTCAAGTTGGGTATATAGGCAGCTTCAGTATAATATGCCTTAGCAATC CACTTAAAAAAAAAAAGATCTCTGTTGAGATATAAGCCAAAAAGACATTTGATGATATTCAGTATTTCTTTTTTTTTGA GACGGAGTTTCACTCGTTACCCAGGCTGGAGTGCCATAATGGCATGATCTCAGCTCACCACAACCTCCACCTCCCGG GTTCAAGCAATTCTCCTGCCTCAGCCTCCCGAGTAGCTGGGATTACAGGTGTGCAGCACCACACCCAGCTAATTTTGT ATTTTTAGTAGAGACGGGGTTTCTCCATGTTGGTCAGGCTGGTCTCGAACTCCTGACCTCAGGTGATCTGCCCGCCTC GGCCTCCCAAAGTGCTGGGATTACAGGCATGAGCCACCGCACCCGGCCGATATTCAGTATTTCTAAAACACTACTAGA GTTCGTAAGAGTGACCAAGTCCAAGGTAAAAATAGAAAAATCCAGCACTTGTTCATCAGCAATAGCCAATTAGAAAA TGTAACGGGAGGGAAAAAAACTGCCACCATAATTGCAGCAAAAATAAAAGTTATCTCAGAATAATCCTAATAAGCAC TGTGAGTGTCATCAGGCACATGGTGTGTTCTTATAGTCCCAGCTACTCAGGAGGCTGAAGGTACGAGGACAGCCTGA ACCCAGGAGTTCAAGACCAGTCTAGGCAACATAGTAAGACCCCATCTCTAAACAACTAAATAAATAATTTTTTAAAAA AGAAATGTGAGTGATATATATGGAAGAGGCACAAAACTGCTCAGAGGTATAAAAAGATAATCTGAATATGAGACGTAA TGTGCTATTGGATGGGAAAACCAGATGTTGTAATGAAGTCACTTCTCCCTAAATACAACCTGAACAGGATTTTACTTG GAATTTTACAAAACGAGACTAAAGAATACATTTTGGTGAAATAAATTATCAGCAATATAAAATGATCTAACAAGAAAA CTGAAAGGAGGAACTAGCCTTACCTGACGTATAATGTCATATAAGGAAACAAAAGTCAAGAGTAGGTCCTGGGAGGA GGGGCTAGAACTGGGTGTTAGTGTTTTATGGGGCAGTTTCCCCGTGGGATGATGAAAAAGTTCTGGAGACAGATTGT GGTGATGGTTTGCAAAACAATGTGATTGTACTTAATGCCTCTAAATGGTATGCTGAAAAATTACTAAAACTGTAAGTTT TATGTTATGTATATCTTACCACAATAATTTTTAAAAGTAGATACCTGGATAAGAACAGAGTAGATAGCAATGGATGGAA CCTGTTCTATATGAGCATGTATTTTGAAAAAGGGAAAATTTAACCAGTAAGTGTTTGAAAGGTTGAATAGCTGACTGT CAGTCTAGAAAAACAAAATTAGACCACTCTCAAAATACATGAACATAAATCTGATGGCTTAAATAATATTTTAAAAATA TTCTTAAATTTAGAGTAAAATGGAATTTTATTTTAGAAAAATAAAATGGAATTAAATATTTATCAAATATTCAAGCCAAG GATAAATCACAGAAGAAAATTATAGCATATTTTATTGCATGAAGTTAGAAACTTGAAAGTGAAACAAAGCAAAATACT ACCTTAAGAAAATGTGTATTCGATATCACAGAGTTAATATTATTCAGTGAAGACTACACATAAATAAATAAGGAACACA TTAGATTTTAGTATGTAAATGGACAAAATACATGAAGAGACAATTTTTTTTTTTTTTTGAGACGGAGTTTTACTCTGTC ACCCAGGCTGGAGTGCAGTGGTGCCATCTCGGCTCACTGCAACCTCCACCTCCCCAGTTCAAGCGATTCTCCTGCCT CAGCCACCCAAGTAGCTGGGACTACAGGTGTGCACCACCACGCCTAGCTAATTTTTTGTATTTTTAGTAGAGATGGGG TTTCACCCTGTTAGCCAGCATGGTCTTGATCTCCTGACCTTGTATCCGCCCGCCTTGGCCTCCCAAAGTGCTGGGATT ACAAGCATGAGCCACTGCACCTGGCCGACAATTTGTATATGTGAAAGCTAGAGAGAGTGAAATTCAGTAAATATATTT TTTAAAGTTCAGCCTTAATTGTAATTAAAGATACGCAAATTAAAACAGCAACAGTGCATCTTTTTGTCGGTTAATTAGC CAAAAAAAGAGCAACTTACATGATATCATGTGCTGATATTCACAGATTATGGATGACACTACATTAACAGCTATTTATA AAACTATTTGTCATTATGTATTAAGGTCCTAAAACACGTTGGGAGAGGAAAGAGCTCTTCCTTGAAGTAGAACTCCAT GTGAGAAATTGCAATGGACTTTGGTAAATCACCATTTTATTTTATTTATTTTTTATTTTATTTTTTTTTGAGACTGAGTCT CATTCTGTTGTCCAGGCTGGAGTGCAGTATCGCGATCTTGGCTCACTGCAACCTCCACCTCCCAGGTTCAAGTGAGTA GCTGGGATTCCAGGTATGCACCACCACACCTGGCTAATTTTTTTTATTTTTAGTAGAGACAGGGTTTCACCATGTTGGT CAGGCTGGTCTCGAACTCCTGACCTCAGGTGATCCACCCGCCTCGGCCTCCTGAAGTGCTGAGATTACAGGCGTGAG CCACCATGCCCGGCCTGGTAAATCACCATTTATAGCCATCGTCATAAACATTGGATTGTGAAATCTTTAGCAGAAGG GGAAAGTGAGGTTTGATAAGATGCAGGATATTTTAATCATATTAAAAAGTCTCTTCACAGATTGTGGCAAATGTCAAA AAATAGTGAATTGGGTAAAGAGAAAACAGGAGTTCTTGCAGCTTTTCTCTAAGTTTTGAATTATTTCAAGATACATTTT AAATGTTCCCTGTTAACTACAGTCGATTTCAGGGAATTTTTTTTTTTTTTTTTTTTTTTGAGACAGAGTCTCTCTCTGTC GCCCAGGTTGGAGTGCAGTGGCATGATCTCCGCTCACTGCAAGCTCAATGCAAGCTCCGCCTCCCAGGTTCACGCCA TTCTCCCGCCTCAGCCTCCCAAGTAGCTGGGACTACAGGCGCCTGCCACCACGCCTGGCTAATTTTTGGTATTCTTAG TAGAGACGGGGTTTCACCGTGTTAGCCAGGATGGTCTCCATCTCCTGACCTCGTGATCCGCCCACCTCAGCCTCCCA AAGTGCTGGGATTACAGGCGGAAGCCACCGCGCACGGCCGGGAATATTATTTTTAAAGCCAAAATCCAACCTGGGCA CAGTGGCTCACGCCTGTAATCCCAGCACTTTGGGAGGCTGAGGCGGGCCGATCACTTGAGGCCAGGAGTTTGAGAC CAGCCTGACCAACATGGCGAAACCCCATCTCTACTAAAAAATATGAAAATTAGCTGGATGTGGTGGTGCTTGCCTGTA ATCCCACCTACTCAGGAGGCTGAGACAGGAGAATCGCTTGAACCTGGGAGACATAGCTTGCAGTGAGCTGAGATTG CACCACTGTACTCCAGCACTTTGGGAGGCCAAGGAGGGAGGGTCACTTGAGGTGAGGAGTTCCACATCAGCGTGGC CAACATGGTGAAACCCCGTCTCTACTAAAAATATGAAAATTAGCCAAGGGTGATGGCATGCGCCTGTAATCTCAGCTG CTCAGGAGGCTGAGGCAGGAGAATTGCTTGAACCTGGGAGATGGAGGTTGCAGTAAGCTGAGATCACACCACTGCA CTCCAGCCTGGGTGAAGAGTGAGACTGTCTCAAAACAACTATATAAAACAACTATATAAAGAGCTATATGCTGAAGA GAAGAATCCCCAAACAATTCTACCTAAAATCTAAGCTATTTTAAAAGCTTTAACAAATGTAGGGGACTGGGTAATGTT GTACTAGAGCTTGTTTCTGAGATTAGCCCATGTACATTGGTAGAGTGTAGCCTTTGTGGCTCACCTGTTTTTACTTTGC AATGTCTGCTCCCACAGGTGGGCTCGGTGGTGGCCGTGGGCTGGATCCGCTCCCGCAAGGCTGTGGACTGGCGCCTC |
| 153 | Human XPR1 protein sequence | MKFAEHLSAHITPEWRKQYIQYEAFKDMLYSAQDQAPSVEVTDEDTVKRYFAKFEEKFFQTCEKELAKINTFYSEKLAE AQRRFATLQNELQSSLDAQKESTGVTTLRQRRKPVFHLSHEERVQHRNIKDLKLAFSEFYLSLILLQNYQNLNFTGFRKIL KKHDKILETSRGADWRVAHVEVAPFYTCKKINQLISETEAVVTNELEDGDRQKAMKRLRVPPLGAAQPAPAWTTFRVGL FCGIFIVLNITLVLAAVFKLETDRSIWPLIRIYRGGFLLIEFLFLLGINTYGWRQAGVNHVLIFELNPRSNLSHQHLFEIAGFL GILWCLSLLACFFAPISVIPTYVYPLA LYGFMVFFLINPTKTFYYKSRFWLLKLLFRVFTAPFHKVGFADFWLADQLNSLSVILMDLEYMICFYSLE LKWDESKGLLPNNSEESGICHKYTYGVRAIVQCIPAWLRFIQCLRRYRDTKRAFPHLVNAGKYSTTFFMVTFAALYSTHK ERGHSDTMVFFYLWIVFYIISSCYTLIWDLKMDWGLFDKNAGENTFLREEIVYPQKAYYYCAIIEDVILRFAWTIQISITST TLLPHSGDIIATVFAPLEVFRRFVWNFFRLENEHLNNCGEFRAVRDIS VAPLNADDQTLLEQMMDQDDGVRNRQKNRSWKYNQSISLRRPRLASQSKARDTKVLIEDTDDEANT |
| 154 | Human MYORG protein sequence | MLQNPQEKSQAYPRRRRPGCYAYRQNPEAIAAAMYTFLPDNFSPAKPKPSKDLKPLLGSAVLGLLLVLAAVVAWCYYS VSLRKAERLRAELLDLKAGGFSIRNQKGEQVFRLAFRSGALDLDSCSRDGALLGCSLTADGLPLHFFIQTVRPKDTVMCY RVRWEEAAPGRAVEHAMFLGDAAAHWYGGAEMRTQHWPIRLDGQQEPQPFVTSDVYSSDAAFGGILERYWLSSRAAA IKVNDSVPFHLGWNSTERSLRLQARYHDTPYKPPAGRAAAPELSYRVCVGSDVTSIHKYMVRRYFNKPSRVPAPEAFRDP IWSTWALYGRAVDQDKVLRFAQQIRLHHFNSSHLEIDDMYTPAYGDFDFDEVKFPNASDMFRRLRDAGFRVTLWVHPFV NYNSSRFGEGVERELFVREPTGRLPALVRWWNGIGAVLDFTHPKARDWFQGHLRRLRSRYSVASFKFDAGEVSYLPRDF STYRPLPDPSVWSRRYTEMALPFFSLAEVRVGYQSQNISCFFRLVDRDSVWGYDLGLRSLIPAVLTVSMLGYPFILPDMVG GNAVPQRTAGGDVPERELYIRWLEVAAFMPAMQFSIPPWRYDAEVVAIAQKFAALRASLVAPLLLELAGEVTDTGDPIVR PLWWIAPGDETAHRIDSQFLIGDTLLVAPVLEPGKQERDVYLPAGKWRSYKGELFDKTPVLLTDYPVDLDEIAYFTWAS |
| 155 | Human PiT2 protein sequence | |
| 156 | *gfaABC1D* promoter sequence | TAACATATCCTGGTGTGGAGTAGGGGACGCTGCTCTGACAGAGGCTCGGGGGCCTGAGCTGGCTCTGTGAGCTGGG GAGGAGGCAGACAGCCAGGCCTTGTCTGCAAGCAGACCTGGCAGCATTGGGCTGGCCGCCCCCCAGGGCCTCCTCT TCATGCCCAGTGAATGACTCACCTTGGCACAGACACAATGTTCGGGGTGGGCACAGTGCCTGCTTCCCGCCGCACCC CAGCCCCCCTCAAATGCCTTCCGAGAAGCCCATTGAGCAGGGGGCTTGCATTGCACCCCAGCCTGACAGCCTGGCAT CTTGGGATAAAAGCAGCACAGCCCCCTAGGGGCTGCCCTTGCTGTGTGGCGCCACCGGCGGTGGAGAACAAGGCTC TATTCAGCCTGTGCCCAGGAAAGGGGATCAGGGGATGCCCAGGCATGGACAGTGGGTGGCAGGGGGGGAGAGGAG GGCTGTCTGCTTCCCAGAAGTCCAAGGACACAAATGGGTGAGGGGAGAGCTCTCCCCATAGCTGGGCTGCGGCCCA ACCCCACCCCCTCAGGCTATGCCAGGGGGTGTTGCCAGGGGCACCCGGGCATCGCCAGTCTAGCCCACTCCTTCATA AAGCCCTCGCATCCCAGGAGCGAGCAGAGCCAGAGCAGGTTGGAGAGGAGACGCATCACCTCCGCTGCTCGCAAG CTTTATTGCGGTAGTTTATCACAGTTAAATTGCTAACGCAGTCAGTGCTTCTGACACAACAGTCTCGAACTTAAGCTG CAGAAGTTGGTCGTGAGGCACTGGGCAGGTAAGTATCAAGGTTACAAGACAGGTTTAAGGAGACCAATAGAAACTG GGCTTGTCGAGACAGAGAAGACTCTTGCGTTTCTGATAGGCACCTATTGGTCTTACTGACATCCACTTTGCCTTTCTC TCCACAGGTGTCCACTCCCAGTTCAATTACAGCTCTTAAGGCTAGAGTACTTAATACGACTCACTATAGGCTAGC |

## Claims

1. Use of an intracerebral phosphate homeostasis promoter in the manufacture of a medicament for preventing and/or treating a cerebral calcification disease.

2. The use according to claim 1, wherein the intracerebral phosphate homeostasis promoter is an agent capable of regulating alternative splicing in an intron of the *SLC20A2* gene.

3. The use according to claim 2, wherein the alternative splicing comprises cryptic exon inclusion, and the agent regulates (e.g., inhibits or enhances) the splicing of the cryptic exon from pre-mRNA of the *SLC20A2* gene;
preferably, the agent thereby regulates (e.g., inhibits or enhances) the level of mature mRNA processed from the pre-mRNA, and/or regulates (e.g., inhibits or enhances) the expression of PiT2 protein.

4. The use according to any one of claims 2-3, wherein the alternative splicing comprises alternative splicing in intron 2;
preferably, the alternative splicing in intron 2 is a cryptic exon inclusion between exon 2 and exon 3;
preferably, the cryptic exon is located at c.289+901 to c.289+1035 of the *SLC20A2* gene;
preferably, the cryptic exon has the sequence as shown in SEQ ID NO: 2.

5. The use according to any one of claims 2-3, wherein the alternative splicing comprises alternative splicing in intron 10;
preferably, the alternative splicing in intron 10 is a cryptic exon inclusion between exon 10 and 11;
preferably, the cryptic exon is located at c.1795-1703 to c.1795-1627 of the *SLC20A2* gene;
preferably, the cryptic exon has the sequence as shown in SEQ ID NO: 3.

6. The use according to any one of claims 2-5, wherein the agent specifically binds to a target region in the *SLC20A2* gene or pre-mRNA thereof, wherein the target region is located within an intron region between two canonical exon regions, and wherein the intron region comprises the cryptic exon;
preferably, the target region is located within the region from up to about 100 nucleotides upstream of the cryptic exon to up to about 100 nucleotides downstream of the cryptic exon;
preferably, the target region is selected from a region spanning the cryptic exon splice site, a region within the cryptic exon, a region within 100 nucleotides upstream of the cryptic exon, or a region within 100 nucleotides downstream of the cryptic exon;
preferably, the target region is selected from a region upstream of the cryptic exon 5' splice acceptor site (SA), a region spanning the 5' splice acceptor site, a region within the cryptic exon, a region spanning the 3' splice donor site, or a region downstream of the 3' splice donor site (SD).

7. The use according to claim 6, wherein the target region is located within the region c.289+801 to c.289+1135 of the *SLC20A2* gene or pre-mRNA thereof;
preferably, the target region is located within the sequence as shown in SEQ ID NO: 8.

8. The use according to claim 6, wherein the target region is located within the region c.1795-1803 to c.1795-1527 of the *SLC20A2* gene or pre-mRNA thereof;
preferably, the target region is located within the sequence as shown in SEQ ID NO: 9.

9. The use according to any one of claims 2-8, wherein the agent is selected from antisense oligonucleotides (ASOs) or a gene editing tool (e.g., CRISPR/Cas, base editor, or non-Cas protein-dependent RNA editing tool);
preferably, the agent is an antisense oligonucleotide (ASO);
preferably, the agent is a DNA editor, such as CRISPR/Cas9;
preferably, the agent is an RNA editor, such as CRISPR/Cas13
preferably, the agent is a base editor Adenine/Cytosine base editor;
preferably, the agent is a non-Cas protein-dependent RNA editing tool.

10. The use according to claim 9, wherein the agent is an antisense oligonucleotide (ASO) that specifically binds to the target region in *SLC20A2* pre-mRNA;
preferably, the ASO consists of 10~30 (e.g., 15~30, 15~25, 18~30, 18~25, 20~25) consecutive nucleotides; preferably consists of 18~25 consecutive nucleotides;
preferably, the ASO comprises a modification of backbone nucleotides and nucleotide linkage, a modification of the ribose, a modification of the nucleobase, or a combination thereof;
preferably, the ASO specifically binds to a target region located within the region c.289+801 to c.289+1135 of *SLC20A2* pre-mRNA; preferably, the ASO has at least 80% (e.g., at least 85%, at least 90%, at least 95%, at least 99%, or 100%) complementarity to a nucleotide sequence within SEQ ID NO: 8;
preferably, the ASO comprises a nucleotide sequence as shown in any one of SEQ ID NOs: 13-46, and 63-65, or a sequence having at least 80% (e.g., at least 85%, at least 90%, at least 95%, at least 99%, or 100%) identity thereto;
preferably, the ASO specifically binds to a target region located within the region c.1795-1803 to c.1795-1527 of *SLC20A2* pre-mRNA; preferably, the ASO has at least 80% (e.g., at least 85%, at least 90%, at least 95%, at least 99%, or 100%) complementarity to a nucleotide sequence within SEQ ID NO: 9;
preferably, the ASO comprises a nucleotide sequence as shown in any one of SEQ ID NOs: 47-61, and 66-68, or a sequence having at least 80% (e.g., at least 85%, at least 90%, at least 95%, at least 99%, or 100%) identity thereto.

11. The use according to claim 9, wherein the agent is a gene editing tool, such as a DNA gene editing system or an RNA base editing tool;
preferably, the gene editing tool is a CRISPR/Cas system comprising a Cas protein and a guide RNA (gRNA), and the gRNA is capable of binding to the target region in the *SLC20A2* gene or pre-mRNA thereof;
preferably, the gRNA binds to the target region to recruit the binding of an editor complex and exert editing activity;
preferably, the target region is located within the region c.289+801 to c.289+1135 of *SLC20A2* pre-mRNA; or located within the region c.1795-1803 to c.1795-1527 of *SLC20A2* pre-mRNA;
preferably, the gene editing tool disrupts the function of the cryptic exon's splice acceptor (SA) or splice donor (SD) by mutating their sequences in the genome or pre-mRNA sequence, thereby inhibiting the integration of the cryptic exon into the mRNA;
preferably, the gene editing tool is a DNA base editor comprising (i) a fusion protein comprising a DNA base editing domain (e.g., a deaminase) and a DNA binding domain (e.g., a Cas protein, such as Cas9 or Cas12) and (ii) a guide RNA (gRNA), the gRNA capable of binding to the target region in the *SLC20A2* gene;
preferably, the gene editing tool is an RNA base editing tool; preferably, the RNA base editing tool comprises: a CRISPR/Cas-based RNA base editor, or a non-CRISPR/Cas-dependent editor, such as an oligonucleotide-based base editor.

12. The use according to claim 1, wherein the intracerebral phosphate homeostasis promoter is an astrocyte phosphate transport promoter.

13. The use according to claim 12, wherein the intracerebral phosphate homeostasis promoter is selected from the group consisting of:
(Z1) a PiT2 protein, a nucleic acid molecule encoding PiT2 protein, an expression vector expressing PiT2 protein, a PiT2 promoter, or a combination thereof;
(Z2) a MYORG protein, a nucleic acid molecule encoding MYORG protein, an expression vector expressing MYORG protein, a MYORG promoter, or a combination thereof;
(Z3) a XPR1 protein, a nucleic acid molecule encoding XPR1 protein, an expression vector expressing XPR1 protein, an XPR1 promoter, or a combination thereof;
(Z4) any combination of Z1~Z3 above.

14. The use according to claim 12 or 13, wherein the promoter in Z1~Z3 comprises a small molecule compound, gene editing reagent, small nucleic acid drug (such as an antisense nucleotide drug), or a combination thereof.

15. The use according to any one of claims 12-14, wherein the astrocyte phosphate transport promoter is an expression vector comprising an exogenous gene and an astrocyte-selectively expressed enhancer element and/or an astrocyte-selectively expressed promoter operably linked thereto, and the exogenous gene is selected from a nucleic acid molecule encoding PiT2 protein, MYORG protein, or XPR1 protein, or a combination thereof;
preferably, the exogenous gene is a nucleic acid molecule encoding PiT2 protein;
preferably, the expression vector is a viral vector, such as AAV;
preferably, the astrocyte-selectively expressed promoter is *gfaABC1D.*

16. The use according to any one of claims 12-14, wherein the astrocyte phosphate transport promoter is a small nucleic acid drug, and the small nucleic acid drug is conjugated to a targeting molecule which has targeting specificity to astrocyte;
preferably, the small nucleic acid drug is an ASO;
preferably, the small nucleic acid drug is fully or partially encapsulated within the targeting molecule, or conjugated to the targeting molecule;
preferably, the targeting molecule is selected from an antibody or antibody fragment thereof, a polysaccharide, an aptamer, or a nanoparticle.

17. The use according to any one of claims 1-16, wherein the cerebral calcification disease comprises Primary Familial Brain calcification (PFBC), Primary Basal Ganglia Calcification (PBGC), or other type of cerebral calcification related to intracerebral phosphate homeostasis deficiency (such as upregulation of cerebrospinal fluid inorganic phosphate).

18. A method for preventing and/or treating a cerebral calcification disease, comprising administering an effective dose of an intracerebral phosphate homeostasis promoter to a subject in need thereof;
preferably, the intracerebral phosphate homeostasis promoter is as defined in any one of claims 2-16;
preferably, the cerebral calcification disease comprises Primary Basal Ganglia Calcification (PBGC), Primary Familial Brain calcification (PFBC), or other type of cerebral calcification related to intracerebral phosphate homeostasis deficiency (such as upregulation of cerebrospinal fluid inorganic phosphate).

19. A medicine kit for treating a cerebral calcification disease, wherein the medicine kit comprises:
(a) a first container, and a first detection reagent for detecting a genetic mutation located in the first container, in which the first detection reagent is used for detecting the presence of the genetic mutation in a gene selected from the group consisting of: *PiT2, MYORG, XPR1,* or a combination thereof; and
(b) a second container, and a pharmaceutical composition located in the second container, in which the pharmaceutical composition comprises the astrocyte phosphate transport promoter as defined in any one of claims 13-16 and a pharmaceutically acceptable carrier and/or excipient;
optionally, the medicine kit may further comprise a third container, and a solution located in the third container for aiding in the dissolution or *in vivo* delivery of the drug in the second container.

20. A method for screening a candidate drug for preventing and/or treating a cerebral calcification disease, wherein the method comprises the following steps:
(a) in a test group, adding a test drug to a culture system of astrocytes or cell line derived therefrom, and measuring the phosphate transport level (E1) and/or activity (A1) in the cells of the test group; in a control group, not adding the test drug to the culture system of the same cells, and measuring the phosphate transport level (E0) and/or activity (A0) in the cells of the control group;
wherein, if the phosphate transport level (E1) and/or activity (A1) in the cells of the test group is significantly higher than that of the control group, it indicates that the test drug promotes the expression and/or activity of phosphate transport level and is a candidate drug for preventing and/or treating the cerebral calcification disease.

21. Use of an agent capable of regulating alternative splicing in an intron of the *SLC20A2* gene in the manufacture of a medicament for preventing and/or treating a disease related to PiT2 functional deficiency in a subject; preferably, the agent is as defined in any one of claims 3-11.

22. A method for preventing and/or treating a disease related to PiT2 functional deficiency in a subject, comprising administering an effective dose of an agent capable of regulating alternative splicing in an intron of the *SLC20A2* gene to a subject in need thereof;
preferably, the agent is as defined in any one of claims 3-11.

23. The use according to claim 21 or the method according to claim 22, wherein the PiT2 functional deficiency involves a mutation in the *SLC20A2* gene;
preferably, the mutation is selected from a non-synonymous mutation, nonsense mutation, frameshift mutation, splicing mutation, or structural variation;
preferably, the mutation is located in an exon region, and the subject is heterozygous for the mutation in the exon region;
preferably, the mutation is located in the target region within an intron, and the subject may be heterozygous, homozygous, or compound heterozygous for the intron mutation located within the target region;
preferably, the target region is located within the region c.289+801 to c.289+1135 of SLC20A2 pre-mRNA, or within the region c.1795-1803 to c.1795-1527 of *SLC20A2* pre-mRNA;
preferably, the PiT2 functional deficiency is *SLC20A2* haploinsufficiency.

24. The use according to claim 21 or the method according to claim 22, wherein the disease related to PiT2 functional deficiency benefits from modulation of functional PiT2 protein or RNA expression;
preferably, the disease is a cerebral calcification, such as Primary Familial Brain calcification (PFBC) or Primary Basal Ganglia Calcification (PBGC);
preferably, the disease is a disease related to Pi balance abnormalities, such as a chronic kidney disease, cardiac valve calcification, arterial vascular calcification, and/or skeletal disease related to phosphate homeostasis imbalance.

25. An agent capable of specifically binding to a target region in the *SLC20A2* gene or pre-mRNA thereof, and the target region is located within an intron region between two canonical exon regions, and wherein the intron region comprises a cryptic exon;
preferably, the cryptic exon is located within intron 2; preferably, the cryptic exon is located at c.289+901 to c.289+1035 of the *SLC20A2* gene or pre-mRNA thereof; preferably, the target region is located within the region c.289+801 to c.289+1135 of the *SLC20A2* gene or pre-mRNA thereof;
preferably, the cryptic exon is located within intron 10; preferably, the cryptic exon is located at c.1795-1703 to c.1795-1627 of the *SLC20A2* gene or pre-mRNA thereof; preferably, the target region is located within the region c.1795-1803 to c.1795-1527 of the *SLC20A2* gene or pre-mRNA thereof;
preferably, the target region has the characteristics defined in any one of claims 6-8.

26. The agent according to claim 25, wherein the agent has the characteristics defined in any one of claims 9-10.

27. A pharmaceutical composition comprising the agent according to any one of claims 25-26 or a combination thereof, and a pharmaceutically acceptable carrier and/or excipient.

28. Use of the agent according to any one of claims 25-26 or the pharmaceutical composition according to claim 27 in the manufacture of a medicament for preventing and/or treating a disease related to PiT2 functional deficiency in a subject;
preferably, the disease related to PiT2 functional deficiency is a cerebral calcification, such as Primary Familial Brain calcification (PFBC) or Primary Basal Ganglia Calcification (PBGC);
preferably, the disease related to PiT2 functional deficiency is a disease related to Pi balance abnormalities, such as a chronic kidney disease, cardiac valve calcification, arterial vascular calcification, and/or skeletal disease related to phosphate homeostasis imbalance;
preferably, the medicament treats the disease by regulating the expression of PiT2 protein or functional RNA in the subject's cell.

29. A method for preventing and/or treating a disease related to PiT2 functional deficiency in a subject, comprising administering the agent according to any one of claims 25-26 or the pharmaceutical composition according to claim 27 to a subject in need thereof;
preferably, the disease related to PiT2 functional deficiency is a cerebral calcification, such as Primary Familial Brain calcification (PFBC) or Primary Basal Ganglia Calcification (PBGC);
preferably, the disease related to PiT2 functional deficiency is a disease related to Pi balance abnormalities, such as a chronic kidney disease, cardiac valve calcification, arterial vascular calcification, and/or skeletal disease related to phosphate homeostasis imbalance.

30. The use according to claim 28 or the method according to claim 29, wherein the subject comprises a mutation in the *SLC20A2* gene;
preferably, the mutation is selected from a non-synonymous mutation, nonsense mutation, frameshift mutation, splicing mutation, or structural variation;
preferably, the mutation is located in an exon region, and the subject is heterozygous for the mutation in the exon region;
preferably, the mutation is located in the target region within an intron, and the subject may be heterozygous, homozygous, or compound heterozygous for the intron mutation located within the target region;
preferably, the target region is located within the region c.289+801 to c.289+1135 of SLC20A2 pre-mRNA, or within the region c.1795-1803 to c.1795-1527 of *SLC20A2* pre-mRNA;
preferably, the PiT2 functional deficiency is *SLC20A2* haploinsufficiency.

31. A method for regulating PiT2 protein expression in a cell, comprising contacting the cell *in vitro* with an agent capable of regulating alternative splicing within an intron of the *SLC20A2* gene;
preferably, the agent capable of regulating alternative splicing within an intron of the *SLC20A2* gene is as defined in any one of claims 2-11.

32. A method for regulating PiT2 protein expression in a cell, comprising contacting the cell *in vitro* with the agent according to any one of claims 25-26;
preferably, the cell comprises a mutation in the *SLC20A2* gene;
preferably, the mutation is selected from a non-synonymous mutation, nonsense mutation, frameshift mutation, splicing mutation, or structural variation; preferably, the mutation is located in an exon region, and the cell is heterozygous for the mutation in the exon region; preferably, the mutation is located in the target region within an intron, and the cell may be heterozygous, homozygous, or compound heterozygous for the intron mutation located within the target region.

33. A method for identifying an agent capable of regulating PiT2 expression in a cell, the method comprising: i) identifying an agent targeting a target region in the PiT2 gene or pre-mRNA thereof, wherein the target region is located within an intron region between two canonical exon regions, and wherein the intron region comprises a cryptic exon, and the target region is within the region from up to about 100 nucleotides upstream of the cryptic exon to up to about 100 nucleotides downstream of the cryptic exon; ii) delivering the agent identified in step i) to the cell; and iii) measuring the mRNA and protein expression levels of PiT2 in the cell of step ii);
preferably, the method further comprises: iv) comparing the expression level measured in step iii) with the PiT2 expression level in a cell treated with a control method;
preferably, the expression level is measured by RT-PCR, qPCR, or by Western blot;
preferably, the target region is selected from a region upstream of the cryptic exon 5' splice acceptor site (SA), a region spanning the 5' splice acceptor site, a region within the cryptic exon, a region spanning the 3' splice donor site, or a region downstream of the 3' splice donor site (SD);
preferably, the cryptic exon is located at c.289+901 to c.289+1035 of the *SLC20A2* gene; preferably, the target region is located within the region c.289+801 to c.289+1135 of the *SLC20A2* gene or pre-mRNA thereof; preferably, the target region is located within the sequence as shown in SEQ ID NO: 8;
preferably, the cryptic exon is located at c.1795-1703 to c.1795-1627 of the *SLC20A2* gene; preferably, the target region is located within the region c.1795-1803 to c.1795-1527 of the *SLC20A2* gene or pre-mRNA thereof; preferably, the target region is located within the sequence as shown in SEQ ID NO: 9.

34. A method for preparing a cerebral calcification model, wherein the method comprises modifying a target genome such that the intron sequence between exon2 and exon3 in endogenous *SLC20A2* gene is wholly or partially replaced by the corresponding human *SLC20A2* gene intron genomic fragment, or such that the intron sequence between endogenous *SLC20A2* gene exon10 and exon11 is wholly or partially replaced by the corresponding human *SLC20A2* gene intron genomic fragment, wherein the corresponding human *SLC20A2* gene intron genomic fragment comprises a mutation that alters *SLC20A2* gene splicing, thereby providing the cerebral calcification model;
preferably, the corresponding human *SLC20A2* gene intron genomic fragment at least comprises a cryptic exon;
preferably, the corresponding human *SLC20A2* gene intron genomic fragment at least comprises the region from about 100 nucleotides upstream of the cryptic exon to about 100 nucleotides downstream of the cryptic exon; for example, the replaced human *SLC20A2* gene intron genomic fragment between exon2 and exon3 comprises the sequence as shown in SEQ ID NO: 10; or the replaced human *SLC20A2* gene intron genomic fragment between exonl0 and exonll comprises the sequence as shown in SEQ ID NO: 11;
preferably, the mutation that alters *SLC20A2* gene splicing causes or enhances the alternative splicing in intron 2 or intron 10 as defined in any preceding claim;
preferably, the replacement method comprises: gene knockout, gene editing, RNA interference, epigenetic suppression, or any combination thereof;
preferably, the model is an animal model, for example a mammalian model, such as a rodent model (e.g., mouse or rat model) or a non-human primate model;
preferably, the model is a mammalian cell model, for example, human epidermal fibroblast or brain glial cell.

35. A genetically modified non-human animal, whose genome comprises: the intron sequence between endogenous *SLC20A2* gene exon2 and exon3 being replaced by the corresponding human *SLC20A2* gene intron genomic fragment, or the intron sequence between endogenous *SLC20A2* gene exon10 and exon11 being replaced by the corresponding human *SLC20A2* gene intron genomic fragment, wherein the corresponding human *SLC20A2* gene intron genomic fragment comprises a mutation that alters *SLC20A2* gene splicing;
preferably, the mutation that alters *SLC20A2* gene splicing causes or enhances the alternative splicing in intron 2 or intron 10 as defined in any preceding claim;
preferably, the replaced human *SLC20A2* gene intron genomic fragment between exon2 and exon3 comprises the sequence as shown in SEQ ID NO: 10;
preferably, the replaced human *SLC20A2* gene intron genomic fragment between exonl0 and exonll comprises the sequence as shown in SEQ ID NO: 11;
preferably, the non-human animal is a rodent, such as a mouse or rat;
preferably, the non-human animal is a non-human primate;
preferably, the genetically modified non-human animal is an animal model of cerebral calcification.

36. A genetically modified cell, whose genome comprises: the intron sequence between endogenous *SLC20A2* gene exon2 and exon3 being replaced by the corresponding human *SLC20A2* gene intron genomic fragment, or the intron sequence between endogenous *SLC20A2* gene exon10 and exonll being replaced by the corresponding human *SLC20A2* gene intron genomic fragment, wherein the corresponding human *SLC20A2* gene intron genomic fragment comprises a mutation that alters *SLC20A2* gene splicing;
preferably, the corresponding human *SLC20A2* gene intron genomic fragment at least comprises a cryptic exon;
preferably, the corresponding human *SLC20A2* gene intron genomic fragment at least comprises the region from about 100 nucleotides upstream of the cryptic exon to about 100 nucleotides downstream of the cryptic exon; for example, the replaced human *SLC20A2* gene intron genomic fragment between exon2 and exon3 comprises the sequence as shown in SEQ ID NO: 10; or the replaced human *SLC20A2* gene intron genomic fragment between exonl0 and exonll comprises the sequence as shown in SEQ ID NO: 11;
preferably, the mutation that alters *SLC20A2* gene splicing causes or enhances the alternative splicing in intron 2 or intron 10 as defined in any preceding claim;
preferably, the corresponding human *SLC20A2* gene intron genomic fragment is introduced exogenously relative to the cell;
preferably, the cell is a mammalian cell;
preferably, the cell is a human epidermal fibroblast or brain glial cell;
preferably, the genetically modified cell is a cell model of cerebral calcification.

37. Use of a genetically modified non-human animal or cell, which comprises: (i) for indicating and analyzing the stage of cerebral calcification disease onset, or for use as a model for studying intracerebral phosphate homeostasis imbalance or cerebral calcification disease targets; (ii) for use as a model for screening a candidate drug or treatment regimen for treating a cerebral calcification disease or disease related to intracerebral phosphate homeostasis imbalance; and/or (iii) for use as a model for screening a candidate drug or treatment regimen for a disease related to phosphate homeostasis imbalance;
wherein, the genome of the genetically modified non-human animal or cell comprises: the intron sequence between endogenous *SLC20A2* gene exon2 and exon3 being replaced by the corresponding human *SLC20A2* gene intron genomic fragment, or the intron sequence between endogenous *SLC20A2* gene exon10 and exon11 being replaced by the corresponding human *SLC20A2* gene intron genomic fragment, wherein the corresponding human *SLC20A2* gene intron genomic fragment comprises a mutation that alters *SLC20A2* gene splicing;
preferably, the corresponding human *SLC20A2* gene intron genomic fragment at least comprises a cryptic exon;
preferably, the corresponding human *SLC20A2* gene intron genomic fragment at least comprises the region from about 100 nucleotides upstream of the cryptic exon to about 100 nucleotides downstream of the cryptic exon; for example, the replaced human *SLC20A2* gene intron genomic fragment between exon2 and exon3 comprises the sequence as shown in SEQ ID NO: 10; or the replaced human *SLC20A2* gene intron genomic fragment between exonl0 and exonll comprises the sequence as shown in SEQ ID NO: 11;
preferably, the mutation that alters *SLC20A2* gene splicing causes or enhances the alternative splicing in intron 2 or intron 10 as defined in any preceding claim.

38. A nucleic acid construct comprising a human *SLC20A2* gene minigene fragment sequence, wherein the minigene fragment sequence comprises exon2 and exon3 and the intron sequence between them, or comprising exon10 and exon11 and the intron sequence between them, wherein the intron sequence comprises a mutation that alters *SLC20A2* gene splicing;
preferably, the nucleic acid construct comprises a promoter operably linked to the minigene fragment sequence;
preferably, the mutation that alters *SLC20A2* gene splicing causes or enhances the alternative splicing in intron 2 or intron 10 as defined in any preceding claim;
preferably, the minigene fragment sequence comprises the sequence as shown in SEQ ID NO: 4 or 5;
preferably, a polyA signal sequence is included downstream of the minigene fragment sequence;
preferably, the nucleic acid construct further comprises a reporter gene, such as a fluorescent protein (e.g., GFP, mCherry) or luciferase.

39. A method for selecting an agent capable of regulating *SLC20A2* mRNA splicing, comprising: providing (i) a cell comprising the nucleic acid construct according to claim 38, (ii) the genetically modified non-human animal according to claim 35 or the genetically modified cell according to claim 36, or (iii) the cerebral calcification model obtained by the method according to claim 34; contacting a test reagent with any one of (i)-(iii); and detecting alternative splicing of *SLC20A2* mRNA;
preferably, the cell is from, for example, a mammal, such as a rodent (e.g., mouse or rat), a non-human primate, or a human;
preferably, selecting a test reagent capable of inhibiting alternative splicing (e.g., reducing the amount of expression product with cryptic exon inclusion) is selected, thereby increasing the production of functional PiT2 protein;
preferably, selecting a test reagent capable of enhancing alternative splicing (e.g., increasing the amount of expression product with cryptic exon inclusion) is selected, thereby reducing the production of functional PiT2 protein;
preferably, the detection comprises analyzing the length of the *SLC20A2* mRNA expression product;
preferably, the alternative splicing is the alternative splicing in intron 2 as defined in any preceding claim; preferably, the detection comprises PCR analysis using primers spanning exon2 and exon3; preferably, an amplicon containing a cryptic exon inclusion of approximately 135 nt in length is produced;
preferably, the alternative splicing is the alternative splicing in intron 10 as defined in any preceding claim; preferably, the detection comprises PCR analysis using primers spanning exon10 and exon11; preferably, an amplicon containing a cryptic exon inclusion of approximately 76 nt in length is produced;
preferably, the method comprises contacting a test reagent *in vitro* with a cell containing the nucleic acid construct and detecting alternative splicing of the nucleic acid construct; preferably, the detection comprises analyzing the length of the mRNA expression product of the nucleic acid construct or analyzing the expression of a reporter gene contained in the nucleic acid construct.
